# EUROPEAN PATENT SPECIFICATION

(11) **EP 3 034 628 B1**
(45) Date of publication and mention of the grant of the patent: **24.10.2018**
(21) Application number: 16153959.8
(22) Date of filing: 13.09.2011
(51) Int. Cl.: C12Q 1/68

(54) **DIAGNOSIS OF CANCER BY MEANS OF METHYLATION MARKER**
KREBSDIAGNOSE MIT HILFE VON METHYLIERUNGSMARKERN
DIAGNOSTIC DU CANCER AU MOYEN DE METHYLATION MARKER

(30) Priority: 13.09.2010 AU 2010904116
(43) Date of publication of application: 22.06.2016
(62) Divisional of application: 11824349.2
(73) Proprietor: Clinical Genomics Pty Ltd, North Ryde, New South Wales 2113 (AU); COMMONWEALTH SCIENTIFIC AND INDUSTRIAL RESEARCH ORGANISATION, Campbell, ACT 2612 (AU)
(72) Inventor: Ross, Jason, Carlingford, New South Wales 2118 (AU); Drew, horace, Putney, New South Wales 2112 (AU); MOLLOY, Peter Laurence, Chatswood, New South Wales 2067 (AU); Mitchell, Susan, Gladesville, New South Wales 2111 (AU); Duesing, Konsta, Padstow Heights, New South Wales 2211 (AU); Xu, Zheng-Zhou, Forestville, New South Wales 2087 (AU); Buckley, Michael, Pennant Hills, New South Wales 2120 (AU)
(74) Representative: Maschio, Antonio

(56) References cited:
- WO-A1-2010/062914
- ZHOU WEN ET AL: "Functional evidence for a nasopharyngeal carcinoma-related gene BCAT1 located at 12p12", ONCOLOGY RESEARCH, PERGAMON PRESS, NEW YORK, NY, US, vol. 16, no. 9, 1 January 2007 (2007-01-01), pages 405-413, XP008136954, ISSN: 0965-0407
- JU WOONG ET AL: "Identification of Genes With Differential Expression in Chemoresistant Epithelial Ovarian Cancer Using High-Density Oligonucleotide Microarrays", ONCOLOGY RESEARCH, PERGAMON PRESS, NEW YORK, NY, US, vol. 18, no. 2-3, 1 January 2009 (2009-01-01), pages 47-56, XP009150472, ISSN: 0965-0407
- MARTJE TÖNJES ET AL: "BCAT1 promotes cell proliferation through amino acid catabolism in gliomas carrying wild-type IDH1", NATURE MEDICINE., vol. 19, no. 7, 23 June 2013 (2013-06-23), pages 901-908, XP055252145, US ISSN: 1078-8956, DOI: 10.1038/nm.3217
- SUSAN M MITCHELL ET AL: "A panel of genes methylated with high frequency in colorectal cancer", BMC CANCER, BIOMED CENTRAL, LONDON, GB, vol. 14, no. 1, 31 January 2014 (2014-01-31), page 54, XP021175595, ISSN: 1471-2407, DOI: 10.1186/1471-2407-14-54
- ERIN L SYMONDS ET AL: "The relationship between the degree of aberrant methylation in colorectal cancer tissue and appearance of tumor-derived DNA in blood.", JOURNAL OF CLINICAL ONCOLOGY, vol. 35, no. 4_suppl, 1 February 2017 (2017-02-01), pages 579-579, XP055441836, US ISSN: 0732-183X, DOI: 10.1200/JCO.2017.35.4_suppl.579
- SUSANNE K. PEDERSEN ET AL: "A Two-Gene Blood Test for Methylated DNA Sensitive for Colorectal Cancer", PLOS ONE, vol. 10, no. 4, 30 April 2015 (2015-04-30) , page e0125041, XP055252713, DOI: 10.1371/journal.pone.0125041
- GRAEME P. YOUNG ET AL: "A cross-sectional study comparing a blood test for methylated BCAT1 and IKZF1 tumor-derived DNA with CEA for detection of recurrent colorectal cancer", CANCER MEDICINE, vol. 5, no. 10, 1 October 2016 (2016-10-01), pages 2763-2772, XP055441855, GB ISSN: 2045-7634, DOI: 10.1002/cam4.868
- SUSANNE K PEDERSEN ET AL: "Evaluation of an assay for methylated BCAT1 and IKZF1 in plasma for detection of colorectal neoplasia", BMC CANCER, BIOMED CENTRAL, LONDON, GB, vol. 15, no. 1, 6 October 2015 (2015-10-06), page 654, XP021229326, ISSN: 1471-2407, DOI: 10.1186/S12885-015-1674-2
- ERIN L SYMONDS ET AL: "A Blood Test for Methylated BCAT1 and IKZF1 vs. a Fecal Immunochemical Test for Detection of Colorectal Neoplasia", CLINICAL AND TRANSLATIONAL GASTROENTEROLOGY, vol. 7, no. 1, 1 January 2016 (2016-01-01) , pages e137-e137, XP055441849, DOI: 10.1038/ctg.2015.67

### SEQUENCE LISTING

<110> CLINICAL GENOMICS PTY. LTD.
   COMMONWEALTH SCIENTIFIC AND INDUSTRIAL RESEARCH
   ORGANISATION
   ROSS, Jason
   DREW, Horace
   BUCKLEY, Michael
   MOLLOY, Peter
   MITCHELL, Susan
   DUESING, Konsta
   BROWN, Glenn
<120> Diagnostic method
<130> 35014615/TDO
<150> 2010904116
   <151> 2010-09-13
<160> 82
<170> PatentIn version 3.5
<210> 1
   <211> 21
   <212> DNA
   <213> Artificial
<220>
   <223> CG linker-upper
<400> 1
   cgcgtatcac cgactgccct t 21
<210> 2
   <211> 17
   <212> DNA
   <213> Artificial
<220>
   <223> CG linker-lower
<400> 2
   gggcagtcgg tgatacg 17
<210> 3
   <211> 21
   <212> DNA
   <213> Artificial
<220>
   <223> CA linker
<400> 3
   cagcaatcac cgactgccct t 21
<210> 4
   <211> 17
   <212> DNA
   <213> Artificial
<220>
   <223> CA linker
<400> 4
   gggcagtcgg tgattgc 17
<210> 5
   <211> 11
   <212> DNA
   <213> Artificial
<220>
   <223> CG competitor
<400> 5
   acagagtcgt a 11
<210> 6
   <211> 12
   <212> DNA
   <213> Artificial
<220>
   <223> CG competitor
<400> 6
   cgtacgactc tg 12
<210> 7
   <211> 11
   <212> DNA
   <213> Artificial
<220>
   <223> CA competitor
<400> 7
   agtgatcagc a 11
<210> 8
   <211> 12
   <212> DNA
   <213> Artificial
<220>
   <223> CA competitor
<400> 8
   catgctgatc ac 12
<210> 9
   <211> 29
   <212> DNA
   <213> Artificial
<220>
   <223> P2-N6 primer
<220>
   <221> misc_feature
   <222> (24)..(29)
   <223> n is a, c, g, or t
<400> 9
   ctgccccggg ttcctcattc tctnnnnnn 29
<210> 10
   <211> 21
   <212> DNA
   <213> Artificial
<220>
   <223> P2-long primer
<400> 10
   ctgccccggg ttcctcattc t 21
<210> 11
   <211> 24
   <212> DNA
   <213> Artificial
<220>
   <223> SolP2-AB linker
<400> 11
   cctaccccac attcctcatt ctct 24
<210> 12
   <211> 25
   <212> DNA
   <213> Artificial
<220>
   <223> SolP2-BP linker
<400> 12
   ttggatgggg tgtaaggagt aagag 25
<210> 13
   <211> 41
   <212> DNA
   <213> Artificial
<220>
   <223> SolP1 AM linker
<400> 13
   mmamtamgmm tmmgmtttmm tmtmtatggg magtmggtga t 41
<210> 14
   <211> 44
   <212> DNA
   <213> Artificial
<220>
   <223> SolP1 BC linker
<400> 14
   cggtgatgcg gaggcgaaag gagagatacc cgtcagccac taat 44
<210> 15
   <211> 251
   <212> DNA
   <213> human
<400> 15
   gatgggtgtt gggacatgga gtccccagcc tgtgctgctg acgccctgtg gtggctagcc 60
   gctctttaga caccagacgc tgccttccac ttcctcctct cacttcttat tcgggcacca 120
   ctgacgacag agacttcggc tgggccattc ccgcctctcc taccgcctgg cagccatggt 180
   aacagcctgg gggatggtcc ctaagatggg gtcagagaac ggctgagcat ggccatcccc 240
   ccagccctga a 251
<210> 16
   <211> 299
   <212> DNA
   <213> human
<400> 16
   caggaagctg cagcagaagg aggaggcggc ggccaccccg gaccccgccg cccggactcc     60
   cgactcggaa gtcgcgcccg ccgctccggt cccgaccccg ggaccccctg ccgcagccgc    120
   cacccctggg cccccagcgg acgagctgta cgcggcgctg gaggactatc accctgccga    180
   gctgtaccgc gcgctcgccg tgtccggggg caccctgccc cgccgaaagg tgcgtccccc    240
   gcccgccttc aggatctgct cagcccctct ccgactccct acagggcctg ctgactccg     299
<210> 17
   <211> 258
   <212> DNA
   <213> human
<400> 17
   gacagagaca gccccaggca agttgaaggt ccgagagccc ccggtgggag aagcgggccg     60
   gtggctgcgc cgcgtgcgtt ctcactctga ggaagtgcgt ggggagccgc tgactccgga    120
   tagcacaccc ttccgagggg actccccgat tcctgggctg ggggcctgcc gcctggcccc     180
   acgtctgacg tacggggcgc gagggccact gctccctgga cttctgtcgg aaccggacgc    240
   agtgggaggg gtcgcagg                                                  258
<210> 18
   <211> 233
   <212> DNA
   <213> human
<400> 18
   aacttgcatg cagtcaaaca ggaaagctgg cgcggggggg atggtgtgcg gaggaaggga     60
   ccccgaagtt tccaggtcat ccctggtctc gaggagcgcg cggagtggag ccgttccctc    120
   gcgggcccga cccaggctcc cccctcctgc caccctttcc cctccgtcct tcccagacca    180
   cgcagcaaga cccgccgagg gcgaagaagt tgggagtgac ttcaggggcc tct           233
<210> 19
   <211> 221
   <212> DNA
   <213> human
<400> 19
   ccgcaggact gggaatccag tggccggcgt cccagcattt tcggacgcgt tccgtgcctc 60
   ccccagtatc cccttgaggg atcgcctact gcgattccac gtagtgtcgg ttacctcgcc 120
   ctagccgcag ggagcatcaa acaaaagaac ttttgcaaac ttttctctct ggacactcgc 180
   gggtctctta aatctggccc ccatctcccc agaacttgca t 221
<210> 20
   <211> 237
   <212> DNA
   <213> human
<400> 20
   aaaggaggct cggtccacca atgcgcgatc gtagcctaca gggaccccca acgaaccccg 60
   gcccagagaa tgcacctggc cgtgccacgc gcgacccccg aggggcaggc ggacgggtcc 120
   aggcgcggac aaaggggcct ctcggggcgc ccaagctcgg ggcgggacgc cgggagcgga 180
   gctggccggg aactcacctt cgcagcggcc cggctgtgct cctcgtggag cagaagg 237
<210> 21
   <211> 254
   <212> DNA
   <213> human
<400> 21
   tgcgttacat aatggaggat taaagaaaac agtgtccctt ggcttaaaac aaatggtgtc 60
   ctcttagtct cccgtcccag tgggcgttag atgtcggggc aggcggctgc acacttaatt 120
   ctccgcgggg gcattggcct gtctgccggt ccaaatcatc cattttcctt ggtctgactg 180
   caaggtcggt gcttaaactt cggacggctg gtgaattgtg cggcgggcgc ggggccctgg 240
   gaggcagccc cctc 254
<210> 22
   <211> 186
   <212> DNA
   <213> human
<400> 22
   gaacttgccg atgtggcagg tgtacagtcc gtggctaggc aggaaggcgg ggtgttgcag 60
   cggcgccccg gcggagggcc cgtgcgcgcc ggggtggccc gcgggtggtg gtggcgaagc 120
   cttgggcgca ccgtcggggc tcgtggctgt ctccgccagc gaccagatct tgggcttgcc 180
   gtgcgg 186
<210> 23
   <211> 193
   <212> DNA
   <213> human
<400> 23
   gaccgaggcc ttgtctccat tgagtgcccc cggggcaata gcgaccggcg gctgcggtgg        60
   tggaggtgca ggaaggtgag ggccatgggg cgcggcgtgg ggagcgccaa cgcccaggaa       120
   ggagcgcatg ttgagcaggg agccctgtgc gaggaatgcg ctgttggtcc agttggagaa       180
   cttgccgatg tgg                                                          193
<210> 24
   <211> 191
   <212> DNA
   <213> human
<400> 24
   ggagtccggt gtgctggaga gccgcagcgg ggtgaactcc cggccccgtc tctgcgtagg        60
   aggtggttcg caaagtggcc ccgggagccg ggactggtac ccggttcccc acggcaccgt       120
   ccggagctct ccagccacga ggcgcagaag tggcctgcca gcgccttccc aggctcaggg       180
   aggcgaggag g                                                            191
<210> 25
   <211> 166
   <212> DNA
   <213> human
<400> 25
   gggcgccgag tggaaactgc tcacagagtc ggagaagcgg ccgttcatcg acgaggccaa        60
   gcgtctacgc gccatgcaca tgaaggagca ccccgactac aagtaccggc cgcggcgcaa       120
   gcccaagacg ctgctcaaga aggacaagtt cgccttcccg gtgccc                      166
<210> 26
   <211> 270
   <212> DNA
   <213> human
<400> 26
   tgagagcccc ctttggcact tggcagcacg cggcggcggg ctcctcggct caacttcgag        60
   gagtctccgc gacgcaactt ttggggacgc tttgcattta agagagaacg accgaggagg       120
   aggagcgctc tgcccggccg ccgctacctg cggggagctc accagcaaac gccactgcag       180
   acgaaggacc caaagaacgt aaagggcaaa ctgccgccgc ggggaggggg caccgccgag       240
   aagttagagt gtcccagaga caacctgctc                                        270
<210> 27
   <211> 311
   <212> DNA
   <213> human
<400> 27
   gcggtcagtt acatgtgctg gtgtccagcc gctgcctctc tttccccggt cggagcgcgg      60
   gggtgcgtgt gccctcactc tcacgaatac agacggagag cttcccagaa ggaaaaacaa     120
   attcgtcata atgttcagcg cagccgagag tttccaacaa gaaagcagct ccagtcgtgg     180
   catgatgact cagggtggcg ttttaaacgc tgaataaacc cattccttag ctctttaata     240
   aatcgctaat aacagcaaaa acagccttct ccccgaaccg cgccacacca aaggcttccc     300
   aagttgggct g                                                          311
<210> 28
   <211> 253
   <212> DNA
   <213> human
<400> 28
   atagcagggt ttacagggca aagagaggag aacctggaaa tcgtgtcctg gggggcaaga      60
   gtgcgttgcc cagtcacaag tgtgctgtag aggtgggaga ggacaaggac ccatttccac     120
   atttcaaccc tccgtttcta tcgctgcccc tggaggactc agctgctaac gccgagctcg     180
   tttccacgcg gctctggtcc tagtcgggga atccgggtgg ccgcgcgacg cgagagaact     240
   gctgggatgc gtg                                                        253
<210> 29
   <211> 222
   <212> DNA
   <213> human
<400> 29
   gatgcgtggc cctggtatgg gcgcacccag cgtccggggc gcaggttctc tcagcccagc      60
   ttctcctccc actcactcgc tcggatctcc tccctccttc acatcccccg cccccgggac     120
   cgcgaggctc cctccccgca ccggccagtg agtacacaaa gccgcgggtg aggggaagct     180
   tcgcaggcgt gcacggagca gtgagatcac tggcgttata aa                        222
<210> 30
   <211> 181
   <212> DNA
   <213> human
<400> 30
   gctgctgaag gagtgagagg gaccttagag cagagcgggc ggtgcctgcg cagtgcgtga 60
   ttacgcgagc cgggcgtggc ccagggttcc ggcgggtaag agcaggagtg tgcgcaggcg 120
   caaagattgc gctctccccc tctcctcgcc tagagaccag aagcgtccct agttactagt 180
   t 181
<210> 31
   <211> 280
   <212> DNA
   <213> human
<400> 31
   gttttgggtg gtttgggagt ccgagctcga gggctgggcc aggaagggca ggcgaggcgg 60
   gcggctccga cgcgggtcgc gaaggcccag ccgcgtcctc tgtccccagg acgactacat 120
   ttcccagagg ccagcggggc gcgcgtactc gagtctgcgg ggcggaggcc gcggctcggg 180
   gctgctgggc gttcggggcg gcttggggcg gcgggaccac tggagtgagc tgtgggagag 240
   atgggggtgt gcctgtgtgt aaaagatctg tcagagtgtg 280
<210> 32
   <211> 253
   <212> DNA
   <213> human
<400> 32
   ggggaggggg aggaggccag ttgaaaagta gaggccgagg acagagttag acactcgttg 60
   tctaacggcc ctgccggagg ggctccgcct gtcagactgc gtcaccctct cgccaagggt 120
   cccccagacg ggcgggggct ccttgcggcc ggctagctac tctccccgcc tccctgcgcc 180
   ctcgccctcc gccccgctgt acccgcagag cgcgcggccc agcgcagccc cttttggcac 240
   cgaggtttcc agg 253
<210> 33
   <211> 251
   <212> DNA
   <213> human
<400> 33
   ggtttcaggc tgcctaggac cggaaccagg gatcgcaccg cagcccaagg taccagtttc 60
   gggcgggcgg cctggcccgg cagggagatg aggtcccctt tcttaacagc aagctaacgc 120
   ggcggtcccc tggagctgct cagcgtccca gcttctaacc agctcctatg gaatccaggt 180
   gcggcttaaa tctcgcacac tggtccccta aactttcaaa accacgttta tgggcaaagc 240
   cctgccggct g 251
<210> 34
   <211> 205
   <212> DNA
   <213> human
<400> 34
   ggctccctca gcgatgtgga ggaccttcaa gaggtggaga tgttggaatg tgacgggttg 60
   aaaatggatt cgaacaagga atttgtgact tccaacgaga gcaccgagga gagctccaac 120
   tgcgagaatg ggtctcccca gaagggccgc ggcggcctgg gcaagaggag gaaggcgccc 180
   accaagaaga gccccctgag cgggg 205
<210> 35
   <211> 240
   <212> DNA
   <213> human
<400> 35
   gaggaaggcg cccaccaaga agagccccct gagcggggtc agccaggagg ggaagcaggt 60
   ccagcgcaac gccgccaacg cgcgagagcg ggcccgcatg cgagtgctga gcaaggcctt     120
   ctccagactc aagaccaccc tgccctgggt gccccccgac accaagctct ccaagctgga     180
   cacgctcagg ctggcgtcca gctacatcgc ccacttgagg cagatcctgg ctaacgacaa 240
<210> 36
   <211> 281
   <212> DNA
   <213> human
<400> 36
   gggatccctc ctctgtgcca gctcaacaca cttgcaaact tttaattggg ctgccacgtc 60
   aaaccctcgc ataaatcact cccgctccgg gggctggtag ggcagagagc gagggccgtg 120
   tctagaccgc ccgcctccgg tccctggagg ctctaggccc gcggtggtcg agatgtgtaa 180
   gtcaccgccg cgcccacacc cccactccca gccccgcgcg cactcgcgcc caccgcaggc 240
   gggagtgcgg gcgcgcctgg actgcagctg cagccccggg a 281
<210> 37
   <211> 225
   <212> DNA
   <213> human
<400> 37
   aggctttcaa ggggaaactc tgactcgttg tctgcatacc tccgccctcc gcatcctccc 60
   gccctccccc gccctttcca agtttggaaa cactatggga cacgtcggag tgctctgcag 120
   ccccgacatg ggcagaattt gcagatctcc ggcgatcaaa gcaggagctg tccagtcccg 180
   cctcacctgt gccctgtagg ccacttgtag ggtggagggc tccag 225
<210> 38
   <211> 205
   <212> DNA
   <213> human
<400> 38
   ggaggcaccc tagggccagg gaaacttttg ccgtataaat agggcagatc cgggctttat 60
   tattttagca ccacggcagc aggaggtttc ggctaagttg gaggtactgg ccacgactgc 120
   atgcccgcgc ccgccaggtg atacctccgc cggtgaccca ggggctctgc gacacaagga 180
   gtctgcatgt ctaagtgcta gacat 205
<210> 39
   <211> 198
   <212> DNA
   <213> human
<400> 39
   gagggaggac atggaggcag ccacctagct cagcggagac gcggagccca cagcagcgcc 60
   ctccggagcc ctaacacgtc gctgccacca tccgcgccgg gactccgcag ccgagctcgg 120
   ccgcccgcag gacgctccag gagcgtcgcg gaccgggcgg cacgggacgc tgcggggctg 180
   agctcaagag cccaggtt 198
<210> 40
   <211> 282
   <212> DNA
   <213> human
<400> 40
   agtctgttac tcggtctggc tgaccccgcc ggtgtctctg tgcatccatg ctacctttcc       60
   ctattaccca cccccttccc agatccgagc agtccgccgg cccgcgcgga cccagagcaa      120
   gaagagggcg aggaagaaga tgcctcggcc cggccgcaac acgtacagcg accagaagcc 180
   gccctactcg tacatctcgc tgaccgctat ggccatccag agctctcccg agaagatgct 240
   gccgctgagc gagatctaca agttcatcat ggaccgcttc cc 282
<210> 41
   <211> 211
   <212> DNA
   <213> human
<400> 41
   gagttcatgt tcgaggaggg ctcctttcgg cggcggccgc gcggcttccg aaggaaatgc 60
   caggcgctca agcccatgta cagcatgatg aacgggctcg gcttcaacca cctcccggac 120
   acctacggct tccagggctc ggccggcggc ctctcgtgcc cgcccaacag cctggcgctg 180
   gagggcggcc tgggcatgat gaacggccac t 211
<210> 42
   <211> 320
   <212> DNA
   <213> human
<400> 42
   gtccggcccg tccaaggcca agaagaccaa cgccggcatc cggcgcccgg agaagccgcc 60
   ctattcctac atcgcgctca tcgtcatggc catccagagt tcacccacca agcgcctgac 120
   gctgagcgag atctaccagt tcctgcagag ccgcttcccc ttcttccggg gctcctacca 180
   gggctggaag aactccgtgc gccacaacct ctcgctcaac gagtgcttca tcaagctacc 240
   caagggcctt gggcggcccg gcaagggcca ctactggacc atcgacccgg ccagcgagtt 300
   catgttcgag gagggctcct 320
<210> 43
   <211> 316
   <212> DNA
   <213> human
<400> 43
   ccctgcccag ccactcggtg ccccacctgc cttccaacgg cggccactcg tacatgggcg 60
   gctgcggcgg cgcggcggcc ggcgagtacc cgcaccacga cagctcggtg cccgcctccc 120
   cgctgctgcc caccggcgcc ggtggggtca tggagccgca cgccgtctac tcgggctcgg 180
   cggcggcctg gccgccctcg gcgtccgcgg cgctcaacag cggcgcctct tatatcaagc 240
   agcagcccct gtccccctgt aaccccgcgg ccaaccccct gtccggcagc ctctccacgc 300
   actccctgga gcagcc 316
<210> 44
   <211> 212
   <212> DNA
   <213> human
<400> 44
   ggttgggccc gggatcagag ctttgcttgg aagttctctg ggtggcagag actcgggcaa 60
   ccggagcctg agcctcccgc ggggaagagc tcggactccg gaggtcgcgc cgccttgggc 120
   ttgaattcag tccttcctta ctactcgtgg ctttgcttac atcatttaac tctccatttc 180
   cttgtctata cagtgggctc ataaggaggc gg 212
<210> 45
   <211> 187
   <212> DNA
   <213> human
<400> 45
   cttccaggga ggcccaggga ggcagcggag agcgaagtcc tgctcggcgg agagacctcg 60
   aggagagtat ggggaaagga atgaatgctg cggagcgccc ctctgggctc cacccaagcc 120
   tcggaggcgg gacggtgggc tccgtcccga ccccttaggc agctggaccg atacctcctg 180
   gatcaga 187
<210> 46
   <211> 205
   <212> DNA
   <213> human
<400> 46
   gaagattcca agaggaatgg agggcttgtg gctccagctc ggacggtctc tggggcaagg 60
   aggtgcagga ggctgcgctg ttggatttct cctcggggtg agcagggccg ggctgtcctg 120
   ccggctctgg agcgcatatg ccgagccagc gcgcacttgg gtagggctaa ctgcgttcag 180
   ctttggcagg agctctgctc ttcct 205
<210> 47
   <211> 102
   <212> DNA
   <213> human
<400> 47
   agagtaagag agagcagccc atctaataga gtgtcccgga ggccagcgcc agcgggtgct 60
   gtaaggagcc cggcggcggc aggtgggaat tgattgagct gg 102
<210> 48
   <211> 165
   <212> DNA
   <213> human
<400> 48
   gtgcagctca ggcaggtcta gtgcatggca gcgccgtatt tacctgtgtt tgtgtcaatc 60
   ccagcgaggc ggccagctcg gcgcgttccg gcaaggcgag gtactgagtc ttctgaaacc 120
   ttctctgtaa tgcggccagc tgaaagctgg aataaatagt cctgg 165
<210> 49
   <211> 180
   <212> DNA
   <213> human
<400> 49
   ctgcccagcc ttccccctgt ccatgtacct ggctggttgg tggcgcttgg gacgcggttg 60
   taggcgccgc cgtactggtg gtaggagcta gcgtagctat agtcggcata agctttggct 120
   gggtagctcc cggcggagcc gttcacgccg tgatactgat actggtaggg gttgagagct 180
<210> 50
   <211> 164
   <212> DNA
   <213> human
<400> 50
   gtgaccagca ctttgtactg ctggggccgc tccagggaga agactctccc ttccaccgct 60
   tttctccctc gccggtttcc tggaccaccc ttcctcccca ccgcccggtt gcggtaactc 120
   agttaccgcg ctgggctgcg gttcccagga gggagggttc ggtt 164
<210> 51
   <211> 284
   <212> DNA
   <213> human
<400> 51
   ctggaatggg tgatctggga cgcggctcaa ttccctagag cataaggagg gaattgcgct 60
   ttttcttctt cattcgagaa atgatcggct cccgccagtc tgcgtgcttt cactcaacag 120
   cagctgcaag ccctaggcca gcgtcgcaga aacggccagg aaccccactt tcctacccgg 180
   gggccagcag cgatcgctgg ggctagcaag cgtgcagagg gggaaagaag gaaatcggct 240
   tccttcccgg acccctcctt cctcttccta ccaggaaggg aaaa 284
<210> 52
   <211> 159
   <212> DNA
   <213> human
<400> 52
   aaagtgggag gggctcaagg acccagagga aggacgtgtt tcacaggcgg aaagccgcgg 60
   ggacaatggc gagagcgacc gccctagccc cgggcggacc cgcaaccctc acactcgcgc 120
   gcccccgcgt tagtgctcga actggtctcc gcctttccg 159
<210> 53
   <211> 355
   <212> DNA
   <213> human
<400> 53
   agtgtgtcag tcactaggca gtgagacgcc aggcttaggg cgaaaaaagt tttcctggaa 60
   ctcgggagct ggtggggagt ctctgtcacg gactgagggt tttttgtgtt tttaaatggg 120
   agtctctggg taaaggggcg gggcgaggag gcgaacgaga caaccttagt attgtttgaa 180
   ttgaagcatt gcgagggaac aatggcagaa aaaccgcgtt ttctcccaag tcctgttccg 240
   gccctggaag agctgctccg ggcgccacac tgggatcctg ttcgccgagc ctgcccctcc 300
   acgccggccg tgcacagccc tgccgatacc gtgggtgcag ctggcgactg cactg 355
<210> 54
   <211> 329
   <212> DNA
   <213> human
<400> 54
   gatggagtag ccctgccctg tggcagccgg ggcccaggtg gattcctcct gggaacgacc 60
   cctccctcct cagcgcgcac tgtgccagag cagtacacag gtacaaaggg caaacgtgtt 120
   catgatttca tttgaattct gttgttttta catcgttctg ccctgatgta aaacaaacca 180
   ggattttaaa ggcagtgagt gatgaggagc tactcccact tggggaagag ctgctaactt 240
   tacttttccg ctgctgttgg ttccggttca aagctcggtt tgggaagtaa gaggaggact 300
   cggagacttt gaccagagca ctccaggcc 329
<210> 55
   <211> 364
   <212> DNA
   <213> human
<400> 55
   agaaagctga agtgacctgt gagactcctc tccacctcac ccgcggccaa aacgcccagc 60
   gccgggtctg ggtttgcgaa cggctcagcc caggccctgc gccagagcac cgcacccggg 120
   ggcggacggc cctgcagctc cccagcctct tcccgcatca actagctgac gcccgggcgc 180
   actggcttcc cccgctcccg tttcgtatta attagttgac gttcgggcgt attggttttt 240
   ttcgttttcg ttttgtatta attagttgat gtttgggtgt attggttttt tttgtttttg 300
   cccgcacgaa agccctgccc gacgtctgca gtcagcatcg cccgcactgc ctagggaggg 360
   tttg 364
<210> 56
   <211> 205
   <212> DNA
   <213> human
<400> 56
   cccgcactgc ctagggaggg tttgcgccga gacccagtgg ccgagtgagc cacagagtcg 60
   ctggtcctgg ggacgctgtc ttctaccgct ccccaccgtc ctcctctttc ctgtatctca 120
   ccccggtacc ccgcaccctt ctccccgctc ggccccgcgc actctgctca gactttacaa 180
   gcactttctc ggggcaccca ggact 205
<210> 57
   <211> 304
   <212> DNA
   <213> human
<400> 57
   acaagcactt tctcggggca cccaggactc gacgcctcct ccgcgccgcc agcggcgctc 60
   gccacccttg gcacgccgga gggaccgcct ccggccccct gcgcggcgtt tctcagggga 120
   ctcccagccc atccctcacc cagctgtccg ggtcccgacg ccttaccttt gccgaactgg 180
   gggacaccga ggctgagccc gaccaagagc cagacggcta ccgagcgctt catgatcccg 240
   tctcccggac gtgaccccgg ctggtcaggg gtcgtcgcgg agggcagtgt agccgaggtg 300
   gcag 304
<210> 58
   <211> 309
   <212> DNA
   <213> human
<400> 58
   ggggactccc agcccatccc tcacccagct gtccgggtcc cgacgcctta cctttgccga 60
   actgggggac accgaggctg agcccgacca agagccagac ggctaccgag cgcttcatga 120
   tcccgtctcc cggacgtgac cccggctggt caggggtcgt cgcggagggc agtgtagccg 180
   aggtggcagc gcagggcagc agcagactcc gcccctacta aagaattcaa gaagacgttc 240
   tctttcctca gcgctttgtt aaacaaattc ttggagaggg cgagagtggt gactaaagag 300
   aggagcctt 309
<210> 59
   <211> 275
   <212> DNA
   <213> human
<400> 59
   gagagtggtg actaaagaga ggagcctttc ctcccctttt gcctgcgctc cggcgcgcgg 60
   aggtgggtga gctccgggga gccgccggcg ggctcagccc tccgctgcgg gtgggtcccg 120
   gcagaggcgc ggcgggcggg gcgcgggctc ccgcggccgc cccgggtctg ctgcgcgggc 180
   aggcagaccc accggcagac aggcggaccg ggcgctcggc tgtcgctgtt ctccgcgcgg 240
   cggctgcggg gcttctgact tggagaacaa tgaag 275
<210> 60
   <211> 249
   <212> DNA
   <213> human
<400> 60
   actggttttc tctcgggcca gggcctccgg ggcaaccgtc tccagcgcgc attcttggtg 60
   caggtggaac agctttctgc tccggtaggg cttcacctat cgcgggagag gttaatctcg 120
   gatctaaacc tcgcagccgc agagcgggct aaaaccgcta ctccacctct tcccatttct 180
   cccctcccca cctcaagaca aaaagtccca ggccgggcag gacctgatca cctctgcctc 240
   ctcccactg 249
<210> 61
   <211> 247
   <212> DNA
   <213> human
<400> 61
   ccctaccact ccgggaactc tgtgatcttc aggaccggcg ggtttcaagt ggcgctgttt 60
   tcggtctctc ctgaggtcgc aaccctcttc ccacgaacaa cagcccacag gctccgcagc 120
   tcgccttggt ctcaacaacc gcgcagtact tgggtgctta agcgagtccc ctaccacata 180
   gtccagggtt gggggcgggg gagatggggc tgggagggga cagtggccag gagtagcgga 240
   aagagaa 247
<210> 62
   <211> 229
   <212> DNA
   <213> human
<400> 62
   aaggaccttg taagagaaag ggagaaaaga ttttgtgtgt ggagcgtgcc tcgtaaggtt 60
   tcgtgcttag gagaagttgg gaggaggcag ctcgcctaga gtctttacgg accgaattcg 120
   gagtttattt cgaacactat gcatcaagcc aaagaaaagc ggggccagtt tgggtttgcg 180
   cctaacttaa ttccgatacg cgcgtcaaaa tgttgtgtag gctggggcc 229
<210> 63
   <211> 211
   <212> DNA
   <213> human
<400> 63
   gagggagaga aaagaatact gaaaataaag ctggcggccg cgggctactg cctgcgtttg 60
   tgtgcgtgtg ccctgggtgt gtggtgtttg tgcctgggcg tgcgatttaa tggagcgcct 120
   ctctgcctct ccagtgcggc cagagctcgc ttcgcgcacc cacccctgcc gaggagccta 180
   cttgctgcag cccaatgcat tgtgtaagac g 211
<210> 64
   <211> 254
   <212> DNA
   <213> human
<400> 64
   ccgaggagcc tacttgctgc agcccaatgc attgtgtaag acgcgacctg ttatggccac 60
   cactacttcc gggttctagc attctggtcg gaatccacct ctccgcctgt gcaacacaca 120
   ctttacacac gcacggggac tgcaagcggg cagcatcgat cgtggctcct ttaagacaaa 180
   ctcagacaga catttttttt aaccctcctt ctctaatctc cttccagtgc agcacttgca 240
   aagagggaga gaga 254
<210> 65
   <211> 195
   <212> DNA
   <213> human
<400> 65
   cagcctcaga accccctgct gggaaacatc tgtcgctcta ggcccttgac gtcctgaggt       60
   tagagagcag cggtactact aggagggacg ccggcttggc taggacaccc tgcactccct      120
   ggggtcagga acgggaagga atggtcagaa acagatgatc agccacaatg atagatggtc      180
   agagacagaa atttt                                                       195
<210> 66
   <211> 300
   <212> DNA
   <213> human
<400> 66
   caacttttgg acacatctgg gcagttgcta agggctcttg ccaagcgtct aggtaagcct       60
   ttggcaaaca gctacagggg tcgtttttgc taatgtagga ctttgggaac ggtgctctgg      120
   cacacaattt gggtaaactt atgcgcgcat aaggatggga gggagcttgg tcaaagcggg      180
   gcttggcaaa tttctaggaa ccttcggcac agatccggag agggaccttt aaacaaacag      240
   ctcgaggggg ccatttggga agtgattggg gcaggaggga agcagagagc gcatcttttt      300
<210> 67
   <211> 172
   <212> DNA
   <213> human
<400> 67
   ggcgaggaag ctccataaaa gccctgtcgc gacccgctct ctgcacccca tccgctggct       60
   ctcacccctc ggagacgctc gcccgacagc atagtacttg ccgcccagcc acgcccgcgc      120
   gccagccacc gtgagtgcta cgacccgtct gtctaggggt gggagcgaac gg              172
<210> 68
   <211> 193
   <212> DNA
   <213> human
<400> 68
   ttacctttta gcagatatgg agggagaacc cgggaccgct atcccaaggc agctggcggt       60
   ctccctgcgg gtcgccgcct tgaggcccag gaagcggtgc gcggtaggaa ggtttccccg      120
   gcagcgccat cgagtgagga atccctggag ctctagagcc ccgcgccctg ccacctccct      180
   ggattcttgg gct                                                         193
<210> 69
   <211> 205
   <212> DNA
   <213> human
<400> 69
   gggagaaaag tgacccagca ggaagaactt ccaattcggt tttgaatgct aaactggcgg      60
   ggcccccacc ttgcactctc gccgcgcgct tcttggtccc tgagacttcg aacgaagttg     120
   cgcgaagttt tcaggtggag cagaggggca ggtcccgacc ggacggcgcc cggagcccgc     180
   aaggtggtgc tagccactcc tgggt                                           205
<210> 70
   <211> 174
   <212> DNA
   <213> human
<400> 70
   acaaatggat ggatgagtga atgaatgaac gaaggaacaa gtgaccgctc gaccttctac      60
   agcgacagcg gcgcaggcgc tcccgcacca acagcccccc accccgccac cgccagaggg     120
   cgcagacagg ccgggtcccg agtgtctgtc ccaggccccc gccccacagg atcc           174
<210> 71
   <211> 260
   <212> DNA
   <213> human
<400> 71
   tcgtcctctt gtgcagacca ggctggccgt gcaccagcga gtgtgcaccg agaccccaag      60
   ggcgtggggt ctccgcgccc gggctctatc tgtccccgac cacccccgag ccctccccgg     120
   gctcaccatg ccgcggtaca gctcctccag gcgcccgtcg atccacttct ccacgtccag     180
   ccgccgctgc agctcccgcc ggtcatactt gacggtgacg cgcgcgtgcc gcttctgcag     240
   ccccccggga ctgccccctg                                                 260
<210> 72
   <211> 17
   <212> DNA
   <213> human
<400> 72
   cggaagtcgc gttcgtc 17
<210> 73
   <211> 20
   <212> DNA
   <213> human
<400> 73
   gcgtacaact cgtccgctaa 20
<210> 74
   <211> 23
   <212> DNA
   <213> human
<400> 74
   gaatgtattt ggtcgtgtta cgc 23
<210> 75
   <211> 14
   <212> DNA
   <213> human
<400> 75
   cccgacgtcc cgcc 14
<210> 76
   <211> 23
   <212> DNA
   <213> human
<400> 76
   tttagtattt tcggacgcgt ttc 23
<210> 77
   <211> 26
   <212> DNA
   <213> human
<400> 77
   gaaataaccg acactacgta aaatcg 26
<210> 78
   <211> 21
   <212> DNA
   <213> human
<400> 78
   cggagacgtt cgttcgatag t 21
<210> 79
   <211> 22
   <212> DNA
   <213> human
<400> 79
   caaacgaatc gtaacactca cg 22
<210> 80
   <211> 23
   <212> DNA
   <213> human
<400> 80
   ctaaacccgt ccgcctaccc ctc 23
<210> 81
   <211> 20
   <212> DNA
   <213> human
<400> 81
   cggattttcg attcggaagt 20
<210> 82
   <211> 250
   <212> DNA
   <213> human
<400> 82
   tagcgcagga tgagggaggc agcccatcct caccgccggt ctcggtccgc gagacgcggg    60
   gacagcgcgg tgcgcggccc gcatgcaggg gtgaccggag ggccgtcccc cccacgaccc   120
   ggaaagaagg agagcctccc gttaggcccc tgtgggtgct ccttggccga ggagctcggt   180
   cttcgctctc ccaccctccc cctttctact cccaagcagg agagcgtgca gccctagcct   240
   gcacaaggcg                                                          250

## Description

### FIELD OF THE INVENTION

The present invention relates generally to nucleic acid molecules in respect of which changes to DNA methylation levels are indicative of the onset or predisposition to the onset of a neoplasm. More particularly, the present invention is directed to nucleic acid molecules in respect of which changes to DNA methylation levels are indicative of the onset and/or progression of a large intestine neoplasm, such as an adenoma or adenocarcinoma. The DNA methylation status of the present invention is useful in a range of applications including, but not limited to, those relating to the diagnosis and/or monitoring of colorectal neoplasms, such as colorectal adenocarcinomas. Accordingly, in a related aspect the present invention is directed to a method of screening for the onset, predisposition to the onset and/or progression of a neoplasm by screening for modulation in DNA methylation of one or more nucleic acid molecules.

### BACKGROUND OF THE INVENTION

Colorectal cancer includes cancerous growths in the colon, rectum and appendix. With 655,000 deaths worldwide per year, it is the fourth most common form of cancer in the United States and the third leading cause of cancer-related death in the Western world. Colorectal cancers arise from adenomatous polyps in the colon. These mushroom-shaped growths are usually benign, but some develop into cancer over time. Localized colon cancer is usually diagnosed through colonoscopy.

Invasive cancers that are confined within the wall of the colon (TNM stages I and II) are curable with surgery. If untreated, they spread to regional lymph nodes (stage III), where up to 73% are curable by surgery and chemotherapy. Cancer that metastasizes to distant sites (stage IV) is usually not curable, although chemotherapy can extend survival, and in rare cases, surgery and chemotherapy together have seen patients through to a cure (Markowitz and Bertagnolli, 2009, N. Engl. J. Med. 361(25): 2449-60). Radiation is used with rectal cancer.

Colorectal cancer is preceded by adenomas. Adenomas are benign tumours, or neoplasms, of epithelial origin which are derived from glandular tissue or exhibit clearly defined glandular structures. Some adenomas show recognisable tissue elements, such as fibrous tissue (fibroadenomas) and epithelial structure, while others, such as bronchial adenomas, produce active compounds that might give rise to clinical syndromes.

Adenomas may progress to become an invasive neoplasm and are then termed adenocarcinomas. Accordingly, adenocarcinomas are defined as malignant epithelial tumours arising from glandular structures, which are constituent parts of many organs of the body. The term adenocarcinoma is also applied to tumours showing a glandular growth pattern. These tumours may be sub-classified according to the substances that they produce, for example mucus secreting and serous adenocarcinomas, or to the microscopic arrangement of their cells into patterns, for example papillary and follicular adenocarcinomas. These carcinomas may be solid or cystic (cystadenocarcinomas). Each organ may produce tumours showing a variety of histological types, for example the ovary may produce both mucinous and cystadenocarcinoma.

Adenomas in different organs behave differently. In general, the overall chance of carcinoma being present within an adenoma (i.e. a focus of cancer having developed within a benign lesion) is approximately 5%. However, this is related to size of an adenoma. For instance, in the large bowel (colon and rectum specifically) occurrence of a cancer within an adenoma is rare in adenomas of less than 1 centimetre. Such a development is estimated at 40 to 50% in adenomas which are greater than 4 centimetres and show certain histopathological change such as villous change, or high grade dysplasia. Adenomas with higher degrees of dysplasia have a higher incidence of carcinoma. In any given colorectal adenoma, the predictors of the presence of cancer now or the future occurrence of cancer in the organ include size (especially greater than 9mm) degree of change from tubular to villous morphology, presence of high grade dysplasia and the morphological change described as "serrated adenoma". In any given individual, the additional features of increasing age, familial occurrence of colorectal adenoma or cancer, male gender or multiplicity of adenomas, predict a future increased risk for cancer in the organ - so-called risk factors for cancer. Except for the presence of adenomas and its size, none of these is objectively defined and all those other than number and size are subject to observer error and to confusion as to precise definition of the feature in question. Because such factors can be difficult to assess and define, their value as predictors of current or future risk for cancer is imprecise.

Once a sporadic adenoma has developed, the chance of a new adenoma occurring is approximately 30% within 26 months.

The symptoms of colorectal cancer depend on the location of tumor in the bowel, and whether is has metastasised. Unfortunately, many of the symptoms may occur in other diseases as well, and hence symptoms may not be conclusively diagnostic of colorectal cancer.

Local symptoms are more likely if the tumor is located closer to the anus. There may be a change in bowel habit (new-onset constipation or diarrhea in the absence of another cause), a feeling of incomplete defecation and reduction in diameter of stools. Tenesmus and change in stool shape are both characteristic of rectal cancer. Lower gastrointestinal bleeding, including the passage of bright red blood in the stool, may indicate colorectal cancer, as may the increased presence of mucus. Melena, black stool with a tarry appearance, normally occurs in upper gastrointestinal bleeding (such as from a duodenal ulcer), but is sometimes encountered in colorectal cancer when the disease is located in the beginning of the large bowl.

A tumor that is large enough to fill the entire lumen of the bowel may cause bowel obstruction. This situation is characterized by constipation, abdominal pain, abdominal distension and vomiting. This occasionally leads to the obstructed and distended bowel perforating and causing peritonitis.

Certain local effects of colorectal cancer occur when the disease has become more advanced. A large tumor is more likely to be noticed on feeling the abdomen, and it may be noticed by a doctor on physical examination. The disease may invade other organs, and may cause blood or air in the urine or vaginal discharge.

If a tumor has caused chronic occult bleeding, iron deficiency anaemia may occur. This may be experienced as fatigue, palpitations and noticed as pallor. Colorectal cancer may also lead to weight loss, generally due to a decreased appetite.

More unusual constitutional symptoms are an unexplained fever and one of several paraneoplastic syndromes. The most common paraneoplastic syndrome is thrombosis, usually deep vein thrombosis.

Colorectal cancer most commonly spreads to the liver. This may go unnoticed, but large deposits in the liver may cause jaundice and abdominal pain (due to stretching of the capsule). If the tumor deposit obstructs the bile duct, the jaundice may be accompanied by other features of biliary obstruction, such as pale stools.

Colorectal cancer can take many years to develop and early detection of colorectal cancer greatly improves the prognosis. Even modest efforts to implement colorectal cancer screening methods can result in a drop in cancer deaths. Despite this, colorectal cancer screening rates remain low. There are currently several different tests available for this purpose:
- Digital rectal exam: The doctor inserts a lubricated, gloved finger into the rectum to feel for abnormal areas. It only detects tumors large enough to be felt in the distal part of the rectum but is useful as an initial screening test.
- Faecal occult blood test: a test for blood in the stool. Two types of tests can be used for detecting occult blood in stools i.e. guaiac based (chemical test) and immunochemical. The sensitivity of immunochemical testing is superior to that of chemical testing without an unacceptable reduction in specificity (Weitzel JN (December 1999). "Genetic cancer risk assessment. Putting it all together". Cancer 86 (11 Suppl): 2483-92).
- Endoscopy:
   ∘ Sigmoidoscopy: A lit probe (sigmoidoscope) is inserted into the rectum and lower colon to check for polyps and other abnormalities.
   ∘ Colonoscopy: A lit probe called a colonoscope is inserted into the rectum and the entire colon to look for polyps and other abnormalities that may be caused by cancer. A colonoscopy has the advantage that if polyps are found during the procedure they can be removed immediately. Tissue can also be taken for biopsy.
- Double contrast barium enema (DCBE): First, an overnight preparation is taken to cleanse the colon. An enema containing barium sulfate is administered, then air is insufflated into the colon, distending it. The result is a thin layer of barium over the inner lining of the colon which is visible on X-ray films. A cancer or a precancerous polyp can be detected this way. This technique can miss the (less common) flat polyp.
- Virtual colonoscopy replaces X-ray films in the double contrast barium enema (above) with a special computed tomography scan and requires special workstation software in order for the radiologist to interpret. This technique is approaching colonoscopy in sensitivity for polyps. However, any polyps found must still be removed by standard colonoscopy.
- Standard computed axial tomography is an x-ray method that can be used to determine the degree of spread of cancer, but is not sensitive enough to use for screening. Some cancers are found in CAT scans performed for other reasons.
- Blood tests: Measurement of the patient's blood for elevated levels of certain proteins can give an indication of tumor load. In particular, high levels of carcinoembryonic antigen (CEA) in the blood can indicate metastasis of adenocarcinoma. While hese tests are frequently false positive or false negative, and are not recommended for screening, they can be useful to assess disease recurrence. CA19-9 and CA 242 biomarkers can indicate e-selectin related metastatic risks, help follow therapeutic progress, and assess disease recurrence. Recently, an assay for detection in plasma of methylated sequences of the Septin 9 gene has also become available to assist in diagnosis of colorectal cancer.
- Positron emission tomography (PET) is a 3-dimensional scanning technology where a radioactive sugar is injected into the patient, the sugar collects in tissues with high metabolic activity, and an image is formed by measuring the emission of radiation from the sugar. Because cancer cells often have very high metabolic rates, this can be used to differentiate benign and malignant tumors. PET is not used for screening and does not (yet) have a place in routine workup of colorectal cancer cases.
- Stool DNA testing is an emerging technology in screening for colorectal cancer. Premalignant adenomas and cancers shed DNA markers from their cells which are not degraded during the digestive process and remain stable in the stool. Capture, followed by PCR amplifies the DNA to detectable levels for assay.
- High C-Reactive Protein levels as risk marker

Despite the existence of these tests, diagnosis remains problematic. Most of the more sensitive tests are quite invasive and expensive and therefore uptake by patients is low. There is therefore an ongoing need to develop simpler and more informative diagnostic protocols or aids to diagnosis that enable one to direct colonoscopy at people more likely to have developed adenomas or carcinomas. A simple and accurate screening test would enable much more widely applicable screening systems to be set up.

In work leading up to the present invention it has been determined that changes to the methylation of certain genes is indicative of the development of neoplasms of the large intestine, such as adenomas and adenocarcinomas. Still further, the identification of specific genomic DNA cytosine nucleotides which become hypermethylated has enabled the development of very simple and specific amplification reactions for routine use in the context of diagnosis. Diagnosis can therefore be made based on screening for one or more of a panel of these differentially methylated genes. Accordingly, the inventors have identified a panel of genes which facilitate the diagnosis of adenocarcinoma and adenoma development and/or the monitoring of conditions characterised by the development of these types of neoplasms.

### SUMMARY OF THE INVENTION

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integers or steps.

As used herein, the term "derived from" shall be taken to indicate that a particular integer or group of integers has originated from the species specified, but has not necessarily been obtained directly from the specified source. Further, as used herein the singular forms of "a", "and" and "the" include plural referents unless the context clearly dictates otherwise.

Unless otherwise defined, all technical and scientific terms used herein have the same meaning as commonly understood by one of ordinary skill in the art to which this invention belongs.

The subject specification contains nucleotide sequence information prepared using the programme PatentIn Version 3.5, presented herein after the bibliography. Each nucleotide sequence is identified in the sequence listing by the numeric indicator <210> followed by the sequence identifier (eg. <210>1, <210>2, etc). The length, type of sequence (DNA, etc) and source organism for each sequence is indicated by information provided in the numeric indicator fields <211>, <212> and <213>, respectively. Nucleotide sequences referred to in the specification are identified by the indicator SEQ ID NO: followed by the sequence identifier (eg. SEQ ID NO:1, SEQ ID NO:2, etc.). The sequence identifier referred to in the specification correlates to the information provided in numeric indicator field <400> in the sequence listing, which is followed by the sequence identifier (eg. <400>1, <400>2, etc). That is SEQ ID NO:1 as detailed in the specification correlates to the sequence indicated as <400>1 in the sequence listing.

One aspect of the present invention is directed to a method of screening for the onset or predisposition to the onset of or monitoring a large intestine neoplasm in an individual, said method comprising assessing the methylation status of a DNA region selected from:
(i) the region defined by any one or more of Hg19 coordinates and 2kb upstream of the transcription start site:

| | |
|---|---|
| (1) chr12:52400748..52409671 | (34) chr10:101292690..101296281 |
| (2) chr5:3596168..3601517 | (35) chr4:4190530..4228621 |
| (3) chr13:95361876..95364389 | (36) chr12:54943404..54973023 |
| (4) chr4:81187742..81212171 | (37) chr5:176047210..176057557 |
| (5) chr19:57019212..57040270 | (38) chr12:22346325..22487648 |
| (6) chr3:33191537..33260707 | (39) chr19:56894648..56904889 |
| (7) chr15:60296421..60298142 | (40) chr20:21491648..21494664 |
| (8) chr13:28494168..28500451 | (41) chr1:50883225..50889141 |
| (9) chr7:96649702..96654143, | (42) chr7:27180996..27183287 |
| (10) chr8:140,811,770-141,537,860 | (43) chr11:2016406..2019065 |
| (11) chr5:2746279..2751769 | (44) chr14:57267425..57277184 |
| (12) chr18:55102917..55158530 | (45) chr4:126237567..126414087 |
| (13) chr20:37353101..37358016 | (46) chr8:23559964..23563922 |
| (14) chr8:2792875..4852328 | (47) chr10:131633547..131762091 |
| (15) chr16:66613351..66622178 | (48) chr4:62362839..62938168 |
| (16) chr5:37815753..37839782 | (49) chr1:47901689..47906363 |
| (17) chr1:63788730..63790797 | (50) chr17:77768176..77770890 |
| (18) chr15:37156644..37178734 | (51) chr17:93598762..93604831 |
| (19) chr7:27139973..27142394 | (52) chr1:33789224..33841194 |
| (20) chr20:21686297..21696620 | (53) chr9:124,004,679-124,030,840 |
| (21) chr16:51169886..51185183 | (54) chr4:158141736..158287227 |
| (22) chr12:85253267..85306606 | (55) chr12:9445136..9462559 |
| (23) chr8:6357172..6420784 | (56) chr12:24964278..25102308 |
| (24) chr14:85996488..86094270 | (57) chrX:21542357..21690352 |
| (25) chr2:182541194..182545381 | (58) chr20:52769988..52790516 |
| (26) chr7:30951468..30965131 | (59) chr3:172162951..172166203 |
| (27) chr8:131792547..132052835 | (60) chr13:28366780..28368089 |
| (28) chr3:128749292..128759583 | (61) chr7:50344378...50472799 |
| (29) chr10:101088856..101154087 | (62) chr7:149412148..149431664 |
| (30) chr7:27282164..27286192 | (63) chr7:24323809..24331477 |
| (31) chr10:129535538..129539450 | (64) chr4:30722037..31148421 |
| (32) chr19:49316274..49339934 | (65) chr10:47083534..47088320 |
| (33) chr6:391752..411443; or | |

(ii) the gene region, including 2kb upstream of any one or more of:

| | | | |
|---|---|---|---|
| (1) GRASP | (18) ANGPT2 | (35) NKX2-6 | (52) HOXA5 |
| (2) IRX1 | (19) LHX6 | (36) PAX1 | (53) GDNF |
| (3) SOX21 | (20) NEUROD1 | (37) FOXD2 | (54) FAT4 |
| (4) FGF5 | (21) AC149644.1 | (38) SLC6A15 | (55) HOXA2 |
| (5) ZNF471 | (22) CCDC48 | (39) PHC2 | (56) LPHN3 |
| (6) SUSD5 | (23) EVX1 | (40) FLRT2 | (57) ADCYAP1 |
| (7) FOXB1 | (24) GHSR | (41) GATA2 | (58) GRIA2 |
| (8) PDX1 | (25) HSD17B14 | (42) ADCY8 | (59) AQP1 |
| (9) DLX5 | (26) KRBA1 | (43) CNNM1 | (60) BCAT1 |
| (10) ONECUT2 | (27) OTOP1 | (44) IKZF1 | (61) CYP24A1 |
| (11) DMRTA2 | (28) PPYR1 | (45) NKX2-3 | (62) FOXI2 |
| (12) CMTM2 | (29) SRMS | (46) PCDH7 | (63) GSX1 |
| (13) OTX2 | (30) ZNF582 | (47) SNCB | (64) IRF4 |
| (14) LOC145845 | (31) IRX2 | (48) ST8SIA1 | (65) NPY |
| (15) EBF3 | (32) CSMD1 | (49) TRAPPC9 | (66) PDE1B |
| (16) SALL1 | (33) MIR675,H19 | (50) NKX2-2 | |
| (17) CBX8 | (34) FOXD3 | (51) SLC32A1 | |

in a biological sample from said individual wherein a higher level of methylation of the DNA regions of group (i) and/or (ii) relative to control levels is indicative of a large intestine neoplasm or a predisposition to the onset of a large intestine neoplastic state.

In another aspect there is provided a method of screening for the onset or predisposition to the onset of or monitoring a large intestine neoplasm in an individual, said method comprising assessing the methylation status of a DNA region selected from:
(i) the region defined by any one or more of Hg19 coordinates and 2kb upstream of the transcription start site:

| | |
|---|---|
| (1) chr12:52400748..52409671 | (6) chr3:33191537..33260707 |
| (2) chr5:3596168..3601517 | (7) chr15:60296421..60298142 |
| (3) chr13:95361876..95364389 | (8) chr13:28494168..28500451 |
| (4) chr4:81187742..81212171 | (9) chr7:96649702..96654143 |
| (5) chr19:57019212..57040270 | |

(ii) the gene region, including 2kb upstream of any one or more of:

| | | | |
|---|---|---|---|
| (1) GRASP | (4) FGF5 | (6) SUSD5 | (8) PDX1 |
| (2) IRX1 | (5) ZNF471 | (7) FOXB1 | (9) DLX5 |
| (3) SOX21 | | | |

in a biological sample from said individual wherein a higher level of methylation of the DNA regions of group (i) and/or (ii) relative to control levels is indicative of a large intestine neoplasm or a predisposition to the onset of a large intestine neoplastic state.

The subregions which have been determined to exhibit particular utility are listed below with reference to the gene and chromosomal region within which they are found:
(1) Chr12:52400748-52409671 (GRASP) subregions Chr12:52399672-52399922 (SEQ ID NO:15), Chr12:52400821-52401119 (SEQ ID NO:16) and Chr12:52401407-52401664 (SEQ ID NO:17).
(2) Chr8:97505882-97624037 (SDC2) subregions Chr8:97506813-97507045 (SEQ ID NO:18) and Chr8:97507037-97507257 (SEQ ID NO:19).
(3) Chr13:110959094-110959330 (SEQ ID NO:20) (COL4A1).
(4) Chr5:3596168-3601517 (IRX1) subregions Chr5:3597227-3597480 (SEQ ID NO:21), Chr5:3599990-3600175 (SEQ ID NO:22), Chr5:3600160-3600352 (SEQ ID NO:23) and Chr5:3594657-3594847 (SEQ ID NO:24).
(5) Chr13:95361879-95364389 (SOX21) subregions Chr13:95364013-95364178 (SEQ ID NO:25) and Chr13:95364515-95364784 (SEQ ID NO:26).
(6) Chr4:81187742-81212171 (FGF5) subregions Chr4:81186918-81187228 (SEQ ID NO:27), Chr4:81187326-81187578 (SEQ ID NO:28) and Chr4:81187571-81187792 (SEQ ID NO:29).
(7) Chr19:57019212-57040269 (ZNF471) subregions Chr19:57018955-57019135 (SEQ ID NO:30) and Chr19:57019294-57019573 (SEQ ID NO:31).
(8) Chr3:33191537-33260707 (SUSD5) subregion Chr3:33260566-33260818 (SEQ ID NO:32).
(9) Chr2:56093097-56151298 (EFEMP1) subregion Chr2:56150356-56150606 (SEQ ID NO:33).
(10) Chr6:134210259-134216675 (TCF21) subregions Chr6:134210545-134210749 (SEQ ID NO:34), Chr6:134210712-134210951 (SEQ ID NO:35) and Chr6:134210994-134211274 (SEQ ID NO:36).
(11) Chr7:94023873-94060544 (COL1A2) subregions Chr7:94023751-94023975 (SEQ ID NO:37) and Chr7:94024141-94024345 (SEQ ID NO:38).
(12) Chr15:60296421-60298142 (FOXB1) subregions Chr15:60296522-60296719 (SEQ ID NO:39) and Chr15:60297024-60297305 (SEQ ID NO:40).
(13) Chr16:86544133-86548070 (FOXF1) subregions Chr16:86544560-86544770 (SEQ ID NO:41), Chr16:86544265-86544584 (SEQ ID NO:42) and Chr16:86544795-86545110 (SEQ ID NO:43).
(14) Chr13:28494168-28500451 (PDX1) subregions Chr13:28502100-28502311 (SEQ ID NO:44), Chr13:28502417-28502603 (SEQ ID NO:45) and Chr13:28503006-28503210 (SEQ ID NO:46).
(15) Chr7:96649702-96654143 (DLX5) subregions Chr7:96650026-96650127 (SEQ ID NO:47), Chr7:96651454-96651618 (SEQ ID NO:48) and Chr7:96653553-96653732 (SEQ ID NO:49).
(16) Chr21:28208606-28217728 (ADAMTS1) subregions Chr21:28217946-28218109 (SEQ ID NO:50), Chr21:28218494-28218777 (SEQ ID NO:51)and Chr21:28218859-28219017 (SEQ ID NO:52).
(17) Chr13:110959631-111165373 (COL4A2) subregions Chr13:110960787-110961141 (SEQ ID NO:53); Chr13:110961331-110961659 (SEQ ID NO:54) and Chr13:110959932-110960181 (SEQ ID NO:82);
(18) Chr5:83238126-83680611 (EDIL3) subregions Chr5:83679544-83679807 (SEQ ID NO:55), Chr5:83679784-83679988 (SEQ ID NO:56), Chr5:83679960-83680263 (SEQ ID NO:57), Chr5:83680075-83680383 (SEQ ID NO:58) and Chr5:83680356-83680630 (SEQ ID NO:59).
(19) Chr15:48700503-48937985 (FBN1) subregion Chr15:48938136-48938384 (SEQ ID NO:60).
(20) Chr1:47901689-47906363 (FOXD2) subregions Chr1:47899091-47899337 (SEQ ID NO:61) and Chr1:47909944-47910172 (SEQ ID NO:62).
(21) Chr2:66662532-66799891 (MEIS1) subregions Chr2:66662009-66662219 (SEQ ID NO:63) and Chr2:66662177-66662430 (SEQ ID NO:64).
(22) Chr16:55513081-55540586 (MMP2) subregions Chr16:55512662-55512856 (SEQ ID NO:65) and Chr16:55513916-55514215 (SEQ ID NO:66).
(23) Chr7:24323807-24331484 (NPY) subregions Chr7:24323765-24323936 (SEQ ID NO:67), Chr7:24324150-24324342 (SEQ ID NO:68) and Chr7:24324513-24324717 (SEQ ID NO:69).
(24) Chr19:38741877-38747172 (PPP1R14A) subregions Chr19:38747251-38747424 (SEQ ID NO:70) and Chr19:38746653-38746912 (SEQ ID NO:71).

In still another embodiment there is provided a method of screening for the onset or predisposition to the onset of or monitoring a large intestine neoplasm in an individual, said method comprising assessing the methylation of one or more cytosine residues selected from:
*Chr12:52400748-52409671 (GRASP)*

| | | | |
|---|---|---|---|
| Chr12:52399713 | Chr12:52399731 | Chr12:52399749 | Chr12:52399783 |
| Chr12:52399796 | Chr12:52399808 | Chr12:52399823 | Chr12:52399835 |
| Chr12:52399891 | | | |
| | | | |
| Chr12:52400847 | Chr12:52400850 | Chr12:52400859 | Chr12:52400866 |
| Chr12:52400869 | Chr12:52400873 | Chr12:52400881 | Chr12:52400886 |
| Chr12:52400893 | Chr12:52400895 | Chr12:52400899 | Chr12:52400902 |
| Chr12:52400907 | Chr12:52400913 | Chr12:52400919 | Chr12:52400932 |
| Chr12:52400938 | Chr12:52400958 | Chr12:52400962 | Chr12:52400971 |
| Chr12:52400973 | Chr12:52400976 | Chr12:52400998 | Chr12:52401008 |
| Chr12:52401010 | Chr12:52401012 | Chr12:52401016 | Chr12:52401019 |
| Chr12:52401025 | Chr12:52401041 | Chr12:52401044 | Chr12:52401053 |
| Chr12:52401060 | Chr12:52401064 | Chr12:52401092 | Chr12:52401118 |
| | | | |
| Chr12:52401438 | Chr12:52401448 | Chr12:52401460 | Chr12:52401465 |
| Chr12:52401474 | Chr12:52401477 | Chr12:52401479 | Chr12:52401483 |
| Chr12:52401504 | Chr12:52401514 | Chr12:52401523 | Chr12:52401540 |
| Chr12:52401553 | Chr12:52401576 | Chr12:52401588 | Chr12:52401595 |
| Chr12:52401599 | Chr12:52401604 | Chr12:52401606 | Chr12:52401634 |
| Chr12:52401640 | Chr12:52401644 | Chr12:52401659 | |

*Chr8:97505882-97624037 (SDC2)*

| | | | |
|---|---|---|---|
| Chr8:97506843 | Chr8:97506845 | Chr8:97506861 | Chr8:97506876 |
| Chr8:97506902 | Chr8:97506909 | Chr8:97506911 | Chr8:97506913 |
| Chr8:97506924 | Chr8:97506932 | Chr8:97506934 | Chr8:97506940 |
| Chr8:97506977 | Chr8:97506993 | Chr8:97507005 | Chr8:97507008 |
| Chr8:97507014 | | | |
| | | | |
| Chr8:97507038 | Chr8:97507061 | Chr8:97507064 | Chr8:97507078 |
| Chr8:97507082 | Chr8:97507084 | Chr8:97507089 | Chr8:97507119 |
| Chr8:97507128 | Chr8:97507136 | Chr8:97507144 | Chr8:97507153 |
| Chr8:97507162 | Chr8:97507214 | Chr8:97507216 | |

*Chr13:110959094-110959330 (COL4A1).*

| | | | |
|---|---|---|---|
| Chr13:97507038 | Chr13:97507061 | Chr13:97507064 | Chr13:97507078 |
| Chr13:97507082 | Chr13:97507084 | Chr13:97507089 | Chr13:97507119 |
| Chr13:97507128 | Chr13:97507136 | Chr13:97507144 | Chr13:97507153 |
| Chr13:97507162 | Chr13:97507214 | Chr13:97507216 | |

*Chr5:3596168-3601517 (IRX1)*

| | | | |
|---|---|---|---|
| Chr5:3597229 | Chr5:3597299 | Chr5:3597311 | Chr5:3597321 |
| Chr5:3597330 | Chr5:3597350 | Chr5:3597352 | Chr5:3597373 |
| Chr5:3597413 | Chr5:3597427 | Chr5:3597431 | Chr5:3597447 |
| Chr5:3597450 | Chr5:3597454 | Chr5:3597456 | |
| | | | |
| Chr5:3599998 | Chr5:3600019 | Chr5:3600037 | Chr5:3600050 |
| Chr5:3600058 | Chr5:3600061 | Chr5:3600070 | Chr5:3600074 |
| Chr5:3600076 | Chr5:3600079 | Chr5:3600089 | Chr5:3600091 |
| Chr5:3600104 | Chr5:3600116 | Chr5:3600121 | Chr5:3600124 |
| Chr5:3600131 | Chr5:3600143 | Chr5:3600149 | Chr5:3600169 |
| Chr5:3600173 | | | |
| | | | |
| Chr5:3600163 | Chr5:3600190 | Chr5:3600201 | Chr5:3600205 |
| Chr5:3600208 | Chr5:3600214 | Chr5:3600250 | Chr5:3600252 |
| Chr5:3600255 | Chr5:3600264 | Chr5:3600270 | Chr5:3600284 |
| Chr5:3600309 | Chr5:3600318 | Chr5:3600345 | |
| | | | |
| Chr5:3594663 | Chr5:3594679 | Chr5:3594684 | Chr5:3594697 |
| Chr5:3594703 | Chr5:3594711 | Chr5:3594725 | Chr5:3594738 |
| Chr5:3594745 | Chr5:3594758 | Chr5:3594768 | Chr5:3594774 |
| Chr5:3594778 | Chr5:3594794 | Chr5:3594799 | Chr5:3594818 |
| Chr5:3594840 | | | |

*Chr13:95361879-95364389 (SOX21)*

| | | | |
|---|---|---|---|
| Chr13:95364016 | Chr13:95364019 | Chr13:95364042 | Chr13:95364050 |
| Chr13:95364054 | Chr13:95364061 | Chr13:95364064 | Chr13:95364074 |
| Chr13:95364080 | Chr13:95364082 | Chr13:95364106 | Chr13:95364119 |
| Chr13:95364123 | Chr13:95364125 | Chr13:95364128 | Chr13:95364141 |
| Chr13:95364163 | Chr13:95364171 | | |
| | | | |
| Chr13:95364543 | Chr13:95364545 | Chr13:95364548 | Chr13:95364551 |
| Chr13:95364560 | Chr13:95364571 | Chr13:95364582 | Chr13:95364584 |
| Chr13:95364587 | Chr13:95364602 | Chr13:95364623 | Chr13:95364627 |
| Chr13:95364640 | Chr13:95364649 | Chr13:95364653 | Chr13:95364656 |
| Chr13:95364665 | Chr13:95364684 | Chr13:95364696 | Chr13:95364712 |
| Chr13:95364729 | Chr13:95364732 | Chr13:95364734 | Chr13:95364748 |
| Chr13:95364751 | | | |

*Chr4:81187742-81212171 (FGF5)*

| | | | |
|---|---|---|---|
| Chr4:81186919 | Chr4:81186947 | Chr4:81186964 | Chr4:81186968 |
| Chr4:81186973 | Chr4:81186975 | Chr4:81186983 | Chr4:81187001 |
| Chr4:81187011 | Chr4:81187041 | Chr4:81187056 | Chr4:81187062 |
| Chr4:81187093 | Chr4:81187116 | Chr4:81187125 | Chr4:81187161 |
| Chr4:81187191 | Chr4:81187196 | Chr4:81187198 | |
| | | | |
| Chr4:81187367 | Chr4:81187389 | Chr4:81187458 | Chr4:81187467 |
| Chr4:81187495 | Chr4:81187498 | Chr4:81187504 | Chr4:81187512 |
| Chr4:81187514 | Chr4:81187530 | Chr4:81187539 | Chr4:81187547 |
| Chr4:81187549 | Chr4:81187551 | Chr4:81187554 | Chr4:81187556 |
| Chr4:81187575 | | | |
| | | | |
| Chr4:81187575 | Chr4:81187592 | Chr4:81187601 | Chr4:81187605 |
| Chr4:81187610 | Chr4:81187648 | Chr4:81187652 | Chr4:81187679 |
| Chr4:81187685 | Chr4:81187691 | Chr4:81187693 | Chr4:81187707 |
| Chr4:81187712 | Chr4:81187733 | Chr4:81187735 | Chr4:81187752 |
| Chr4:81187758 | Chr4:81187764 | Chr4:81187784 | |

*Chr19:57019212-57040269 (ZNF471)*

| | | | |
|---|---|---|---|
| Chr19:57018990 | Chr19:57018994 | Chr19:57019003 | Chr19:57019010 |
| Chr19:57019018 | Chr19:57019020 | Chr19:57019025 | Chr19:57019029 |
| Chr19:57019044 | Chr19:57019047 | Chr19:57019067 | Chr19:57019073 |
| Chr19:57019084 | Chr19:57019101 | Chr19:57019118 | |
| | | | |
| Chr19:57019315 | Chr19:57019321 | Chr19:57019346 | Chr19:57019351 |
| Chr19:57019355 | Chr19:57019361 | Chr19:57019364 | Chr19:57019366 |
| Chr19:57019371 | Chr19:57019373 | Chr19:57019385 | Chr19:57019387 |
| Chr19:57019405 | Chr19:57019428 | Chr19:57019433 | Chr19:57019435 |
| Chr19:57019437 | Chr19:57019443 | Chr19:57019451 | Chr19:57019456 |
| Chr19:57019463 | Chr19:57019465 | Chr19:57019470 | Chr19:57019483 |
| Chr19:57019487 | Chr19:57019492 | Chr19:57019502 | Chr19:57019505 |

*Chr3:33191537-33260707 (SUSD5)*

| | | | |
|---|---|---|---|
| Chr3:33260601 | Chr3:33260621 | Chr3:33260631 | Chr3:33260640 |
| Chr3:33260651 | Chr3:33260665 | Chr3:33260676 | Chr3:33260694 |
| Chr3:33260698 | Chr3:33260711 | Chr3:33260715 | Chr3:33260732 |
| Chr3:33260742 | Chr3:33260748 | Chr3:33260755 | Chr3:33260760 |
| Chr3:33260769 | Chr3:33260776 | Chr3:33260778 | Chr3:33260780 |
| Chr3:33260788 | Chr3:33260806 | | |

*Chr2:56093097-56151298 (EFEMP1)*

| | | | |
|---|---|---|---|
| Chr2:56150376 | Chr2:56150389 | Chr2:56150394 | Chr2:56150415 |
| Chr2:56150419 | Chr2:56150423 | Chr2:56150433 | Chr2:56150473 |
| Chr2:56150475 | Chr2:56150478 | Chr2:56150499 | Chr2:56150537 |
| Chr2:56150549 | Chr2:56150580 | Chr2:56150601 | |

*Chr6:134210259-134216675 (TCF21)*

| | | | |
|---|---|---|---|
| Chr6:134210556 | Chr6:134210598 | Chr6:134210615 | Chr6:134210640 |
| Chr6:134210649 | Chr6:134210667 | Chr6:134210692 | Chr6:134210694 |
| Chr6:134210697 | Chr6:134210720 | Chr6:134210745 | |
| Chr6:134210720 | Chr6:134210745 | Chr6:134210776 | Chr6:134210781 |
| Chr6:134210784 | Chr6:134210790 | Chr6:134210792 | Chr6:134210794 |
| Chr6:134210800 | Chr6:134210806 | Chr6:134210812 | Chr6:134210868 |
| Chr6:134210894 | Chr6:134210906 | Chr6:134210919 | Chr6:134210946 |
| | | | |
| Chr6:134211050 | Chr6:134211061 | Chr6:134211076 | Chr6:134211081 |
| Chr6:134211103 | Chr6:134211110 | Chr6:134211121 | Chr6:134211125 |
| Chr6:134211131 | Chr6:134211153 | Chr6:134211155 | Chr6:134211162 |
| Chr6:134211179 | Chr6:134211182 | Chr6:134211184 | Chr6:134211208 |
| Chr6:134211210 | Chr6:134211212 | Chr6:134211218 | Chr6:134211220 |
| Chr6:134211227 | Chr6:134211233 | Chr6:134211241 | Chr6:134211245 |
| Chr6:134211247 | Chr6:134211270 | | |

*Chr7:94023873-94060544 (COL1A2)*

| | | | |
|---|---|---|---|
| Chr7:94024172 | Chr7:94024191 | Chr7:94024214 | Chr7:94024230 |
| Chr7:94024254 | Chr7:94024266 | Chr7:94024268 | Chr7: 94024272 |
| Chr7:94024288 | Chr7:94024291 | Chr7:94024310 | |

*Chr15:60296421-60298142 (FOXB1)*

| | | | |
|---|---|---|---|
| Chr15:60296555 | Chr15:60296561 | Chr15:60296563 | Chr15:60296578 |
| Chr15:60296585 | Chr15:60296598 | Chr15:60296601 | Chr15:60296614 |
| Chr15:60296616 | Chr15:60296619 | Chr15:60296627 | Chr15:60296633 |
| Chr15:60296639 | Chr15:60296643 | Chr15:60296647 | Chr15:60296654 |
| Chr15:60296665 | Chr15:60296668 | Chr15:60296670 | Chr15:60296675 |
| Chr15:60296679 | Chr15:60296684 | Chr15:60296689 | Chr15:60296694 |
| | | | |
| Chr15:60297035 | Chr15:60297050 | Chr15:60297053 | Chr15:60297109 |
| Chr15:60297118 | Chr15:60297121 | Chr15:60297126 | Chr15:60297128 |
| Chr15:60297130 | Chr15:60297152 | Chr15:60297169 | Chr15:60297174 |
| Chr15:60297178 | Chr15:60297185 | Chr15:60297192 | Chr15:60297203 |
| Chr15:60297212 | Chr15:60297221 | Chr15:60297228 | Chr15:60297252 |
| Chr15:60297266 | Chr15:60297273 | Chr15:60297298 | |

*Chr16:86544133-86548070 (FOXF1)*

| | | | |
|---|---|---|---|
| Chr16:86544571 | Chr16:86544587 | Chr16:86544590 | Chr16:86544593 |
| Chr16:86544597 | Chr16:86544599 | Chr16:86544601 | Chr16:86544608 |
| Chr16:86544624 | Chr16:86544652 | Chr16:86544658 | Chr16:86544675 |
| Chr16:86544685 | Chr16:86544699 | Chr16:86544703 | Chr16:86544706 |
| Chr16:86544714 | Chr16:86544720 | Chr16:86544735 | Chr16:86544745 |
| Chr16:86544763 | | | |
| | | | |
| Chr16:86544268 | Chr16:86544273 | Chr16:86544295 | Chr16:86544298 |
| Chr16:86544305 | Chr16:86544308 | Chr16:86544312 | Chr16:86544321 |
| Chr16:86544337 | Chr16:86544339 | Chr16:86544346 | Chr16:86544377 |
| Chr16:86544384 | Chr16:86544391 | Chr16:86544416 | Chr16:86544431 |
| Chr16:86544460 | Chr16:86544464 | Chr16:86544477 | Chr16:86544484 |
| Chr16:86544518 | Chr16:86544523 | Chr16:86544547 | Chr16:86544552 |
| Chr16:86544559 | Chr16:86544571 | | |
| | | | |
| Chr16:86544810 | Chr16:86544832 | Chr16:86544835 | Chr16:86544843 |
| Chr16:86544853 | Chr16:86544859 | Chr16:86544862 | Chr16:86544865 |
| Chr16:86544867 | Chr16:86544870 | Chr16:86544874 | Chr16:86544877 |
| Chr16:86544885 | Chr16:86544892 | Chr16:86544900 | Chr16:86544907 |
| Chr16:86544915 | Chr16:86544928 | Chr16:86544931 | Chr16:86544934 |
| Chr16:86544951 | Chr16:86544955 | Chr16:86544958 | Chr16:86544966 |
| Chr16:86544972 | Chr16:86544975 | Chr16:86544978 | Chr16:86544987 |
| Chr16:86544993 | Chr16:86544996 | Chr16:86545000 | Chr16:86545002 |
| Chr16:86545005 | Chr16:86545015 | Chr16:86545018 | Chr16:86545060 |
| Chr16:86545062 | Chr16:86545078 | Chr16:86545092 | |

*Chr13:28494168-28500451 (PDX1)*

| | | | |
|---|---|---|---|
| Chr13:28502109 | Chr13:28502153 | Chr13:28502161 | Chr13:28502177 |
| Chr13:28502179 | Chr13:28502191 | Chr13:28502198 | Chr13:28502205 |
| Chr13:28502207 | Chr13:28502210 | Chr13:28502245 | Chr13:28502309 |
| | | | |
| Chr13:28502442 | Chr13:28502449 | Chr13:28502461 | Chr13:28502464 |
| Chr13:28502475 | Chr13:28502507 | Chr13:28502512 | Chr13:28502538 |
| Chr13:28502544 | Chr13:28502549 | Chr13:28502559 | Chr13:28502564 |
| Chr13:28502585 | | | |
| | | | |
| Chr13:28503045 | Chr13:28503049 | Chr13:28503081 | Chr13:28503099 |
| Chr13:28503114 | Chr13:28503127 | Chr13:28503138 | Chr13:28503147 |
| Chr13:28503155 | Chr13:28503157 | Chr13:28503179 | |

*Chr7:96649702-96654143 (DLX5)*

| | | | |
|---|---|---|---|
| Chr7:96650062 | Chr7:96650072 | Chr7:96650078 | Chr7:96650096 |
| Chr7:96650099 | Chr7:96650102 | | |
| | | | |
| Chr7:96651485 | Chr7:96651488 | Chr7:96651518 | Chr7:96651523 |
| Chr7:96651532 | Chr7:96651535 | Chr7:96651537 | Chr7:96651542 |
| Chr7:96651550 | Chr7:96651586 | | |
| | | | |
| Chr7:96653596 | Chr7:96653605 | Chr7:96653607 | Chr7:96653617 |
| Chr7:96653620 | Chr7:96653623 | Chr7:96653644 | Chr7:96653656 |
| Chr7:96653683 | Chr7:96653686 | Chr7:96653692 | Chr7:96653698 |
| Chr7:96653701 | | | |

*Chr21:28208606-28217728 (ADAMTS1)*

| | | | |
|---|---|---|---|
| Chr21:28217973 | Chr21:28218002 | Chr21:28218015 | Chr21:28218018 |
| Chr21:28218047 | Chr21:28218051 | Chr21:28218057 | Chr21:28218072 |
| Chr21:28218074 | Chr21:28218084 | Chr21:28218105 | |
| | | | |
| Chr21:28218514 | Chr21:28218516 | Chr21:28218550 | Chr21:28218568 |
| Chr21:28218579 | Chr21:28218586 | Chr21:28218596 | Chr21:28218635 |
| Chr21:28218638 | Chr21:28218646 | Chr21:28218671 | Chr21:28218684 |
| Chr21:28218688 | Chr21:28218704 | Chr21:28218729 | Chr21:28218741 |
| | | | |
| Chr21:28218893 | Chr21:28218906 | Chr21:28218914 | Chr21:28218916 |
| Chr21:28218928 | Chr21:28218934 | Chr21:28218938 | Chr21:28218949 |
| Chr21:28218953 | Chr21:28218959 | Chr21:28218974 | Chr21:28218976 |
| Chr21:28218978 | Chr21:28218984 | Chr21:28218986 | Chr21:28218996 |
| Chr21:28219008 | Chr21:28219016 | | |

*Chr13:110959631-111165373 (COL4A2)*

| | | | |
|---|---|---|---|
| Chr13:110960813 | Chr13:110960827 | Chr13:110960849 | Chr13:110960875 |
| Chr13:110960925 | Chr13:110960930 | Chr13:110960938 | Chr13:110960978 |
| Chr13:110961001 | Chr13:110961003 | Chr13:110961025 | Chr13:110961045 |
| Chr13:110961049 | Chr13:110961069 | Chr13:110961072 | Chr13:110961088 |
| Chr13:110961091 | Chr13:110961095 | Chr13:110961110 | Chr13:110961116 |
| Chr13:110961131 | Chr13:110960786 | | |
| | | | |
| Chr13:110961358 | Chr13:110961386 | Chr13:110961404 | Chr13:110961406 |
| Chr13:110961445 | Chr13:110961579 | Chr13:110961594 | Chr13:110961606 |
| Chr13:110961631 | | | |
| | | | |
| Chr13:110959935 | Chr13:110959965 | Chr13:110959968 | Chr13:110959974 |
| Chr13:110959979 | Chr13:110959981 | Chr13:110959986 | Chr13:110959988 |
| Chr13:110959997 | Chr13:110959999 | Chr13:110960004 | Chr13:110960006 |
| Chr13:110960011 | Chr13:110960027 | Chr13:110960035 | Chr13:110960046 |
| Chr13:110960051 | Chr13:110960071 | Chr13:110960099 | Chr13:110960108 |
| Chr13:110960115 | Chr13:110960156 | Chr13:110960180 | |

*Chr5:83238126-83680611 (EDIL3)*

| | | | |
|---|---|---|---|
| Chr5:83679825 | Chr5:83679827 | Chr5:83679836 | Chr5:83679843 |
| Chr5:83679846 | Chr5:83679860 | Chr5:83679864 | Chr5:83679883 |
| Chr5:83679894 | Chr5:83679900 | Chr5:83679906 | Chr5:83679910 |
| Chr5:83679937 | Chr5:83679953 | Chr5:83679957 | Chr5:83679961 |
| Chr5:83679976 | Chr5:83679982 | Chr5:83679987 | Chr5:83680003 |
| Chr5:83680007 | Chr5:83680011 | Chr5:83680026 | Chr5:83680032 |
| Chr5:83680086 | Chr5:83680090 | Chr5:83680103 | Chr5:83680106 |
| Chr5:83680122 | Chr5:83680126 | | |
| | | | |
| Chr5:83679546 | Chr5:83679568 | Chr5:83679571 | Chr5:83679585 |
| Chr5:83679602 | Chr5:83679617 | Chr5:83679630 | Chr5:83679640 |
| Chr5:83679667 | Chr5:83679675 | Chr5:83679689 | Chr5:83679693 |
| Chr5:83679699 | Chr5:83679701 | Chr5:83679733 | |
| | | | |
| Chr5:83679973 | Chr5:83679989 | Chr5:83679992 | Chr5:83680001 |
| Chr5:83680003 | Chr5:83680006 | Chr5:83680012 | Chr5:83680015 |
| Chr5:83680019 | Chr5:83680033 | Chr5:83680036 | Chr5:83680045 |
| Chr5:83680051 | Chr5:83680061 | Chr5:83680063 | Chr5:83680066 |
| Chr5:83680108 | Chr5:83680115 | Chr5:83680118 | Chr5:83680132 |
| Chr5:83680147 | Chr5:83680159 | Chr5:83680174 | Chr5:83680181 |
| Chr5:83680185 | Chr5:83680198 | Chr5:83680205 | Chr5:83680209 |
| Chr5:83680217 | Chr5:83680232 | Chr5:83680235 | Chr5:83680237 |
| Chr5:83680253 | | | |
| | | | |
| Chr5:83680108 | Chr5:83680115 | Chr5:83680118 | Chr5:83680132 |
| Chr5:83680147 | Chr5:83680159 | Chr5:83680174 | Chr5:83680181 |
| Chr5:83680185 | Chr5:83680198 | Chr5:83680205 | Chr5:83680209 |
| Chr5:83680217 | Chr5:83680232 | Chr5:83680235 | Chr5:83680237 |
| Chr5:83680253 | Chr5:83680264 | Chr5:83680284 | Chr5:83680310 |
| Chr5:83680326 | Chr5:83680355 | | |
| Chr5:83680401 | Chr5:83680406 | Chr5:83680409 | Chr5:83680411 |
| Chr5:83680413 | Chr5:83680430 | Chr5:83680438 | Chr5:83680441 |
| Chr5:83680444 | Chr5:83680458 | Chr5:83680463 | Chr5:83680474 |
| Chr5:83680483 | Chr5:83680485 | Chr5:83680488 | Chr5:83680492 |
| Chr5:83680497 | Chr5:83680499 | Chr5:83680507 | Chr5:83680509 |
| Chr5:83680513 | Chr5:83680518 | Chr5:83680529 | Chr5:83680531 |
| Chr5:83680548 | Chr5:83680559 | Chr5:83680564 | Chr5:83680568 |
| Chr5:83680572 | Chr5:83680579 | Chr5:83680589 | Chr5:83680591 |
| Chr5:83680593 | Chr5:83680596 | Chr5:83680602 | |

*Chr15:48700503-48937985 (FBN1)*

| | | | |
|---|---|---|---|
| Chr15:48938149 | Chr15:48938163 | Chr15:48938172 | Chr15:48938181 |
| Chr15:48938183 | Chr15:48938218 | Chr15:48938236 | Chr15:48938238 |
| Chr15:48938254 | Chr15:48938267 | Chr15:48938273 | Chr15:48938280 |
| Chr15:48938291 | Chr15:48938349 | | |

*Chr1:47901689-47906363 (FOXD2)*

| | | | |
|---|---|---|---|
| Chr1:47899102 | Chr1:47899126 | Chr1:47899129 | Chr1:47899143 |
| Chr1:47899152 | Chr1:47899168 | Chr1:47899184 | Chr1:47899205 |
| Chr1:47899212 | Chr1:47899230 | Chr1:47899232 | Chr1:47899253 |
| Chr1:47899286 | Chr1:47899327 | | |
| | | | |
| Chr1:47909988 | Chr1:47909995 | Chr1:47910005 | Chr1:47910036 |
| Chr1:47910051 | Chr1:47910056 | Chr1:47910062 | Chr1:47910074 |
| Chr1:47910103 | Chr1:47910122 | Chr1:47910137 | Chr1:47910142 |
| Chr1:47910144 | Chr1:47910146 | | |

*Chr2:66662532-66799891 (MEIS1)*

| | | | |
|---|---|---|---|
| Chr2:66662043 | Chr2:66662047 | Chr2:66662049 | Chr2:66662063 |
| Chr2:66662073 | Chr2:66662107 | Chr2:66662111 | Chr2:66662124 |
| Chr2:66662145 | Chr2:66662156 | Chr2:66662161 | Chr2:66662163 |
| Chr2:66662178 | Chr2:66662218 | | |
| | | | |
| Chr2:66662178 | Chr2:66662218 | Chr2:66662220 | Chr2:66662246 |
| Chr2:66662265 | Chr2:66662280 | Chr2:66662306 | Chr2:66662310 |
| Chr2:66662323 | Chr2:66662333 | Chr2:66662337 | |

*Chr16:55513081-55540586 (MMP2)*

| | | | |
|---|---|---|---|
| Chr16:55512695 | Chr16:55512711 | Chr16:55512732 | Chr16:55512750 |
| Chr16:55512753 | Chr16:55512793 | | |
| | | | |
| Chr16:55513961 | Chr16:55513997 | Chr16:55514025 | Chr16:55514059 |
| Chr16:55514061 | Chr16:55514092 | Chr16:55514120 | Chr16:55514131 |
| Chr16:55514158 | Chr16:55514205 | | |

*Chr7:24323807-24331484 (NPY)*

| | | | |
|---|---|---|---|
| Chr7:24323767 | Chr7:24323792 | Chr7:24323794 | Chr7:24323799 |
| Chr7:24323817 | Chr7:24323834 | Chr7:24323840 | Chr7:24323844 |
| Chr7:24323848 | Chr7:24323866 | Chr7:24323876 | Chr7:24323880 |
| Chr7:24323882 | Chr7:24323884 | Chr7:24323894 | Chr7:24323905 |
| Chr7:24323910 | Chr7:24323930 | Chr7:24323934 | |
| | | | |
| Chr7:24324180 | Chr7:24324186 | Chr7:24324206 | Chr7:24324217 |
| Chr7:24324222 | Chr7:24324225 | Chr7:24324244 | Chr7:24324249 |
| Chr7:24324251 | Chr7:24324268 | Chr7:24324274 | Chr7:24324280 |
| Chr7:24324311 | Chr7:24324313 | | |
| | | | |
| Chr7:24324549 | Chr7:24324570 | Chr7:24324592 | Chr7:24324595 |
| Chr7:24324597 | Chr7:24324599 | Chr7:24324621 | Chr7:24324625 |
| Chr7:24324633 | Chr7:24324635 | Chr7:24324668 | Chr7:24324672 |
| Chr7:24324676 | Chr7:24324679 | Chr7:24324683 | Chr7:24324690 |

*Chr19:38741877-38747172 (PPP1R14A)*

| | | | |
|---|---|---|---|
| Chr19:38747280 | Chr19:38747296 | Chr19:38747300 | Chr19:38747313 |
| Chr19:38747319 | Chr19:38747322 | Chr19:38747328 | Chr19:38747334 |
| Chr19:38747355 | Chr19:38747361 | Chr19:38747371 | Chr19:38747382 |
| Chr19:38747389 | Chr19:38747410 | | |
| | | | |
| Chr19:38746654 | Chr19:38746680 | Chr19:38746690 | Chr19:38746701 |
| Chr19:38746715 | Chr19:38746726 | Chr19:38746728 | Chr19:38746732 |
| Chr19:38746749 | Chr19:38746759 | Chr19:38746770 | Chr19:38746784 |
| Chr19:38746786 | Chr19:38746804 | Chr19:38746808 | Chr19:38746811 |
| Chr19:38746826 | Chr19:38746834 | Chr19:38746837 | Chr19:38746849 |
| Chr19:38746852 | Chr19:38746865 | Chr19:38746871 | Chr19:38746873 |
| Chr19:38746875 | Chr19:38746877 | Chr19:38746882 | Chr19:38746898 |

or a corresponding cytosine at position n+1 on the opposite DNA strand, in a biological sample from said individual wherein a higher level of methylation of one or more of said residues relative to the methylation level of a corresponding residue in a control sample is indicative of a large intestine neoplasm or a predisposition to the onset of a neoplastic state.

In yet another aspect the present invention is directed to a method of screening for the onset or predisposition to the onset of or monitoring a large intestine neoplasm in an individual, said method comprising assessing the level of expression of a DNA region selected from:
(i) the region defined by any one or more of Hg19 coordinates and 2kb upstream of the transcription start site:

| | |
|---|---|
| (1) chr12:52400748..52409671 | (34) chr10:101292690..101296281 |
| (2) chr5:3596168..3601517 | (35) chr4:4190530..4228621 |
| (3) chr13:95361876..95364389 | (36) chr12:54943404..54973023 |
| (4) chr4:81187742..81212171 | (37) chr5:176047210..176057557 |
| (5) chr19:57019212..57040270 | (38) chr12:22346325..22487648 |
| (6) chr3:33191537..33260707 | (39) chr19:56894648..56904889 |
| (7) chr15:60296421..60298142 | (40) chr20:21491648..21494664 |
| (8) chr13:28494168..28500451 | (41) chr1:50883225..50889141 |
| (9) chr7:96649702..96654143, | (42) chr7:27180996..27183287 |
| (10) chr8:140,811,770-141,537,860 | (43) chr11:2016406..2019065 |
| (11) chr5:2746279..2751769 | (44) chr14:57267425..57277184 |
| (12) chr18:55102917..55158530 | (45) chr4:126237567..126414087 |
| (13) chr20:37353101..37358016 | (46) chr8:23559964..23563922 |
| (14) chr8:2792875..4852328 | (47) chr10:131633547..131762091 |
| (15) chr16:66613351..66622178 | (48) chr4:62362839..62938168 |
| (16) chr5:37815753..37839782 | (49) chr1:47901689..47906363 |
| (17) chr1:63788730..63790797 | (50) chr17:77768176..77770890 |
| (18) chr15:37156644..37178734 | (51) chr17:93598762..93604831 |
| (19) chr7:27139973..27142394 | (52) chr1:33789224..33841194 |
| (20) chr20:21686297..21696620 | (53) chr9:124,004,679-124,030,840 |
| (21) chr16:51169886..51185183 | (54) chr4:158141736..158287227 |
| (22) chr12:85253267..85306606 | (55) chr12:9445136..9462559 |
| (23) chr8:6357172..6420784 | (56) chr12:24964278..25102308 |
| (24) chr14:85996488..86094270 | (57) chrX:21542357..21690352 |
| (25) chr2:182541194..182545381 | (58) chr20:52769988..52790516 |
| (26) chr7:30951468..30965131 | (59) chr3:172162951..172166203 |
| (27) chr8:131792547..132052835 | (60) chr13:28366780..28368089 |
| (28) chr3:128749292..128759583 | (61) chr7:50344378...50472799 |
| (29) chr10:101088856..101154087 | (62) chr7:149412148..149431664 |
| (30) chr7:27282164..27286192 | (63) chr7:24323809..24331477 |
| (31) chr10:129535538..129539450 | (64) chr4:30722037..31148421 |
| (32) chr19:49316274..49339934 | (65) chr10:47083534..47088320 |
| (33) chr6:391752..411443 | |

(ii) the gene region, including 2kb upstream of any one or more of:

| | | | |
|---|---|---|---|
| (1) GRASP | (18) ANGPT2 | (35) NKX2-6 | (52) HOXA5 |
| (2) IRX1 | (19) LHX6 | (36) PAX1 | (53) GDNF |
| (3) SOX21 | (20) NEUROD1 | (37) FOXD2 | (54) FAT4 |
| (4) FGF5 | (21) AC149644.1 | (38) SLC6A15 | (55) HOXA2 |
| (5) ZNF471 | (22) CCDC48 | (39) PHC2 | (56) LPHN3 |
| (6) SUSD5 | (23) EVX1 | (40) FLRT2 | (57) ADCYAP1 |
| (7) FOXB1 | (24) GHSR | (41) GATA2 | (58) GRIA2 |
| (8) PDX1 | (25) HSD17B14 | (42) ADCY8 | (59) AQP1 |
| (9) DLX5 | (26) KRBA1 | (43) CNNM1 | (60) BCAT1 |
| (10) ONECUT2 | (27) OTOP1 | (44) IKZF1 | (61) CYP24A1 |
| (11) DMRTA2 | (28) PPYR1 | (45) NKX2-3 | (62) FOXI2 |
| (12) CMTM2 | (29) SRMS | (46) PCDH7 | (63) GSX1 |
| (13) OTX2 | (30) ZNF582 | (47) SNCB | (64) IRF4 |
| (14) LOC145845 | (31) IRX2 | (48) ST8SIA1 | (65) NPY |
| (15) EBF3 | (32) CSMD1 | (49) TRAPPC9 | (66) PDE1B |
| (16) SALL1 | (33) MIR675,H19 | (50) NKX2-2 | |
| (17) CBX8 | (34) FOXD3 | (51) SLC32A1 | |

in a biological sample from said individual wherein a lower level of expression of the DNA regions of group (i) and/or (ii) relative to control levels is indicative of a large intestine neoplasm or a predisposition to the onset of a neoplastic state.

A related aspect of the present invention provides a molecular array, which array comprises a plurality of:
(i) nucleic acid molecules comprising a nucleotide sequence corresponding to any one or more of the neoplastic marker DNA hereinbefore described or a sequence exhibiting at least 80% identity thereto or a functional derivative, fragment, variant or homologue of said nucleic acid molecule; or
(ii) nucleic acid molecules comprising a nucleotide sequence capable of hybridising to any one or more of the sequences of (i) under medium stringency conditions or a functional derivative, fragment, variant or homologue of said nucleic acid molecule; or
(iii) nucleic acid probes or oligonucleotides comprising a nucleotide sequence capable of hybridising to any one or more of the sequences of (i) under medium stringency conditions or a functional derivative, fragment, variant or homologue of said nucleic acid molecule; or
(iv) probes capable of binding to any one or more of the proteins encoded by the nucleic acid molecules of (i) or a derivative, fragment or, homologue thereof
wherein the level of expression of said marker genes of (i) or proteins of (iv) is indicative of the neoplastic state of a cell or cellular subpopulation derived from the large intestine.

### BRIEF DESCRIPTION OF THE DRAWINGS

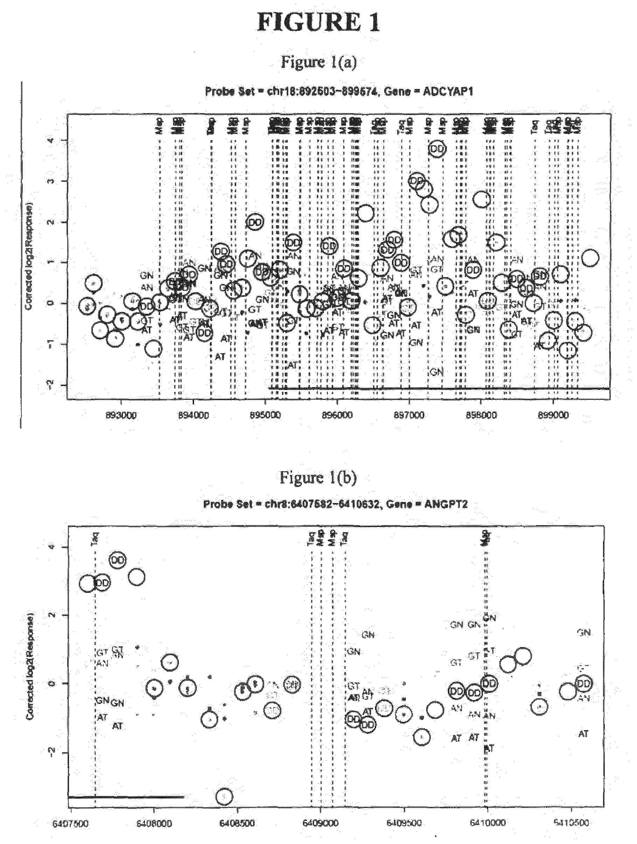

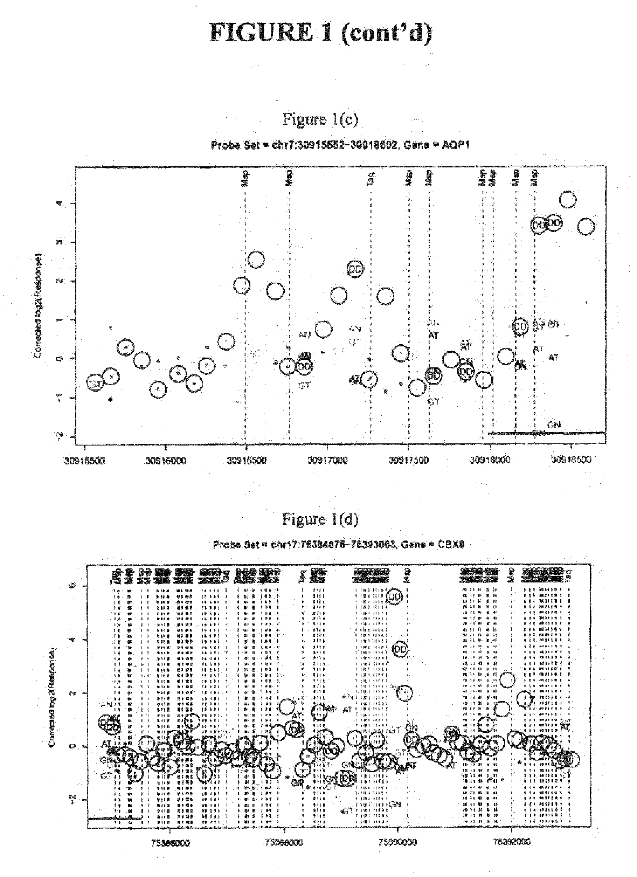

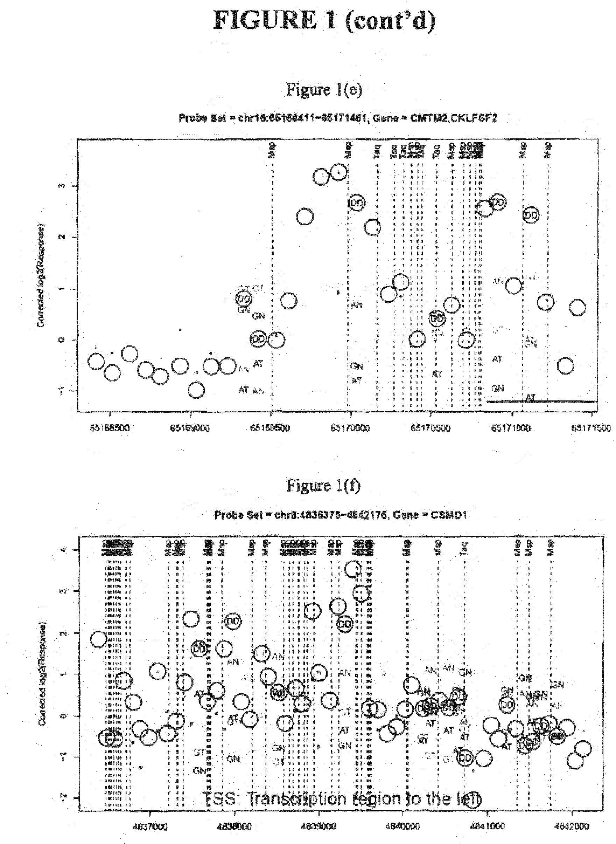

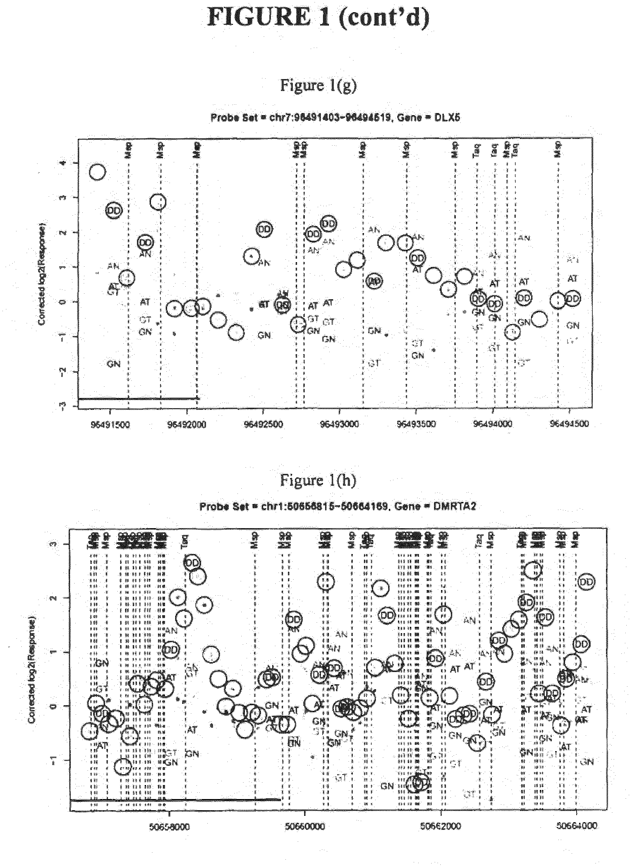

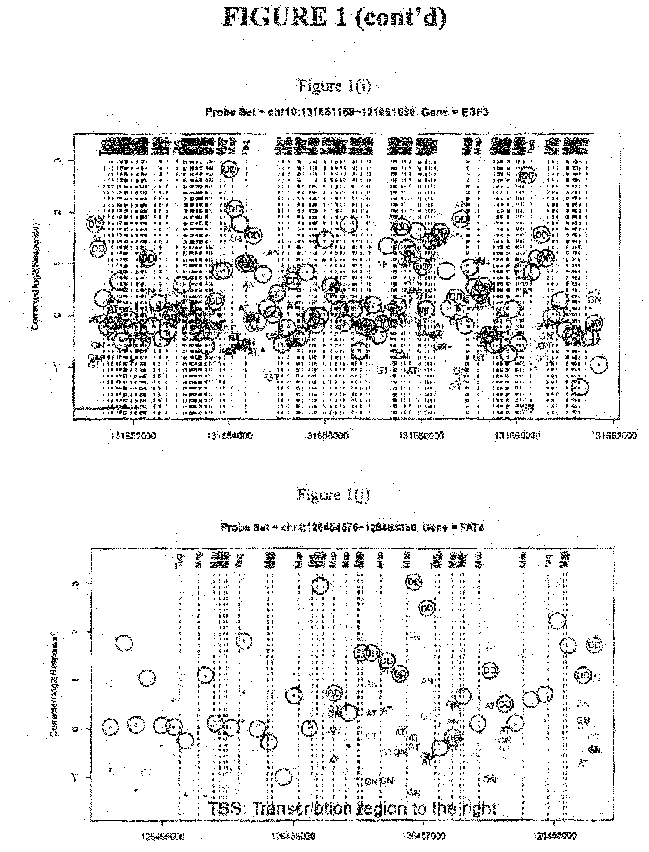

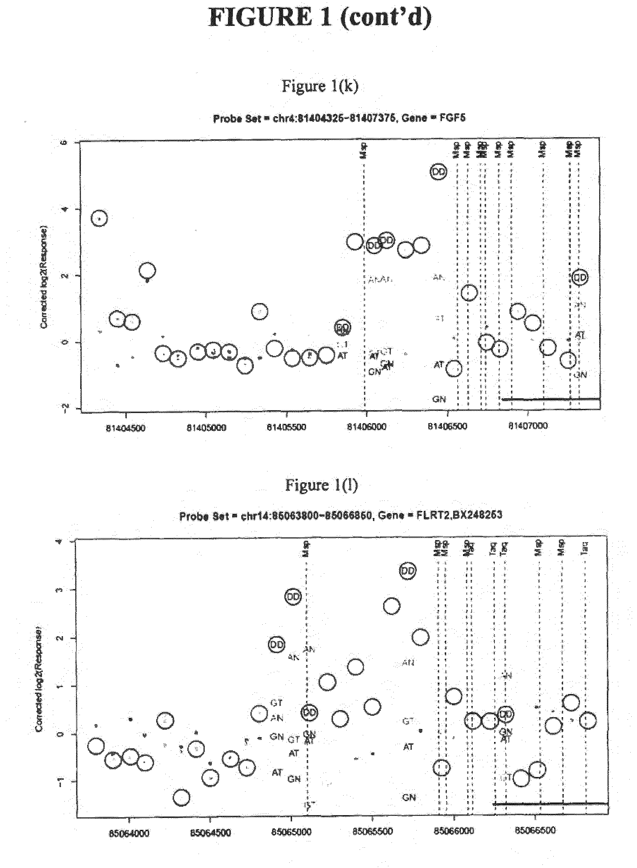

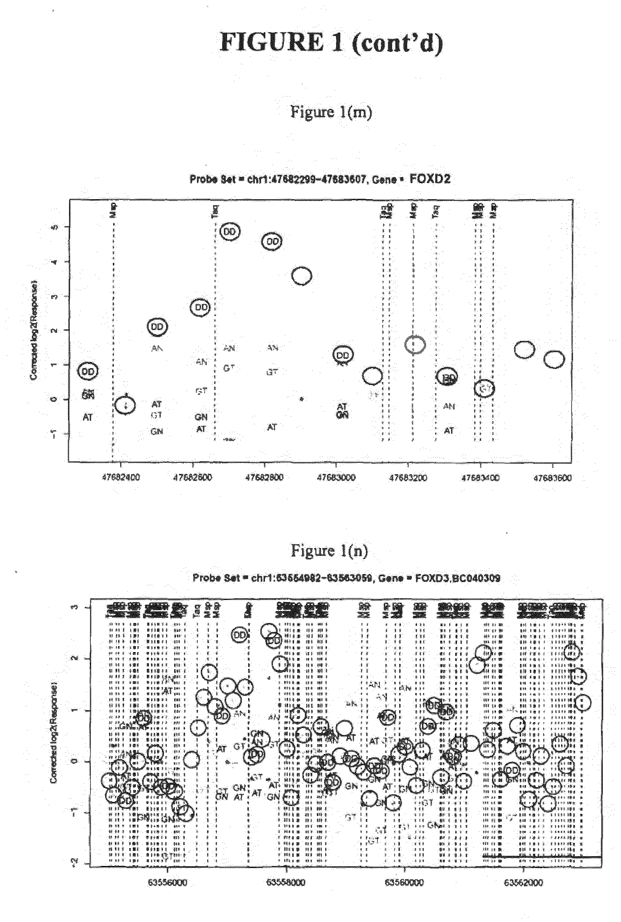

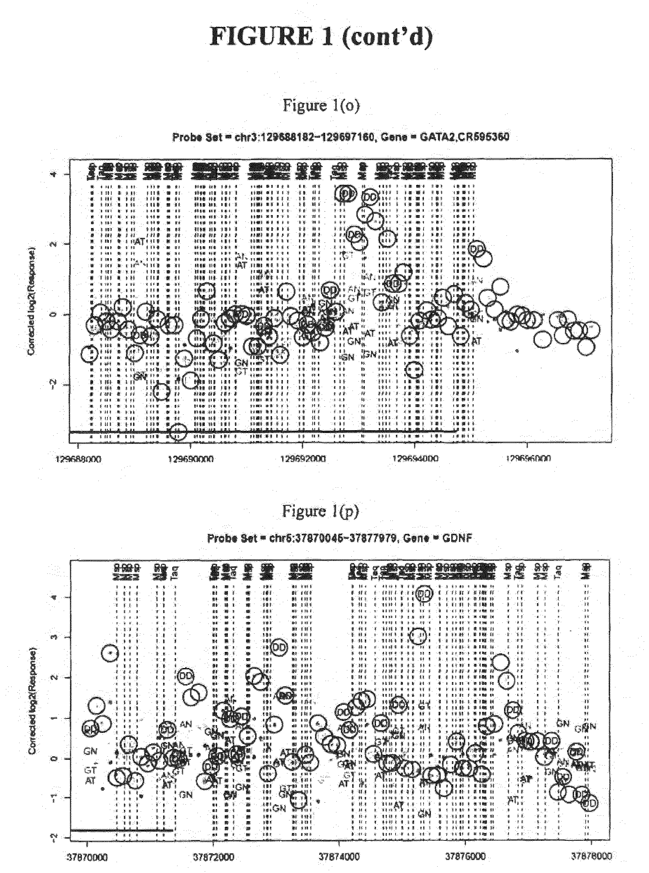

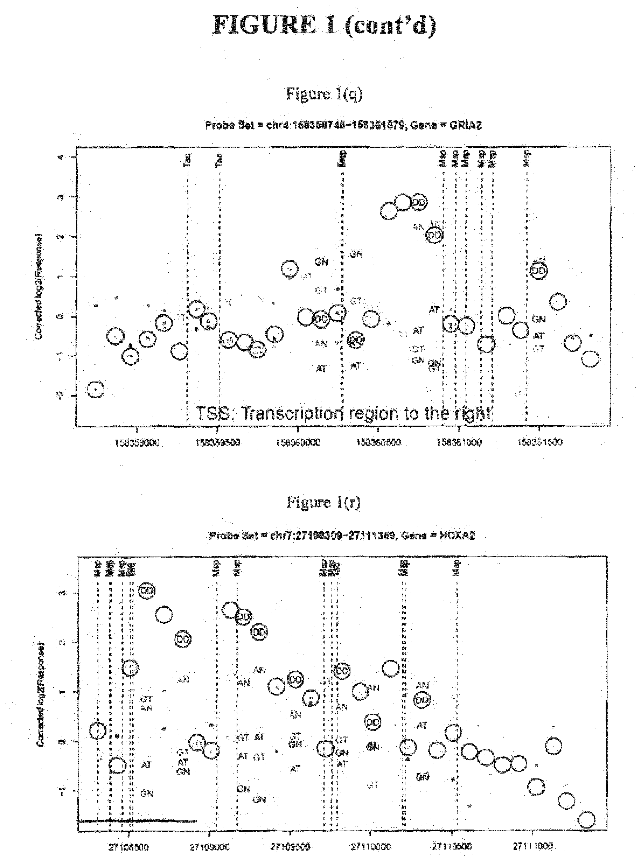

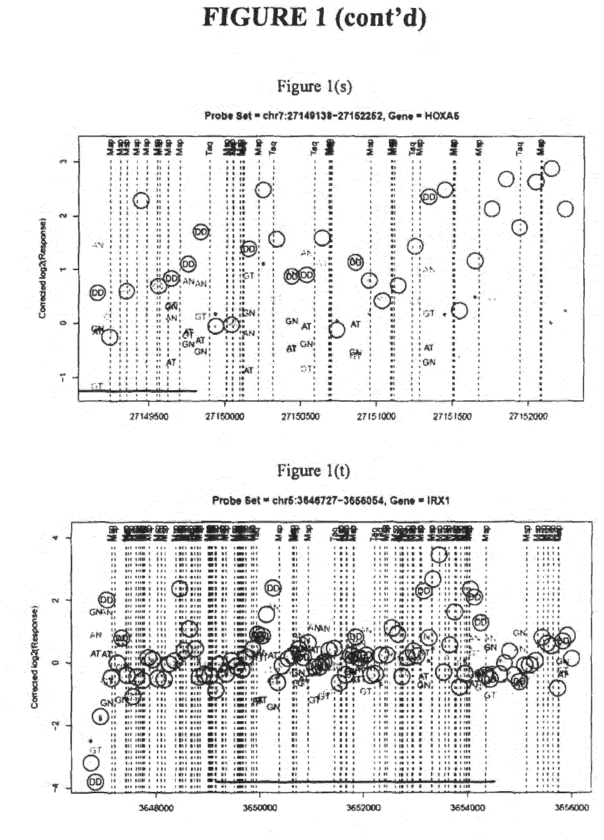

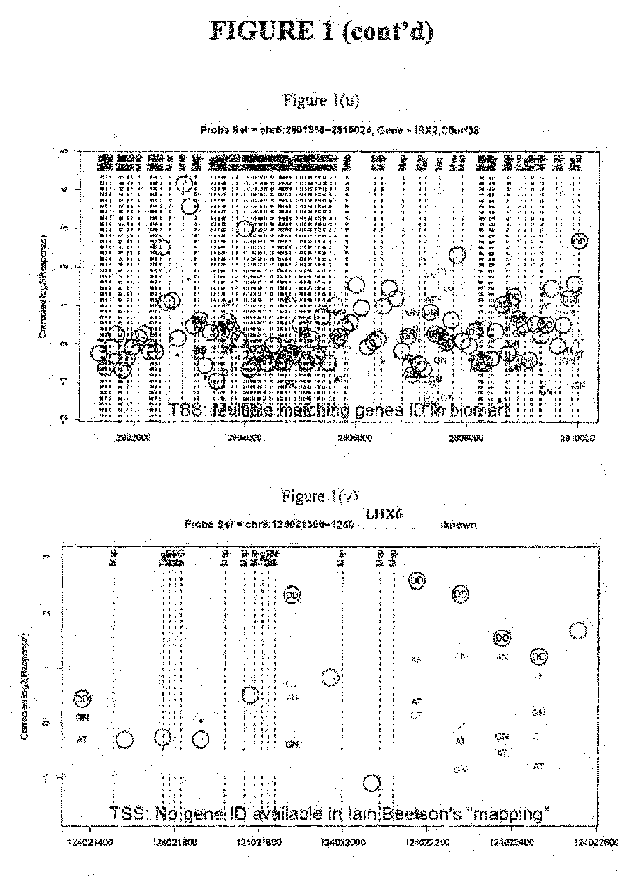

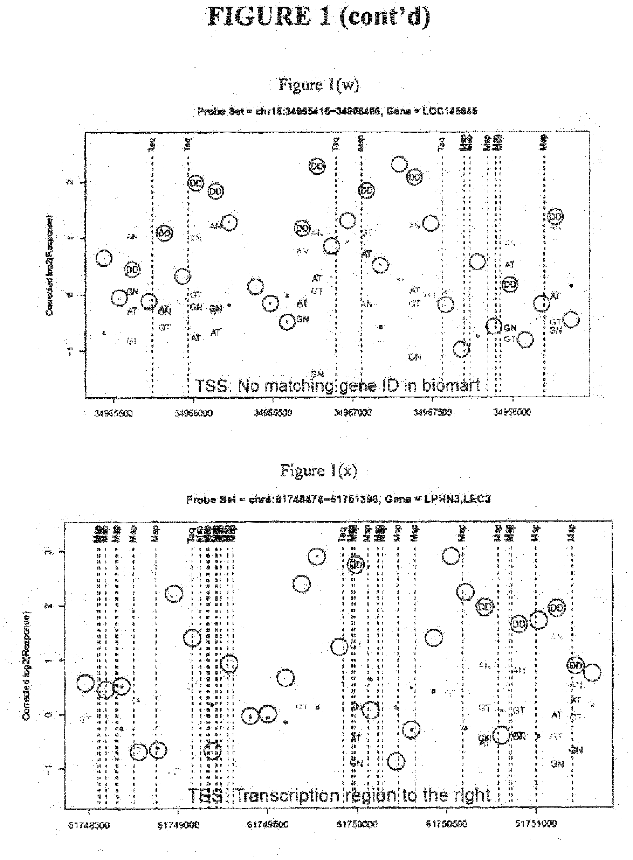

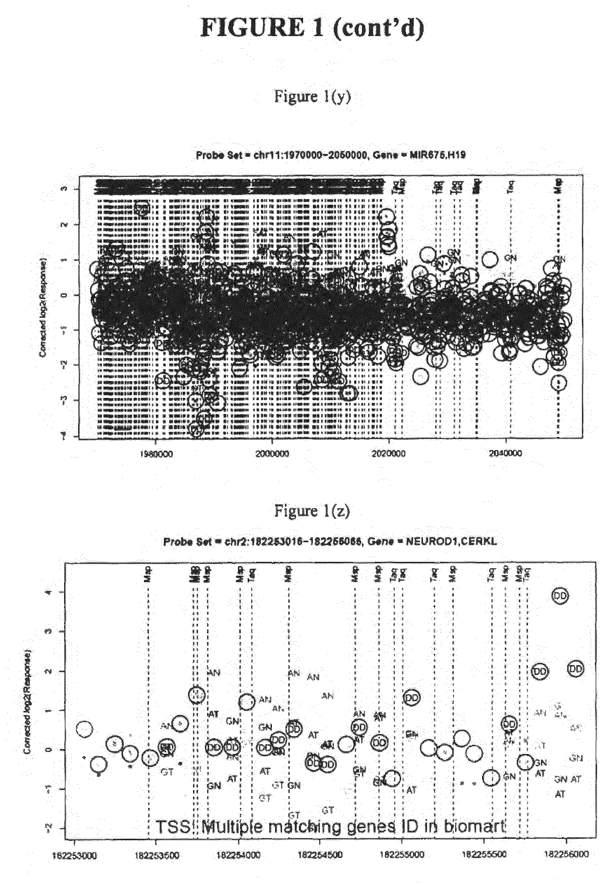

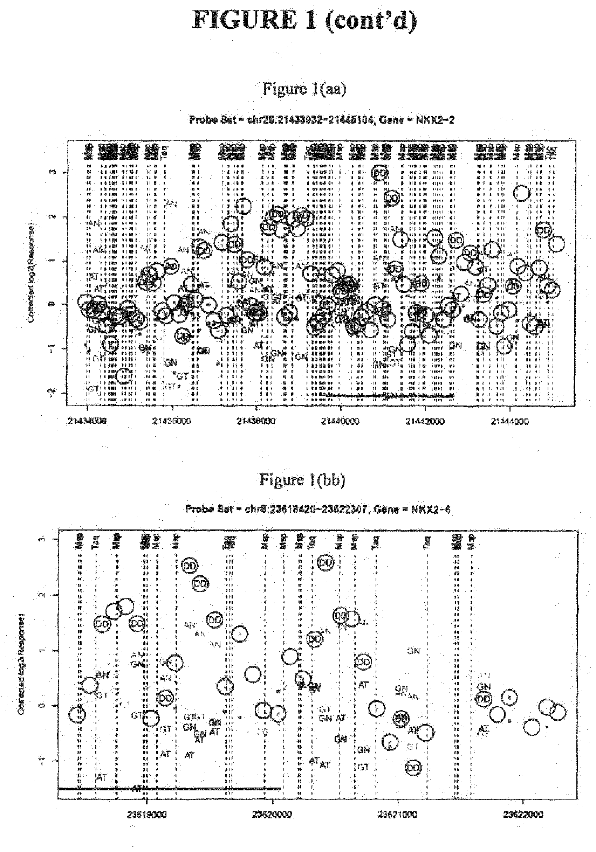

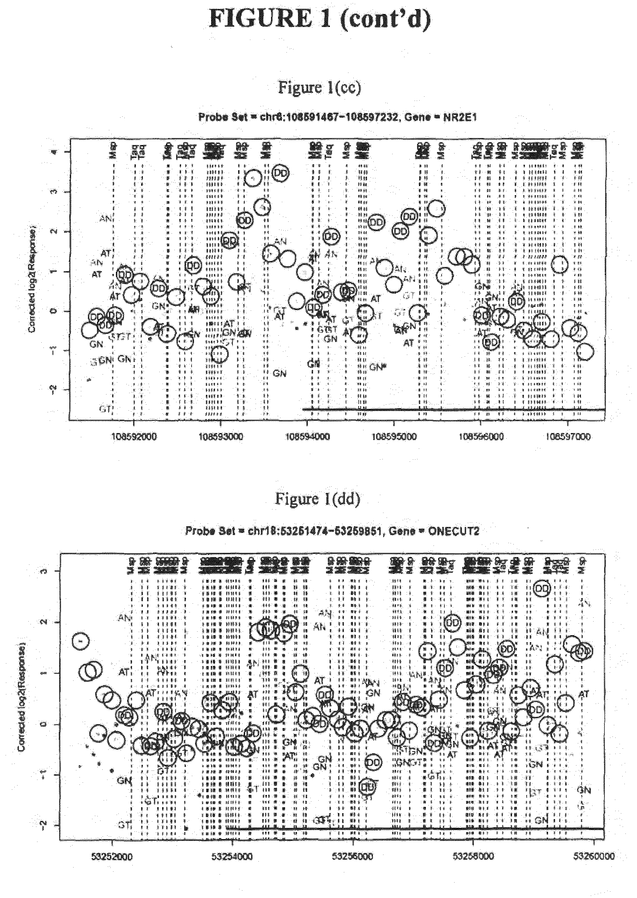

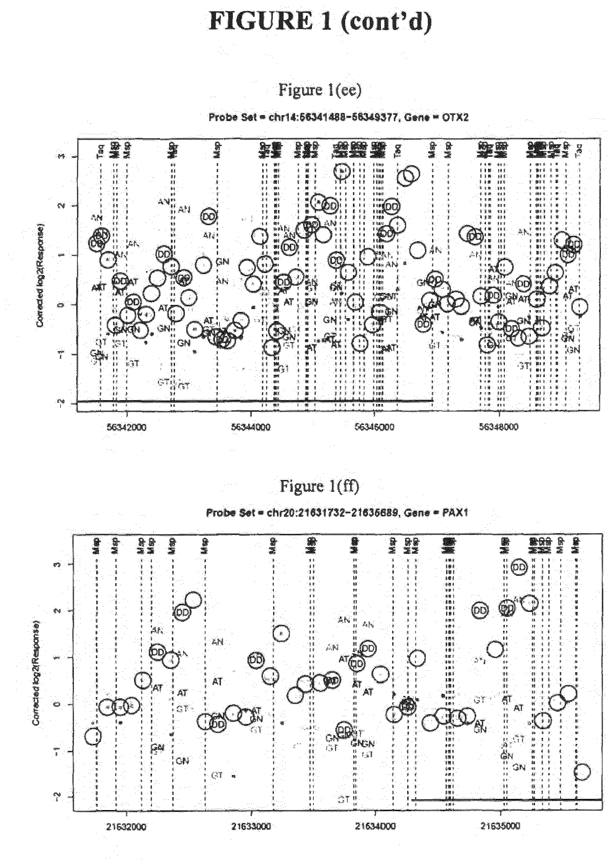

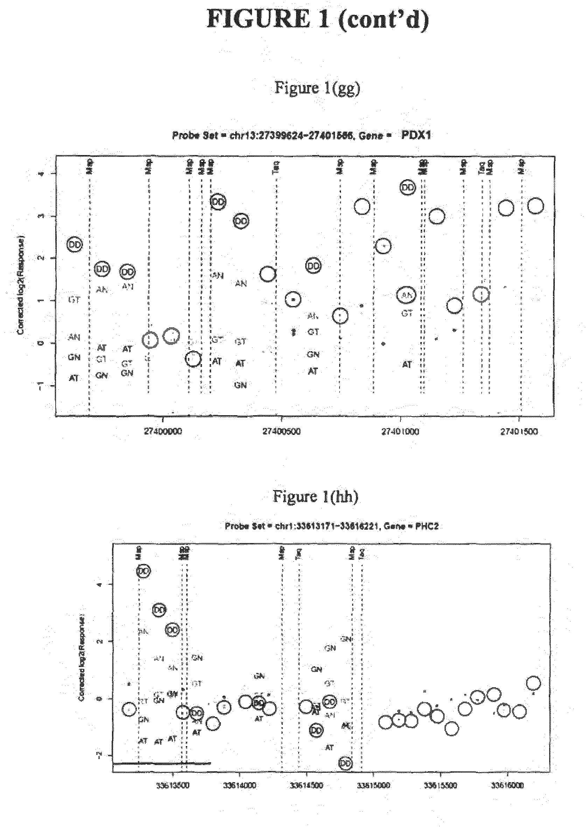

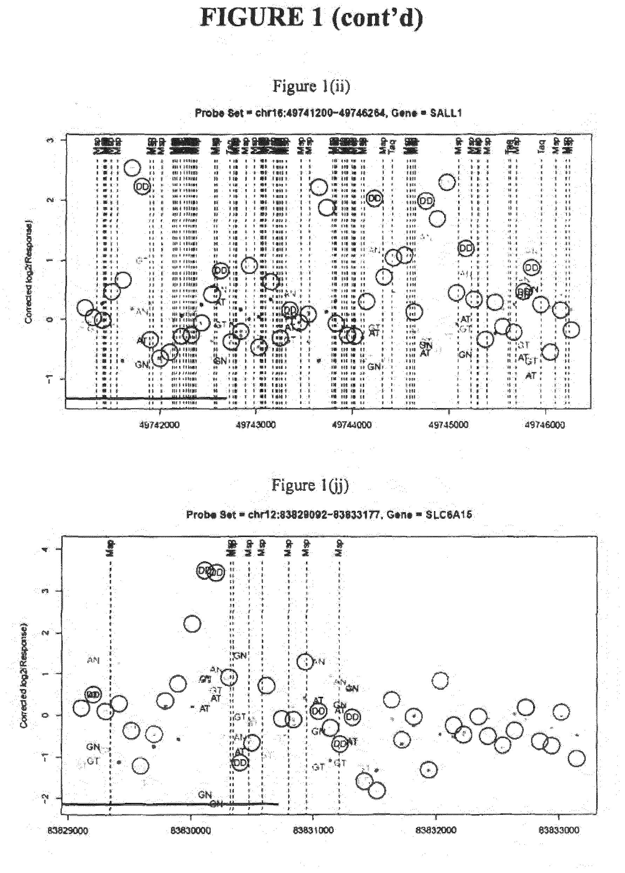

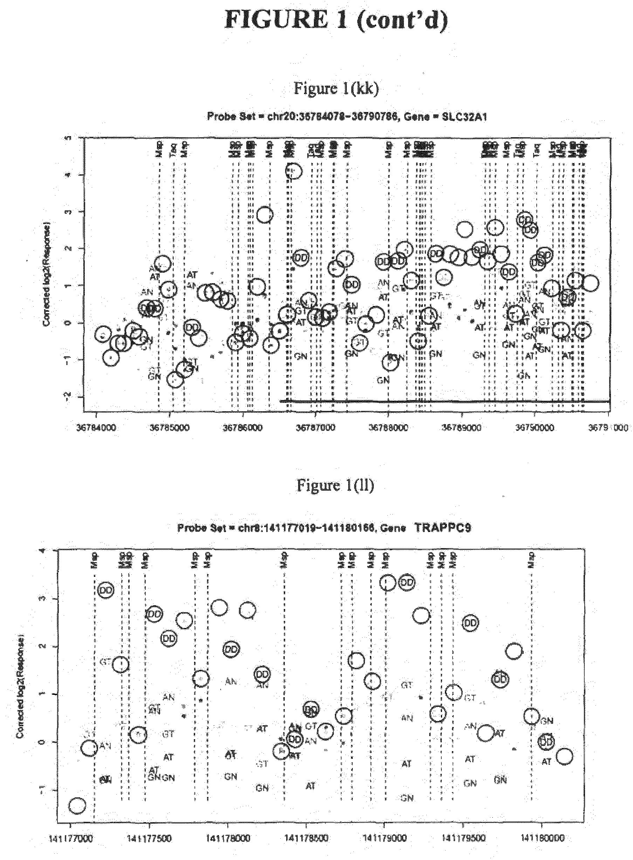

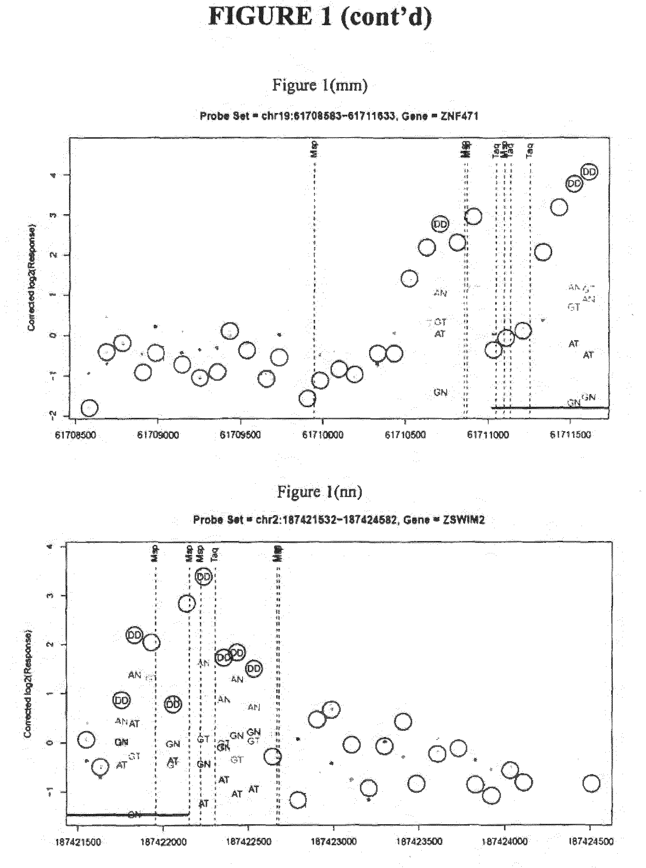

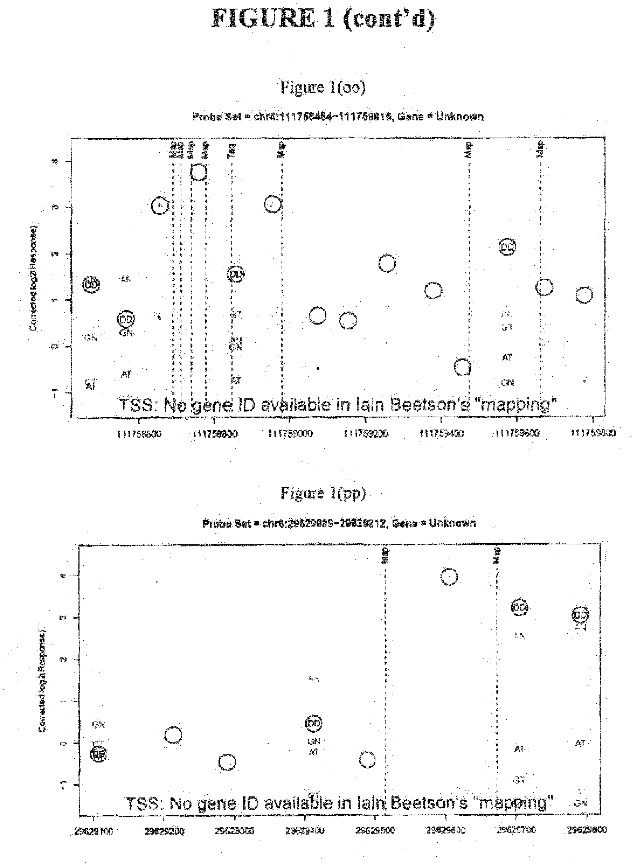

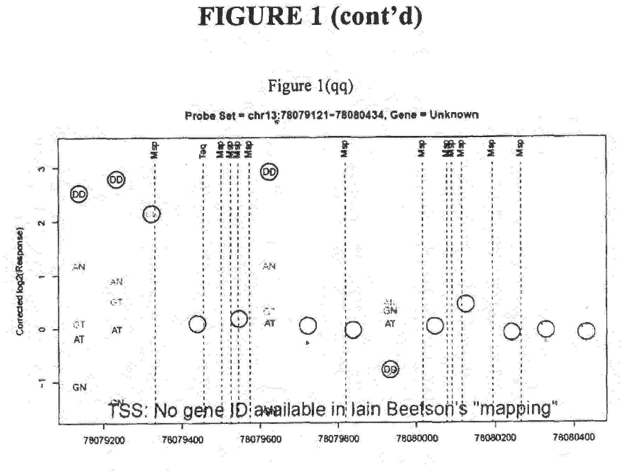

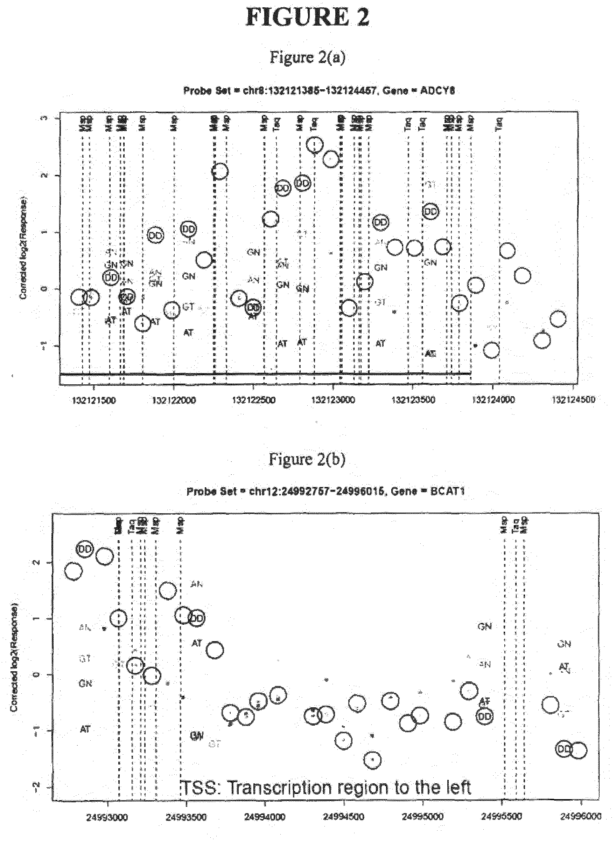

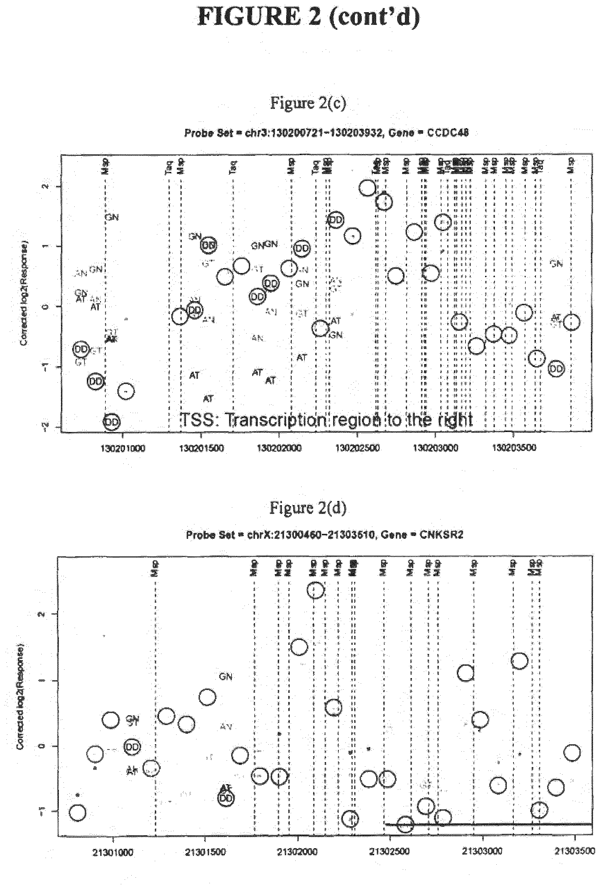

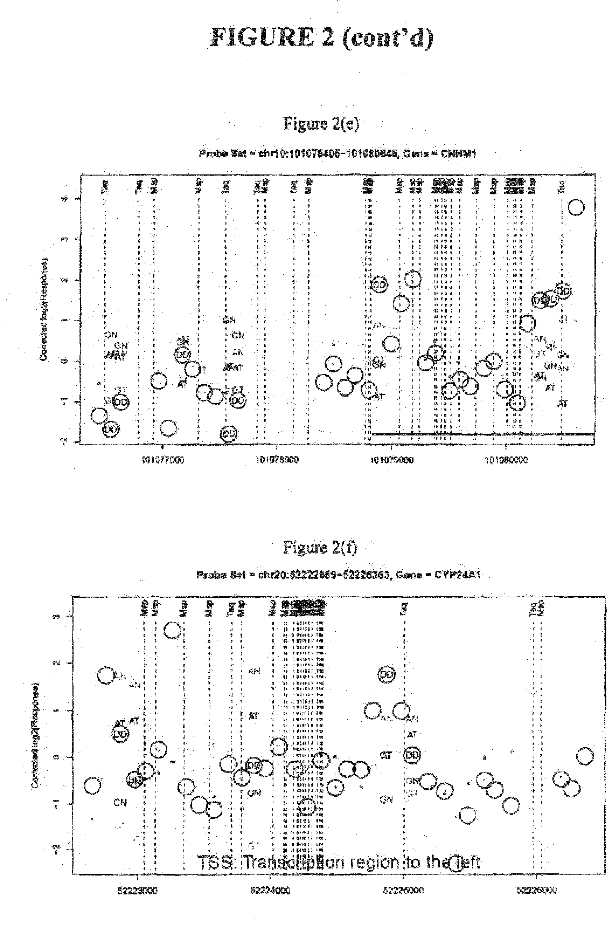

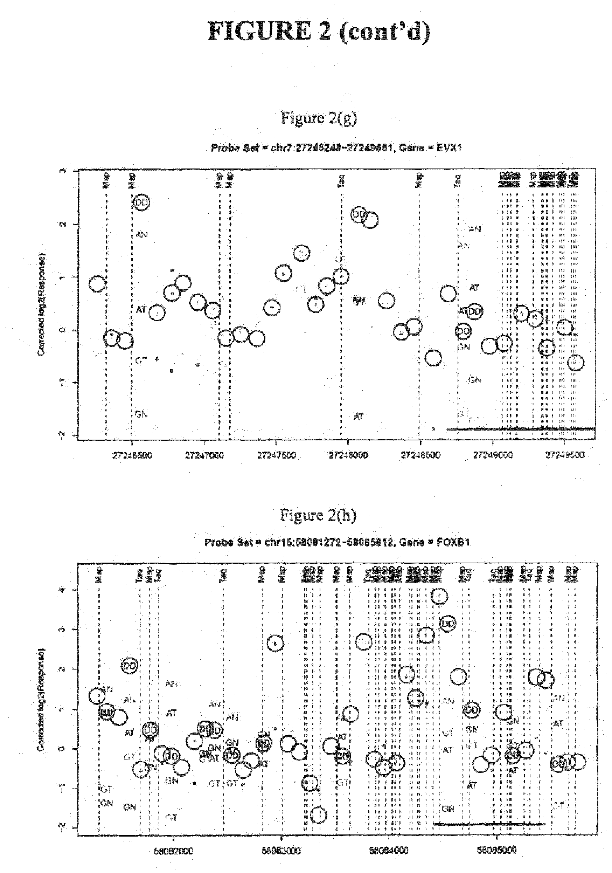

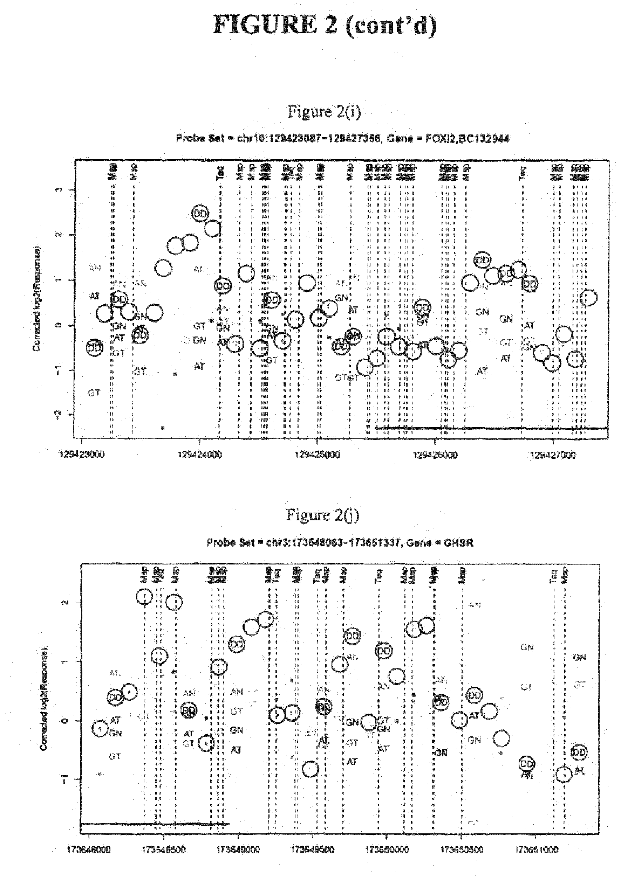

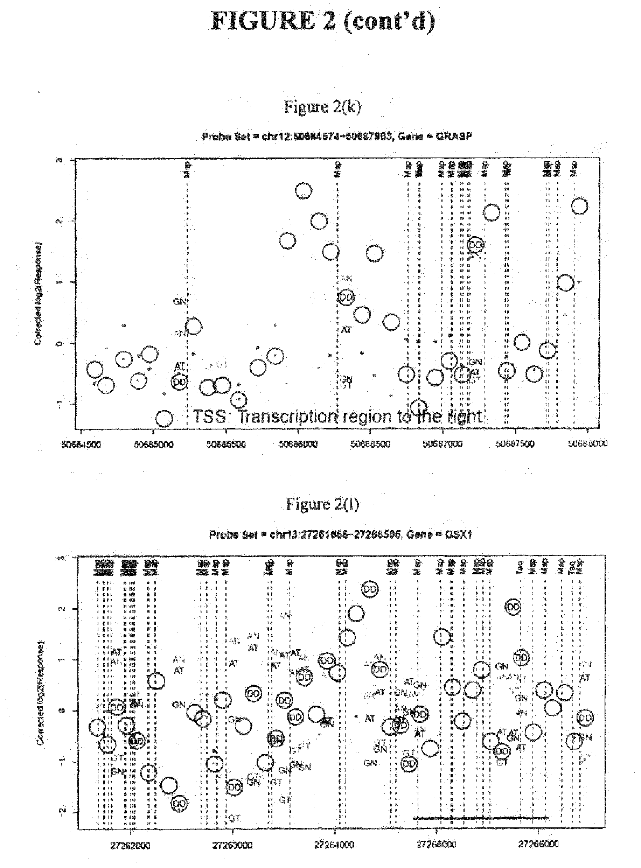

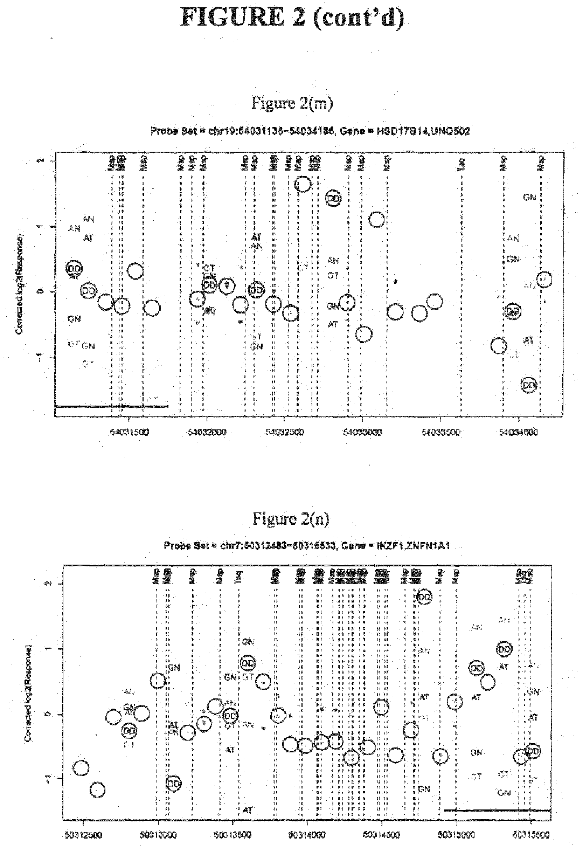

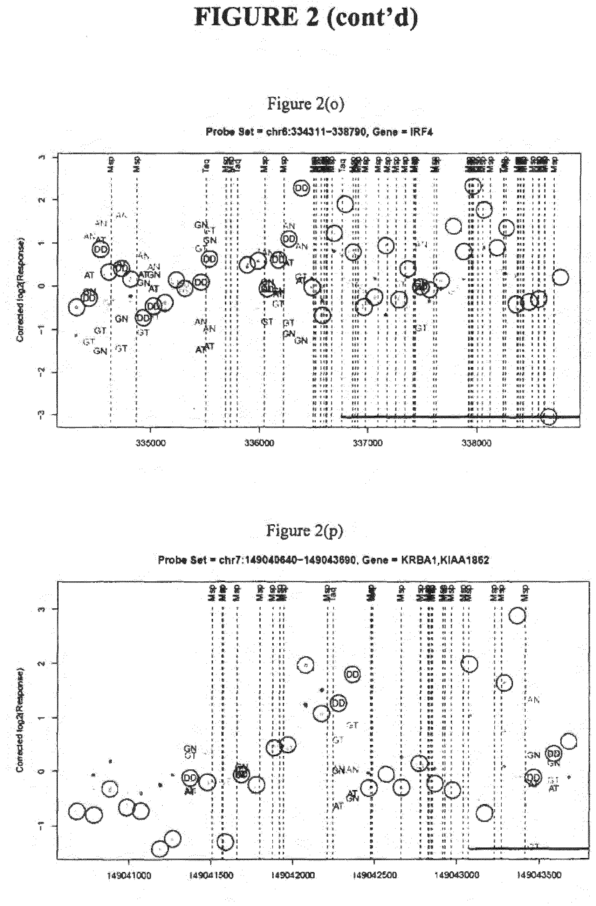

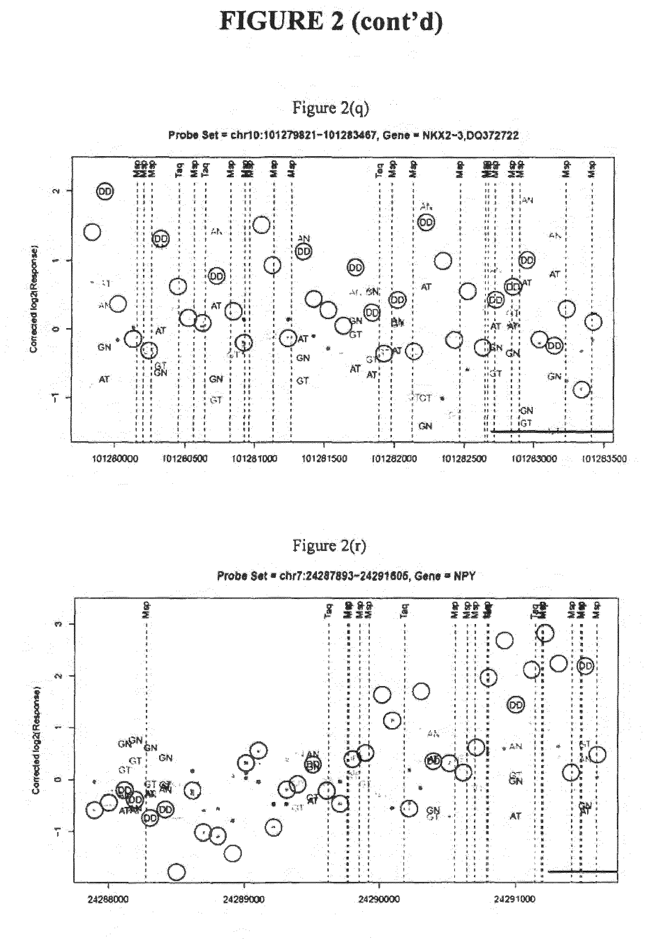

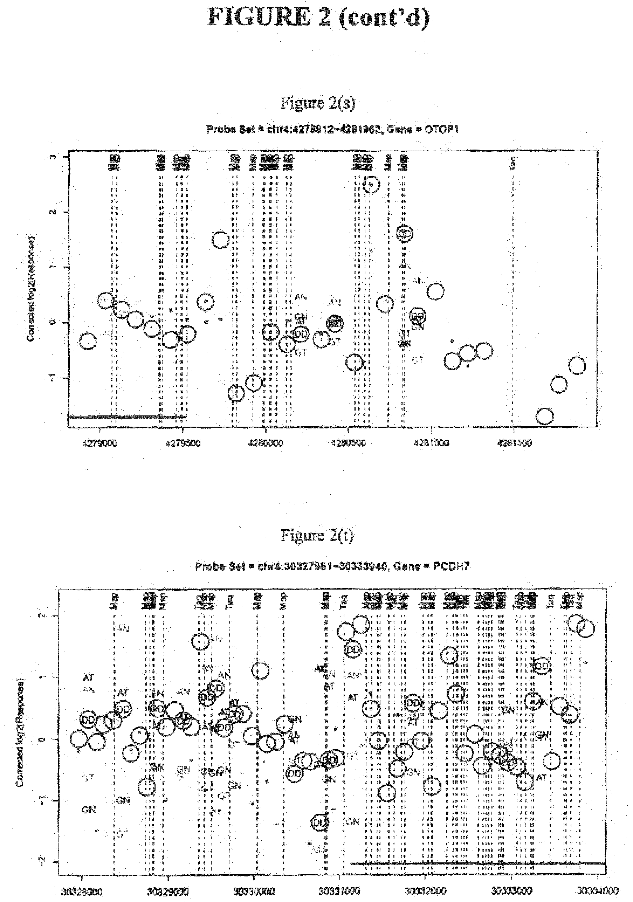

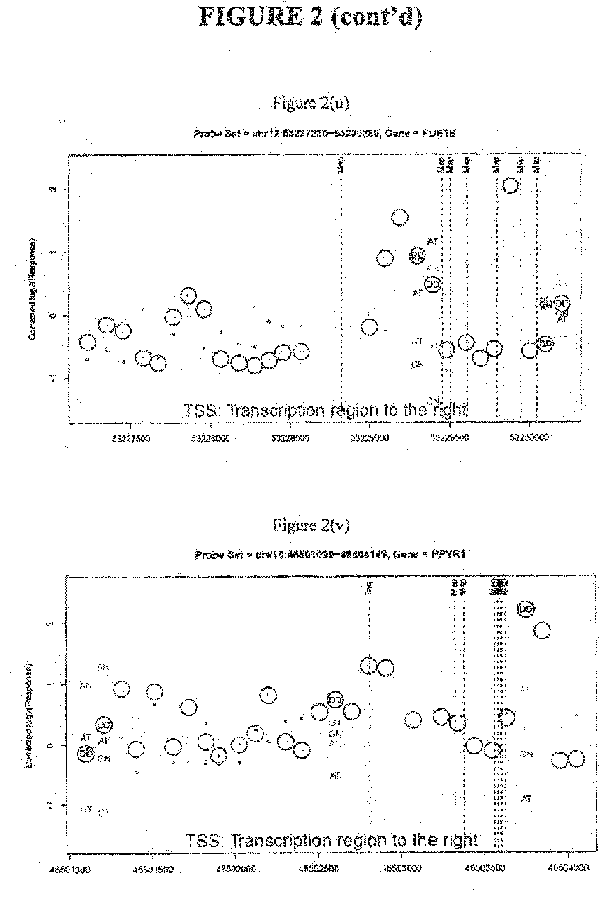

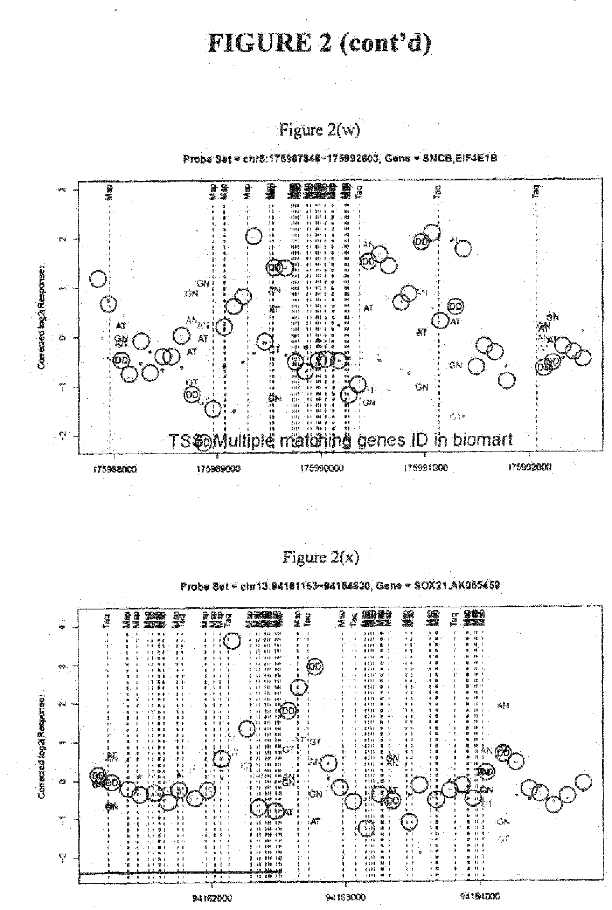

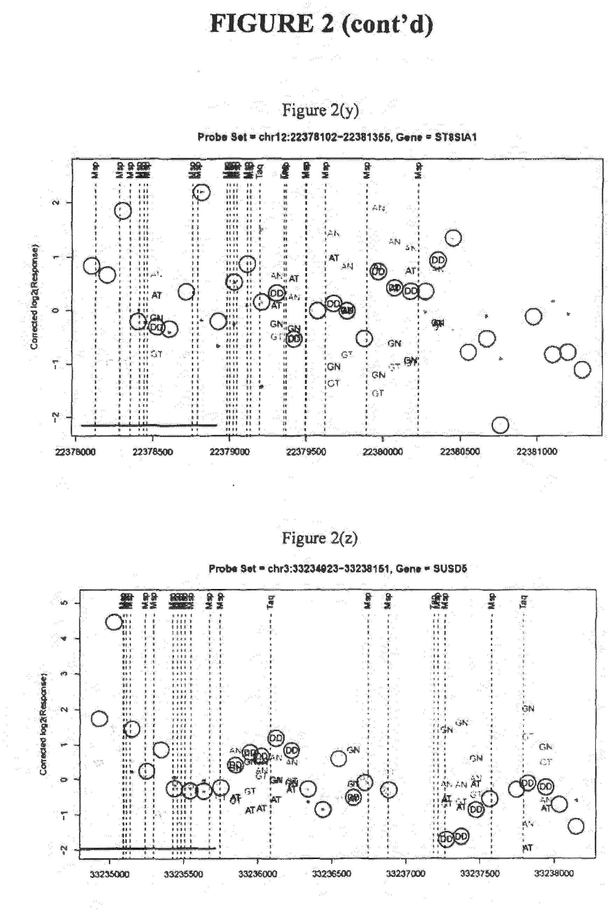

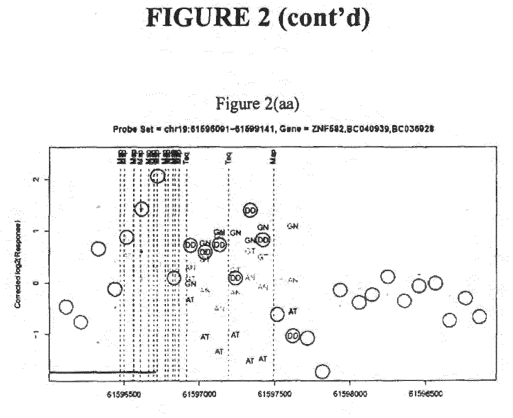

**Figures 1** **and** **2** depict the differential methylation plots for the genes shown in Table 3 and 4, respectively. The chromosome co-ordinates of the tiled regions are shown on the X-axis. The positions of TaqI and MspI restriction sites are indicated by dashed vertical lines. On the Y-axis is shown (log2 scale) the hybridisation intensities. In a column above each probe position are represented the signals of the methylated fractions of normal and cancer DNAs (GN and GT respectively) and their unmethylated fractions (AN and AT respectively). The "double-differences" (GT-GN) - (AT-AN), that represents the differential methylation between cancer and normal is shown as black DD for probes with the highest 17% quality score, as grey DD for those in the top 43% and as grey dots for those probes with lower quality scores or that lie beyond 300 bp from a TaqI or MspI restriction site. All DD probe points are circled for clarity.

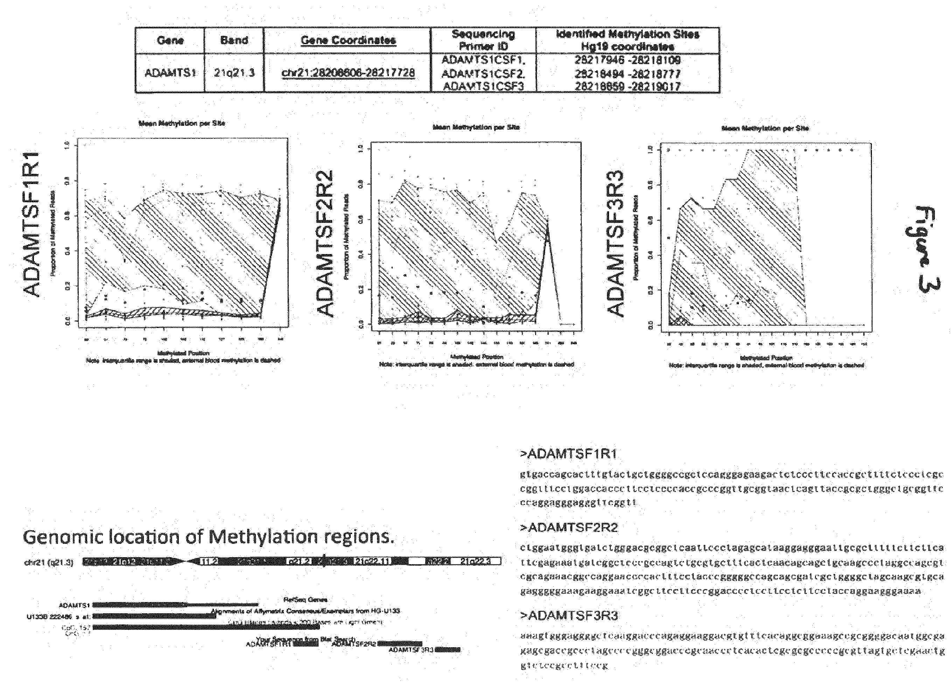

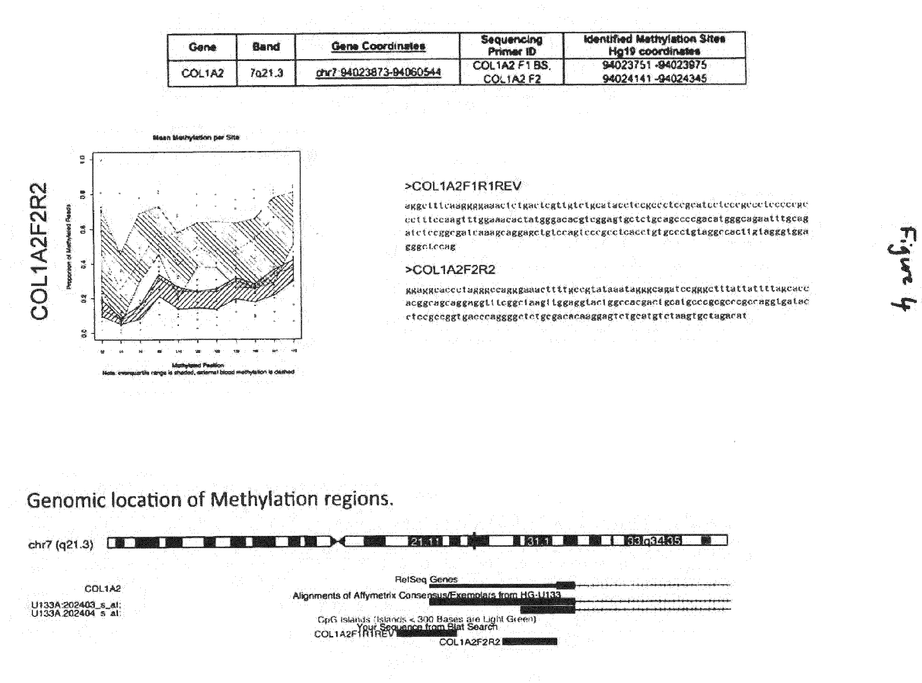

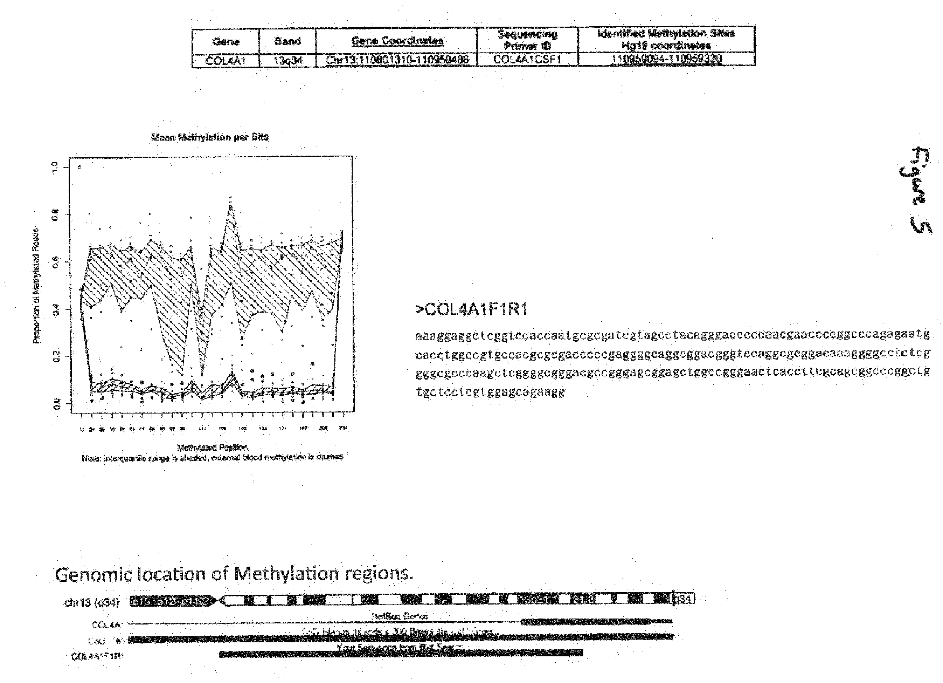

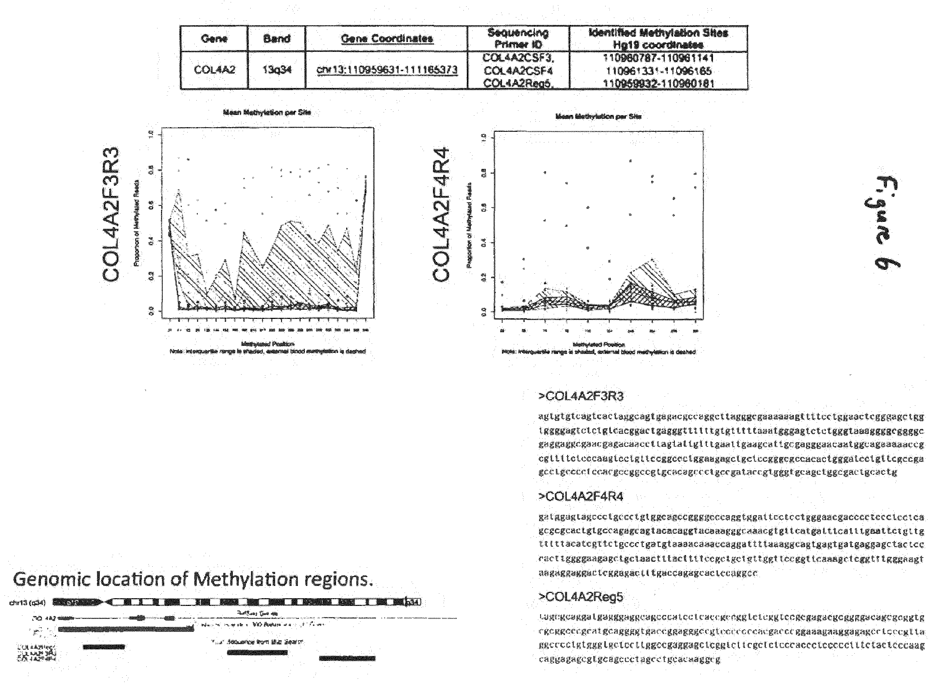

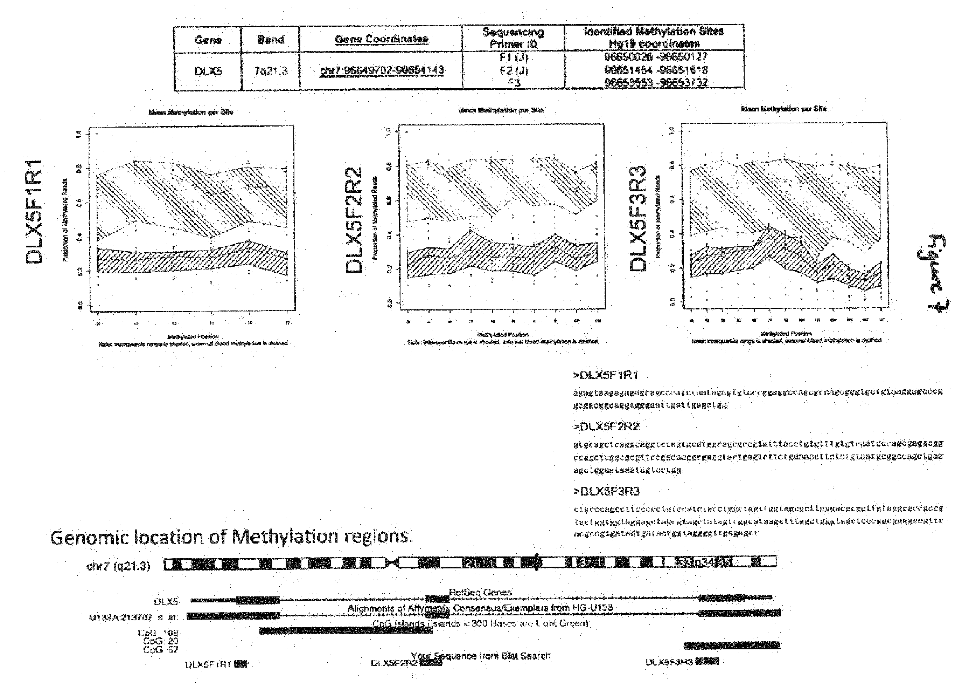

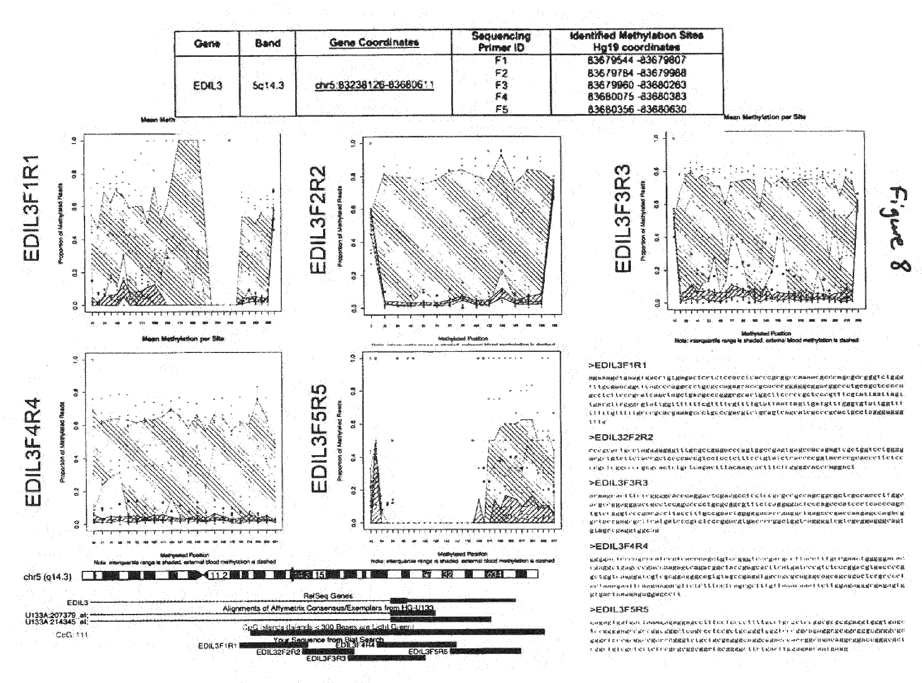

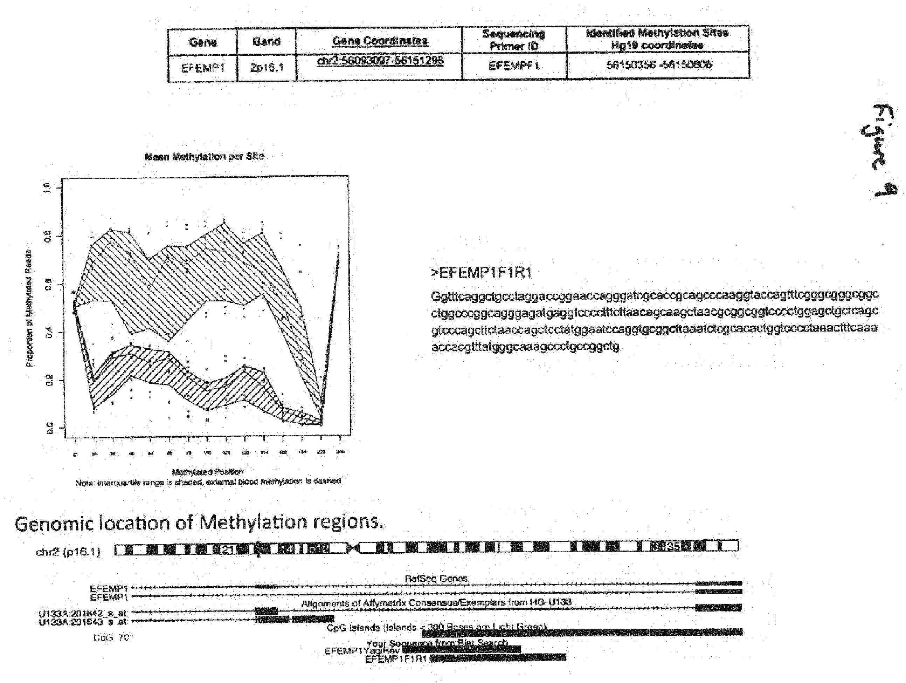

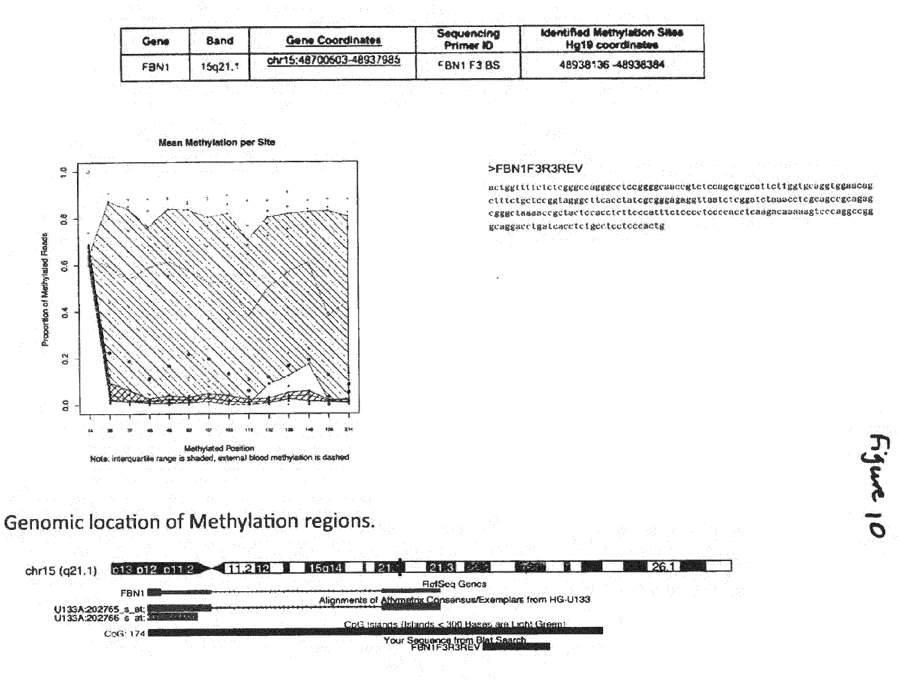

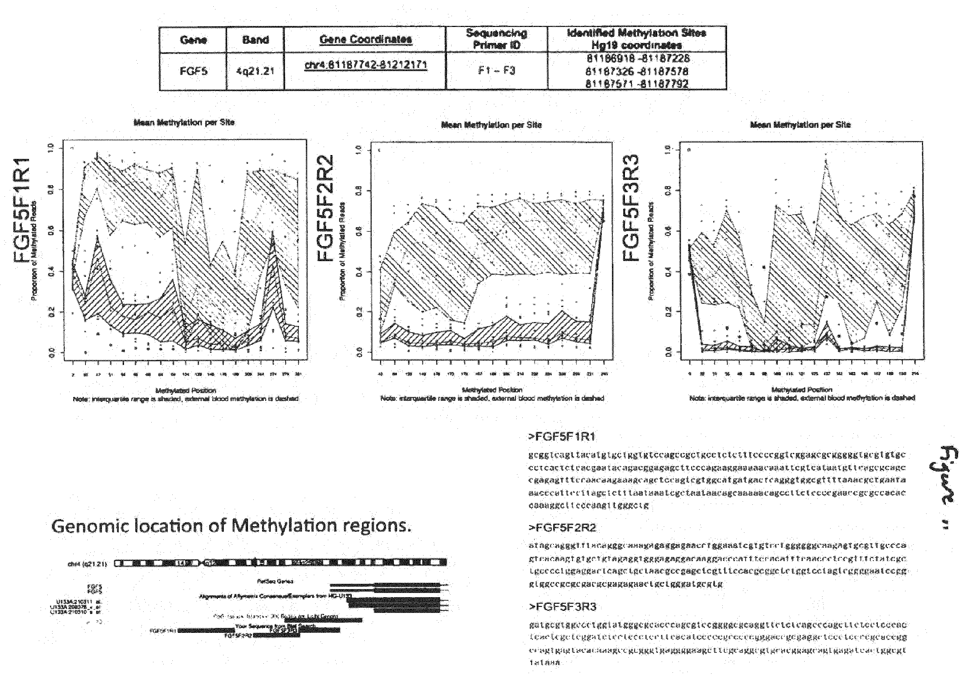

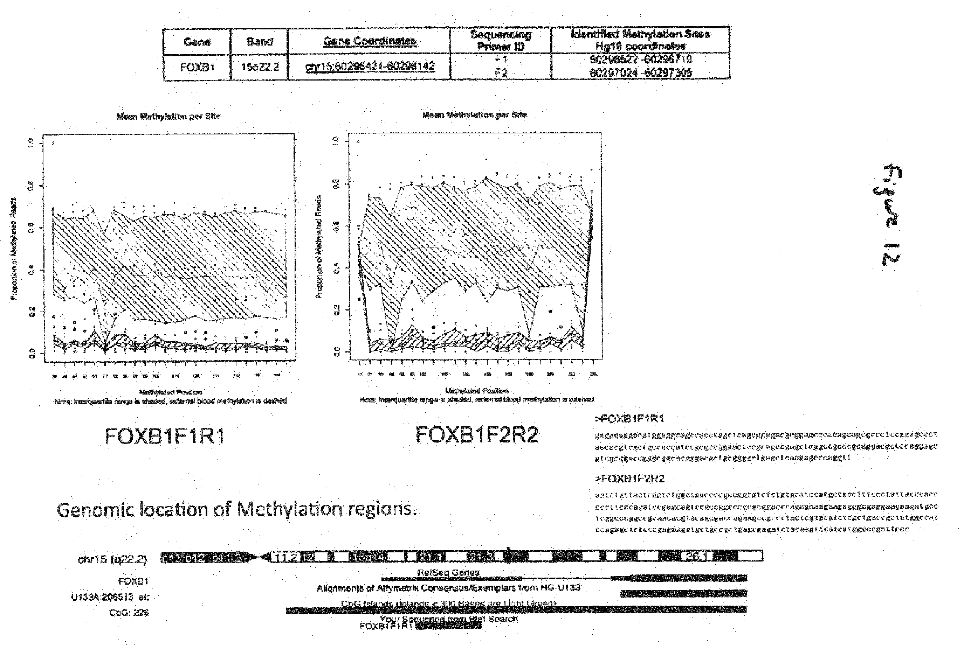

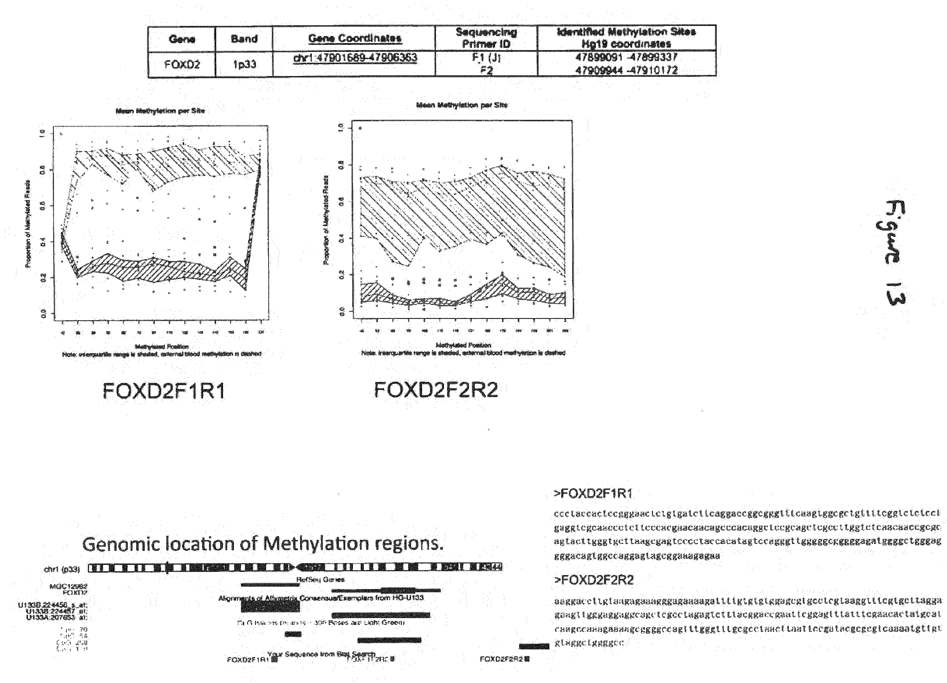

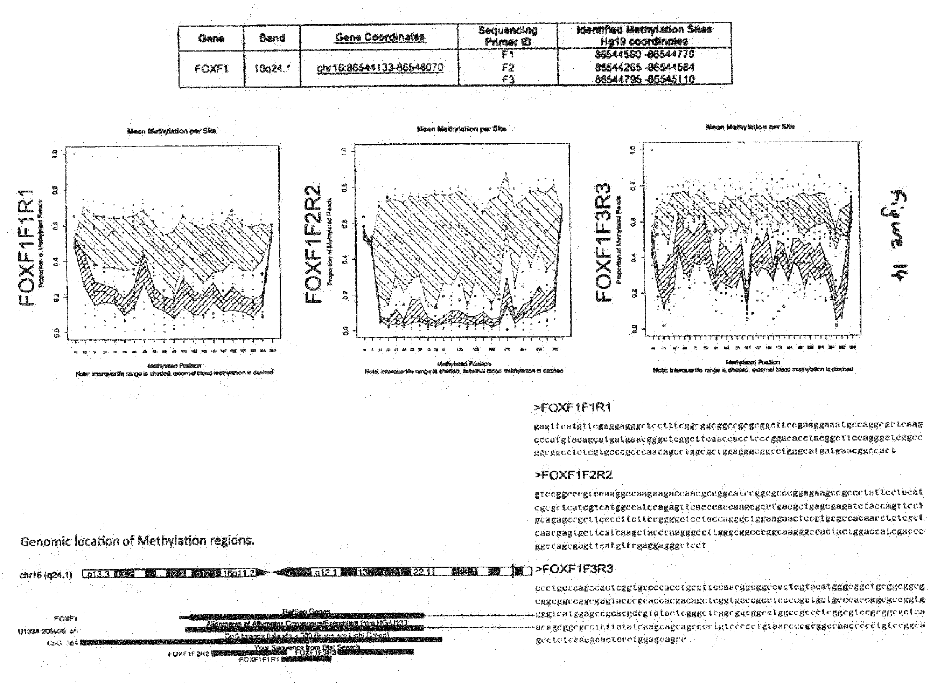

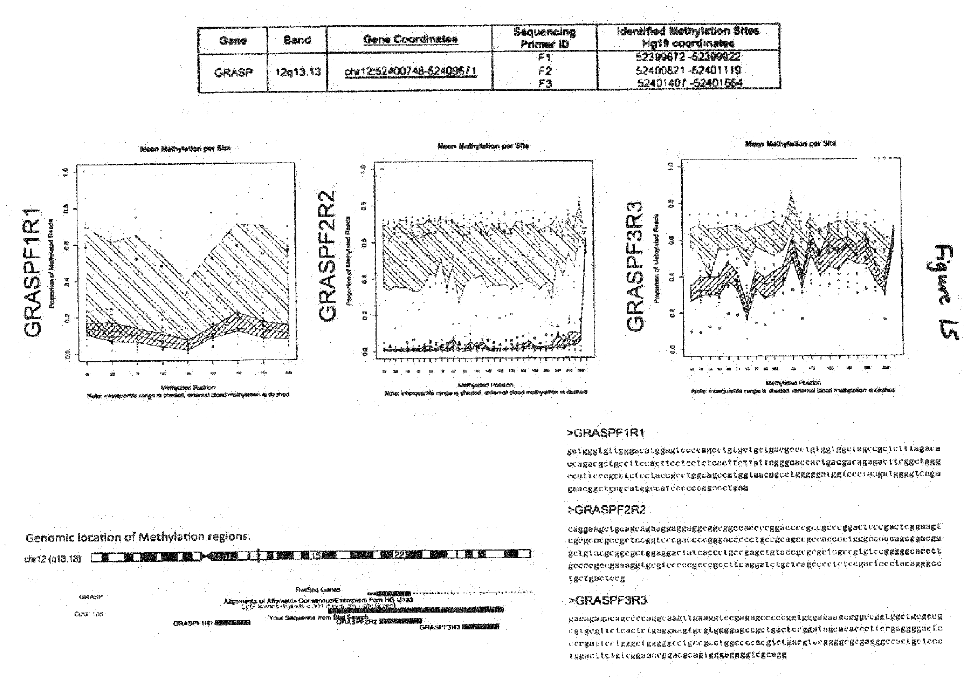

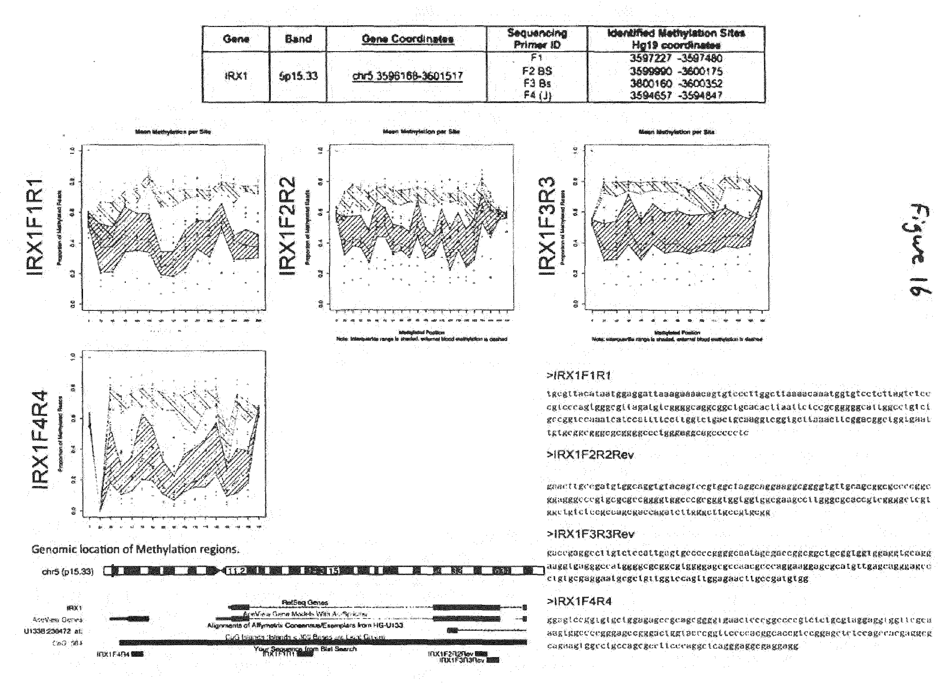

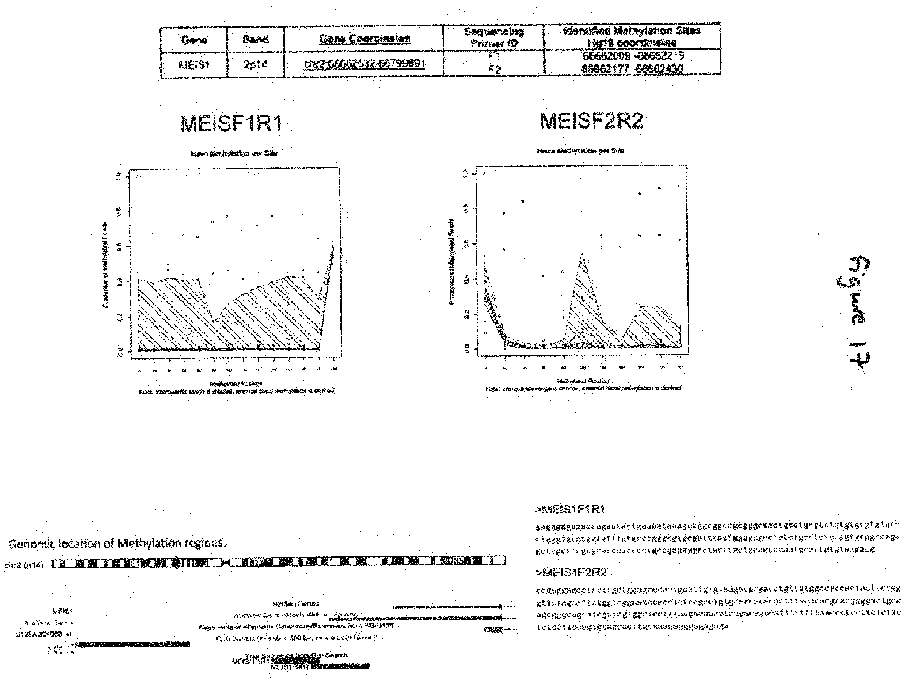

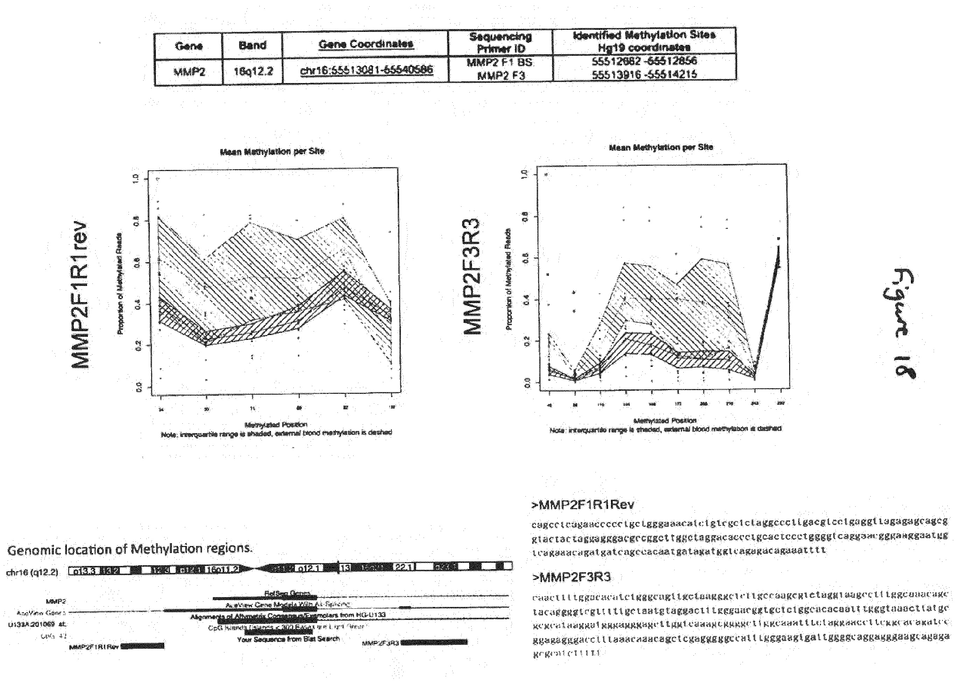

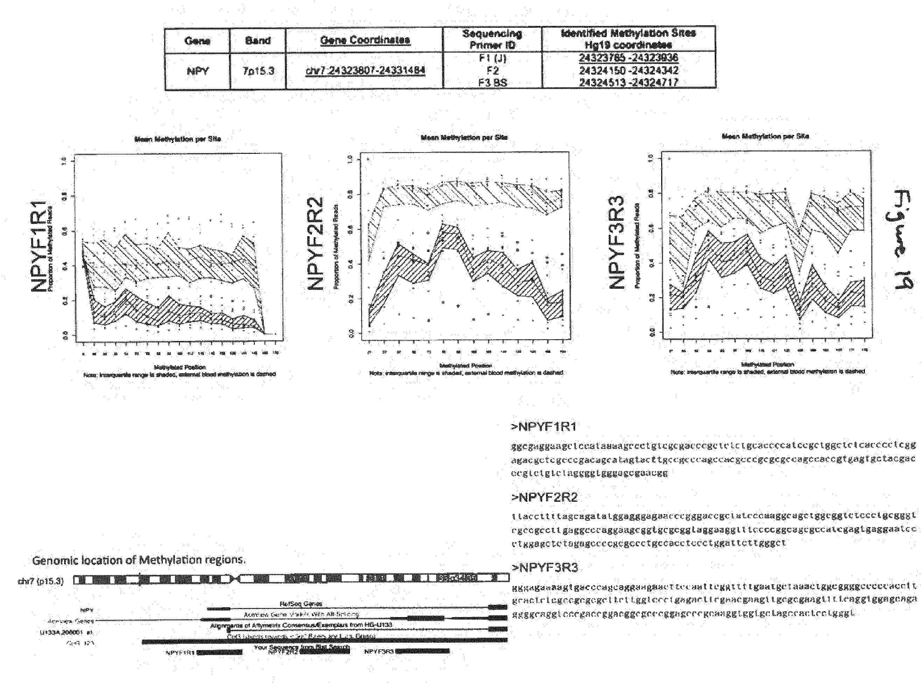

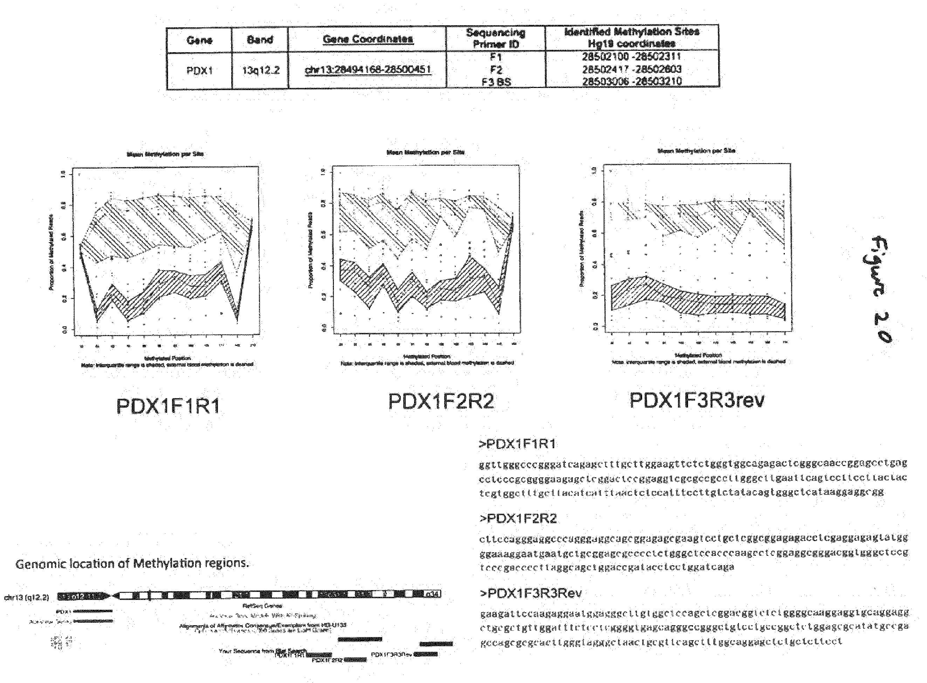

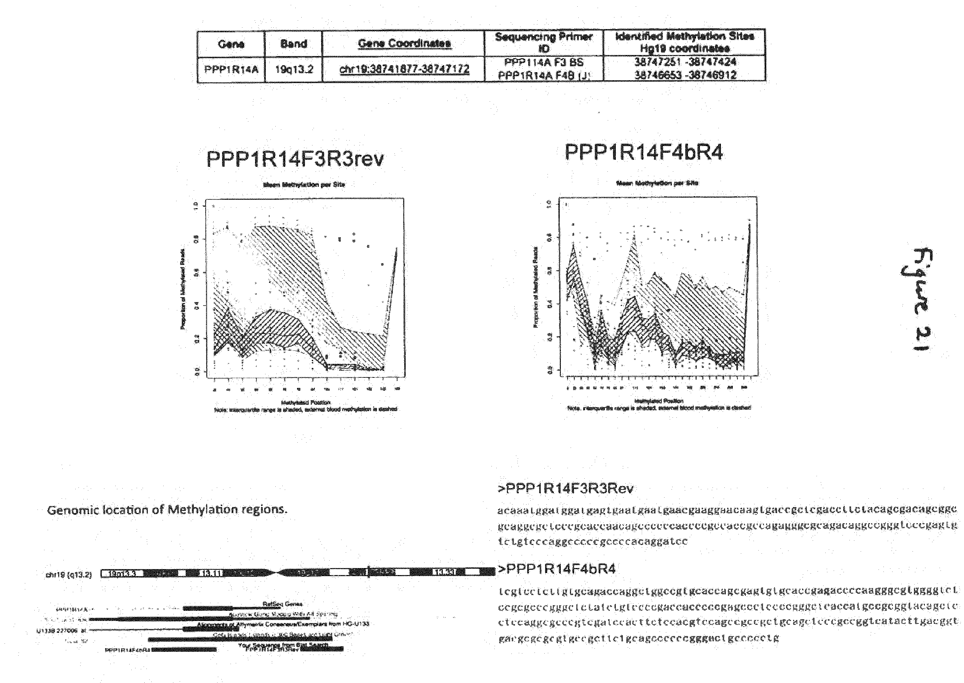

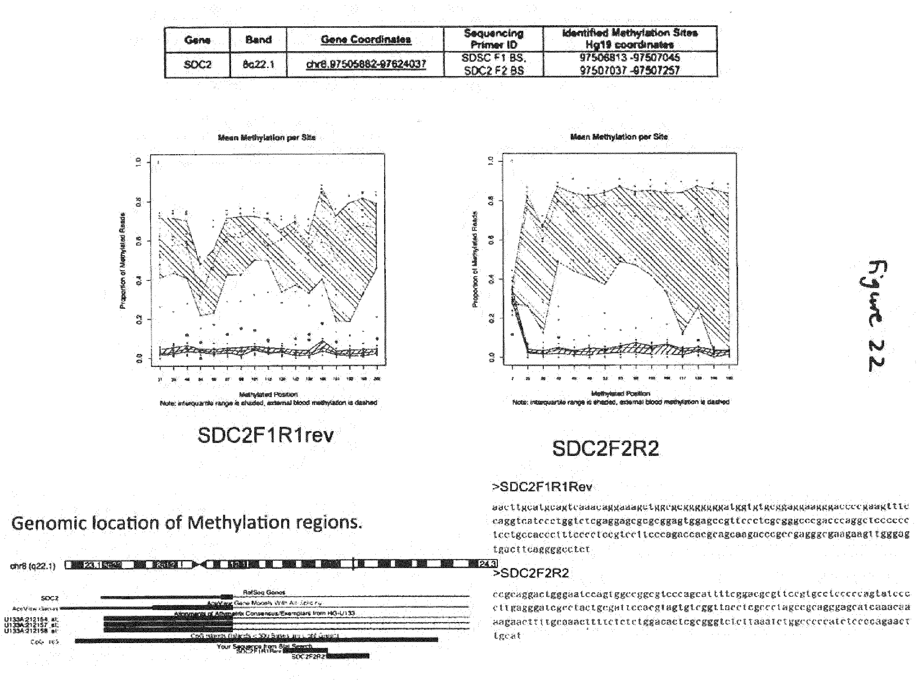

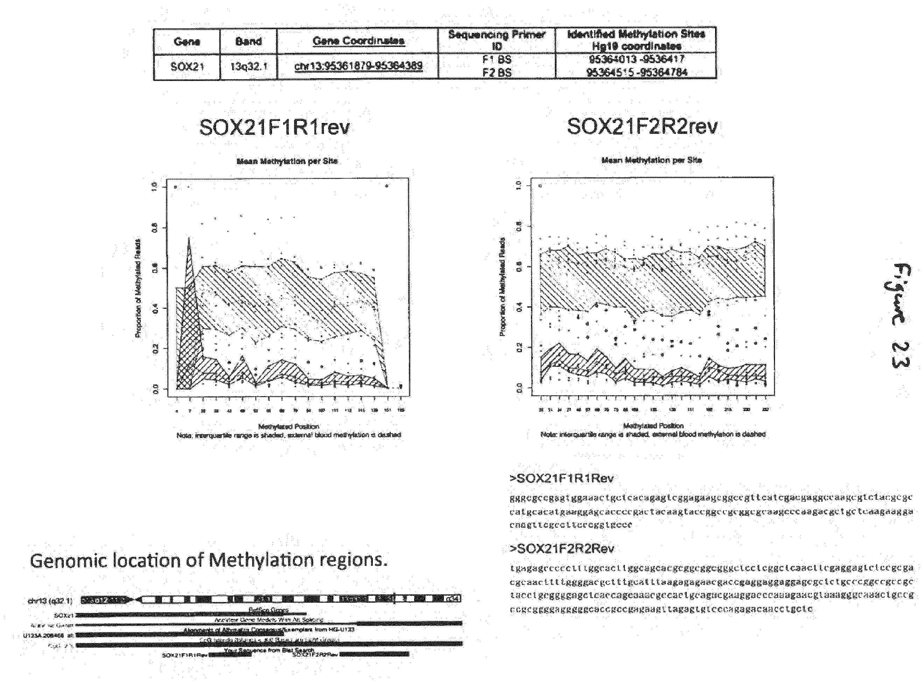

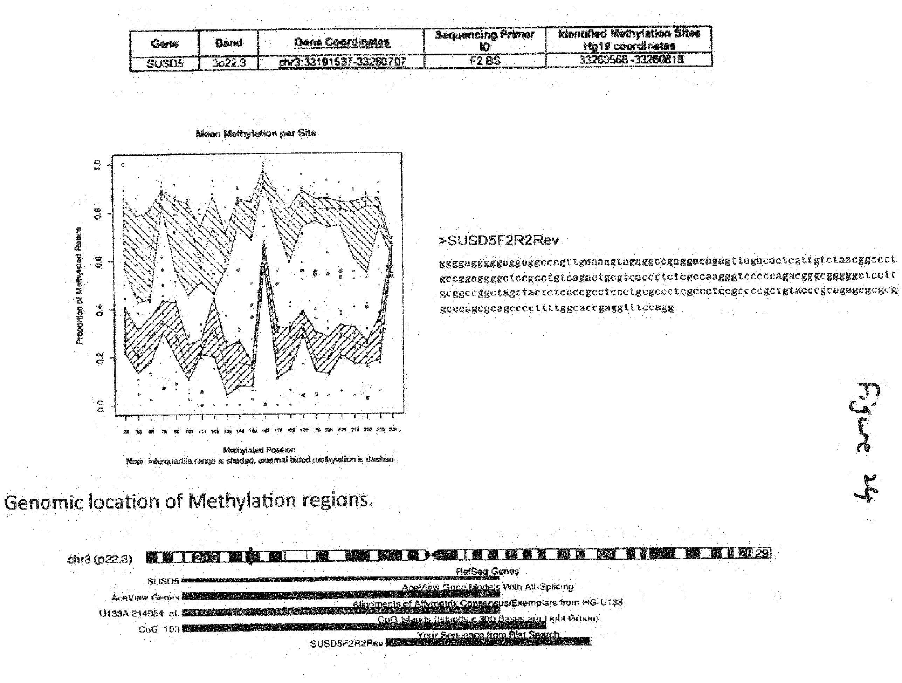

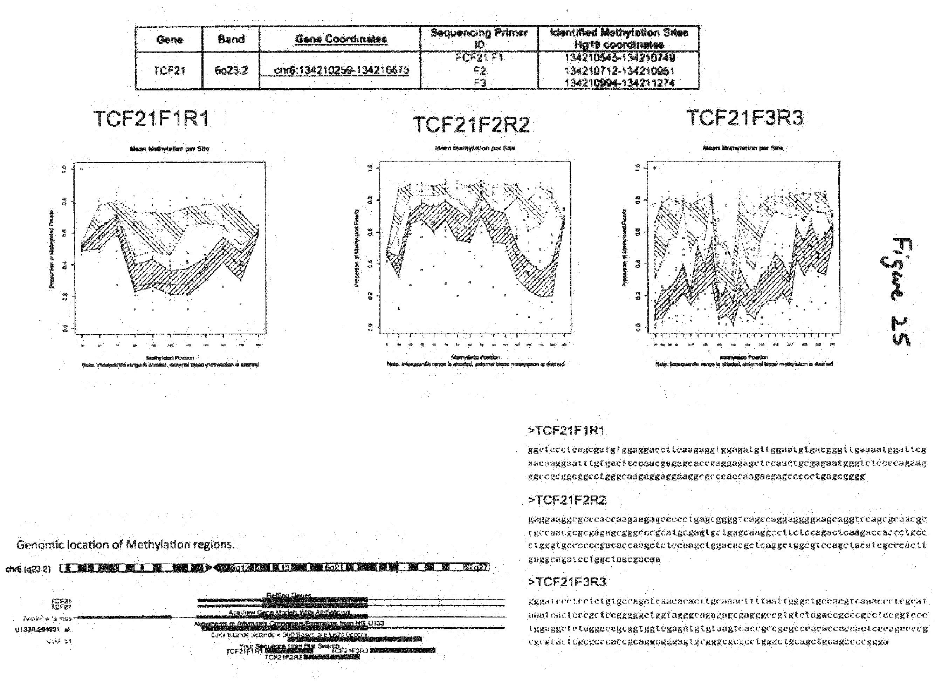

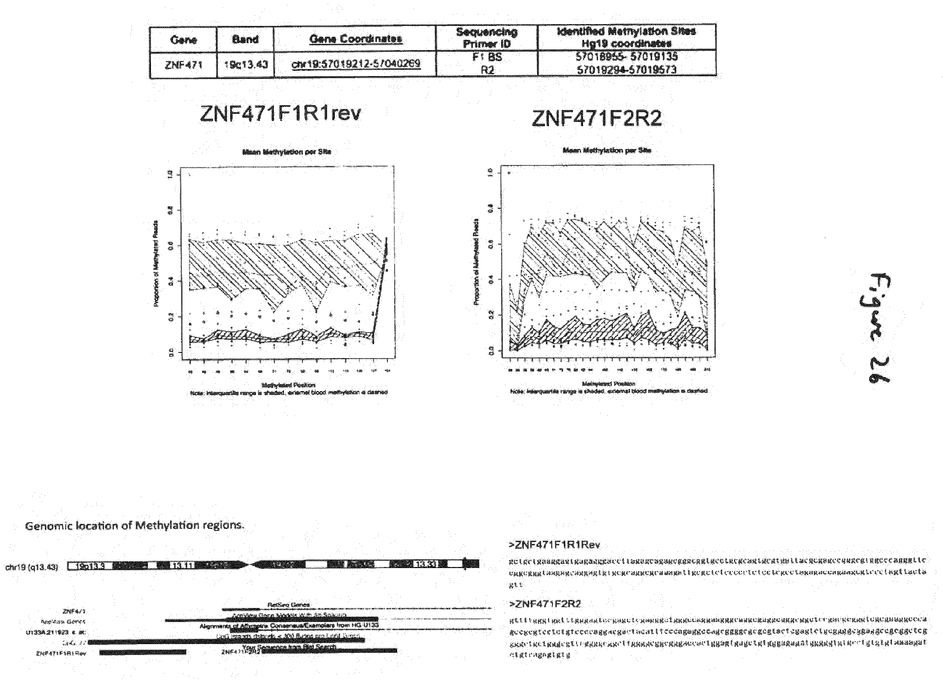

**Figures 3-27** show the profiles of DNA methylation obtained from bisulphite sequence analysis of one or more amplicons from 24 genes. In each figure the upper box shows the chromosomal co-ordinates of the gene (Hg19 sequence) and of the individual amplicons. The DNA methylation plots show the fraction of methylated cytosine (Y-axis) at each CpG site as determined by sequence analysis. The positions of each CpG site within the amplicons are shown on the X-axis. Individual grey dots represent methylation fractions at each CpG site for the 10 cancer DNAs; the average methylation of the ten samples is shown by the continuous grey line and the interquartile range by the shaded area. Similarly, the methylation fraction of normal DNA samples is shown by black dots, the average methylation and interquartile range by the black line and shaded black area respectively. The methylation fraction in blood DNA is shown by a light grey dashed line (this is often obscured by the methylation data for normal tissue DNA). The figures also include the sequence region for each PCR amplicon, prior to bisulphite modification. In some cases have been designed to the bottom strand relative to the direction of gene transcription and these are labelled BS. The pictograms at the bottom of the figures show the chromosomal positions of the genes and maps that show the location and exon structure of the gene transcripts, the position of Affymetrix U133 Plus probesets and location of CpG islands, as well as the location of the different amplified regions.

### DETAILED DESCRIPTION OF THE INVENTION

The present invention is predicated, in part, on the elucidation of DNA methylation status which characterises large intestine neoplasms. This finding has now facilitated the development of routine means of screening for the onset or predisposition to the onset of or monitoring a large intestine neoplasm based on increased methylation of certain genes relative to control levels.

In accordance with the present invention, it has been determined that certain genes are modulated, in terms of differential changes to their levels of methylation, depending on whether or not the cell in issue is neoplastic or not. It should be understood that the genes in issue are described herein both by reference to their name and their chromosomal coordinates. The chromosomal coordinates of the tiling regions of Nimblegen promoter tiling arrays are consistent with the human genome database version Hg18 (referred to herein as "Hg18 coordinates" and are detailed in Tables 3 and 4). To the extent that chromosomal coordinates corresponding to gene names are listed, these are consistent with the human genome database version Hg19 which was released in February 2009 (herein referred to as "Hg19 coordinates").

Accordingly, one aspect of the present invention is directed to a method of screening for the onset or predisposition to the onset of or monitoring a large intestine neoplasm in an individual, said method comprising assessing the methylation status of a DNA region selected from:
(i) the region defined by any one or more of Hg19 coordinates and 2kb upstream of the transcription start site:

| | |
|---|---|
| (1) chr12:52400748..52409671 | (34) chr10:101292690..101296281 |
| (2) chr5:3596168..3601517 | (35) chr4:4190530..4228621 |
| (3) chr13:95361876..95364389 | (36) chr12:54943404..54973023 |
| (4) chr4:81187742..81212171 | (37) chr5:176047210..176057557 |
| (5) chr19:57019212..57040270 | (38) chr12:22346325..22487648 |
| (6) chr3:33191537..33260707 | (39) chr19:56894648..56904889 |
| (7) chr15:60296421..60298142 | (40) chr20:21491648..21494664 |
| (8) chr13:28494168..28500451 | (41) chr1:50883225..50889141 |
| (9) chr7:96649702..96654143, | (42) chr7:27180996..27183287 |
| (10) chr8:140,811,770-141,537,860 | (43) chr11:2016406..2019065 |
| (11) chr5:2746279..2751769 | (44) chr14:57267425..57277184 |
| (12) chr18:55102917..55158530 | (45) chr4:126237567..126414087 |
| (13) chr20:37353101..37358016 | (46) chr8:23559964..23563922 |
| (14) chr8:2792875..4852328 | (47) chr10:131633547..131762091 |
| (15) chr16:66613351..66622178 | (48) chr4:62362839..62938168 |
| (16) chr5:37815753..37839782 | (49) chr1:47901689..47906363 |
| (17) chr1:63788730..63790797 | (50) chr17:77768176..77770890 |
| (18) chr15:37156644..37178734 | (51) chr17:93598762..93604831 |
| (19) chr7:27139973..27142394 | (52) chr1:33789224..33841194 |
| (20) chr20:21686297..21696620 | (53) chr9:124,004,679-124,030,840 |
| (21) chr16:51169886..51185183 | (54) chr4:158141736..158287227 |
| (22) chr12:85253267..85306606 | (55) chr12:9445136..9462559 |
| (23) chr8:6357172..6420784 | (56) chr12:24964278..25102308 |
| (24) chr14:85996488..86094270 | (57) chrX:21542357..21690352 |
| (25) chr2:182541194..182545381 | (58) chr20:52769988..52790516 |
| (26) chr7:30951468..30965131 | (59) chr3:172162951..172166203 |
| (27) chr8:131792547..132052835 | (60) chr13:28366780..28368089 |
| (28) chr3:128749292..128759583 | (61) chr7:50344378...50472799 |
| (29) chr10:101088856..101154087 | (62) chr7:149412148..149431664 |
| (30) chr7:27282164..27286192 | (63) chr7:24323809..24331477 |
| (31) chr10:129535538..129539450 | (64) chr4:30722037..31148421 |
| (32) chr19:49316274..49339934 | (65) chr10:47083534..47088320 |
| (33) chr6:391752..411443; or | |

(ii) the gene region, including 2kb upstream of any one or more of:

| | | | |
|---|---|---|---|
| (1) GRASP | (18) ANGPT2 | (35) NKX2-6 | (52) HOXA5 |
| (2) IRX1 | (19) LHX6 | (36) PAX1 | (53) GDNF |
| (3) SOX21 | (20) NEUROD1 | (37) FOXD2 | (54) FAT4 |
| (4) FGF5 | (21) AC149644.1 | (38) SLC6A15 | (55) HOXA2 |
| (5) ZNF471 | (22) CCDC48 | (39) PHC2 | (56) LPHN3 |
| (6) SUSD5 | (23) EVX1 | (40) FLRT2 | (57) ADCYAP1 |
| (7) FOXB1 | (24) GHSR | (41) GATA2 | (58) GRIA2 |
| (8) PDX1 | (25) HSD17B14 | (42) ADCY8 | (59) AQP1 |
| (9) DLX5 | (26) KRBA1 | (43) CNNM1 | (60) BCAT1 |
| (10) ONECUT2 | (27) OTOP1 | (44) IKZF1 | (61) CYP24A1 |
| (11) DMRTA2 | (28) PPYR1 | (45) NKX2-3 | (62) FOXI2 |
| (12) CMTM2 | (29) SRMS | (46) PCDH7 | (63) GSX1 |
| (13) OTX2 | (30) ZNF582 | (47) SNCB | (64) IRF4 |
| (14) LOC145845 | (31) IRX2 | (48) ST8SIA1 | (65) NPY |
| (15) EBF3 | (32) CSMD1 | (49) TRAPPC9 | (66) PDE1B |
| (16) SALL1 | (33) MIR675,H19 | (50) NKX2-2 | |
| (17) CBX8 | (34) FOXD3 | (51) SLC32A1 | |

in a biological sample from said individual wherein a higher level of methylation of the DNA regions of group (i) and/or (ii) relative to control levels is indicative of a large intestine neoplasm or a predisposition to the onset of a large intestine neoplastic state.

Reference to "large intestine" should be understood as a reference to a cell derived from one of the eight anatomical regions of the large intestine, which regions commence after the terminal region of the ileum, these being:
(i) the cecum;
(ii) the ascending colon;
(iii) the transverse colon;
(iv) the descending colon;
(v) the sigmoid colon;
(vi) the rectum;
(vii) the splenic flexure; and
(viii) the hepatic flexure.

Reference to "neoplasm" should be understood as a reference to a lesion, tumour or other encapsulated or unencapsulated mass or other form of growth which comprises neoplastic cells. A "neoplastic cell" should be understood as a reference to a cell exhibiting abnormal growth. The term "growth" should be understood in its broadest sense and includes reference to proliferation. In this regard, an example of abnormal cell growth is the uncontrolled proliferation of a cell. Another example is failed apoptosis in a cell, thus prolonging its usual life span. The neoplastic cell may be a benign cell or a malignant cell. In a preferred embodiment, the subject neoplasm is an adenoma or an adenocarcinoma. Without limiting the present invention to any one theory or mode of action, an adenoma is generally a benign tumour of epithelial origin which is either derived from epithelial tissue or exhibits clearly defined epithelial structures. These structures may take on a glandular appearance. It can comprise a malignant cell population within the adenoma, such as occurs with the progression of a benign adenoma or benign neoplastic legion to a malignant adenocarcinoma.

Preferably, said neoplastic cell is an adenoma or adenocarcinoma and even more preferably a colorectal adenoma or adenocarcinoma.

Reference to "DNA region" should be understood as a reference to a specific section of genomic DNA. These DNA regions are specified either by reference to a gene name or a set of chromosomal coordinates. Both the gene names and the chromosomal coordinates would be well known to, and understood by, the person of skill in the art. As detailed hereinbefore, the chromosomal coordinates for regions assayed on Nimblegen promoter tiling arrays correspond to the Hg18 version of the genome, while those describing the associated gene symbol correspond to the Hg19 version of the genome. In general, a gene can be routinely identified by reference to its name, via which both its sequences and chromosomal location can be routinely obtained, or by reference to its chromosomal coordinates, via which both the gene name and its sequence can also be routinely obtained.

Reference to each of the genes/DNA regions detailed above should be understood as a reference to all forms of these molecules and to fragments or variants thereof. As would be appreciated by the person of skill in the art, some genes are known to exhibit allelic variation between individuals or single nucleotide polymorphisms. SNPs encompass insertions and deletions of varying size and simple sequence repeats, such as dinucleotide and trinucleotide repeats. Variants include nucleic acid sequences from the same region sharing at least 90%, 95%, 98%, 99% sequence identity i.e. having one or more deletions, additions, substitutions, inverted sequences etc. relative to the DNA regions described herein. Accordingly, the present invention should be understood to extend to such variants which, in terms of the present diagnostic applications, achieve the same outcome despite the fact that minor genetic variations between the actual nucleic acid sequences may exist between individuals. The present invention should therefore be understood to extend to all forms of DNA which arise from any other mutation, polymorphic or allelic variation.

It should be understood that the "individual" who is the subject of testing may be any human or non-human mammal. Examples of non-human mammals includes primates, livestock animals (e.g. horses, cattle, sheep, pigs, donkeys), laboratory test animals (e.g. mice, rats, rabbits, guinea pigs), companion animals (e.g. dogs, cats) and captive wild animals (e.g. deer, foxes).

Preferably the mammal is a human.

The panel of genes which has been identified demonstrates increased methylation in large intestine neoplastic cells relative to corresponding non-neoplastic cells. This increased methylation is, in some cases, localised to a few specific CpG sites within the DNA region while in other cases it is observed across a wide range of CpG sites. However, although specific regions from all genes exhibit increased methylation and are therefore useful diagnostic markers, several of these genes provide particularly good sensitivity and specificity, either or both because elevated methylation is observed in a higher proportion of neoplasms or because their greater difference in level of DNA methylation between neoplastic tissue and either normal colon tissue or peripheral blood leukocytes.

According to this embodiment there is provided a method of screening for the onset or predisposition to the onset of or monitoring a large intestine neoplasm in an individual, said method comprising assessing the methylation status of a DNA region selected from:
(i) the region defined by any one or more of Hg19 coordinates and 2kb upstream of the transcription start site:

| | |
|---|---|
| (1) chr12:52400748..52409671 | (6) chr3:33191537..33260707 |
| (2) chr5:3596168..3601517 | (7) chr15:60296421..60298142 |
| (3) chr13:95361876..95364389 | (8) chr13:28494168..28500451 |
| (4) chr4:81187742..81212171 | (9) chr7:96649702..96654143 |
| (5) chr19:57019212..57040270 | |

(ii) the gene region, including 2kb upstream of any one or more of:

| | | | |
|---|---|---|---|
| (1) GRASP | (4) FGF5 | (6) SUSD5 | (8) PDX1 |
| (2) IRX1 | (5) ZNF471 | (7) FOXB1 | (9) DLX5 |
| (3) SOX21 | | | |

in a biological sample from said individual wherein a higher level of methylation of the DNA regions of group (i) and/or (ii) relative to control levels is indicative of a large intestine neoplasm or a predisposition to the onset of a large intestine neoplastic state.

In accordance with these aspects, in yet another embodiment said control level is a non-neoplastic level.

According to these aspects of the present invention, said large intestine tissue is preferably colorectal tissue.

In still another embodiment, the neoplasm is malignant, such as a carcinoma.

In a further embodiment, the neoplasm is non-malignant, such as an adenoma.

Without limiting the present invention to any one theory or mode of action, although measuring the methylation levels across these DNA regions is diagnostic of a large intestine neoplastic condition, it has been determined that discrete subregions are particularly useful in this regard since these subregions contain a high density of CpG dinucleotides which are frequently hypermethylated in large intestine neoplasias, such as colorectal cancers. To this end, it has also been determined that in the context of a further 15 DNA regions which were previously known to be downregulated in terms of their level of expression in the context of large intestine neoplasms, there in fact occur discrete subregions which exhibit a particularly high density of hypermethylated CpG dinucleotides. This finding renders these subregions a particularly useful target for analysis since it both simplifies the screening process due to a shorter more clearly defined region of DNA requiring analysis and, further, the fact that the results from these regions will provide a significantly more definitive result in relation to the presence, or not, of hypermethylation than would be obtained if analysis was performed across the DNA region as a whole. This finding therefore both simplifies the screening process and increases the sensitivity and specificity of large intestine neoplasia diagnosis.

The subregions which have been determined to exhibit particular utility are listed below with reference to the gene and chromosomal region within which they are found:
(1) Chr12:52400748-52409671 (GRASP) subregions Chr12:52399672-52399922 (SEQ ID NO:15), Chr12:52400821-52401119 (SEQ ID NO:16) and Chr12:52401407-52401664 (SEQ ID NO:17).
(2) Chr8:97505882-97624037 (SDC2) subregions Chr8:97506813-97507045 (SEQ ID NO:18) and Chr8:97507037-97507257 (SEQ ID NO:19).
(3) Chr13:110959094-110959330 (SEQ ID NO:20) (COL4A1).
(4) Chr5:3596168-3601517 (IRX1) subregions Chr5:3597227-3597480 (SEQ ID NO:21), Chr5:3599990-3600175 (SEQ ID NO:22), Chr5:3600160-3600352 (SEQ ID NO:23) and Chr5:3594657-3594847 (SEQ ID NO:24).
(5) Chr13:95361879-95364389 (SOX21) subregions Chr13:95364013-95364178 (SEQ ID NO:25) and Chr13:95364515-95364784 (SEQ ID NO:26).
(6) Chr4:81187742-81212171 (FGF5) subregions Chr4:81186918-81187228 (SEQ ID NO:27), Chr4:81187326-81187578 (SEQ ID NO:28) and Chr4:81187571-81187792 (SEQ ID NO:29).
(7) Chr19:57019212-57040269 (ZNF471) subregions Chr19:57018955-57019135 (SEQ ID NO:30) and Chr19:57019294-57019573 (SEQ ID NO:31).
(8) Chr3:33191537-33260707 (SUSD5) subregion Chr3:33260566-33260818 (SEQ ID NO:32).
(9) Chr2:56093097-56151298 (EFEMP1) subregion Chr2:56150356-56150606 (SEQ ID NO:33).
(10) Chr6:134210259-134216675 (TCF21) subregions Chr6:134210545-134210749 (SEQ ID NO:34), Chr6:134210712-134210951 (SEQ ID NO:35) and Chr6:134210994-134211274 (SEQ ID NO:36).
(11) Chr7:94023873-94060544 (COL1A2) subregions Chr7:94023751-94023975 (SEQ ID NO:37) and Chr7:94024141-94024345 (SEQ ID NO:38).
(12) Chr15:60296421-60298142 (FOXB1) subregions Chr15:60296522-60296719 (SEQ ID NO:39) and Chr15:60297024-60297305 (SEQ ID NO:40).
(13) Chr16:86544133-86548070 (FOXF1) subregions Chr16:86544560-86544770 (SEQ ID NO:41), Chr16:86544265-86544584 (SEQ ID NO:42) and Chr16:86544795-86545110 (SEQ ID NO:43).
(14) Chr13:28494168-28500451 (PDX1) subregions Chr13:28502100-28502311 (SEQ ID NO:44), Chr13:28502417-28502603 (SEQ ID NO:45) and Chr13:28503006-28503210 (SEQ ID NO:46).
(15) Chr7:96649702-96654143 (DLX5) subregions Chr7:96650026-96650127 (SEQ ID NO:47), Chr7:96651454-96651618 (SEQ ID NO:48) and Chr7:96653553-96653732 (SEQ ID NO:49).
(16) Chr21:28208606-28217728 (ADAMTS1) subregions Chr21:28217946-28218109 (SEQ ID NO:50), Chr21:28218494-28218777 (SEQ ID NO:51)and Chr21:28218859-28219017 (SEQ ID NO:52).
(17) Chr13:110959631-111165373 (COL4A2) subregions Chr13:110960787-110961141 (SEQ ID NO:53); Chr13:110961331-110961659 (SEQ ID NO:54) and Chr13:110959932-110960181 (SEQ ID NO:82);
(18) Chr5:83238126-83680611 (EDIL3) subregions Chr5:83679544-83679807 (SEQ ID NO:55), Chr5:83679784-83679988 (SEQ ID NO:56), Chr5:83679960-83680263 (SEQ ID NO:57), Chr5:83680075-83680383 (SEQ ID NO:58) and Chr5:83680356-83680630 (SEQ ID NO:59).
(19) Chr15:48700503-48937985 (FBN1) subregion Chr15:48938136-48938384 (SEQ ID NO:60).
(20) Chr1:47901689-47906363 (FOXD2) subregions Chr1:47899091-47899337 (SEQ ID NO:61) and Chr1:47909944-47910172 (SEQ ID NO:62).
(21) Chr2:66662532-66799891 (MEIS1) subregions Chr2:66662009-66662219 (SEQ ID NO:63) and Chr2:66662177-66662430 (SEQ ID NO:64).
(22) Chr16:55513081-55540586 (MMP2) subregions Chr16:55512662-55512856 (SEQ ID NO:65) and Chr16:55513916-55514215 (SEQ ID NO:66).
(23) Chr7:24323807-24331484 (NPY) subregions Chr7:24323765-24323936 (SEQ ID NO:67), Chr7:24324150-24324342 (SEQ ID NO:68) and Chr7:24324513-24324717 (SEQ ID NO:69).
(24) Chr19:38741877-38747172 (PPP1R14A) subregions Chr19:38747251-38747424 (SEQ ID NO:70) and Chr19:38746653-38746912 (SEQ ID NO:71).

According to this embodiment there is therefore provided a method of screening for the onset or predisposition to the onset of or monitoring a large intestine neoplasm in an individual, said method comprising assessing the methylation status of one or more DNA regions selected from regions defined by Hg19 coordinates:

| | | | |
|---|---|---|---|
| (1) | Chr12:52399672-52399922 | (30) | Chr13:28502100-28502311 |
| (2) | Chr12:52400821-52401119 | (31) | Chr13:28502417-28502603 |
| (3) | Chr12:52401407-52401664 | (32) | Chr13:28503006-28503210 |
| (4) | Chr8:97506813-97507045 | (33) | Chr7:96650026-96650127 |
| (5) | Chr8:97507037-97507257 | (34) | Chr7:96651454-96651618 |
| (6) | Chr13:110959094-110959330 | (35) | Chr7:96653553-96653732 |
| (7) | Chr5:3597227-3597480 | (36) | Chr21:28217946-28218109 |
| (8) | Chr5:3599990-3600175 | (37) | Chr21:28218494-28218777 |
| (9) | Chr5:3600160-3600352 | (38) | Chr21:28218859-28219017 |
| (10) | Chr5:3594657-3594847 | (39) | Chr13:110960787-110961141 |
| (11) | Chr13:95364013-95364178 | (40) | Chr13:110961331-110961659 |
| (12) | Chr13:95364515-95364784 | (41) | Chr5:83679544-83679807 |
| (13) | Chr4:81186918-81187228 | (42) | Chr5:83679784-83679988 |
| (14) | Chr4:81187326-81187578 | (43) | Chr5:83679960-83680263 |
| (15) | Chr4:81187571-81187792 | (44) | Chr5:83680075-83680383 |
| (16) | Chr19:57018955-57019135 | (45) | Chr5:83680356-83680630 |
| (17) | Chr19:57019294-57019573 | (46) | Chr15:48938136-48938384 |
| (18) | Chr3:33260566-33260818 | (47) | Chr1:47899091-47899337 |
| (19) | Chr2:56150356-56150606 | (48) | Chr1:47909944-47910172 |
| (20) | Chr6:134210545-134210749 | (49) | Chr2:66662009-66662219 |
| (21) | Chr6:134210712-134210951 | (50) | Chr2:66662177-66662430 |
| (22) | Chr6:134210994-134211274 | (51) | Chr16:55512662-55512856 |
| (23) | Chr7:94023751-94023975 | (52) | Chr16:55513916-55514215 |
| (24) | Chr7:94024141-94024345 | (53) | Chr7:24323765-24323936 |
| (25) | Chr15:60296522-60296719 | (54) | Chr7:24324150-24324342 |
| (26) | Chr15:60297024-60297305 | (55) | Chr7:24324513-24324717 |
| (27) | Chr16:86544560-86544770 | (56) | Chr19:38747251-38747424 |
| (28) | Chr16:86544265-86544584 | (57) | Chr19:38746653-38746912 |
| (29) | Chr16:86544795-86545110 | (58) | Chr13:110959932-110960181 |

in a biological sample from said individual when a higher level of methylation of said DNA region relative to control levels is indicative of a large intestine neoplasia or a predisposition to the onset of a large intestine neoplastic state.

In another embodiment, said neoplastic cell is an adenoma or adenocarcinoma and even more preferably a colorectal adenoma or adenocarcinoma.

In one particular embodiment there is therefore provided a method of screening for the onset or predisposition to the onset of or monitoring or monitoring a large intestine neoplasm in an individual, said method comprising assessing the methylation status of one or more DNA regions selected from regions defined by Hg19 coordinates:

| | | | |
|---|---|---|---|
| (1) | Chr12:52399672-52399922 | (19) | Chr2:56150356-56150606 |
| (2) | Chr12:52400821-52401119 | (20) | Chr6:134210545-134210749 |
| (3) | Chr12:52401407-52401664 | (21) | Chr6:134210712-134210951 |
| (4) | Chr8:97506813-97507045 | (22) | Chr6:134210994-134211274 |
| (5) | Chr8:97507037-97507257 | (23) | Chr7:94023751-94023975 |
| (6) | Chr13:110959094-110959330 | (24) | Chr7:94024141-94024345 |
| (7) | Chr5:3597227-3597480 | (25) | Chr15:60296522-60296719 |
| (8) | Chr5:3599990-3600175 | (26) | Chr15:60297024-60297305 |
| (9) | Chr5:3600160-3600352 | (27) | Chr16:86544560-86544770 |
| (10) | Chr5:3594657-3594847 | (28) | Chr16:86544265-86544584 |
| (11) | Chr13:95364013-95364178 | (29) | Chr16:86544795-86545110 |
| (12) | Chr13:95364515-95364784 | (30) | Chr13:28502100-28502311 |
| (13) | Chr4:81186918-81187228 | (31) | Chr13:28502417-28502603 |
| (14) | Chr4:81187326-81187578 | (32) | Chr13:28503006-28503210 |
| (15) | Chr4:81187571-81187792 | (33) | Chr7:96650026-96650127 |
| (16) | Chr19:57018955-57019135 | (34) | Chr7:96651454-96651618 |
| (17) | Chr19:57019294-57019573 | (35) | Chr7:96653553-96653732 |
| (18) | Chr3:33260566-33260818 | | |

in a biological sample from said individual when a higher level of methylation of said DNA region relative to control levels is indicative of a large intestine neoplasia or a predisposition to the onset of a large intestine neoplastic state.

In yet another embodiment said DNA regions are selected from regions defined by Hg19 coordinates:

| | | | |
|---|---|---|---|
| (1) | Chr12:52399672-52399922 | (18) | Chr3:33260566-33260818 |
| (2) | Chr12:52400821-52401119 | (19) | Chr2:56150356-56150606 |
| (4) | Chr8:97506813-97507045 | (20) | Chr6:134210545-134210749 |
| (5) | Chr8:97507037-97507257 | (21) | Chr6:134210712-134210951 |
| (6) | Chr13:110959094-110959330 | (24) | Chr7:94024141-94024345 |
| (10) | Chr5:3594657-3594847 | (25) | Chr15:60296522-60296719 |
| (11) | Chr13:95364013-95364178 | (26) | Chr15:60297024-60297305 |
| (12) | Chr13:95364515-95364784 | (28) | Chr16:86544265-86544584 |
| (13) | Chr4:81186918-81187228 | (30) | Chr13:28502100-28502311 |
| (16) | Chr19:57018955-57019135 | (31) | Chr13:28502417-28502603 |
| (17) | Chr19:57019294-57019573 | (35) | Chr7:96653553-96653732 |

In another embodiment, said neoplastic cell is an adenoma or adenocarcinoma and even more preferably a colorectal adenoma or adenocarcinoma.

Without limiting the present invention to any one theory or mode of action, DNA methylation is universal in bacteria, plants, and animals. DNA methylation is a type of chemical modification of DNA that is stable over rounds of cell division but does not involve changes in the underlying DNA sequence of the organism. Chromatin and DNA modifications are two important features of epigenetics and play a role in the process of cellular differentiation, allowing cells to stably maintain different characteristics despite containing the same genomic material. In eukaryotic organisms DNA methylation occurs only at the number 5 carbon of the cytosine pyrimidine ring. In mammals, DNA methylation occurs mostly at the number 5 carbon of the cytosine of a CpG dinucleotide. CpG dinucleotides comprise approximately 1% human genome.

70-80% of all CpGs are methylated. CpGs may be grouped in clusters called "CpG islands" that are present in the 5' regulatory regions of many genes and are frequently unmethylated. In many disease processes such as cancer, gene promoters and/or CpG islands acquire abnormal hypermethylation, which is associated with heritable transcriptional silencing. DNA methylation may impact the transcription of genes in two ways. First, the methylation of DNA may itself physically impede the binding of transcriptional proteins to the gene, thus blocking transcription. Second, methylated DNA may be bound by proteins known as Methyl-CpG-binding domain proteins (MBDs). MBD proteins then recruit additional proteins to the locus, such as histone deacetylases and other chromatin remodelling proteins that can modify histones, thereby forming compact, inactive chromatin termed silent chromatin. This link between DNA methylation and chromatin structure is very important. In particular, loss of Methyl-CpG-binding Protein 2 (MeCP2) has been implicated in Rett syndrome and Methyl-CpG binding domain protein 2 (MBD2) mediates the transcriptional silencing of hypermethylated genes in cancer.

In humans, the process of DNA methylation is carried out by three enzymes, DNA methyltransferase 1, 3a and 3b (DNMT1, DNMT3a, DNMT3b). It is thought that DNMT3a and DNMT3b are the *de novo* methyltransferases that set up DNA methylation patterns early in development. DNMT1 is the proposed maintenance methyltransferase that is responsible for copying DNA methylation patterns to the daughter strands during DNA replication. DNMT3L is a protein that is homologous to the other DNMT3s but has no catalytic activity. Instead, DNMT3L assists the *de novo* methyltransferases by increasing their ability to bind to DNA and stimulating their activity. Finally, DNMT2 has been identified as an "enigmatic" DNA methylstransferase homolog, containing all 10 sequence motifs common to all DNA methyltransferases; however, DNMT2 may not methylate DNA but instead has been shown to methylate a small RNA.

"Methylation status" should therefore be understood as a reference to the presence, absence and/or quantity of methylation at a particular nucleotide, or nucleotides, within a DNA region. The methylation status of a particular DNA sequence (e.g. DNA region as described herein) can indicate the methylation state of every base in the sequence or can indicate the methylation state of a subset of the base pairs (e.g., of cytosines or the methylation state of one or more specific restriction enzyme recognition sequences) within the sequence, or can indicate information regarding regional methylation density within the sequence without providing precise information of where in the sequence the methylation occurs. The methylation status can optionally be represented or indicated by a "methylation value." A methylation value can be generated, for example, by quantifying the amount of intact DNA present following restriction digestion with a methylation dependent restriction enzyme. In this example, if a particular sequence in the DNA is quantified using quantitative PCR, an amount of template DNA approximately equal to a mock treated control indicates the sequence is not highly methylated whereas an amount of template substantially less than occurs in the mock treated sample indicates the presence of methylated DNA at the sequence. Accordingly, a value, i.e., a methylation value, for example from the above described example, represents the methylation status and can thus be used as a quantitative indicator of the methylation status. This is of particular use when it is desirable to compare the methylation status of a sequence in a sample to a threshold value.

The method of the present invention is predicated on the comparison of the level of methylation of specific DNA regions of a biological sample with the control methylation levels of these DNA regions. The "control level" is the "normal level", which is the level of methylation of the DNA region of a corresponding large intestine cell or cellular population which is not neoplastic or in another biological sample from which DNA may be isolated for assay.

The normal (or "non-neoplastic") methylation level may be determined using non-neoplastic tissues derived from the same individual who is the subject of testing. However, it would be appreciated that this may be quite invasive for the individual concerned and it is therefore likely to be more convenient to analyse the test results relative to a standard result which reflects individual or collective results obtained from individuals other than the patient in issue. This latter form of analysis is in fact the preferred method of analysis since it enables the design of kits which require the collection and analysis of a single biological sample, being a test sample of interest. The standard results which provide the normal methylation level may be calculated by any suitable means which would be well known to the person of skill in the art. For example, a population of normal tissues can be assessed in terms of the level of methylation of the genes of the present invention, thereby providing a standard value or range of values against which all future test samples are analysed. It should also be understood that the normal level may be determined from the subjects of a specific cohort and for use with respect to test samples derived from that cohort. Accordingly, there may be determined a number of standard values or ranges which correspond to cohorts which differ in respect of characteristics such as age, gender, ethnicity or health status. Said "normal level" may be a discrete level or a range of levels. An increase in the methylation level of the subject genes relative to normal levels is indicative of the tissue being neoplastic.

The term "methylation" shall be taken to mean the presence of a methyl group added by the action of a DNA methyl transferase enzyme to a cytosine base or bases in a region of nucleic acid, e.g. genomic DNA. As described herein, there are several methods known to those skilled in the art for determining the level or degree of methylation of nucleic acid.

By "higher level" is meant that there are a higher number of methylated CpG dinucleotides in the subject diagnosed than in a control sample, that is, either the proportion of DNA molecules methylated at a particular CpG site is higher or there are a higher number of separate CpG sites methylated in the subject. It should be understood that the terms "enhanced" and "increased" are used interchangeably with the term "higher". The present invention is not to be limited by a precise number of methylated residues that are considered to be diagnostic of neoplasia in a subject, because some variation between patient samples will occur. The present invention is also not limited by positioning of the methylated residue.

Nevertheless, a number of specific cytosine residues which undergo hypermethylation within these subregions have also been identified. In another embodiment, therefore, a screening method can be employed which is specifically directed to assessing the methylation status of one or more of either these residues or the corresponding cytosine at position n+1 on the opposite DNA strand.

To this end, listed below are the cytosine residues which have been identified in this regard. It should be appreciated by the person of skill in the art that these individual residues are numbered by reference to Hg19, which also corresponds to the numbering of the specific subregions listed hereinbefore and which can be further identified when the coordinate numbering for each subregion is applied to the corresponding subregion sequences which are provided in the sequence listing. It should be understood that these residues have been identified in the context of the subregion DNA. However, here are other residues which are hypermethylated outside the subregions themselves but within the larger DNA region from which the subregions derive. Accordingly, these specified residues represent a particularly useful subset of individual cytosine residues which undergo hypermethylation within the context of the DNA regions and subregions herein disclosed. These individual residues are grouped below according to the DNA region within which they occur. These DNA regions are identified by reference to both the Hg19 chromosomal coordinates and the gene region name.

According to this embodiment there is therefore provided a method of screening for the onset or predisposition to the onset of or monitoring a large intestine neoplasm in an individual, said method comprising assessing the methylation of one or more cytosine residues selected from:
*Chr12:52400748-52409671 (GRASP)*

| | | | |
|---|---|---|---|
| Chr12:52399713 | Chr12:52399731 | Chr12:52399749 | Chr12:52399783 |
| Chr12:52399796 | Chr12:52399808 | Chr12:52399823 | Chr12:52399835 |
| Chr12:52399891 | | | |
| | | | |
| Chr12:52400847 | Chr12:52400850 | Chr12:52400859 | Chr12:52400866 |
| Chr12:52400869 | Chr12:52400873 | Chr12:52400881 | Chr12:52400886 |
| Chr12:52400893 | Chr12:52400895 | Chr12:52400899 | Chr12:52400902 |
| Chr12:52400907 | Chr12:52400913 | Chr12:52400919 | Chr12:52400932 |
| Chr12:52400938 | Chr12:52400958 | Chr12:52400962 | Chr12:52400971 |
| Chr12:52400973 | Chr12:52400976 | Chr12:52400998 | Chr12:52401008 |
| Chr12:52401010 | Chr12:52401012 | Chr12:52401016 | Chr12:52401019 |
| Chr12:52401025 | Chr12:52401041 | Chr12:52401044 | Chr12:52401053 |
| Chr12:52401060 | Chr12:52401064 | Chr12:52401092 | Chr12:52401118 |
| | | | |
| Chr12:52401438 | Chr12:52401448 | Chr12:52401460 | Chr12:52401465 |
| Chr12:52401474 | Chr12:52401477 | Chr12:52401479 | Chr12:52401483 |
| Chr12:52401504 | Chr12:52401514 | Chr12:52401523 | Chr12:52401540 |
| Chr12:52401553 | Chr12:52401576 | Chr12:52401588 | Chr12:52401595 |
| Chr12:52401599 | Chr12:52401604 | Chr12:52401606 | Chr12:52401634 |
| Chr12:52401640 | Chr12:52401644 | Chr12:52401659 | |

*Chr8:97505882-97624037 (SDC2)*

| | | | |
|---|---|---|---|
| Chr8:97506843 | Chr8:97506845 | Chr8:97506861 | Chr8:97506876 |
| Chr8:97506902 | Chr8:97506909 | Chr8:97506911 | Chr8:97506913 |
| Chr8:97506924 | Chr8:97506932 | Chr8:97506934 | Chr8:97506940 |
| Chr8:97506977 | Chr8:97506993 | Chr8:97507005 | Chr8:97507008 |
| Chr8:97507014 | | | |
| | | | |
| Chr8:97507038 | Chr8:97507061 | Chr8:97507064 | Chr8:97507078 |
| Chr8:97507082 | Chr8:97507084 | Chr8:97507089 | Chr8:97507119 |
| Chr8:97507128 | Chr8:97507136 | Chr8:97507144 | Chr8:97507153 |
| Chr8:97507162 | Chr8:97507214 | Chr8:97507216 | |

*Chr13:110959094-110959330 (COL4A1).*

| | | | |
|---|---|---|---|
| Chr13:97507038 | Chr13:97507061 | Chr13:97507064 | Chr13:97507078 |
| Chr13:97507082 | Chr13:97507084 | Chr13:97507089 | Chr13:97507119 |
| Chr13:97507128 | Chr13:97507136 | Chr13:97507144 | Chr13:97507153 |
| Chr13:97507162 | Chr13:97507214 | Chr13:97507216 | |

*Chr5:3596168-3601517 (IRX1)*

| | | | |
|---|---|---|---|
| Chr5:3597229 | Chr5:3597299 | Chr5:3597311 | Chr5:3597321 |
| Chr5:3597330 | Chr5:3597350 | Chr5:3597352 | Chr5:3597373 |
| Chr5:3597413 | Chr5:3597427 | Chr5:3597431 | Chr5:3597447 |
| Chr5:3597450 | Chr5:3597454 | Chr5:3597456 | |
| | | | |
| Chr5:3599998 | Chr5:3600019 | Chr5:3600037 | Chr5:3600050 |
| Chr5:3600058 | Chr5:3600061 | Chr5:3600070 | Chr5:3600074 |
| Chr5:3600076 | Chr5:3600079 | Chr5:3600089 | Chr5:3600091 |
| Chr5:3600104 | Chr5:3600116 | Chr5:3600121 | Chr5:3600124 |
| Chr5:3600131 | Chr5:3600143 | Chr5:3600149 | Chr5:3600169 |
| Chr5:3600173 | | | |
| | | | |
| Chr5:3600163 | Chr5:3600190 | Chr5:3600201 | Chr5:3600205 |
| Chr5:3600208 | Chr5:3600214 | Chr5:3600250 | Chr5:3600252 |
| Chr5:3600255 | Chr5:3600264 | Chr5:3600270 | Chr5:3600284 |
| Chr5:3600309 | Chr5:3600318 | Chr5:3600345 | |
| | | | |
| Chr5:3594663 | Chr5:3594679 | Chr5:3594684 | Chr5:3594697 |
| Chr5:3594703 | Chr5:3594711 | Chr5:3594725 | Chr5:3594738 |
| Chr5:3594745 | Chr5:3594758 | Chr5:3594768 | Chr5:3594774 |
| Chr5:3594778 | Chr5:3594794 | Chr5:3594799 | Chr5:3594818 |
| Chr5:3594840 | | | |

*Chr13:95361879-95364389 (SOX21)*

| | | | |
|---|---|---|---|
| Chr13:95364016 | Chr13:95364019 | Chr13:95364042 | Chr13:95364050 |
| Chr13:95364054 | Chr13:95364061 | Chr13:95364064 | Chr13:95364074 |
| Chr13:95364080 | Chr13:95364082 | Chr13:95364106 | Chr13:95364119 |
| Chr13:95364123 | Chr13:95364125 | Chr13:95364128 | Chr13:95364141 |
| Chr13:95364163 | Chr13:95364171 | | |
| | | | |
| Chr13:95364543 | Chr13:95364545 | Chr13:95364548 | Chr13:95364551 |
| Chr13:95364560 | Chr13:95364571 | Chr13:95364582 | Chr13:95364584 |
| Chr13:95364587 | Chr13:95364602 | Chr13:95364623 | Chr13:95364627 |
| Chr13:95364640 | Chr13:95364649 | Chr13:95364653 | Chr13:95364656 |
| Chr13:95364665 | Chr13:95364684 | Chr13:95364696 | Chr13:95364712 |
| Chr13:95364729 | Chr13:95364732 | Chr13:95364734 | Chr13:95364748 |
| Chr13:95364751 | | | |

*Chr4:81187742-81212171 (FGF5)*

| | | | |
|---|---|---|---|
| Chr4:81186919 | Chr4:81186947 | Chr4:81186964 | Chr4:81186968 |
| Chr4:81186973 | Chr4:81186975 | Chr4:81186983 | Chr4:81187001 |
| Chr4:81187011 | Chr4:81187041 | Chr4:81187056 | Chr4:81187062 |
| Chr4:81187093 | Chr4:81187116 | Chr4:81187125 | Chr4:81187161 |
| Chr4:81187191 | Chr4:81187196 | Chr4:81187198 | |
| | | | |
| Chr4:81187367 | Chr4:81187389 | Chr4:81187458 | Chr4:81187467 |
| Chr4:81187495 | Chr4:81187498 | Chr4:81187504 | Chr4:81187512 |
| Chr4:81187514 | Chr4:81187530 | Chr4:81187539 | Chr4:81187547 |
| Chr4:81187549 | Chr4:81187551 | Chr4:81187554 | Chr4:81187556 |
| Chr4:81187575 | | | |
| | | | |
| Chr4:81187575 | Chr4:81187592 | Chr4:81187601 | Chr4:81187605 |
| Chr4:81187610 | Chr4:81187648 | Chr4:81187652 | Chr4:81187679 |
| Chr4:81187685 | Chr4:81187691 | Chr4:81187693 | Chr4:81187707 |
| Chr4:81187712 | Chr4:81187733 | Chr4:81187735 | Chr4:81187752 |
| Chr4:81187758 | Chr4:81187764 | Chr4:81187784 | |

*Chr19:57019212-57040269 (ZNF471)*

| | | | |
|---|---|---|---|
| Chr19:57018990 | Chr19:57018994 | Chr19:57019003 | Chr19:57019010 |
| Chr19:57019018 | Chr19:57019020 | Chr19:57019025 | Chr19:57019029 |
| Chr19:57019044 | Chr19:57019047 | Chr19:57019067 | Chr19:57019073 |
| Chr19:57019084 | Chr19:57019101 | Chr19:57019118 | |
| | | | |
| Chr19:57019315 | Chr19:57019321 | Chr19:57019346 | Chr19:57019351 |
| Chr19:57019355 | Chr19:57019361 | Chr19:57019364 | Chr19:57019366 |
| Chr19:57019371 | Chr19:57019373 | Chr19:57019385 | Chr19:57019387 |
| Chr19:57019405 | Chr19:57019428 | Chr19:57019433 | Chr19:57019435 |
| Chr19:57019437 | Chr19:57019443 | Chr19:57019451 | Chr19:57019456 |
| Chr19:57019463 | Chr19:57019465 | Chr19:57019470 | Chr19:57019483 |
| Chr19:57019487 | Chr19:57019492 | Chr19:57019502 | Chr19:57019505 |

*Chr3:33191537-33260707 (SUSD5)*

| | | | |
|---|---|---|---|
| Chr3:33260601 | Chr3:33260621 | Chr3:33260631 | Chr3:33260640 |
| Chr3:33260651 | Chr3:33260665 | Chr3:33260676 | Chr3:33260694 |
| Chr3:33260698 | Chr3:33260711 | Chr3:33260715 | Chr3:33260732 |
| Chr3:33260742 | Chr3:33260748 | Chr3:33260755 | Chr3:33260760 |
| Chr3:33260769 | Chr3:33260776 | Chr3:33260778 | Chr3:33260780 |
| Chr3:33260788 | Chr3:33260806 | | |

*Chr2:56093097-56151298 (EFEMP1)*

| | | | |
|---|---|---|---|
| Chr2:56150376 | Chr2:56150389 | Chr2:56150394 | Chr2:56150415 |
| Chr2:56150419 | Chr2:56150423 | Chr2:56150433 | Chr2:56150473 |
| Chr2:56150475 | Chr2:56150478 | Chr2:56150499 | Chr2:56150537 |
| Chr2:56150549 | Chr2:56150580 | Chr2:56150601 | |

*Chr6:134210259-134216675 (TCF21)*

| | | | |
|---|---|---|---|
| Chr6:134210556 | Chr6:134210598 | Chr6:134210615 | Chr6:134210640 |
| Chr6:134210649 | Chr6:134210667 | Chr6:134210692 | Chr6:134210694 |
| Chr6:134210697 | Chr6:134210720 | Chr6:134210745 | |
| | | | |
| Chr6:134210720 | Chr6:134210745 | Chr6:134210776 | Chr6:134210781 |
| Chr6:134210784 | Chr6:134210790 | Chr6:134210792 | Chr6:134210794 |
| Chr6:134210800 | Chr6:134210806 | Chr6:134210812 | Chr6:134210868 |
| Chr6:134210894 | Chr6:134210906 | Chr6:134210919 | Chr6:134210946 |
| | | | |
| Chr6:134211050 | Chr6:134211061 | Chr6:134211076 | Chr6:134211081 |
| Chr6:134211103 | Chr6:134211110 | Chr6:134211121 | Chr6:134211125 |
| Chr6:134211131 | Chr6:134211153 | Chr6:134211155 | Chr6:134211162 |
| Chr6:134211179 | Chr6:134211182 | Chr6:134211184 | Chr6:134211208 |
| Chr6:134211210 | Chr6:134211212 | Chr6:134211218 | Chr6:134211220 |
| Chr6:134211227 | Chr6:134211233 | Chr6:134211241 | Chr6:134211245 |
| Chr6:134211247 | Chr6:134211270 | | |

*Chr7:94023873-94060544 (COL1A2)*

| | | | |
|---|---|---|---|
| Chr7:94024172 | Chr7:94024191 | Chr7:94024214 | Chr7:94024230 |
| Chr7:94024254 | Chr7:94024266 | Chr7:94024268 | Chr7: 94024272 |
| Chr7:94024288 | Chr7:94024291 | Chr7:94024310 | |

*Chr15:60296421-60298142 (FOXB1)*

| | | | |
|---|---|---|---|
| Chr15:60296555 | Chr15:60296561 | Chr15:60296563 | Chr15:60296578 |
| Chr15:60296585 | Chr15:60296598 | Chr15:60296601 | Chr15:60296614 |
| Chr15:60296616 | Chr15:60296619 | Chr15:60296627 | Chr15:60296633 |
| Chr15:60296639 | Chr15:60296643 | Chr15:60296647 | Chr15:60296654 |
| Chr15:60296665 | Chr15:60296668 | Chr15:60296670 | Chr15:60296675 |
| Chr15:60296679 | Chr15:60296684 | Chr15:60296689 | Chr15:60296694 |
| | | | |
| Chr15:60297035 | Chr15:60297050 | Chr15:60297053 | Chr15:60297109 |
| Chr15:60297118 | Chr15:60297121 | Chr15:60297126 | Chr15:60297128 |
| Chr15:60297130 | Chr15:60297152 | Chr15:60297169 | Chr15:60297174 |
| Chr15:60297178 | Chr15:60297185 | Chr15:60297192 | Chr15:60297203 |
| Chr15:60297212 | Chr15:60297221 | Chr15:60297228 | Chr15:60297252 |
| Chr15:60297266 | Chr15:60297273 | Chr15:60297298 | |

*Chr16:86544133-86548070 (FOXF1)*

| | | | |
|---|---|---|---|
| Chr16:86544571 | Chr16:86544587 | Chr16:86544590 | Chr16:86544593 |
| Chr16:86544597 | Chr16:86544599 | Chr16:86544601 | Chr16:86544608 |
| Chr16:86544624 | Chr16:86544652 | Chr16:86544658 | Chr16:86544675 |
| Chr16:86544685 | Chr16:86544699 | Chr16:86544703 | Chr16:86544706 |
| Chr16:86544714 | Chr16:86544720 | Chr16:86544735 | Chr16:86544745 |
| Chr16:86544763 | | | |
| | | | |
| Chr16:86544268 | Chr16:86544273 | Chr16:86544295 | Chr16:86544298 |
| Chr16:86544305 | Chr16:86544308 | Chr16:86544312 | Chr16:86544321 |
| Chr16:86544337 | Chr16:86544339 | Chr16:86544346 | Chr16:86544377 |
| Chr16:86544384 | Chr16:86544391 | Chr16:86544416 | Chr16:86544431 |
| Chr16:86544460 | Chr16:86544464 | Chr16:86544477 | Chr16:86544484 |
| Chr16:86544518 | Chr16:86544523 | Chr16:86544547 | Chr16:86544552 |
| Chr16:86544559 | Chr16:86544571 | | |
| Chr16:86544810 | Chr16:86544832 | Chr16:86544835 | Chr16:86544843 |
| Chr16:86544853 | Chr16:86544859 | Chr16:86544862 | Chr16:86544865 |
| Chr16:86544867 | Chr16:86544870 | Chr16:86544874 | Chr16:86544877 |
| Chr16:86544885 | Chr16:86544892 | Chr16:86544900 | Chr16:86544907 |
| Chr16:86544915 | Chr16:86544928 | Chr16:86544931 | Chr16:86544934 |
| Chr16:86544951 | Chr16:86544955 | Chr16:86544958 | Chr16:86544966 |
| Chr16:86544972 | Chr16:86544975 | Chr16:86544978 | Chr16:86544987 |
| Chr16:86544993 | Chr16:86544996 | Chr16:86545000 | Chr16:86545002 |
| Chr16:86545005 | Chr16:86545015 | Chr16:86545018 | Chr16:86545060 |
| Chr16:86545062 | Chr16:86545078 | Chr16:86545092 | |

*Chr13:28494168-28500451 (PDXI)*

| | | | |
|---|---|---|---|
| Chr13:28502109 | Chr13:28502153 | Chr13:28502161 | Chr13:28502177 |
| Chr13:28502179 | Chr13:28502191 | Chr13:28502198 | Chr13:28502205 |
| Chr13:28502207 | Chrl3:28502210 | Chr13:28502245 | Chr13:28502309 |
| | | | |
| Chr13:28502442 | Chr13:28502449 | Chr13:28502461 | Chr13:28502464 |
| Chr13:28502475 | Chr13:28502507 | Chr13:28502512 | Chr13:28502538 |
| Chr13:28502544 | Chr13:28502549 | Chr13:28502559 | Chr13:28502564 |
| Chr13:28502585 | | | |
| | | | |
| Chr13:28503045 | Chr13:28503049 | Chr13:28503081 | Chr13:28503099 |
| Chr13:28503114 | Chr13:28503127 | Chr13:28503138 | Chr13:28503147 |
| Chr13:28503155 | Chr13:28503157 | Chr13:28503179 | |

*Chr7:96649702-96654143 (DLX5)*

| | | | |
|---|---|---|---|
| Chr7:96650062 | Chr7:96650072 | Chr7:96650078 | Chr7:96650096 |
| Chr7:96650099 | Chr7:96650102 | | |
| | | | |
| Chr7:96651485 | Chr7:96651488 | Chr7:96651518 | Chr7:96651523 |
| Chr7:96651532 | Chr7:96651535 | Chr7:96651537 | Chr7:96651542 |
| Chr7:96651550 | Chr7:96651586 | | |
| | | | |
| Chr7:96653596 | Chr7:96653605 | Chr7:96653607 | Chr7:96653617 |
| Chr7:96653620 | Chr7:96653623 | Chr7:96653644 | Chr7:96653656 |
| Chr7:96653683 | Chr7:96653686 | Chr7:96653692 | Chr7:96653698 |
| Chr7:96653701 | | | |

*Chr21:28208606-28217728 (ADAMTS1)*

| | | | |
|---|---|---|---|
| Chr21:28217973 | Chr21:28218002 | Chr21:28218015 | Chr21:28218018 |
| Chr21:28218047 | Chr21:28218051 | Chr21:28218057 | Chr21:28218072 |
| Chr21:28218074 | Chr21:28218084 | Chr21:28218105 | |
| | | | |
| Chr21:28218514 | Chr21:28218516 | Chr21:28218550 | Chr21:28218568 |
| Chr21:28218579 | Chr21:28218586 | Chr21:28218596 | Chr21:28218635 |
| Chr21:2821863 8 | Chr21:28218646 | Chr21:28218671 | Chr21:28218684 |
| Chr21:28218688 | Chr21:28218704 | Chr21:28218729 | Chr21:28218741 |
| | | | |
| Chr21:28218893 | Chr21:28218906 | Chr21:28218914 | Chr21:28218916 |
| Chr21:28218928 | Chr21:28218934 | Chr21:2821893 8 | Chr21:28218949 |
| Chr21:28218953 | Chr21:28218959 | Chr21:28218974 | Chr21:28218976 |
| Chr21:28218978 | Chr21:28218984 | Chr21:28218986 | Chr21:28218996 |
| Chr21:28219008 | Chr21:28219016 | | |

*Chr13:110959631-111165373 (COL4A2)*

| | | | |
|---|---|---|---|
| Chr13:110960813 | Chr13:110960827 | Chr13:110960849 | Chr13:110960875 |
| Chr13:110960925 | Chr13:110960930 | Chr13:110960938 | Chr13:110960978 |
| Chr13:110961001 | Chr13:110961003 | Chr13:110961025 | Chr13:110961045 |
| Chr13:110961049 | Chr13:110961069 | Chr13:110961072 | Chr13:110961088 |
| Chr13:110961091 | Chr13:110961095 | Chr13:110961110 | Chr13:110961116 |
| Chr13:110961131 | Chr13:110960786 | | |
| | | | |
| Chr13:110961358 | Chr13:110961386 | Chr13:110961404 | Chr13:110961406 |
| Chr13:110961445 | Chr13:110961579 | Chr13:110961594 | Chr13:110961606 |
| Chr13:110961631 | | | |
| | | | |
| Chr13:110959935 | Chr13:110959965 | Chr13:110959968 | Chr13:110959974 |
| Chr13:110959979 | Chr13:110959981 | Chr13:110959986 | Chr13:110959988 |
| Chr13:110959997 | Chr13:110959999 | Chr13:110960004 | Chr13:110960006 |
| Chr13:110960011 | Chr13:110960027 | Chr13:110960035 | Chr13:110960046 |
| Chr13:110960051 | Chr13:110960071 | Chr13:110960099 | Chr13:110960108 |
| Chr13:110960115 | Chr13:110960156 | Chr13:110960180 | |

*Chr5:83238126-83680611 (EDIL3)*

| | | | |
|---|---|---|---|
| Chr5:83679825 | Chr5:83679827 | Chr5:83679836 | Chr5:83679843 |
| Chr5:83679846 | Chr5:83679860 | Chr5:83679864 | Chr5:83679883 |
| Chr5:83679894 | Chr5:83679900 | Chr5:83679906 | Chr5:83679910 |
| Chr5:83679937 | Chr5:83679953 | Chr5:83679957 | Chr5:83679961 |
| Chr5:83679976 | Chr5:83679982 | Chr5:83679987 | Chr5:83680003 |
| Chr5:83680007 | Chr5:83680011 | Chr5:83680026 | Chr5:83680032 |
| Chr5:83680086 | Chr5:83680090 | Chr5:83680103 | Chr5:83680106 |
| Chr5:83680122 | Chr5:83680126 | | |
| | | | |
| Chr5:83679546 | Chr5:83679568 | Chr5:83679571 | Chr5:83679585 |
| Chr5:83679602 | Chr5:83679617 | Chr5:83679630 | Chr5:83679640 |
| Chr5:83679667 | Chr5:83679675 | Chr5:83679689 | Chr5:83679693 |
| Chr5:83679699 | Chr5:83679701 | Chr5:83679733 | |
| | | | |
| Chr5:83679973 | Chr5:83679989 | Chr5:83679992 | Chr5:83680001 |
| Chr5:83680003 | Chr5:83680006 | Chr5:83680012 | Chr5:83680015 |
| Chr5:83680019 | Chr5:83680033 | Chr5:83680036 | Chr5:83680045 |
| Chr5:83680051 | Chr5:83680061 | Chr5:83680063 | Chr5:83680066 |
| Chr5:83680108 | Chr5:83680115 | Chr5:83680118 | Chr5:83680132 |
| Chr5:83680147 | Chr5:83680159 | Chr5:83680174 | Chr5:83680181 |
| Chr5:83680185 | Chr5:83680198 | Chr5:83680205 | Chr5:83680209 |
| Chr5:83680217 | Chr5:83680232 | Chr5:83680235 | Chr5:83680237 |
| Chr5:83680253 | | | |
| | | | |
| Chr5:83680108 | Chr5:83680115 | Chr5:83680118 | Chr5:83680132 |
| Chr5:83680147 | Chr5:83680159 | Chr5:83680174 | Chr5:83680181 |
| Chr5:83680185 | Chr5:83680198 | Chr5:83680205 | Chr5:83680209 |
| Chr5:83680217 | Chr5:83680232 | Chr5:83680235 | Chr5:83680237 |
| Chr5:83680253 | Chr5:83680264 | Chr5:83680284 | Chr5:83680310 |
| Chr5:83680326 | Chr5:83680355 | | |
| | | | |
| Chr5:83680401 | Chr5:83680406 | Chr5:83680409 | Chr5:83680411 |
| Chr5:83680413 | Chr5:83680430 | Chr5:83680438 | Chr5:83680441 |
| Chr5:83680444 | Chr5:83680458 | Chr5:83680463 | Chr5:83680474 |
| Chr5:83680483 | Chr5:83680485 | Chr5:83680488 | Chr5:83680492 |
| Chr5:83680497 | Chr5:83680499 | Chr5:83680507 | Chr5:83680509 |
| Chr5:83680513 | Chr5:83680518 | Chr5:83680529 | Chr5:83680531 |
| Chr5:83680548 | Chr5:83680559 | Chr5:83680564 | Chr5:83680568 |
| Chr5:83680572 | Chr5:83680579 | Chr5:83680589 | Chr5:83680591 |
| Chr5:83680593 | Chr5:83680596 | Chr5:83680602 | |

*Chr15:48700503-48937985 (FBN1)*

| | | | |
|---|---|---|---|
| Chr15:48938149 | Chr15:48938163 | Chr15:48938172 | Chr15:48938181 |
| Chr15:48938183 | Chr15:48938218 | Chr15:48938236 | Chr15:48938238 |
| Chr15:48938254 | Chr15:48938267 | Chr15:48938273 | Chr15:48938280 |
| Chr15:48938291 | Chr15:48938349 | | |

*Chrl:47901689-47906363 (FOXD2)*

| | | | |
|---|---|---|---|
| Chr1:47899102 | Chr1:47899126 | Chr1:47899129 | Chr1:47899143 |
| Chr1:47899152 | Chr1:47899168 | Chr1:47899184 | Chr1:47899205 |
| Chr1:47899212 | Chr1:47899230 | Chr1:47899232 | Chr1:47899253 |
| Chr1:47899286 | Chr1:47899327 | | |
| | | | |
| Chr1:47909988 | Chr1:47909995 | Chr1:47910005 | Chr1:47910036 |
| Chr1:47910051 | Chr1:47910056 | Chr1:47910062 | Chr1:47910074 |
| Chr1:47910103 | Chr1:47910122 | Chr1:47910137 | Chr1:47910142 |
| Chr1:47910144 | Chr1:47910146 | | |

*Chr2:66662532-66799891 (MEIS1)*

| | | | |
|---|---|---|---|
| Chr2:66662043 | Chr2:66662047 | Chr2:66662049 | Chr2:66662063 |
| Chr2:66662073 | Chr2:66662107 | Chr2:66662111 | Chr2:66662124 |
| Chr2:66662145 | Chr2:66662156 | Chr2:66662161 | Chr2:66662163 |
| Chr2:66662178 | Chr2:66662218 | | |
| | | | |
| Chr2:66662178 | Chr2:66662218 | Chr2:66662220 | Chr2:66662246 |
| Chr2:66662265 | Chr2:66662280 | Chr2:66662306 | Chr2:66662310 |
| Chr2:66662323 | Chr2:66662333 | Chr2:66662337 | |

*Chr16:55513081-55540586 (MMP2)*

| | | | |
|---|---|---|---|
| Chr16:55512695 | Chr16:55512711 | Chr16:55512732 | Chr16:55512750 |
| Chr16:55512753 | Chr16:55512793 | | |
| | | | |
| Chr16:55513961 | Chr16:55513997 | Chr16:55514025 | Chr16:55514059 |
| Chr16:55514061 | Chr16:55514092 | Chr16:55514120 | Chr16:55514131 |
| Chr16:55514158 | Chr16:55514205 | | |

*Chr7:24323807-24331484 (NPY)*

| | | | |
|---|---|---|---|
| Chr7:24323767 | Chr7:24323792 | Chr7:24323794 | Chr7:24323799 |
| Chr7:24323817 | Chr7:24323834 | Chr7:24323840 | Chr7:24323844 |
| Chr7:24323848 | Chr7:24323866 | Chr7:24323876 | Chr7:24323880 |
| Chr7:24323882 | Chr7:24323884 | Chr7:24323894 | Chr7:24323905 |
| Chr7:24323910 | Chr7:24323930 | Chr7:24323934 | |
| Chr7:24324180 | Chr7:24324186 | Chr7 :24324206 | Chr7:24324217 |
| Chr7:24324222 | Chr7:24324225 | Chr7 :24324244 | Chr7 :24324249 |
| Chr7:24324251 | Chr7:24324268 | Chr7:24324274 | Chr7 :24324280 |
| Chr7:24324311 | Chr7:24324313 | | |
| | | | |
| Chr7:24324549 | Chr7:24324570 | Chr7 :24324592 | Chr7 :24324595 |
| Chr7:24324597 | Chr7:24324599 | Chr7:24324621 | Chr7 :24324625 |
| Chr7:24324633 | Chr7:24324635 | Chr7 :24324668 | Chr7 :24324672 |
| Chr7:24324676 | Chr7:24324679 | Chr7 :24324683 | Chr7 :24324690 |

*Chr19:38741877-38747172 (PPP1R14A)*

| | | | |
|---|---|---|---|
| Chr19:38747280 | Chr19:38747296 | Chr19:38747300 | Chr19:38747313 |
| Chr19:38747319 | Chr19:38747322 | Chr19:38747328 | Chr19:38747334 |
| Chr19:38747355 | Chr19:38747361 | Chr19:38747371 | Chr19:38747382 |
| Chr19:38747389 | Chrl9:38747410 | | |
| | | | |
| Chr19:38746654 | Chr19:38746680 | Chr19:38746690 | Chr19:38746701 |
| Chr19:38746715 | Chr19:38746726 | Chr19:38746728 | Chr19:38746732 |
| Chr19:38746749 | Chr19:38746759 | Chr19:38746770 | Chr19:38746784 |
| Chr19:38746786 | Chr19:38746804 | Chr19:38746808 | Chr19:38746811 |
| Chr19:38746826 | Chr19:38746834 | Chr19:38746837 | Chr19:38746849 |
| Chr19:38746852 | Chr19:38746865 | Chr19:38746871 | Chr19:38746873 |
| Chr19:38746875 | Chr19:38746877 | Chr19:38746882 | Chr19:38746898 |

or a corresponding cytosine at position n+1 on the opposite DNA strand, in a biological sample from said individual wherein a higher level of methylation of one or more of said residues relative to the methylation level of a corresponding residue in a control sample is indicative of a large intestine neoplasm or a predisposition to the onset of a neoplastic state.

In another embodiment said one or more cytosine residues are selected from GRASP, SDC2, COL4A1, IRX1, SOX21, FGF5, ZNF471, SUSD5, EFEMP1, TCF21, COL1A2, FOXB1, FOXF1, PDX1 and DLX5.

In one particular embodiment said one or more cytosine residues are selected from:
*Chr12:52400748-52409671 (GRASP)*

| | | | |
|---|---|---|---|
| Chr12:52399713 | Chr12:52399731 | Chr12:52399749 | Chr12:52399783 |
| Chr12:52399796 | Chr12:52399808 | Chr12:52399823 | Chr12:52399835 |
| Chr12:52399891 | | | |
| | | | |
| Chr12:52400847 | Chr12:52400850 | Chr12:52400859 | Chr12:52400866 |
| Chr12:52400869 | Chr12:52400873 | Chr12:52400881 | Chr12:52400886 |
| Chr12:52400893 | Chr12:52400895 | Chr12:52400899 | Chr12:52400902 |
| Chr12:52400907 | Chr12:52400913 | Chr12:52400919 | Chr12:52400932 |
| Chr12:52400938 | Chr12:52400958 | Chr12:52400962 | Chr12:52400971 |
| Chr12:52400973 | Chr12:52400976 | Chr12:52400998 | Chr12:52401008 |
| Chr12:52401010 | Chr12:52401012 | Chr12:52401016 | Chr12:52401019 |
| Chr12:52401025 | Chr12:52401041 | Chr12:52401044 | Chr12:52401053 |
| Chr12:52401060 | Chr12:52401064 | Chr12:52401092 | Chr12:52401118 |

*Chr8:97505882-97624037 (SDC2)*

| | | | |
|---|---|---|---|
| Chr8:97506843 | Chr8:97506845 | Chr8:97506861 | Chr8:97506876 |
| Chr8:97506902 | Chr8:97506909 | Chr8:97506911 | Chr8:97506913 |
| Chr8:97506924 | Chr8:97506932 | Chr8:97506934 | Chr8:97506940 |
| Chr8:97506977 | Chr8:97506993 | Chr8:97507005 | Chr8:97507008 |
| Chr8:97507014 | | | |
| | | | |
| Chr8:97507038 | Chr8:97507061 | Chr8:97507064 | Chr8:97507078 |
| Chr8:97507082 | Chr8:97507084 | Chr8:97507089 | Chr8:97507119 |
| Chr8:97507128 | Chr8:97507136 | Chr8:97507144 | Chr8:97507153 |
| Chr8:97507162 | Chr8:97507214 | Chr8:97507216 | |

*Chr13:110959094-110959330 (COL4A1).*

| | | | |
|---|---|---|---|
| Chr13:97507038 | Chr13:97507061 | Chr13:97507064 | Chr13:97507078 |
| Chr13:97507082 | Chr13:97507084 | Chr13:97507089 | Chr13:97507119 |
| Chr13:97507128 | Chr13:97507136 | Chr13:97507144 | Chr13:97507153 |
| Chr13:97507162 | Chr13:97507214 | Chr13:97507216 | |
| Chr5:3594663 | Chr5:3594679 | Chr5:3594684 | Chr5:3594697 |
| Chr5:3594703 | Chr5:3594711 | Chr5:3594725 | Chr5:3594738 |
| Chr5:3594745 | Chr5:3594758 | Chr5:3594768 | Chr5:3594774 |
| Chr5:3594778 | Chr5:3594794 | Chr5:3594799 | Chr5:3594818 |
| Chr5:3594840 | | | |

*Chr13:95361879-95364389 (SOX21)*

| | | | |
|---|---|---|---|
| Chr13:95364016 | Chr13:95364019 | Chr13:95364042 | Chr13:95364050 |
| Chr13:95364054 | Chr13:95364061 | Chr13:95364064 | Chr13:95364074 |
| Chr13:95364080 | Chr13:95364082 | Chr13:95364106 | Chr13:95364119 |
| Chr13:95364123 | Chr13:95364125 | Chr13:95364128 | Chr13:95364141 |
| Chr13:95364163 | Chr13:95364171 | | |
| | | | |
| Chr13:95364543 | Chr13:95364545 | Chr13:95364548 | Chr13:95364551 |
| Chr13:95364560 | Chr13:95364571 | Chr13:95364582 | Chr13:95364584 |
| Chr13:95364587 | Chr13:95364602 | Chr13:95364623 | Chr13:95364627 |
| Chr13:95364640 | Chr13:95364649 | Chr13:95364653 | Chr13:95364656 |
| Chr13:95364665 | Chr13:95364684 | Chr13:95364696 | Chr13:95364712 |
| Chr13:95364729 | Chr13:95364732 | Chr13:95364734 | Chr13:95364748 |
| Chr13:95364751 | | | |

*Chr4:81187742-81212171 (FGF5)*

| | | | |
|---|---|---|---|
| Chr4:81186919 | Chr4:81186947 | Chr4:81186964 | Chr4:81186968 |
| Chr4:81186973 | Chr4:81186975 | Chr4:81186983 | Chr4:81187001 |
| Chr4:81187011 | Chr4:81187041 | Chr4:81187056 | Chr4:81187062 |
| Chr4:81187093 | Chr4:81187116 | Chr4:81187125 | Chr4:81187161 |
| Chr4:81187191 | Chr4:81187196 | Chr4:81187198 | |

*Chr19:57019212-57040269 (ZNF471)*

| | | | |
|---|---|---|---|
| Chr19:57018990 | Chr19:57018994 | Chr19:57019003 | Chr19:57019010 |
| Chr19:57019018 | Chr19:57019020 | Chr19:57019025 | Chr19:57019029 |
| Chr19:57019044 | Chr19:57019047 | Chr19:57019067 | Chr19:57019073 |
| Chr19:57019084 | Chr19:57019101 | Chr19:57019118 | |
| Chr19:57019315 | Chr19:57019321 | Chr19:57019346 | Chr19:57019351 |
| Chr19:57019355 | Chr19:57019361 | Chr19:57019364 | Chr19:57019366 |
| Chr19:57019371 | Chr19:57019373 | Chr19:57019385 | Chr19:57019387 |
| Chr19:57019405 | Chr19:57019428 | Chr19:57019433 | Chr19:57019435 |
| Chr19:57019437 | Chr19:57019443 | Chr19:57019451 | Chr19:57019456 |
| Chr19:57019463 | Chr19:57019465 | Chr19:57019470 | Chr19:57019483 |
| Chr19:57019487 | Chr19:57019492 | Chr19:57019502 | Chr19:57019505 |

*Chr3:33191537-33260707 (SUSD5)*

| | | | |
|---|---|---|---|
| Chr3:33260601 | Chr3:33260621 | Chr3:33260631 | Chr3:33260640 |
| Chr3:33260651 | Chr3:33260665 | Chr3:33260676 | Chr3:33260694 |
| Chr3:33260698 | Chr3:33260711 | Chr3:33260715 | Chr3:33260732 |
| Chr3:33260742 | Chr3:33260748 | Chr3:33260755 | Chr3:33260760 |
| Chr3:33260769 | Chr3:33260776 | Chr3:33260778 | Chr3:33260780 |
| Chr3:33260788 | Chr3:33260806 | | |

*Chr2:56093097-56151298 (EFEMP1)*

| | | | |
|---|---|---|---|
| Chr2:56150376 | Chr2:56150389 | Chr2:56150394 | Chr2:56150415 |
| Chr2:56150419 | Chr2:56150423 | Chr2:56150433 | Chr2:56150473 |
| Chr2:56150475 | Chr2:56150478 | Chr2:56150499 | Chr2:56150537 |
| Chr2:56150549 | Chr2:56150580 | Chr2:56150601 | |

*Chr6:134210259-134216675 (TCF21)*

| | | | |
|---|---|---|---|
| Chr6:134210556 | Chr6:134210598 | Chr6:134210615 | Chr6:134210640 |
| Chr6:134210649 | Chr6:134210667 | Chr6:134210692 | Chr6:134210694 |
| Chr6:134210697 | Chr6:134210720 | Chr6:134210745 | |
| | | | |
| Chr6:134210720 | Chr6:134210745 | Chr6:134210776 | Chr6:134210781 |
| Chr6:134210784 | Chr6:134210790 | Chr6:134210792 | Chr6:134210794 |
| Chr6:134210800 | Chr6:134210806 | Chr6:134210812 | Chr6:134210868 |
| Chr6:134210894 | Chr6:134210906 | Chr6:134210919 | Chr6:134210946 |

*Chr7:94023873-94060544 (COL1A2)*

| | | | |
|---|---|---|---|
| Chr7:94024172 | Chr7:94024191 | Chr7:94024214 | Chr7:94024230 |
| Chr7:94024254 | Chr7:94024266 | Chr7:94024268 | Chr7: 94024272 |
| Chr7:94024288 | Chr7:94024291 | Chr7:94024310 | |

*Chr15:60296421-60298142 (FOXB1*

| | | | |
|---|---|---|---|
| Chr15:60296555 | Chr15:60296561 | Chr15:60296563 | Chr15:60296578 |
| Chr15:60296585 | Chr15:60296598 | Chr15:60296601 | Chr15:60296614 |
| Chr15:60296616 | Chr15:60296619 | Chr15:60296627 | Chr15:60296633 |
| Chr15:60296639 | Chr15:60296643 | Chr15:60296647 | Chr15:60296654 |
| Chr15:60296665 | Chr15:60296668 | Chr15:60296670 | Chr15:60296675 |
| Chr15:60296679 | Chr15:60296684 | Chr15:60296689 | Chr15:60296694 |
| | | | |
| Chr15:60297035 | Chr15:60297050 | Chr15:60297053 | Chr15:60297109 |
| Chr15:60297118 | Chr15:60297121 | Chr15:60297126 | Chr15:60297128 |
| Chr15:60297130 | Chr15:60297152 | Chr15:60297169 | Chr15:60297174 |
| Chr15:60297178 | Chr15:60297185 | Chr15:60297192 | Chr15:60297203 |
| Chr15:60297212 | Chr15:60297221 | Chr15:60297228 | Chr15:60297252 |
| Chr15:60297266 | Chr15:60297273 | Chr15:60297298 | |

*Chr16:86544133-86548070 (FOXF1)*

| | | | |
|---|---|---|---|
| Chr16:86544268 | Chr16:86544273 | Chr16:86544295 | Chr16:86544298 |
| Chr16:86544305 | Chr16:86544308 | Chr16:86544312 | Chr16:86544321 |
| Chr16:86544337 | Chr16:86544339 | Chr16:86544346 | Chr16:86544377 |
| Chr16:86544384 | Chr16:86544391 | Chr16:86544416 | Chr16:86544431 |
| Chr16:86544460 | Chr16:86544464 | Chr16:86544477 | Chr16:86544484 |
| Chr16:86544518 | Chr16:86544523 | Chr16:86544547 | Chr16:86544552 |
| Chr16:86544559 | Chr16:86544571 | | |

*Chr13:28494168-28500451 (PDXI)*

| | | | |
|---|---|---|---|
| Chr13:28502109 | Chr13:28502153 | Chr13:28502161 | Chr13:28502177 |
| Chr13:28502179 | Chr13:28502191 | Chr13:28502198 | Chr13:28502205 |
| Chr13:28502207 | Chr13:28502210 | Chr13:28502245 | Chr13:28502309 |
| | | | |
| Chr13:28502442 | Chr13:28502449 | Chr13:28502461 | Chr13:28502464 |
| Chr13:28502475 | Chr13:28502507 | Chr13:28502512 | Chr13:28502538 |
| Chr13:28502544 | Chr13:28502549 | Chr13:28502559 | Chr13:28502564 |
| Chr13:28502585 | | | |

*Chr7:96649702-96654143 (DLX5)*

| | | | |
|---|---|---|---|
| Chr7:96653596 | Chr7:96653605 | Chr7:96653607 | Chr7:96653617 |
| Chr7:96653620 | Chr7:96653623 | Chr7:96653644 | Chr7:96653656 |
| Chr7:96653683 | Chr7:96653686 | Chr7:96653692 | Chr7:96653698 |
| Chr7:96653701 | | | |

The detection method of the present invention can be performed on any suitable biological sample. To this end, reference to a "biological sample" should be understood as a reference to any sample of biological material derived from an animal such as, but not limited to, cellular material, biofluids (eg. blood), faeces, tissue biopsy specimens, surgical specimens or fluid which has been introduced into the body of an animal and subsequently removed (such as, for example, the solution retrieved from an enema wash). The biological sample which is tested according to the method of the present invention may be tested directly or may require some form of treatment prior to testing. For example, a biopsy or surgical sample may require homogenisation prior to testing or it may require sectioning for *in situ* testing of the qualitative expression levels of individual genes. Alternatively, a cell sample may require permeabilisation prior to testing. Further, to the extent that the biological sample is not in liquid form, (if such form is required for testing) it may require the addition of a reagent, such as a buffer, to mobilise the sample.

To the extent that the DNA region of interest is present in a biological sample, the biological sample may be directly tested or else all or some of the nucleic acid present in the biological sample may be isolated prior to testing. In yet another example, the sample may be partially purified or otherwise enriched prior to analysis. For example, to the extent that a biological sample comprises a very diverse cell population, it may be desirable to enrich for a sub-population of particular interest. It is within the scope of the present invention for the target cell population or molecules derived therefrom to be treated prior to testing, for example, inactivation of live virus. It should also be understood that the biological sample may be freshly harvested or it may have been stored (for example by freezing) prior to testing or otherwise treated prior to testing (such as by undergoing culturing).

The choice of what type of sample is most suitable for testing in accordance with the method disclosed herein will be dependent on the nature of the situation. Preferably, said sample is a faecal (stool) sample, enema wash, surgical resection, tissue biopsy or blood sample (e.g. whole blood, serum or plasma).

More preferably, said biological sample is a blood sample, biopsy sample or stool sample.

As detailed hereinbefore, the present invention is designed to screen for a neoplastic cell or cellular population, which is located in the large intestine. Accordingly, reference to "cell or cellular population" should be understood as a reference to an individual cell or a group of cells. Said group of cells may be a diffuse population of cells, a cell suspension, an encapsulated population of cells or a population of cells which take the form of tissue.

Reference to the "onset" of a neoplasm, such as adenoma or adenocarcinoma, should be understood as a reference to one or more cells of that individual exhibiting dysplasia. In this regard, the adenoma or adenocarcinoma may be well developed in that a mass of dysplastic cells has developed. Alternatively, the adenoma or adenocarcinoma may be at a very early stage in that only relatively few abnormal cell divisions have occurred at the time of diagnosis. The present invention also extends to the assessment of an individual's predisposition to the development of a neoplasm, such as an adenoma or adenocarcinoma. Without limiting the present invention in any way, changed methylation levels may be indicative of that individual's predisposition to developing a neoplasia, such as the future development of an adenoma or adenocarcinoma or another adenoma or adenocarcinoma.

Although the preferred method is to assess methylation levels for the purpose of diagnosing neoplasia development or predisposition thereto, the detection of converse changes in the levels of said methylation may be desired under certain circumstances, for example, to monitor the effectiveness of therapeutic or prophylactic treatment directed to modulating a neoplastic condition, such as adenoma or adenocarcinoma development. For example, where elevated levels of methylation indicate that an individual has developed a condition characterised by adenoma or adenocarcinoma development, screening for a decrease in the levels of methylation subsequently to the onset of a therapeutic treatment regime may be utilised to indicate successful clearance of the neoplastic cells. In another example, one can use this method to test the tissue at the margins of a tumour resection in order to determine whether the full margin of the tumour has been removed.

The present method can therefore be used in the diagnosis, prognosis, classification, prediction of disease risk, detection of recurrence of disease, selection of treatment of a number of types of neoplasias and monitoring of neoplasias. A cancer at any stage of progression can be detected, such as primary, metastatic, and recurrent cancers.

The present invention provides methods for determining whether a mammal (e.g., a human) has a neoplasia of the large intestine, whether a biological sample taken from a mammal contains neoplastic cells or DNA derived from neoplastic cells, estimating the risk or likelihood of a mammal developing a neoplasm, monitoring the efficacy of anti-cancer treatment, or selecting the appropriate anti-cancer treatment in a mammal with cancer. Such methods are based on the determination that neoplastic cells have a different methylation status than normal cells in the DNA regions described herein. Accordingly, by determining whether or not a cell contains differentially methylated sequences in the DNA regions as described herein, it is possible to determine that a cell is neoplastic .

The method of the invention can be used to evaluate individuals known or suspected to have a neoplasia or as a routine clinical test, i.e., in an individual not necessarily suspected to have a neoplasia. Further diagnostic assays can be performed to confirm the status of neoplasia in the individual.

Further, the present methods may be used to assess the efficacy of a course of treatment. For example, the efficacy of an anti-cancer treatment can be assessed by monitoring DNA methylation of the sequences described herein over time in a mammal having cancer. For example, a reduction or absence of methylation in any of the diagnostic sequences of the invention in a biological sample taken from a mammal following a treatment, compared to a level in a sample taken from the mammal before, or earlier in, the treatment, indicates efficacious treatment.

The method of the present invention is therefore useful as a one-time test or as an on-going monitor of those individuals thought to be at risk of neoplasia development or as a monitor of the effectiveness of therapeutic or prophylactic treatment regimes directed to inhibiting or otherwise slowing neoplasia development. In these situations, mapping the modulation of methylation levels in any one or more classes of biological samples is a valuable indicator of the status of an individual or the effectiveness of a therapeutic or prophylactic regime which is currently in use. Accordingly, the method of the present invention should be understood to extend to monitoring for increases or decreases in methylation levels in an individual relative to their normal level (as hereinbefore defined), or relative to one or more earlier methylation levels determined from a biological sample of said individual.

The methods for detecting neoplasia can comprise the detection of one or more other cancer-associated polynucleotide or polypeptides sequences. Accordingly, detection of methylation by the method of the invention can be used either alone, or in combination with other screening methods for the diagnosis or prognosis of neoplasia.

Any method for detecting DNA methylation can be used in the methods of the present invention. A number of methods are available for detection of differentially methylated DNA at specific loci in either primary tissue samples or in patient samples such as blood, urine, stool or saliva (reviewed in Kristensen and Hansen Clin Chem. 55:1471-83, 2009; Ammerpohl et al. Biochim Biophys Acta. 1790:847-62, 2009; Shames et al. Cancer Lett. 251:187-98, 2007; Clark et al. Nat Protoc. 1:2353-64, 2006). For analysis of the proportion or extent of DNA methylation in a target gene, DNA is normally treated with sodium bisulfite and regions of interest amplified using primers and PCR conditions that will amplify independently of the methylation status of the DNA. The methylation of the overall amplicon or individual CpG sites can then be assessed by sequencing, including pyrosequencing, restriction enzyme digestion (COBRA) or by melting curve analysis. Alternatively ligation-based methods for analysis of methylation at specific CpG sites may be used. Detection of aberrantly methylated DNA released from tumours and into bodily fluids is being developed as a means of cancer diagnosis. Here, in the case of hypermethylated sequences, it is necessary to use sensitive methods that allow the selective amplification of the methylated DNA sequence from a background of normal cellular DNA that is unmethylated. Such methods based on bisulfite-treated DNA, for example; include methylation selective PCR (MSP), Heavymethyl PCR, Headloop PCR and Helper-dependent chain reaction (PCT/AU2008/001475).

Briefly, in some embodiments, methods for detecting methylation include randomly shearing or randomly fragmenting the genomic DNA, cutting the DNA with a methylation-dependent or methylation-sensitive restriction enzyme and subsequently selectively identifying and/or analyzing the cut or uncut DNA. Selective identification can include, for example, separating cut and uncut DNA (e.g., by size) and quantifying a sequence of interest that was cut or, alternatively, that was not cut. See, e.g., U.S. Pat. No. 7,186,512. Alternatively, the method can encompass amplifying intact DNA after restriction enzyme digestion, thereby only amplifying DNA that was not cleaved by the restriction enzyme in the area amplified. See, e.g., U.S. patent application Ser. Nos. 10/971,986; 11/071,013; and 10/971,339. In some embodiments, amplification can be performed using primers that are gene specific. Alternatively, adaptors can be added to the ends of the randomly fragmented DNA, the DNA can be digested with a methylation-dependent or methylation-sensitive restriction enzyme, intact DNA can be amplified using primers that hybridize to the adaptor sequences. In this case, a second step can be performed to determine the presence, absence or quantity of a particular gene in an amplified pool of DNA. In some embodiments, the DNA is amplified using real-time, quantitative PCR.

In some embodiments, the methods comprise quantifying the average methylation density in a target sequence within a population of genomic DNA. In some embodiments, the method comprises contacting genomic DNA with a methylation-dependent restriction enzyme or methylation-sensitive restriction enzyme under conditions that allow for at least some copies of potential restriction enzyme cleavage sites in the locus to remain uncleaved; quantifying intact copies of the locus; and comparing the quantity of amplified product to a control value representing the quantity of methylation of control DNA, thereby quantifying the average methylation density in the locus compared to the methylation density of the control DNA.

The quantity of methylation of a locus of DNA can be determined by providing a sample of genomic DNA comprising the locus, cleaving the DNA with a restriction enzyme that is either methylation-sensitive or methylation-dependent, and then quantifying the amount of intact DNA or quantifying the amount of cut DNA at the DNA locus of interest. The amount of intact or cut DNA will depend on the initial amount of genomic DNA containing the locus, the amount of methylation in the locus, and the number (i.e., the fraction) of nucleotides in the locus that are methylated in the genomic DNA. The amount of methylation in a DNA locus can be determined by comparing the quantity of intact DNA or cut DNA to a control value representing the quantity of intact DNA or cut DNA in a similarly-treated DNA sample. The control value can represent a known or predicted number of methylated nucleotides. Alternatively, the control value can represent the quantity of intact or cut DNA from the same locus in another (e.g., normal, non-diseased) cell or a second locus.

By using at least one methylation-sensitive or methylation-dependent restriction enzyme under conditions that allow for at least some copies of potential restriction enzyme cleavage sites in the locus to remain uncleaved and subsequently quantifying the remaining intact copies and comparing the quantity to a control, average methylation density of a locus can be determined. A methylation-sensitive enzyme is one which cuts DNA if its recognition sequence is unmethylated while a methylation-dependent enzyme cuts DNA if its recognition sequence is methylated. If the methylation-sensitive restriction enzyme is contacted to copies of a DNA locus under conditions that allow for at least some copies of potential restriction enzyme cleavage sites in the locus to remain uncleaved, then the remaining intact DNA will be directly proportional to the methylation density, and thus may be compared to a control to determine the relative methylation density of the locus in the sample. Similarly, if a methylation-dependent restriction enzyme is contacted to copies of a DNA locus under conditions that allow for at least some copies of potential restriction enzyme cleavage sites in the locus to remain uncleaved, then the remaining intact DNA will be inversely proportional to the methylation density, and thus may be compared to a control to determine the relative methylation density of the locus in the sample. Such assays are disclosed in, e.g., U.S. patent application Ser. No. 10/971,986.

Kits for the above methods can include, e.g., one or more of methylation-dependent restriction enzymes, methylation-sensitive restriction enzymes, amplification (e.g., PCR) reagents, probes and/or primers.

Quantitative amplification methods (e.g., quantitative PCR or quantitative linear amplification) can be used to quantify the amount of intact DNA within a locus flanked by amplification primers following restriction digestion. Methods of quantitative amplification are disclosed in, e.g., U.S. Pat. Nos. 6,180,349; 6,033,854; and 5,972,602, as well as in, e.g., Gibson et al., Genome Research 6:995-1001 (1996); DeGraves, et al., Biotechniques 34(1):106-10, 112-5 (2003); Deiman B, et al., Mol. Biotechnol. 20(2):163-79 (2002). Amplifications may be monitored in "real time."

Additional methods for detecting DNA methylation can involve genomic sequencing before and after treatment of the DNA with bisulfite. See, e.g., Frommer et al., Proc. Natl. Acad. Sci. USA 89:1827-1831 (1992). When sodium bisulfite is contacted to DNA, unmethylated cytosine is converted to uracil, while methylated cytosine is not modified.

In some embodiments, restriction enzyme digestion of PCR products amplified from bisulfite-converted DNA is used to detect DNA methylation. See, e.g., Sadri & Hornsby, Nucl. Acids Res. 24:5058-5059 (1996); Xiong & Laird, Nucleic Acids Res. 25:2532-2534 (1997).

In some embodiments, a methylation-specific PCR ("MSP") reaction is used alone or in combination with other methods to detect DNA methylation. An MSP assay entails initial modification of DNA by sodium bisulfite, converting all unmethylated, but not methylated, cytosines to uracil, and subsequent amplification with primers specific for methylated verses unmethylated DNA. See, Herman et al. Proc. Natl. Acad. Sci. USA 93:9821-9826 (1996); U.S. Pat. No. 5,786,146.

In some embodiments, a MethyLight assay is used alone or in combination with other methods to detect DNA methylation (see, Eads et al., Cancer Res. 59:2302-2306 (1999)). Briefly, in the MethyLight process genomic DNA is converted in a sodium bisulfite reaction (the bisulfite process converts unmethylated cytosine residues to uracil). Amplification of a DNA sequence of interest is then performed using PCR primers that hybridize to CpG dinucleotides. By using primers that hybridize only to sequences resulting from bisulfite conversion of methylated DNA, (or alternatively to unmethylated sequences) amplification can indicate methylation status of sequences where the primers hybridize. Furthermore, the amplification product can be detected with a probe that specifically binds to a sequence resulting from bisulfite treatment of a unmethylated DNA. If desired, both primers and probes can be used to detect methylation status. Thus, kits for use with MethyLight can include sodium bisulfite as well as primers or detectably-labelled probes (including but not limited to Taqman or molecular beacon probes) that distinguish between methylated and unmethylated DNA that have been treated with bisulfite. Other kit components can include, e.g., reagents necessary for amplification of DNA including but not limited to, PCR buffers, deoxynucleotides; and a thermostable polymerase.

In some embodiments, a Ms-SNuPE (Methylation-sensitive Single Nucleotide Primer Extension) reaction is used alone or in combination with other methods to detect DNA methylation (see, Gonzalgo & Jones, Nucleic Acids Res. 25:2529-2531 (1997)). The Ms-SNuPE technique is a quantitative method for assessing methylation differences at specific CpG sites based on bisulfite treatment of DNA, followed by single-nucleotide primer extension (Gonzalgo & Jones, *supra*)*.* Briefly, genomic DNA is reacted with sodium bisulfite to convert unmethylated cytosine to uracil while leaving 5-methylcytosine unchanged. Amplification of the desired target sequence is then performed using PCR primers specific for bisulfite-converted DNA, and the resulting product is isolated and used as a template for methylation analysis at the CpG site(s) of interest.

Typical reagents (e.g., as might be found in a typical Ms-SNuPE-based kit) for Ms-SNuPE analysis can include, but are not limited to: PCR primers for specific gene (or methylation-altered DNA sequence or CpG island); optimized PCR buffers and deoxynucleotides; gel extraction kit; positive control primers; Ms-SNuPE primers for a specific gene; reaction buffer (for the Ms-SNuPE reaction); and detectably-labelled nucleotides. Additionally, bisulfite conversion reagents may include: DNA denaturation buffer; sulfonation buffer; DNA recovery regents or kit (e.g., precipitation, ultrafiltration, affinity column); desulfonation buffer; and DNA recovery components.

Additional methylation detection methods include, but are not limited to, methylated CpG island amplification (see, Toyota et al., Cancer Res. 59:2307-12 (1999)) and those described in, e.g., U.S. Patent Publication 2005/0069879; Rein, et al. Nucleic Acids Res. 26 (10): 2255-64 (1998); Olek, et al. Nat. Genet. 17(3): 275-6 (1997); and PCT Publication No. WO 00/70090.

More detailed information in relation to several of these generally described methods is provided below:

### (a) Probe or Primer Design and/or Production

Several methods described herein for the diagnosis of a neoplasia use one or more probes and/or primers. Methods for designing probes and/or primers for use in, for example, PCR or hybridization are known in the art and described, for example, in Dieffenbach and Dveksler (Eds) (In: PCR Primer: A Laboratory Manual, Cold Spring Harbor Laboratories, NY, 1995). Furthermore, several software packages are publicly available that design optimal probes and/or primers for a variety of assays, e.g. Primer 3 available from the Center for Genome Research, Cambridge, Mass., USA.

Clearly, the potential use of the probe or primer should be considered during its design. For example, should the probe or primer be produced for use in a methylation specific PCR or ligase chain reaction (LCR) assay the nucleotide at the 3' end (or 5' end in the case of LCR) should preferably correspond to a methylated nucleotide in a nucleic acid.

Probes and/or primers useful for detection of a sequence associated with a neoplasia are assessed, for example, to determine those that do not form hairpins, self-prime or form primer dimers (e.g. with another probe or primer used in a detection assay). Furthermore, a probe or primer (or the sequence thereof) is often assessed to determine the temperature at which it denatures from a target nucleic acid (i.e. the melting temperature of the probe or primer, or Tm). Methods for estimating Tm are known in the art and described, for example, in Santa Lucia, Proc. Natl. Acad. Sci. USA, 95: 1460-1465, 1995 or Bresslauer et al., Proc. Natl. Acad. Sci. USA, 83: 3746-3750, 1986.

Methods for producing/synthesizing a probe or primer of the present invention are known in the art. For example, oligonucleotide synthesis is described, in Gait (Ed) (In: Oligonucleotide Synthesis: A Practical Approach, IRL Press, Oxford, 1984). For example, a probe or primer may be obtained by biological synthesis (e.g. by digestion of a nucleic acid with a restriction endonuclease) or by chemical synthesis. For short sequences (up to about 100 nucleotides) chemical synthesis is preferable.

For longer sequences standard replication methods employed in molecular biology are useful, such as, for example, the use of M13 for single stranded DNA as described by Messing, Methods Enzymol, 101, 20-78, 1983. Other methods for oligonucleotide synthesis include, for example, phosphotriester and phosphodiester methods (Narang, et al. Meth. Enzymol 68: 90, 1979) and synthesis on a support (Beaucage, et al. Tetrahedron Letters 22:1859-1862, 1981) as well as phosphoramidate technique, Caruthers, M. H., et al., Methods in Enzymology, Vol. 154, pp. 287-314 (1988), and others described in "Synthesis and Applications of DNA and RNA," S. A. Narang, editor, Academic Press, New York, 1987, and the references cited therein. Probes comprising locked nucleic acid (LNA) are synthesized as described, for example, in Nielsen et al. J. Chem. Soc. Perkin Trans., 1:3423, 1997; Singh and Wengel, Chem. Commun. 1247, 1998. While, probes comprising peptide-nucleic acid (PNA) are synthesized as described, for example, in Egholm et al., Am. Chem. Soc., 114:1895, 1992; Egholm et al., Nature, 365:566, 1993; and Orum et al., Nucl. Acids Res., 21:5332, 1993.

### (b) Methylation-Sensitive Endonuclease Digestion of DNA

In one example, the increased methylation in a sample is determined using a process comprising treating the nucleic acid with an amount of a methylation-sensitive restriction endonuclease enzyme under conditions sufficient for nucleic acid to be digested and then detecting the fragments produced. Exemplary methylation-sensitive endonucleases include, for example, HhaI or HpaII. Preferably, assays include internal controls that are digested with a methylation-insensitive enzyme having the same specificity as the methylation-sensitive enzyme employed. For example, the methylation-insensitive enzyme MspI is an isoschizomer of the methylation-sensitive enzyme HpaII.

### Hybridization Assay Formats

In one example, the digestion of nucleic acid is detected by selective hybridization of a probe or primer to the undigested nucleic acid. Alternatively, the probe selectively hybridizes to both digested and undigested nucleic acid but facilitates differentiation between both forms, e.g., by electrophoresis. Suitable detection methods for achieving selective hybridization to a hybridization probe include, for example, Southern or other nucleic acid hybridization (Kawai et al., Mol. Cell. Biol. 14:7421-7427, 1994; Gonzalgo et al., Cancer Res. 57:594-599, 1997).

Suitable hybridization conditions are determined based on the melting temperature (Tm) of a nucleic acid duplex comprising the probe. The skilled artisan will be aware that optimum hybridization reaction conditions should be determined empirically for each probe, although some generalities can be applied. Preferably, hybridizations employing short oligonucleotide probes are performed at low to medium stringency. In the case of a GC rich probe or primer or a longer probe or primer a high stringency hybridization and/or wash is preferred. A high stringency is defined herein as being a hybridization and/or wash carried out in about 0.1 x SSC buffer and/or about 0.1% (w/v) SDS, or lower salt concentration, and/or at a temperature of at least 65°C., or equivalent conditions. Reference herein to a particular level of stringency encompasses equivalent conditions using wash/hybridization solutions other than SSC known to those skilled in the art.

In accordance with the present example, a difference in the fragments produced for the test sample and a negative control sample is indicative of the subject having a neoplasia. Similarly, in cases where the control sample comprises data from a tumor, cancer tissue or a cancerous cell or pre-cancerous cell, similarity, albeit not necessarily absolute identity, between the test sample and the control sample is indicative of a positive diagnosis (i.e. cancer).

### Amplification Assay Formats

In an alternative example, the fragments produced by the restriction enzyme are detected using an amplification system, such as, for example, polymerase chain reaction (PCR), rolling circle amplification (RCA), inverse polymerase chain reaction (iPCR), in situ PCR (Singer-Sam et al., Nucl. Acids Res. 18:687, 1990), strand displacement amplification (SDA) or cycling probe technology.

Methods of PCR are known in the art and described, for example, by McPherson et al., PCR: A Practical Approach. (series eds, D. Rickwood and B. D. Hames), IRL Press Limited, Oxford. pp 1-253, 1991 and by Dieffenbach (ed) and Dveksler (ed) (In: PCR Primer: A Laboratory Manual, Cold Spring Harbour Laboratories, NY, 1995). Generally, for PCR two non-complementary nucleic acid primer molecules comprising at least about 18 nucleotides in length, and more preferably at least 20-30 nucleotides in length are hybridized to different strands of a nucleic acid template molecule at their respective annealing sites, and specific nucleic acid molecule copies of the template that intervene the annealing sites are amplified enzymatically. Amplification products may be detected, for example, using electrophoresis and detection with a detectable marker that binds nucleic acids. Alternatively, one or more of the oligonucleotides are labelled with a detectable marker (e.g. a fluorophore) and the amplification product detected using, for example, a lightcycler (Perkin Elmer, Wellesley, Mass., USA, Roche Applied Science, Indianapolis, IN, USA).

Strand displacement amplification (SDA) utilizes oligonucleotide primers, a DNA polymerase and a restriction endonuclease to amplify a target sequence. The oligonucleotides are hybridized to a target nucleic acid and the polymerase is used to produce a copy of the region intervening the primer annealing sites. The duplexes of copied nucleic acid and target nucleic acid are then nicked with an endonuclease that specifically recognizes a sequence at the beginning of the copied nucleic acid. The DNA polymerase recognizes the nicked DNA and produces another copy of the target region at the same time displacing the previously generated nucleic acid. The advantage of SDA is that it occurs in an isothermal format, thereby facilitating high-throughput automated analysis.

Cycling Probe Technology uses a chimeric synthetic primer that comprises DNA-RNA-DNA that is capable of hybridizing to a target sequence. Upon hybridization to a target sequence the RNA-DNA duplex formed is a target for RNaseH thereby cleaving the primer. The cleaved primer is then detected, for example, using mass spectrometry or electrophoresis.

For primers that flank or are adjacent to a methylation-sensitive endonuclease recognition site, it is preferred that such primers flank only those sites that are hypermethylated in neoplasia to ensure that a diagnostic amplification product is produced. In this regard, an amplification product will only be produced when the restriction site is not cleaved, i.e., when it is methylated. Accordingly, detection of an amplification product indicates that the CpG dinucleotide/s of interest is/are methylated.

As will be known to the skilled artisan, the precise length of the amplified product will vary depending upon the distance between the primers. Clearly this form of analysis may be used to determine the methylation status of a plurality of CpG dinucleotides provided that each dinucleotide is within a methylation sensitive restriction endonuclease site. In these methods, one or more of the primers may be labelled with a detectable marker to facilitate rapid detection of amplified nucleic acid, for example, a fluorescent label (e.g. Cy5 or Cy3) or a radioisotope (e.g. ³²P).

The amplified nucleic acids are generally analyzed using, for example, non-denaturing agarose gel electrophoresis, non-denaturing polyacrylamide gel electrophoresis, mass spectrometry, liquid chromatography (e.g. HPLC or dHPLC), or capillary electrophoresis. (e.g. MALDI-TOF). High throughput detection methods, such as, for example, matrix-assisted laser desorption/ionization time of flight (MALDI-TOF), electrospray ionization (ESI), mass spectrometry (including tandem mass spectrometry, e.g. LC MS/MS), biosensor technology, evanescent fiber-optics technology or DNA chip technology (e.g., WO98/49557; WO 96/17958; Fodor et al., Science 767-773, 1991; U.S. Pat. No. 5,143,854; and U.S. Pat. No. 5,837,832. Alternatively, amplification of a nucleic acid may be continuously monitored using a melting curve analysis method as described herein and/or in, for example, U.S. Pat. No. 6,174,670.

### (c) Other Assay Formats

In an alternative example, the increased methylation in a sample is determined by performing a process comprising treating chromatin containing the nucleic acid with an amount of DNaseI under conditions sufficient for nucleic acid to be digested and then detecting the fragments produced. This assay format is predicated on the understanding that chromatin containing methylated DNA, e.g., hyper methylated DNA, has a more tightly-closed conformation than non-hyper methylated DNA and, as a consequence, is less susceptible to endonuclease digestion by DNase I.

In accordance with this method , DNA fragments of different lengths are produced by DNase I digestion of methylated compared to non-methylated DNA. Such different DNA fragments are detected, for example, using an assay described earlier. Alternatively, the DNA fragments are detected using PCR-SSCP essentially as described, for example, in Gregory and Feil Nucleic Acids Res., 27, e32i-e32iv, 1999. In adapting PCR-SSCP to the present invention, amplification primers flanking or comprising one or more CpG dinucleotides in a nucleic acid that are resistant to DNase I digestion in a neoplasia sample but not resistant to DNase I digestion in a healthy/normal control or healthy/normal test sample are used to amplify the DNase I-generated fragments. In this case, the production of a specific nucleic acid fragment using DNase I is diagnostic of neoplasia, because the DNA is not efficiently degraded. In contrast, template DNA from a healthy/normal subject sample is degraded by the action of DNase I and, as a consequence, amplification fails to produce a discrete amplification product. Alternative methods to PCR-SSCP, such as for example, PCR-dHPLC are also known in the art and contemplated by the present invention.

### (d) Selective Mutagenesis of Non-Methylated DNA

In an alternative method the increased methylation in a sample is determined using a process comprising treating the nucleic acid with an amount of a compound that selectively mutates a non-methylated cytosine residue within a CpG dinucleotide under conditions sufficient to induce mutagenesis.

Preferred compounds mutate cytosine to uracil or thymidine, such as, for example, a salt of bisulfite, e.g., sodium bisulfite or potassium bisulfite (Frommer et al., Proc. Natl. Acad. Sci. USA 89, 1827-1831, 1992). Bisulfite treatment of DNA is known to distinguish methylated from non-methylated cytosine residues, by mutating cytosine residues that are not protected by methylation, including cytosine residues that are not within a CpG dinucleotide or that are positioned within a CpG dinucleotide that is not subject to methylation.

### Sequence Based Detection

In one example, the presence of one or more mutated nucleotides or the number of mutated sequences is determined by sequencing mutated DNA. One form of analysis comprises amplifying mutated nucleic acid using an amplification reaction described herein, for example, PCR. The amplified product is then directly sequenced or cloned and the cloned product sequenced. Methods for sequencing DNA are known in the art and include for example, the dideoxy chain termination method or the Maxam-Gilbert method (see Sambrook et al., Molecular Cloning, A Laboratory Manual (2nd Ed., CSHP, New York 1989) or Zyskind et al., Recombinant DNA Laboratory Manual, (Acad. Press, 1988)).

As the treatment of nucleic acid with a compound, such as, for example, bisulfite results in non-methylated cytosines being mutated to uracil (and hence thymidine after an amplification process), analysis of the sequence determines the presence or absence of a methylated nucleotide. For example, by comparing the sequence obtained using a control sample or a sample that has not been treated with bisulfite, or the known nucleotide sequence of the region of interest with a treated sample facilitates the detection of differences in the nucleotide sequence. Any thymine residue detected at the site of a cytosine in the treated sample compared to a control or untreated sample may be considered to be caused by mutation as a result of bisulfite treatment. Suitable methods for the detection of methylation using sequencing of bisulfite treated nucleic acid are described, for example, in Frommer et al., Proc. Natl. Acad. Sci. USA 89: 1827-1831, 1992 or Clark et al., Nucl. Acids Res. 22: 2990-2997, 1994.

In another method, the presence of a mutated or non-mutated nucleotide in a bisulfite treated sample is detected using pyrosequencing, such as, for example, as described in Uhlmann et al., Electrophoresis, 23: 4072-4079, 2002. Essentially this method is a form of real-time sequencing that uses a primer that hybridizes to a site adjacent or close to the site of a cytosine that is methylated. Following hybridization of the primer and template in the presence of a DNA polymerase each of four modified deoxynucleotide triphosphates are added separately according to a predetermined dispensation order. Only an added nucleotide that is complementary to the bisulfite treated sample is incorporated and inorganic pyrophosphate (PPi) is liberated. The PPi then drives a reaction resulting in production of detectable levels of light. Such a method allows determination of the identity of a specific nucleotide adjacent to the site of hybridization of the primer.

Methods of solid phase pyrosequencing are known in the art and reviewed in, for example, Landegren et al., Genome Res., 8(8): 769-776, 1998. Such methods enable the high-throughput detection of methylation of a number of CpG dinucleotides.

A related method for determining the sequence of a bisulfite treated nucleotide is methylation-sensitive single nucleotide primer extension (Me-SnuPE) or SNaPmeth. Suitable methods are described, for example, in Gonzalgo and Jones, Nucl. Acids Res., 25:2529-2531 or Uhlmann et al., Electrophoresis, 23:4072-4079, 2002. An oligonucleotide is used that hybridizes to the region of a nucleic acid adjacent to the site of a cytosine that is methylated. This oligonucleotide is then used in a primer extension protocol with a polymerase and a free nucleotide diphosphate or dideoxynucleotide triphosphate that corresponds to either or any of the possible bases that occur at this site following bisulfite treatment (i.e., thymine or cytosine). Preferably, the nucleotide-diphosphate is labelled with a detectable marker (e.g. a fluorophore). Following primer extension, unbound labelled nucleotide diphosphates are removed, e.g. using size exclusion chromatography or electrophoresis, or hydrolyzed, using for example, alkaline phosphatase, and the incorporation of the labelled nucleotide to the oligonucleotide is detected, indicating the base that is present at the site.

Clearly other high throughput sequencing methods are encompassed by the present invention. Such methods include, for example, solid phase minisequencing (as described, for example, in Southern et al., Genomics, 13:1008-1017, 1992), or minisequencing with FRET (as described, for example, in Chen and Kwok, Nucleic Acids Res. 25:347-353, 1997).

### Restriction Endonuclease-Based Assay Format

In one method, the presence of a non-mutated sequence is detected using combined bisulfite restriction analysis (COBRA) essentially as described in Xiong and Laird, Nucl. Acids Res., 25:2532-2534, 2001. This method exploits the differences in restriction enzyme recognition sites between methylated and unmethylated nucleic acid after treatment with a compound that selectively mutates a non-methylated cytosine residue, e.g., bisulfite.

Following bisulfite treatment a region of interest comprising one or more CpG dinucleotides that are methylated and are included in a restriction endonuclease recognition sequence is amplified using an amplification reaction described herein, e.g., PCR. The amplified product is then contacted with the restriction enzyme that cleaves at the site of the CpG dinucleotide for a time and under conditions sufficient for cleavage to occur. A restriction site may be selected to indicate the presence or absence of methylation. For example, the restriction endonuclease TaqI cleaves the sequence TCGA, following bisulfite treatment of a non-methylated nucleic acid the sequence will be TTGA and, as a consequence, will not be cleaved. The digested and/or non-digested nucleic acid is then detected using a detection means known in the art, such as, for example, electrophoresis and/or mass spectrometry. The cleavage or non-cleavage of the nucleic acid is indicative of cancer in a subject. Clearly, this method may be employed in either a positive read-out or negative read-out system for the diagnosis of a cancer.

### Positive Read-Out Assay Format

In one embodiment, the assay format of the invention comprises a positive read-out system in which DNA from a sample that has been treated, for example, with bisulfite is detected as a positive signal. Preferably, the non-hypermethylated DNA from a healthy or normal control subject is not detected or only weakly detected.

In a preferred embodiment, the increased methylation in a subject sample is determined using a process comprising:
(i) treating the nucleic acid with an amount of a compound that selectively mutates a non-methylated cytosine residue under conditions sufficient to induce mutagenesis thereby producing a mutated nucleic acid;
(ii) hybridizing a nucleic acid to a probe or primer comprising a nucleotide sequence that is complementary to a sequence comprising a methylated cytosine residue under conditions such that selective hybridization to the non-mutated nucleic acid occurs; and
(iii) detecting the selective hybridization.

In this context, the term "selective hybridization" means that hybridization of a probe or primer to the non-mutated nucleic acid occurs at a higher frequency or rate, or has a higher maximum reaction velocity, than hybridization of the same probe or primer to the corresponding mutated sequence. Preferably, the probe or primer does not hybridize to the non-methylated sequence carrying the mutation(s) under the reaction conditions used.

### Hybridization-Based Assay Format

In one embodiment, the hybridization is detected using Southern, dot blot, slot blot or other nucleic acid hybridization means (Kawai et al., Mol. Cell. Biol. 14:7421-7427, 1994; Gonzalgo et al., Cancer Res. 57:594-599, 1997). Subject to appropriate probe selection, such assay formats are generally described herein above and apply mutatis mutandis to the presently described selective mutagenesis approach.

Preferably, a ligase chain reaction format is employed to distinguish between a mutated and non-mutated nucleic acid. Ligase chain reaction (described in EP 320,308 and U.S. Pat. No. 4,883,750) uses at least two oligonucleotide probes that anneal to a target nucleic acid in such a way that they are juxtaposed on the target nucleic acid. In a ligase chain reaction assay, the target nucleic acid is hybridized to a first probe that is complementary to a diagnostic portion of the target sequence (the diagnostic probe) e.g., a nucleic acid comprising one or more methylated CpG dinucleotide(s), and with a second probe that is complementary to a nucleotide sequence contiguous with the diagnostic portion (the contiguous probe), under conditions wherein the diagnostic probe remains bound substantially only to the target nucleic acid. The diagnostic and contiguous probes can be of different lengths and/or have different melting temperatures such that the stringency of the hybridization can be adjusted to permit their selective hybridization to the target, wherein the probe having the higher melting temperature is hybridized at higher stringency and, following washing to remove unbound and/or non-selectively bound probe, the other probe having the lower melting temperature is hybridized at lower stringency. The diagnostic probe and contiguous probe are then covalently ligated such as, for example, using T4 DNA ligase, to thereby produce a larger target probe that is complementary to the target sequence, and the probes that are not ligated are removed by modifying the hybridization stringency. In this respect, probes that have not been ligated will selectively hybridize under lower stringency hybridization conditions than probes that have been ligated. Accordingly, the stringency of the hybridization can be increased to a stringency that is at least as high as the stringency used to hybridize the longer probe, and preferably at a higher stringency due to the increased length contributed by the shorter probe following ligation.

In another example, one or both of the probes is labelled such that the presence or absence of the target sequence can be tested by melting the target-probe duplex, eluting the dissociated probe, and testing for the label(s). Where both probes are labelled, different ligands are used to permit distinction between the ligated and unligated probes, in which case the presence of both labels in the same eluate fraction confirms the ligation event. If the target nucleic acid is bound to a solid matrix e.g., in a Southern hybridization, slot blot, dot blot, or microchip assay format, the presence of both the diagnostic and contiguous probes can be determined directly.

Methylation specific microarrays (MSO) are also useful for differentiating between a mutated and non-mutated sequence. A suitable method is described, for example, in Adorjan et al. Nucl. Acids Res., 30: e21, 2002. MSO uses nucleic acid that has been treated with a compound that selectively mutates a non-methylated cytosine residue (e.g., bisulfite) as template for an amplification reaction that amplifies both mutant and non-mutated nucleic acid. The amplification is performed with at least one primer that comprises a detectable label, such as, for example, a fluorophore, e.g., Cy3 or Cy5.

To produce a microarray for detection of mutated nucleic acid oligonucleotides are spotted onto, for example, a glass slide, preferably, with a degree of redundancy (for example, as described in Golub et al., Science, 286:531-537, 1999). Preferably, for each CpG dinucleotide analyzed two different oligonucleotides are used. Each oligonucleotide comprises a sequence N₂-16CGN₂-16 or N₂-16TGN₂-16 (wherein N is a number of nucleotides adjacent or juxtaposed to the CpG dinucleotide of interest) reflecting the methylated or non-methylated status of the CpG dinucleotides.

The labelled amplification products are then hybridized to the oligonucleotides on the microarray under conditions that enable detection of single nucleotide differences. Following washing to remove unbound amplification product, hybridization is detected using, for example, a microarray scanner. Not only does this method allow for determination of the methylation status of a large number of CpG dinucleotides, it is also semi-quantitative, enabling determination of the degree of methylation at each CpG dinucleotide analyzed. As there may be some degree of heterogeneity of methylation in a single sample, such quantification may assist in the diagnosis of cancer.

### Amplification-Based Assay Format

In an alternative example, the hybridization is detected using an amplification system. In methylation-specific PCR formats (MSP; Herman et al. Proc. Natl. Acad. Sci. USA 93:9821-9826, 1992), the hybridization is detected using a process comprising amplifying the bisulfite-treated DNA. Accordingly, by using one or more probe or primer that anneals specifically to the unmutated sequence under moderate and/or high stringency conditions an amplification product is only produced using a sample comprising a methylated nucleotide. Alternate assays that provide for selective amplification of either the methylated or the unmethylated component from a mixture of bisulfite-treated DNA are provided by Cottrell et al., Nucl. Acids Res. 32: e10, 2003 (HeavyMethyl PCR), Rand et al. Nucl. Acids Res. 33:e127, 2005 (Headloop PCR), Rand et al. Epigenetics 1:94-100, 2006 (Bisulfite Differential Denaturation PCR) and PCT/AU07/000389 (End-specific PCR).

Any amplification assay format described herein can be used, such as, for example, polymerase chain reaction (PCR), rolling circle amplification (RCA), inverse polymerase chain reaction (iPCR), in situ PCR (Singer-Sam et al., Nucl. Acids Res. 18, 687, 1990), strand displacement amplification, or cycling probe technology. PCR techniques have been developed for detection of gene mutations (Kuppuswamy et al., Proc. Natl. Acad. Sci. USA 88:1143-1147, 1991) and quantitation of allelic-specific expression (Szabo and Mann, Genes Dev. 9: 3097-3108, 1995; and Singer-Sam et al., PCR Methods Appl. 1: 160-163, 1992). Such techniques use internal primers, which anneal to a PCR-generated template and terminate immediately 5' of the single nucleotide to be assayed. Such as format is readily combined with ligase chain reaction as described herein above. The use of a real-time quantitative assay format is also useful. Subject to the selection of appropriate primers, such assay formats are generally described herein above and apply mutatis mutandis to the presently described selective mutagenesis approach.

Methylation-specific melting-curve analysis (essentially as described in Worm et al., Clin. Chem., 47:1183-1189, 2001) is also contemplated by the present invention. This process exploits the difference in melting temperature in amplification products produced using bisulfite treated methylated or unmethylated nucleic acid. In essence, non-discriminatory amplification of a bisulfite treated sample is performed in the presence of a fluorescent dye that specifically binds to double stranded DNA (e.g., SYBR Green I). By increasing the temperature of the amplification product while monitoring fluorescence the melting properties and thus the sequence of the amplification product is determined. A decrease in the fluorescence reflects melting of at least a domain in the amplification product. The temperature at which the fluorescence decreases is indicative of the nucleotide sequence of the amplified nucleic acid, thereby permitting the nucleotide at the site of one or more CpG dinucleotides to be determined. As the sequence of the nucleic acids amplified using the present invention

The present invention also encompasses the use of real-time quantitative forms of PCR, such as, for example, TaqMan (Holland et al., Proc. Natl. Acad. Sci. USA, 88:7276-7280, 1991; Lee et al., Nucleic Acid Res. 21:3761-3766, 1993) to perform this embodiment. For example, the MethylLight method of Eads et al., Nucl. Acids Res. 28: E32, 2000 uses a modified TaqMan assay to detect methylation of a CpG dinucleotide. Essentially, this method comprises treating a nucleic acid sample with bisulfite and amplifying nucleic acid comprising one or more CpG dinucleotides that are methylated in a neoplastic cell and not in a control sample using an amplification reaction, e.g., PCR. The amplification reaction is performed in the presence of three oligonucleotides, a forward and reverse primer that flank the region of interest and a probe that hybridizes between the two primers to the site of the one or more methylated CpG dinucleotides. The probe is dual labelled with a 5' fluorescent reporter and a 3' quencher (or vice versa). When the probe is intact, the quencher dye absorbs the fluorescence of the reporter due to their proximity. Following annealing of to the PCR product the probe is cleaved by 5' to 3' exonuclease activity of, for example, Taq DNA polymerase. This cleavage releases the reporter from the quencher thereby resulting in an increased fluorescence signal that can be used to estimate the initial template methylation level. By using a probe or primer that selectively hybridizes to unmutated nucleic acid (i.e. methylated nucleic acid) the level of methylation is determined, e.g., using a standard curve.

Alternatively, rather than using a labelled probe that requires cleavage, a probe, such as, for example, a Molecular Beacon is used (see, for example, Mhlanga and Malmberg, Methods 25:463-471, 2001). Molecular beacons are single stranded nucleic acid molecules with a stem-and-loop structure. The loop structure is complementary to the region surrounding the one or more CpG dinucleotides that are methylated in a neoplastic sample and not in a control sample. The stem structure is formed by annealing two "arms" complementary to each other, which are on either side of the probe (loop). A fluorescent moiety is bound to one arm and a quenching moiety that suppresses any detectable fluorescence when the molecular beacon is not bound to a target sequence is bound to the other arm. Upon binding of the loop region to its target nucleic acid the arms are separated and fluorescence is detectable. However, even a single base mismatch significantly alters the level of fluorescence detected in a sample. Accordingly, the presence or absence of a particular base is determined by the level of fluorescence detected. Such an assay facilitates detection of one or more unmutated sites (i.e. methylated nucleotides) in a nucleic acid.

Fluorescently labelled locked nucleic acid (LNA) molecules or fluorescently labelled protein-nucleic acid (PNA) molecules are useful for the detection of nucleotide differences (e.g., as described in Simeonov and Nikiforov, Nucleic Acids Research, 30(17): 1-5, 2002). LNA and PNA molecules bind, with high affinity, to nucleic acid, in particular, DNA. Fluorophores (in particular, rhodomine or hexachlorofluorescein) conjugated to the LNA or PNA probe fluoresce at a significantly greater level upon hybridization of the probe to target nucleic acid. However, the level of increase of fluorescence is not enhanced to the same level when even a single nucleotide mismatch occurs. Accordingly, the degree of fluorescence detected in a sample is indicative of the presence of a mismatch between the LNA or PNA probe and the target nucleic acid, such as, in the presence of a mutated cytosine in a methylated CpG dinucleotide. Preferably, fluorescently labelled LNA or PNA technology is used to detect at least a single base change in a nucleic acid that has been previously amplified using, for example, an amplification method known in the art and/or described herein.

As will be apparent to the skilled artisan, LNA or PNA detection technology is amenable to a high-throughput detection of one or more markers by immobilizing an LNA or PNA probe to a solid support, as described in Orum et al., Clin. Chem. 45:1898-1905, 1999.

Alternatively, a real-time assay, such as, for example, the so-called HeavyMethyl assay (Cottrell et al., Nucl. Acids Res. 32: e10, 2003) is used to determine the presence or level of methylation of nucleic acid in a test sample. Essentially, this method uses one or more non-extendible nucleic acid (e.g., oligonucleotide) blockers that bind to bisulfite-treated nucleic acid in a methylation specific manner (i.e., the blocker/s bind specifically to unmutated DNA under moderate to high stringency conditions). An amplification reaction is performed using one or more primers that may optionally be methylation specific but that flank the one or more blockers. In the presence of unmethylated nucleic acid (i.e., non-mutated DNA) the blocker/s bind and no PCR product is produced. Using a TaqMan assay essentially as described supra the level of methylation of nucleic acid in a sample is determined.

Other amplification based methods for detecting methylated nucleic acid following treatment with a compound that selectively mutates a non-methylated cytosine residue include, for example, methylation-specific single stranded conformation analysis (MS-SSCA) (Bianco et al., Hum. Mutat., 14:289-293, 1999), methylation-specific denaturing gradient gel electrophoresis (MS-DGGE) (Abrams and Stanton, Methods Enzymol., 212:71-74, 1992) and methylation-specific denaturing high-performance liquid chromatography (MS-DHPLC) (Deng et al. Chin. J. Cancer Res., 12:171-191, 2000). Each of these methods use different techniques for detecting nucleic acid differences in an amplification product based on differences in nucleotide sequence and/or secondary structure. Such methods are clearly contemplated by the present invention.

As with other amplification-based assay formats, the amplification product is analyzed using a range of procedures, including gel electrophoresis, gel filtration, mass spectrometry, and in the case of labelled primers, by identifying the label in the amplification product. In an alternative embodiment, restriction enzyme digestion of PCR products amplified from bisulfite-converted DNA is performed essentially as described by Sadri and Hornsby, Nucl. Acids Res. 24:5058-5059, 1996; and Xiong and Laird, Nucl. Acids Res. 25:2532-2534, 1997), to analyze the product formed.

High throughput detection methods, such as, for example, matrix-assisted laser desorption/ionization time of flight (MALDI-TOF), electrospray ionization (ESI), Mass spectrometry (including tandem mass spectrometry, e.g. LC MS/MS), biosensor technology, evanescent fiber-optics technology or DNA chip technology, can also be employed.

As with the other assay formats described herein that utilize hybridization and/or amplification detection systems, combinations of such processes as described herein above are particularly contemplated by the selective mutagenesis-based assay formats of the present invention. In one example, the increased methylation is detected by performing a process comprising:
(i) treating the nucleic acid with an amount of a compound that selectively mutates a non-methylated cytosine residue within a CpG dinucleotide under conditions sufficient to induce mutagenesis thereby producing a mutated nucleic acid;
(ii) hybridizing the nucleic acid to two non-overlapping and non-complementary primers each of which comprises a nucleotide sequence that is complementary to a sequence in the DNA comprising a methylated cytosine residue under conditions such that hybridization to the non-mutated nucleic acid occurs;
(iii) amplifying nucleic acid intervening the hybridized primers thereby producing a DNA fragment consisting of a sequence that comprises a primer sequence;
(iv) hybridizing the amplified DNA fragment to a probe comprising a nucleotide sequence that corresponds or is complementary to a sequence comprising a methylated cytosine residue under conditions such that hybridization to the non-mutated nucleic acid occurs; and detecting the hybridization.

### Negative Read-Out Assays

In another example, the assay format comprises a negative read-out system in which reduced methylation of DNA from a healthy/normal control sample is detected as a positive signal and preferably, methylated DNA from a neoplastic sample is not detected or is only weakly detected.

In a preferred embodiment, the reduced methylation is determined using a process comprising:
(i) treating the nucleic acid with an amount of a compound that selectively mutates a non-methylated cytosine residue within a CpG island under conditions sufficient to induce mutagenesis thereby producing a mutated nucleic acid;
(ii) hybridizing the nucleic acid to a probe or primer comprising a nucleotide sequence that is complementary to a sequence comprising the mutated cytosine residue under conditions such that selective hybridization to the mutated nucleic acid occurs; and
(iii) detecting the selective hybridization.

In this context, the term "selective hybridization" means that hybridization of a probe or primer to the mutated nucleic acid occurs at a higher frequency or rate, or has a higher maximum reaction velocity, than hybridization of the same probe or primer to the corresponding non-mutated sequence. Preferably, the probe or primer does not hybridize to the methylated sequence (or non-mutated sequence) under the reaction conditions used.

### Hybridization-Based Assay Format

In one embodiment the hybridization is detected using Southern, dot blot, slot blot or other nucleic acid hybridization means (Kawai et al., Mol. Cell. Biol. 14:7421-7427, 1994; Gonzalgo et al., Cancer Res. 57:594-599, 1997). Subject to appropriate probe selection, such assay formats are generally described herein above and apply mutatis mutandis to the presently described selective mutagenesis approach. Preferably, a ligase chain reaction format is employed to distinguish between a non-mutated and mutated nucleic acid. In this respect, the assay requirements and conditions are as described herein above for positive read-out assays and apply mutatis mutandis to the present format. However the selection of probes will differ. For negative read-out assays, one or more probes are selected that selectively hybridize to the mutated sequence rather than the non-mutated sequence.

Preferably, the ligase chain reaction probe(s) have 3'-terminal and/or 5'-terminal sequences that comprise a CpG dinucleotide that is not methylated in a healthy control sample, but is hypermethylated in cancer, such that the diagnostic probe and contiguous probe are capable of being ligated only when the cytosine of the CpG dinucleotide is mutated to thymidine e.g., in the case of a non-methylated cytosine residue.

As will be apparent to the skilled artisan the MSO method described supra is amenable to either or both positive and/or negative readout assays. This is because the assay described detects both mutated and non-mutated sequences thereby facilitating determining the level of methylation. However, an assay detecting only methylated or non-methylated sequences is contemplated by the invention.

### Amplification-Based Assay Format

In an alternative example, the hybridization is detected using an amplification system using any amplification assay format as described herein above for positive read-out assay albeit using primers (and probes where applicable) selectively hybridize to a mutated nucleic acid.

In adapting the HeavyMethyl assay described supra to a negative read-out format, the blockers that bind to bisulfite-treated nucleic acid in a methylation specific manner bind specifically to mutated DNA under moderate to high stringency conditions. An amplification reaction is performed using one or more primers that may optionally be methylation specific (i.e. only bind to mutated nucleic acid) but that flank the one or more blockers. In the presence of methylated nucleic acid (i.e., mutated DNA) the blocker/s bind and no PCR product is produced.

In one example, the reduced methylation in the normal/healthy control subject is detected by performing a process comprising:
(i) treating the nucleic acid with an amount of a compound that selectively mutates non-methylated cytosine residues under conditions sufficient to induce mutagenesis thereby producing a mutated nucleic acid;
(ii) hybridizing the nucleic acid to two non-overlapping and non-complementary primers each of which comprises a nucleotide sequence that is complementary to a sequence in the DNA comprising a mutated cytosine residue under conditions such that hybridization to the mutated nucleic acid occurs;
(iii) amplifying nucleic acid intervening the hybridized primers thereby producing a DNA fragment consisting of a sequence that comprises a primer sequence;
(iv) hybridizing the amplified DNA fragment to a probe comprising a nucleotide sequence that corresponds or is complementary to a sequence comprising a mutated cytosine residue under conditions such that hybridization to the mutated nucleic acid occurs; and
(v) detecting the hybridization.

As will be apparent to the skilled artisan a negative read-out assay preferably includes a suitable control sample to ensure that the negative result is caused by methylated nucleic acid rather than a reaction failing.

This invention also provides kits for the detection and/or quantification of the diagnostic sequences of the invention, or expression or methylation thereof using the methods described herein.

For kits for detection of methylation, the kits of the invention can comprise at least one polynucleotide that hybridizes to at least one of the diagnostic sequences of the invention and at least one reagent for detection of gene methylation. Reagents for detection of methylation include, e.g., sodium bisulfite, polynucleotides designed to hybridize to sequence that is the product of a biomarker sequence of the invention if the biomarker sequence is not methylated (e.g., containing at least one C→U conversion), and/or a methylation-sensitive or methylation-dependent restriction enzyme. The kits may also include control natural or synthetic DNA sequences representing methylated or unmethylated forms of the sequence. The kits can provide solid supports in the form of an assay apparatus that is adapted to use in the assay. The kits may further comprise detectable labels, optionally linked to a polynucleotide, e.g., a probe, in the kit. Other materials useful in the performance of the assays can also be included in the kits, including test tubes, transfer pipettes, and the like. The kits can also include written instructions for the use of one or more of these reagents in any of the assays described herein.

As detailed hereinbefore, hypermethylation is associated with transcriptional silencing. Accordingly, in addition to the increased level of methylation of these genes providing a basis upon which to screen for the predisposition to or onset of a large intestine neoplasm, the downregulation in the level of expression of these genes is also diagnostically valuable. In accordance with this aspect of the present invention, reference to a gene "expression product" or "expression of a gene" is a reference to either a transcription product (such as primary RNA or mRNA) or a translation product such as protein. In this regard, one can assess changes to the level of expression of a gene either by screening for changes to the level of expression product which is produced (i.e. RNA or protein), changes to the chromatin proteins with which the gene is associated, for example the presence of histone H3 methylated on lysine at amino acid position number 9 or 27 (repressive modifications) or changes to the DNA itself which acts to downregulate expression, such as changes to the methylation of the DNA. These genes and their gene expression products, whether they be RNA transcripts, changes to the DNA which act to downregulate expression or encoded proteins, are collectively referred to as "neoplastic markers".

Accordingly, another aspect of the present invention is directed to a method of screening for the onset or predisposition to the onset of or monitoring a large intestine neoplasm in an individual, said method comprising assessing the level of expression of a DNA region selected from:
(i) the region defined by any one or more of Hg19 coordinates and 2kb upstream of the transcription start site:

| | |
|---|---|
| (1) chr12:52400748..52409671 | (34) chr10:101292690..101296281 |
| (2) chr5:3596168..3601517 | (35) chr4:4190530..4228621 |
| (3) chr13:95361876..95364389 | (36) chr12:54943404..54973023 |
| (4) chr4:81187742..81212171 | (37) chr5:176047210..176057557 |
| (5) chr19:57019212..57040270 | (38) chr12:22346325..22487648 |
| (6) chr3:33191537..33260707 | (39) chr19:56894648..56904889 |
| (7) chr15:60296421..60298142 | (40) chr20:21491648..21494664 |
| (8) chr13:28494168..28500451 | (41) chr1:50883225..50889141 |
| (9) chr7:96649702..96654143, | (42) chr7:27180996..27183287 |
| (10) chr8:140,811,770-141,537,860 | (43) chr11:2016406..2019065 |
| (11) chr5:2746279..2751769 | (44) chr14:57267425..57277184 |
| (12) chr18:55102917..55158530 | (45) chr4:126237567..126414087 |
| (13) chr20:37353101..37358016 | (46) chr8:23559964..23563922 |
| (14) chr8:2792875..4852328 | (47) chr10:131633547..131762091 |
| (15) chr16:66613351..66622178 | (48) chr4:62362839..62938168 |
| (16) chr5:37815753..37839782 | (49) chr1:47901689..47906363 |
| (17) chr1:63788730..63790797 | (50) chr17:77768176..77770890 |
| (18) chr15:37156644..37178734 | (51) chr17:93598762..93604831 |
| (19) chr7:27139973..27142394 | (52) chr1:33789224..33841194 |
| (20) chr20:21686297..21696620 | (53) chr9:124,004,679-124,030,840 |
| (21) chr16:51169886..51185183 | (54) chr4:158141736..158287227 |
| (22) chr12:85253267..85306606 | (55) chr12:9445136..9462559 |
| (23) chr8:6357172..6420784 | (56) chr12:24964278..25102308 |
| (24) chr14:85996488..86094270 | (57) chrX:21542357..21690352 |
| (25) chr2:182541194..182545381 | (58) chr20:52769988..52790516 |
| (26) chr7:30951468..30965131 | (59) chr3:172162951..172166203 |
| (27) chr8:131792547..132052835 | (60) chr13:28366780..28368089 |
| (28) chr3:128749292..128759583 | (61) chr7:50344378...50472799 |
| (29) chr10:101088856..101154087 | (62) chr7:149412148..149431664 |
| (30) chr7:27282164..27286192 | (63) chr7:24323809..24331477 |
| (31) chr10: 129535538..129539450 | (64) chr4:30722037..31148421 |
| (32) chr19:49316274..49339934 | (65) chr10:47083534..47088320 |
| (33) chr6:391752..411443 | |

(ii) the gene region, including 2kb upstream of any one or more of:

| | | | |
|---|---|---|---|
| (1) GRASP | (18) ANGPT2 | (35) NKX2-6 | (52) HOXA5 |
| (2) IRX1 | (19) LHX6 | (36) PAX1 | (53) GDNF |
| (3) SOX21 | (20) NEUROD1 | (37) FOXD2 | (54) FAT4 |
| (4) FGF5 | (21) AC149644.1 | (38) SLC6A15 | (55) HOXA2 |
| (5) ZNF471 | (22) CCDC48 | (39) PHC2 | (56) LPHN3 |
| (6) SUSD5 | (23) EVX1 | (40) FLRT2 | (57) ADCYAP1 |
| (7) FOXB1 | (24) GHSR | (41) GATA2 | (58) GRIA2 |
| (8) PDX1 | (25) HSD17B14 | (42) ADCY8 | (59) AQP1 |
| (9) DLX5 | (26) KRBA1 | (43) CNNM1 | (60) BCAT1 |
| (10) ONECUT2 | (27) OTOP1 | (44) IKZF1 | (61) CYP24A1 |
| (11) DMRTA2 | (28) PPYR1 | (45) NKX2-3 | (62) FOXI2 |
| (12) CMTM2 | (29) SRMS | (46) PCDH7 | (63) GSX1 |
| (13) OTX2 | (30) ZNF582 | (47) SNCB | (64) IRF4 |
| (14) LOC145845 | (31) IRX2 | (48) ST8SIA1 | (65) NPY |
| (15) EBF3 | (32) CSMD1 | (49) TRAPPC9 | (66) PDE1B |
| (16) SALL1 | (33) MIR675,H19 | (50) NKX2-2 | |
| (17) CBX8 | (34) FOXD3 | (51) SLC32A1 | |

in a biological sample from said individual wherein a lower level of expression of the DNA regions of group (i) and/or (ii) relative to control levels is indicative of a large intestine neoplasm or a predisposition to the onset of a neoplastic state.

The method of this aspect of the present invention is predicated on the comparison of the level of the neoplastic markers of a biological sample with the control levels of these markers. The "control level" may be either a "normal level", which is the level of marker expressed by a corresponding large intestine cell or cellular population which is not neoplastic.

As detailed hereinbefore, the normal (or "non-neoplastic") level may be determined using tissues derived from the same individual who is the subject of testing. However, it would be appreciated that this may be quite invasive for the individual concerned and it is therefore likely to be more convenient to analyse the test results relative to a standard result which reflects individual or collective results obtained from individuals other than the patient in issue.

There is therefore more particularly provided a method of screening for the onset or predisposition to the onset of or monitoring a large intestine neoplasm in an individual, said method comprising assessing the level of expression of one or more genes or transcripts selected from:
(i) the region defined by any one or more of Hg19 coordinates and 2kb upstream of the transcription start site:

| | |
|---|---|
| (1) chr12:52400748..52409671 | (6) chr3:33191537..33260707 |
| (2) chr5:3596168..3601517 | (7) chr15:60296421..60298142 |
| (3) chr13:95361876..95364389 | (8) chr13:28494168..28500451 |
| (4) chr4:81187742..81212171 | (9) chr7:96649702..96654143, |
| (5) chr19:57019212..57040270 | |

(ii) the gene region, including 2kb upstream of any one or more of:

| | | | |
|---|---|---|---|
| (1) GRASP | (4) FGF5 | (6) SUSD5 | (8) PDX1 |
| (2) IRX1 | (5) ZNF471 | (7) FOXB1 | (9) DLX5 |
| (3) SOX21 | | | |

in a biological sample from said individual wherein a lower level of expression of the gene or transcripts of group (i) and/or group (ii) relative to control levels is indicative of a neoplastic large intestine neoplasm or a predisposition to the onset of a neoplastic state.

Preferably, said control level is a non-neoplastic level.

As detailed hereinbefore, the present invention is designed to screen for a neoplastic cell or cellular population, which is located in the large intestine. Accordingly, reference to "cell or cellular population" should be understood as a reference to an individual cell or a group of cells. Said group of cells may be a diffuse population of cells, a cell suspension, an encapsulated population of cells or a population of cells which take the form of tissue.

Reference to "expression" should be understood as a reference to the transcription and/or translation of a nucleic acid molecule. Reference to "RNA" should be understood to encompass reference to any form of RNA, such as primary RNA or mRNA or non-translated RNA (e.g. miRNAs etc.). Without limiting the present invention in any way, the modulation of gene transcription leading to increased or decreased RNA synthesis may also correlate with the translation of some of these RNA transcripts (such as mRNA) to produce a protein product. Accordingly, the present invention also extends to detection methodology which is directed to screening for modulated levels or patterns of the neoplastic marker protein products as an indicator of the neoplastic state of a cell or cellular population. Although one method is to screen for mRNA transcripts and/or the corresponding protein product, it should be understood that the present invention is not limited in this regard and extends to screening for any other form of neoplastic marker expression product such as, for example, a primary RNA transcript.

In terms of screening for the downregulation of expression of a marker it would also be well known to the person of skill in the art that changes which are detectable at the DNA level are indicative of changes to gene expression activity and therefore changes to expression product levels. Such changes include but are not limited to, changes to DNA methylation. Accordingly, reference herein to "screening the level of expression" and comparison of these "levels of expression" to control "levels of expression" should be understood as a reference to assessing DNA factors which are related to transcription, such as gene/DNA methylation patterns. These have, in part, been described in detail hereinbefore.

It would also be known to a person skilled in the art that changes in the structure of chromatin are indicative of changes in gene expression. Silencing of gene expression is often associated with modification of chromatin proteins, methylation of lysines at either or both positions 9 and 27 of histone H3 being well studied examples, while active chromatin is marked by acetylation of lysine 9 of histone H3. Thus association of gene sequences with chromatin carrying repressive or active modifications can be used to make an assessment of the expression level of a gene.

Reference to "nucleic acid molecule" should be understood as a reference to both deoxyribonucleic acid molecules and ribonucleic acid molecules and fragments thereof. The present invention therefore extends to both directly screening for mRNA levels in a biological sample or screening for the complementary cDNA which has been reverse-transcribed from an mRNA population of interest. It is well within the skill of the person of skill in the art to design methodology directed to screening for either DNA or RNA. As detailed above, the method of the present invention also extends to screening for the protein product translated from the subject mRNA or the genomic DNA itself.

In one preferred embodiment, the level of gene expression is measured by reference to genes which encode a protein product and, more particularly, said level of expression is measured at the protein level.

In another particularly preferred embodiment, said gene expression is assessed by the association of DNA with chromatin proteins carrying repressive modifications, for example, methylation of lysines 9 or 27 of histone H3.

The present invention should be understood to encompass methods of detection based on identifying both proteins and/or nucleic acid molecules in one or more biological samples. This may be of particular significance to the extent that some of the neoplastic markers of interest may correspond to genes or gene fragments which do not encode a protein product. Accordingly, to the extent that this occurs it would not be possible to test for a protein and the subject marker would have to be assessed on the basis of transcription expression profiles or changes to genomic DNA.

The term "protein" should be understood to encompass peptides, polypeptides and proteins (including protein fragments). The protein may be glycosylated or unglycosylated and/or may contain a range of other molecules fused, linked, bound or otherwise associated to the protein such as amino acids, lipids, carbohydrates or other peptides, polypeptides or proteins. Reference herein to a "protein" includes a protein comprising a sequence of amino acids as well as a protein associated with other molecules such as amino acids, lipids, carbohydrates or other peptides, polypeptides or proteins.

The proteins encoded by the neoplastic markers of the present invention may be in multimeric form meaning that two or more molecules are associated together. Where the same protein molecules are associated together, the complex is a homomultimer. An example of a homomultimer is a homodimer. Where at least one marker protein is associated with at least one non-marker protein, then the complex is a heteromultimer such as a heterodimer.

Reference to a "fragment" should be understood as a reference to a portion of the subject nucleic acid molecule or protein. This is particularly relevant with respect to screening for modulated RNA levels in stool samples since the subject RNA is likely to have been degraded or otherwise fragmented due to the environment of the gut. One may therefore actually be detecting fragments of the subject RNA molecule, which fragments are identified by virtue of the use of a suitably specific probe.

Although the preferred method is to detect the expression product or DNA changes of the neoplastic markers for the purpose of diagnosing neoplasia development or predisposition thereto, the detection of converse changes in the levels of said markers may be desired under certain circumstances, for example, to monitor the effectiveness of therapeutic or prophylactic treatment directed to modulating a neoplastic condition, such as adenoma or adenocarcinoma development. For example, where reduced expression of the subject markers indicates that an individual has developed a condition characterised by adenoma or adenocarcinoma development, for example, screening for an increase in the levels of these markers subsequently to the onset of a therapeutic regime may be utilised to indicate reversal or other form of improvement of the subject individual's condition. The method of the present invention is therefore useful as a one off test or as an on-going monitor of those individuals thought to be at risk of neoplasia development or as a monitor of the effectiveness of therapeutic or prophylactic treatment regimes directed to inhibiting or otherwise slowing neoplasia development.

Means of assessing the subject expressed neoplasm markers in a biological sample can be achieved by any suitable method, which would be well known to the person of skill in the art. To this end, it would be appreciated that to the extent that one is examining either a homogeneous cellular population (such as a tumour biopsy or a cellular population which has been enriched from a heterogeneous starting population) or a tissue section, one may utilise a wide range of techniques such as *in situ* hybridisation, assessment of expression profiles by microassays, immunoassays and the like (hereinafter described in more detail) to detect the absence of or downregulation of the level of expression of one or more markers of interest. However, to the extent that one is screening a heterogenous cellular population or a bodily fluid in which heterogeneous populations of cells are found, such as a blood sample, the absence of or reduction in level of expression of a particular marker may be undetectable due to the inherent expression of the marker by non-neoplastic cells which are present in the sample. That is, a decrease in the level of expression of a subgroup of cells may not be detectable. In this situation, a more appropriate mechanism of detecting a reduction in a neoplastic subpopulation of the expression levels of one or more markers of the present invention is via indirect means, such as the detection of epigenetic changes.

Methods of detecting changes to gene expression levels (in addition to the methylation analyses hereinbefore described in detail), particularly where the subject biological sample is not contaminated with high numbers of non-neoplastic cells, include but are not limited to:
(i) *In vivo* detection.
   Molecular Imaging may be used following administration of imaging probes or reagents capable of disclosing altered expression of the markers in the intestinal tissues.
   Molecular imaging (Moore et al., BBA, 1402:239-249, 1988; Weissleder et al., Nature Medicine 6:351-355, 2000) is the *in* vivo imaging of molecular expression that correlates with the macro-features currently visualized using "classical" diagnostic imaging techniques such as X-Ray, computed tomography (CT), MRI, Positron Emission Tomography (PET) or endoscopy.
(ii) Detection of downregulation of RNA expression in the cells by Fluorescent *In Situ* Hybridization (FISH), or in extracts from the cells by technologies such as Quantitative Reverse Transcriptase Polymerase Chain Reaction (QRTPCR) or Flow cytometric qualification of competitive RT-PCR products (Wedemeyer et al., Clinical Chemistry 48:9 1398-1405, 2002).
(iii) Assessment of expression profiles of RNA, for example by array technologies (Alon et al., Proc. Natl. Acad. Sci. USA: 96:6745-6750, June 1999).
   A "microarray" is a linear or multi-dimensional array of preferably discrete regions, each having a defined area, formed on the surface of a solid support. The density of the discrete regions on a microarray is determined by the total numbers of target polynucleotides to be detected on the surface of a single solid phase support. As used herein, a DNA microarray is an array of oligonucleotide probes placed onto a chip or other surfaces used to amplify or clone target polynucleotides. Since the position of each particular group of probes in the array is known, the identities of the target polynucleotides can be determined based on their binding to a particular position in the microarray.
   DNA microarray technology make it possible to conduct a large scale assay of a plurality of target nucleic acid molecules on a single solid phase support. U.S. Pat. No. 5,837,832 (Chee et al.) and related patent applications describe immobilizing an array of oligonucleotide probes for hybridization and detection of specific nucleic acid sequences in a sample. Target polynucleotides of interest isolated from a tissue of interest are hybridized to the DNA chip and the specific sequences detected based on the target polynucleotides' preference and degree of hybridization at discrete probe locations. One important use of arrays is in the analysis of differential gene expression, where the profile of expression of genes in different cells or tissues, often a tissue of interest and a control tissue, is compared and any differences in gene expression among the respective tissues are identified. Such information is useful for the identification of the types of genes expressed in a particular tissue type and diagnosis of conditions based on the expression profile.
(iv) Measurement of altered neoplastic marker protein levels in cell extracts, for example by immunoassay.
   Testing for proteinaceous neoplastic marker expression product in a biological sample can be performed by any one of a number of suitable methods which are well known to those skilled in the art. Examples of suitable methods include, but are not limited to, antibody screening of tissue sections, biopsy specimens or bodily fluid samples. To the extent that antibody based methods of diagnosis are used, the presence of the marker protein may be determined in a number of ways such as by Western blotting, ELISA or flow cytometry procedures. These, of course, include both single-site and two-site or "sandwich" assays of the non-competitive types, as well as in the traditional competitive binding assays. These assays also include direct binding of a labelled antibody to a target.
(v) Determining altered expression of protein neoplastic markers on the cell surface, for example by immunohistochemistry.
(vi) Determining altered protein expression based on any suitable functional test, enzymatic test or immunological test in addition to those detailed in points (iv) and (v) above.

A person of ordinary skill in the art could determine, as a matter of routine procedure, the appropriateness of applying a given method to a particular type of biological sample.

A related aspect of the present invention provides a molecular array, which array comprises a plurality of:
(i) nucleic acid molecules comprising a nucleotide sequence corresponding to any one or more of the neoplastic marker DNA hereinbefore described or a sequence exhibiting at least 80% identity thereto or a functional derivative, fragment, variant or homologue of said nucleic acid molecule; or
(ii) nucleic acid molecules comprising a nucleotide sequence capable of hybridising to any one or more of the sequences of (i) under medium stringency conditions or a functional derivative, fragment, variant or homologue of said nucleic acid molecule; or
(iii) nucleic acid probes or oligonucleotides comprising a nucleotide sequence capable of hybridising to any one or more of the sequences of (i) under medium stringency conditions or a functional derivative, fragment, variant or homologue of said nucleic acid molecule; or
(iv) probes capable of binding to any one or more of the proteins encoded by the nucleic acid molecules of (i) or a derivative, fragment or, homologue thereof
wherein the level of expression of said marker genes of (i) or proteins of (iv) is indicative of the neoplastic state of a cell or cellular subpopulation derived from the large intestine.

Preferably, said percent identity is at least 85%, 90%, 91%, 92%, 93%, 94%, 95%, 96%, 97%, 98% or 99%.

"Hybridization" refers to the process by which a nucleic acid strand joins with a complementary strand through base pairing. Hybridization reactions can be sensitive and selective so that a particular sequence of interest can be identified even in samples in which it is present at low concentrations. Stringent conditions can be defined by, for example, the concentrations of salt or formamide in the prehybridization and hybridization solutions, or by the hybridization temperature, and are well known in the art. For example, stringency can be increased by reducing the concentration of salt, increasing the concentration of formamide, or raising the hybridization temperature, altering the time of hybridization, as described in detail, below. In alternative aspects, nucleic acids of the invention are defined by their ability to hybridize under various stringency conditions (e.g., high, medium, and low), as set forth herein.

Reference herein to a low stringency includes and encompasses from at least about 0 to at least about 15% v/v formamide and from at least about 1 M to at least about 2 M salt for hybridization, and at least about 1 M to at least about 2 M salt for washing conditions. Generally, low stringency is at from about 25-30°C to about 42°C. The temperature may be altered and higher temperatures used to replace formamide and/or to give alternative stringency conditions. Alternative stringency conditions may be applied where necessary, such as medium stringency, which includes and encompasses from at least about 16% v/v to at least about 30% v/v formamide and from at least about 0.5 M to at least about 0.9 M salt for hybridization, and at least about 0.5 M to at least about 0.9 M salt for washing conditions, or high stringency, which includes and encompasses from at least about 31% v/v to at least about 50% v/v formamide and from at least about 0.01 M to at least about 0.15 M salt for hybridization, and at least about 0.01 M to at least about 0.15 M salt for washing conditions. In general, washing is carried out Tₘ = 69.3 + 0.41 (G+C)% (Marmur and Doty, J. Mol. Biol. 5:109, 1962). However, the Tₘ of a duplex DNA decreases by 1°C with every increase of 1% in the number of mismatch base pairs (Bonner and Laskey, Eur. J. Biochem. 46:83, 1974). Formamide is optional in these hybridization conditions. Accordingly, particularly preferred levels of stringency are defined as follows: low stringency is 6 x SSC buffer, 0.1% w/v SDS at 25-42°C; a moderate stringency is 2 x SSC buffer, 0.1% w/v SDS at a temperature in the range 20°C to 65°C; high stringency is 0.1 x SSC buffer, 0.1% w/v SDS at a temperature of at least 65°C.

Where nucleic acids of the invention are defined by their ability to hybridize under high stringency, these conditions comprise about 50% formamide at about 37°C to 42°C. In one aspect, nucleic acids of the invention are defined by their ability to hybridize under reduced stringency comprising conditions in about 35% to 25% formamide at about 30°C to 35°C. Alternatively, nucleic acids of the invention are defined by their ability to hybridize under high stringency comprising conditions at 42°C in 50% formamide, 5X SSPE, 0.3% SDS, and a repetitive sequence blocking nucleic acid, such as cot-1 or salmon sperm DNA (e.g., 200 n/ml sheared and denatured salmon sperm DNA). In one aspect, nucleic acids of the invention are defined by their ability to hybridize under reduced stringency conditions comprising 35% formamide at a reduced temperature of 35°C.

Preferably, the subject probes are designed to bind to the nucleic acid or protein to which they are directed with a level of specificity which minimises the incidence of non-specific reactivity. However, it would be appreciated that it may not be possible to eliminate all potential cross-reactivity or non-specific reactivity, this being an inherent limitation of any probe based system.

In terms of the probes which are used to detect the subject proteins, they may take any suitable form including antibodies and aptamers.

A library or array of nucleic acid or protein probes provides rich and highly valuable information. Further, two or more arrays or profiles (information obtained from use of an array) of such sequences are useful tools for comparing a test set of results with a reference, such as another sample or stored calibrator. In using an array, individual probes typically are immobilized at separate locations and allowed to react for binding reactions. Primers associated with assembled sets of markers are useful for either preparing libraries of sequences or directly detecting markers from other biological samples.

A library (or array, when referring to physically separated nucleic acids corresponding to at least some sequences in a library) of gene markers exhibits highly desirable properties. These properties are associated with specific conditions, and may be characterized as regulatory profiles. A profile, as termed here refers to a set of members that provides diagnostic information of the tissue from which the markers were originally derived. A profile in many instances comprises a series of spots on an array made from deposited sequences.

A characteristic patient profile is generally prepared by use of an array. An array profile may be compared with one or more other array profiles or other reference profiles. The comparative results can provide rich information pertaining to disease states, developmental state, receptiveness to therapy and other information about the patient.

Another aspect of the present invention provides a diagnostic kit for assaying biological samples comprising one or more agents for detecting one or more neoplastic markers and reagents useful for facilitating the detection by said agents. Further means may also be included, for example, to receive a biological sample. The agent may be any suitable detecting molecule.

The present invention is further described by reference to the following non-limiting examples.

### EXAMPLE 1

Bisulfite-tag technology was applied to produce methylated and unmethylated genome fractions as described below. Briefly, DNAs were digested with methylation insensitive enzymes MspI and TaqI and treated with sodium bisulfite under non-denaturing conditions, such that the cytosine in the 5'-CG single-stranded overhang left by each restriction enzyme would be converted to uracil if it were unmethylated but would remain unconverted if methylated. Separate linkers with either 5'-CG or 5'-CA overhangs were ligated to provide linkered methylated and unmethylated fractions respectively. After incorporation of a second primer by random-primed copying of a reverse strand this common primer was used in combination with the appropriate forward primer to amplify the methylated and unmethylated fractions.

In detail, tumour and matched normal DNA samples from eight patients were processed and analysed as described below.
1. DNAs from cancer and normal tissues (1 to 2 ug) were sheared by sonication using a Bioruptor UCD-200 sonicator (Diagenode, Belgium) in 300 µL of 10 mM Tris, 0.1 mM EDTA, pH 7.5 at a power setting of "high" for 60 minutes on ice, with alternating cycles of 30 seconds "on" or "off'. A small part of each sample was then applied to a 2% agarose gel, to confirm that a mean size range of 200 to 300 bp had been reached. DNA was precipitated and resuspended in water.
2. The volume was adjusted to 20 µL in Antarctic Phosphatase reaction buffer (New England Biolabs) and DNA de-phosphorylated using 5 units of Antarctic phosphatase at 37°C for 60 min. The enzyme was then inactivated by heating to 65°C for 10 min.
3. Sequential restriction enzyme digestion: 3 µL of 10X New England Biolabs Buffer 4 and 2 µL, 20 units, of *Msp*I enzyme were added and incubation continued at 37°C for 2 hr. 20 units, 2 µL, of *Taq*I and BSA to 100 µg/mL were added to give a volume of 28 µL and incubation continued at 65°C for a further 2 hr. Incubation was stopped by addition of 2 µL of 500 mM EDTA.
4. The cut DNA (1 µg) was reacted with sodium bisulfite, using the MethylEasy kit from Human Genetic Signatures (North Ryde, Australia), but omitting any prior heating or alkali denaturation step. Briefly:
(a) 25-30 uL of restriction digested DNA was combined in a 2 mL tube with 250 µl of sodium bisulfite, then incubated at 37°C for 4 hours while omitting mineral oil.
(b) 20 µg of blue-dye-labelled glycogen was added and DNA precipitated with 750 µL of MethylEasy solution 4 and 1 ml of isopropanol. Following centrifugation and washing of the pellet with 70% ethanol DNA was dissolved in 12 µL of MethylEasy Solution 3. Alternatively excess bisulfite was removed using a small silica column, and sample eluted from the column in 16 µL of MethylEasy Solution 3.
(c) Desulphonation was achieved by heating in MethylEasy solution 3 for 60 minutes at 72° C, while spinning down several times to reduce evaporation.

5. Ligation of CG and CA linkers. Separate ligations were set up with P1-CG or P1-CA linkers (Table 1) as described below. Both P1-CG and P1-CA linkers (Table 1) were modified by incorporation of a biotin at the 3' end of the upper strand to allow subsequent capture.

**Table 1. Sequences of detection facilitation and competitor linkers**

| | | |
|---|---|---|
| P1-CG linker-upper | 5'-pCGCGTATCACCGACTGCCCTT-biotin-3' | SEQ ID NO:1 |
| P1-CG linker-lower | 5' -GGGCAGTCGGTGATACG-3' | SEQ ID NO:2 |
| | | |
| P1-CA linker-upper | 5'-pCAGCAATCACCGACTGCCCTT-biotin-3' | SEQ ID NO:3 |
| P1-CA linker lower | 5'-GGGCAGTCGGTGATTGC-3' | SEQ ID NO:4 |
| | | |
| CG-competitor-upper | 5'-ACAGAGTCGTA-3' | SEQ ID NO:5 |
| CG-competitor-lower | 5'-CGTACGACTCTG-3' | SEQ ID NO:6 |
| | | |
| CA-competitor-upper | 5'-AGTGATCAGCA-3' | SEQ ID NO:7 |
| CA-competitor-lower | 5'-pCATGCTGATCAC-3' | SEQ ID NO: 8 |
| | | |
| P2-N6 primer | 5'-CTGCCCCGGGTTCCTCATTCTCTNNNNNN-3' | SEQ ID NO:9 |
| P2-long primer | 5'-CTGCCCCGGGTTCCTCATTCT-3' | SEQ ID NO:10 |

Mixes of combined linkers and competitors were prepared as in Table 2 below. These amounts, and ratios, are for 1000ng of DNA of average length 200 bp.

**Table 2**

| Ligation | Linker (20 bp) | Competitor (10 bp) |
|---|---|---|
| CG | 200 ng (2:1) | 200 ng (4:1) |
| CA | 1000 ng (10:1) | 100 ng (2:1) |

Ligation reactions with 1 µg of bisulfite treated DNA were set up in 50 µL with 0.6 µL (12 units) of T4 DNA ligase and incubated at room temperature for 5 hr. After ligation, DNA was purified through a Promega Wizard column and eluted with 2 x 50 µL of 10 mM Tris.HCl, 0.1 mM EDTA, pH8. This step removes the unligated linkers.
6. Bead capture and release of single-strand: 25 µL of Dynal M280 streptavidin beads were first washed twice with 500 µl of 2X salt wash buffer (2M NaCl, 10mM Tris pH7.5, 1mM EDTA, 0.2% Tween 20) and resuspended in 100ul of 2x wash buffer. The 100µL of eluted DNA was added and mixed on a shaker at room temperature with the beads for 15min at 500rpm. Beads were magnetically captured for 1 min and the supernatant removed. This was followed with two washes in 500 µL of 1X wash buffer, once with 500 µL of 1 x SSC and then with 500 µL of 0.5 x SSC at 65°C for 3 min with shaking.
7. The beads were then resuspended in 50 µL of 10 mM Tris.HCl, 0.1 mM EDTA, pH8 and transferred to a new tube. After incubation at 98°C for 5 min and chilling for 1 min on ice, the beads were magnetically captured for one minute on ice and the supernatant (50 µL) transferred to a fresh tube. After drying, the DNA was resuspended in 10 µL of water.
8. Random priming .The P2 primer sequence was incorporated by random priming using a P2-N6 primer (Table 1). The 10 µL of eluted single-stranded DNA was mixed with 500 ng (5 µL) of P2-N6 primer, heated at 98°C for 2 min and chilled on ice. To this mix was added, 2.5ul 10x New England Biolabs buffer 2, 0.625ul 10mM dNTPs, 0.625ul Klenow fragment of DNA polymerase (5000u/ul) and the volume adjusted to 25 µL. After incubation on ice for 10 min, then at room temperature for 60 min, the reaction was terminated by heating at 75°C for 20 min.
9. Amplification . After trial reactions using 1 µL of random primed DNA, large to estimate amplification cycles, 300 µL amplification reactions for each of the methylated and unmethylated fractions were set up as follows:
10 µL random primed DNA sample
150 µL Promega GoTaq 2x Hot start mastermix
18 µL P1-CA or P1-CG primer (Table 1, 5uM stock)
18 µL P2-long primer (Table 1, 5uM stock)
7.5 µL Sybr Green (1:3000 dilution of 10000x concentration)
96.5 µL water
Samples were run on a Roche LightCycler 480, SYBR green programme: 95°C 2min x1 cycle, and 95°C 15sec, 60°C 1min x 40 cycles. Fluorescence was monitored and reactions stopped at the top of the exponential phase. DNA was purified using Promega Wizard columns, eluted in 30 µL water and quantified by spectrophotometry.
10. Labelling and hybridisation. 20 µg samples of each fraction were fluorescently labelled using the Nimblegen Dual-Color DNA labelling kit but with the Cy3 and Cy5 random nonamers being substituted with Cy3 and Cy5 random hexamers (Geneworks, Adelaide, Australia). Methylated DNA fractions were labelled with Cy3 and unmethylated with Cy5 dyes.
Methylated fractions of cancer and normal DNAs were co-hybridised with a Nimblegen 720K promoter tiling array following Nimblegen protocols and the unmethylated fractions co-hybridised to a separate array according to manufacturer's protocols. Arrays were scanned on an Axon scanner
In addition to individual samples, four pools of DNA were prepared for fluorescent labelling (as well as four additional pools in which Cy3 label was used for the cancer DNAs and Cy5 for normal)-
Pool 1 combined the methylated DNA fractions from the cancer tissues: cy5 label
Pool 2 combined the methylated DNA fractions from matched normal tissues: cy3 label
Pool 3 combined the unmethylated DNA fractions from the cancer tissues; cy5 label
Pool 4 combined the unmethylated DNA fractions from matched normal tissues: cy3 label

### Identification of differentially methylated genes

Data from microarrays for the eight individual cancer and normal pairs as well as the analysis of pooled DNA and the dye-swap hybridisation with pooled DNAs were combined as described below to identify the most differentially methylated genes. Differential methylation is assessed via a difference-difference score, DD. This defined for each probe as

DD = (Y.meth.tumour - Y.unmeth.tumour) - (Y.meth.normal - Y.unmeth.normal) where each Y value is the base-2 logarithm of the raw probe response value for the given combination of methylation status (meth/unmeth) and disease status (tumour/normal). Larger values of DD mean methylation is increased in the tumour samples relative to normal samples. For each tiled region probes were ranked in order of highest DD as DD1, DD2, DD3 etc.

Two gene lists were created, one that was based on the differential methylation for the two most differential probes in a tiled region (DD2) and one that was based on the top four differential probes (DD4).

For the DD2 list data for where the probe quality as scored by Nimblegen is beyond the threshold of 1.5 were discarded. This threshold accepts about 17% of all probes and minimises the potential for "noise' when only two probes are considered. For each tiled region, the DD2 values for the eight individual samples were combined via a median. Then an overall DD2 value across all samples obtained from the average of three values: this median, and the two pooled sample values, forward and dye-swap. All tiled regions were then ranked by this score, and the top 30 returned.

For the DD4 list where a greater number of probes per tiled region were considered, a quality score threshold of 4 (that accepts about 40% of probes) was used. The overall DD4 value for each tiled region was determined as for DD2 except using the individual DD4 values in each case.

Table 3 shows a list of 44 genes shown to be differentially methylated between cancer and normal DNAs, based on either the level of the differential signal based on the DD2 or DD4 analysis. The list is ordered by the difference based on 4 probes, and then 2 probes. The top 4 ranked probes (and 9 in all) appear in the top 30 gene list derived by both methods. The hg18 coordinates of the tiled regions on the Nimblegen arrays showing differential methylation are given in the first column. Corresponding gene names where available linked to tiled regions and the coordinates of the genes (hg19) are shown in the right two columns. DD plots across the tiled regions for each gene are shown in Figure 1.

**Table 3**

| **Tiled region (hg18)** | **Summary DD2** | **Summary DD4** | **Gene symbols** | **Gene Location Build 37.1 (hg19)** |
|---|---|---|---|---|
| chr13:27399624-27401566 | 3.849 | 3.202 | PDX1 | Chr13, (28494168..28500451) |
| chr19:61708583-61711633 | 2.968 | 3.111 | ZNF471 | Chr19 (57019212..57040270) |
| chr8:141177019-141180166 | 3.369 | 3.023 | TRAPPC9 | Chr8 (140,811,770-141,537,860) |
| chr4:81404325-81407375 | 3.302 | 2.965 | FGF5 | Chr4 (81187742..81212171) |
| chr5:2801368-2810024 | | 2.670 | IRX2 | Chr5 (2746279..2751769) |
| chr20:21433932-21445104 | 2.846 | 2.597 | NKX2-2 | Chr20 (21491648..21494664) |
| chr18:53251474-53259851 | | 2.585 | ONECUT2 | Chr18 (55102917..55158530) |
| chr5:3646727-3656054 | | 2.525 | IRX1 | Chr5 (3596168..3601517) |
| chr20:36784078-36790786 | 2.943 | 2.502 | SLC32A1 | Chr20 (37353101..37358016) |
| chr1 :50656815-50664169 | | 2.487 | DMRTA2 | Chr1 (50883225..50889141) |
| chr8:4836376-4842176 | | 2.480 | CSMD1 | Chr8 (2792875..4852328) |
| chr7:27149138-27152252 | | 2.474 | HOXA5 | Chr7 (27180996..27183287) |
| chr16:65168411-65171461 | | 2.471 | CMTM2 | Chr16 (66613351..66622178) |
| chr11:1970000-2050000 | | 2.462 | MIR675,H19 | Chr11 (2016406..2019065) |
| chr5:37870045-37877979 | 3.052 | 2.455 | GDNF | Chr5 (37815753..37839782) |
| chr14:56341488-56349377 | | 2.450 | OTX2 | Chr14 (57267425..57277184) |
| chr1 :63554982-63563059 | | 2.438 | FOXD3 | Chr1 (63788730..63790797) |
| chr4:126454576-126458380 | 2.791 | 2.437 | FAT4 | Chr4 (126237567..126414087) |
| chr15:34965416-34968466 | | 2.429 | LOC145845 | Chr15 (37156644..37178734) |
| chr8:23618420-23622307 | 3.002 | 2.427 | NKX2-6 | Chr8 (23559964..23563922) |
| chr4:111758454-111759816 | | 2.424 | | |
| chr7:27108309-27111359 | | 2.423 | HOXA2 | Chr7 (27139973..27142394) |
| chr10:131651159-131661686 | | 2.401 | EBF3 | Chr10 (131633547..131762091) |
| chr20:21631732-21635689 | | 2.388 | PAX1 | Chr20 (21686297..21696620) |
| chr4:61748478-61751396 | | 2.385 | LPHN3 | Chr4 (62362839..62938168) |
| chr16:49741200-49746264 | | 2.385 | SALL1 | Chr16 (51169886..51185183) |
| chr1 :47682299-47683607 | 4.570 | | FOXD2 | Chr1 (47901689..47906363) |
| chr7:96488157-96489487 | 3.812 | | DLX5 | Chr7 (96649702..96654143,) |
| chr17:75384875-75393063 | 3.431 | | CBX8 | Chr17 (77768176..77770890) |
| chr12:83829092-83833177 | 3.388 | | SLC6A15 | Chr12 (85253267..85306606) |
| chr18:892503-899574 | 3.303 | | ADCYAP1 | Chr17 (93598762..93604831) |
| chr8:6407582-6410632 | 3.158 | | ANGPT2 | Chr8 (6357172..6420784) |
| chr13:78079121-78080434 | 3.056 | | | |
| chr6:29629089-29629812 | 3.022 | | | |
| chr1:33613171-33616221 | 2.936 | | PHC2 | Chr1 (33789224..33841194) |
| chr9:124021356-124022656 | 2.899 | | LHX6 | Chr9 (124,004,679-124,030,840) |
| chr14:85063800-85066850 | 2.898756 | | FLRT2 | Chr14 (85996488..86094270) |
| chr4:158358745-158361879 | 2.84852 | | GRIA2 | Chr4 (158141736..158287227) |
| chr2:182253016-182256066 | 2.837453 | | NEUROD1 | Chr2 (182541194..182545381) |
| chr3:129688182-129697160 | 2.83441 | | GATA2 | Chr12 (9445136..9462559) |
| chr7:30915552-30918602 | 2.787447 | | AQP1 | Chr7 (30951468..30965131) |

The double-difference plots of these differentially methylated genes are shown in Figure 1. In these plots, low signals are expected where probes lie >300 bp from a restriction site (because of the sheared length of fragments that all have one end located at a restriction site), or where a restriction site lies within a probe, shortening the size of the hybridising region. Extensive differential methylation is seen across the tiled regions of some genes, PDX1 and TRAPP9C for example, while in some cases increased methylation is confined to part of the tiled region, for example the right hand half of ZNF471.

### EXAMPLE 2

Methylated DNA fractions from bisulfite-treated DNA of the colorectal cancer cell lines HCT116, HT29 and SW480 and from DNA isolated from whole blood were prepared using a biotin capture method described below and libraries of methylated DNA were sequenced using the Applied Biosystems SOliD sequencing system. Briefly, DNAs were sheared and modified SOLiD P2 linkers ligated to the sheared DNAs. DNAs were then cut with Csp61 (cut site G'TAC) and ligated with modified SOLiD P1 linkers. DNA was then denatured and treated with sodium bisulfite to convert all unmethylated cytosines to uracil. The bisulfite treated DNA was then copied using a modified P2 primer and the original, uracil-containing bisulfite-treated DNA strand removed. The P1 forward primer was then used to prime forward strand synthesis in the presence of biotin-dCTP. Thus biotin dCTP was incorporated in positions that that contained methylated cytosine in the original DNA, and hence were not converted to uracil. The biotin containing fraction, representing molecules containing methylated cytosines and lying adjacent to Csp61 sites, was captured on magnetic beads and subsequently amplified for high throughput sequencing. Details of the protocol are as below:
1. Genomic DNAs, 2 ug, were sonicated as in Example 1 to produce DNA fragments ranging in size from 90 to 1000 bp, with most molecules ranging between 100 - 230 bp.
   DNA was end-repaired to ensure flush ends and 5' phosphorylation for efficient ligation using the End-it kit (Epicentre Biotechnologies). Reactions were done in 1× End-it buffer, 1 mM ATP, 0.25 mM of each dNTP and 1 µL of End-it enzyme mix (T4 DNA polymerase and polynucleotide kinase). After incubation at room temperature for 45 min reactions were heated to 70°C for 10 min to inactivate the T4 DNA polymerase.
2. Repaired DNA was purified and concentrated with a Qiagen MinElute reaction clean up kit. DNA was eluted in 2 x 16.5 µL of Qiagen Elution Buffer (EB). The eluate was adjusted to 50 µl of NEBuffer 2 (50 mM NaCl, 10 mM Tris-HCl, 10 mM MgCl₂, 1 mM dithiothreitol, pH7.9 @ 25°C) containing 200 µM dATP and incubated with 50 units of 3'→5' exonuclease N-terminal truncated Klenow fragment of DNA polymerase (new England Biolabs). The reaction was stopped by heating at 75°C for 20 min.
3. Repaired, A-tailed DNAs were ligated with annealed linkers SOLP2-AB/SOLP2-BP. The linker is adapted from the Applied Biosystems P2 linker used in its SOLiD high throughput sequencing system, the underlined bases in SolP2-BP having replaced cytosines present in the original sequence. The SolP2-BP sequence contains no cytosines so that subsequent incorporation of biotin dCTP will be restricted to the insert sequence.
   SolP2-AB 5' CCTACCCCACATTCCTCATTCTCT SEQ ID NO:11
   SolP2-BP 3' TTGGATGGGGTGTAAGGAGTAAGAGp SEQ ID NO:12

   Complementary oligonucleotides were combined in a 0.2 mL PCR tube with Quick ligase buffer (New England Biolabs) to give a final 500 µM concentration. The oligonucleotides were annealed by using a thermal cycler as specified in the Applied Biosystems SOLiD library preparation appendices.
   DNA ligations were done in Quick Ligase buffer (66 mM Tris-HCl, 10 mM MgCl₂, 1 mM dithiothreitol, 1mM ATP, 7.5% w/v polyethylene glycol 6000, pH7.5 @ 25°C), using 1 µL of Quick ligase per 40 µL of reaction (New England Biolabs). The ratio of linkers to DNA fragment ends was approximately 10 or 15 to 1. Linkers were removed using a QiaQuick PCR purification kit (Qiagen) and DNA eluted in 40 or 50 µL of EB.
4. Subsequent to the first linker ligation, the DNA was cut with the restriction enzyme Csp61 (G'TAC) that leaves a 5'-TA-3' overhang. DNAs were cut overnight in restriction enzyme buffers provided by the supplier (Fermentas, buffer B) with 10 U of Csp6I.
   These ends were then ligated with the hemimethylated SolP1 linker in which the cytosines on the upper strand SolP1-AM had been replaced with 5-methyl cytosines (shown as "M") and the a 5'-TA-3' overhang, underlined, added to the original SolP2-B sequence.
   SolP1-AM 5' MMAMTAMGMMTMMGMTTTMMTMTMTATGGGMAGTMGGTGAT SEQ ID NO:13
   SolP1-BC 3' CGGTGATGCGGAGGCGAAAGGAGAGATACCCGTCAGCCACTAAT SEQ ID NO:14
5. Following P1 linker ligation, DNA was denatured and treated with sodium bisulfite using a MethylEasy kit (Human Genetic Signatures) and resuspended in 30 µL of HGS reagent 3. In the upper strand, the methyl cytosines in the P1 linker are protected from conversion and the P2 linker strand contains no cytosines, restricting C to U conversion to the insert region. Due to the single stranded nick, after denaturation only the upper strand will have adaptors at both ends.
6. The primer SolP2-AB was used to prime and extend to synthesise a copy of the converted DNA. Briefly, bisulfite-treated DNA (1 µg) was incubated with Invitrogen Platinum Taq DNA polymerase (1 µL, 5 units) in 25 µL Platinum Taq buffer containing 50 µM dNTPs, 2.5 mM MgCl₂ and 500 nmol SolP2-AB primer. After heating at 94°C for 3 min the reaction was incubated for 15 min at 65°C. The 25 µL of primed strand reaction had 2.5 µL of Arctic phosphatase buffer added along with 5 U of Arctic phosphatase (to remove dNTP pool). Incubation was for 30 min at 37°C with subsequent denaturation of the Antarctic phosphatase for 8 min at 65°C.
7. Following synthesis of the complementary strand, the original bisulfite-treated strand was degraded by treatment with USER enzyme mix that cleaves DNA at positions of uracil bases. The remaining strand was then used as a template for synthesis of the strand corresponding to the original bisulfite treated strand, with incorporation of biotin-dCTP for specific tagging of methylated sites. The reaction tube was cooled and a mixture of reagents was added to bring the volume to 50 µL with a final concentration of 100 µM deoxyadenosine-triphosphate, 100 µM deoxyguanosine-triphosphate, 100 µM deoxythymidine-triphosphate and 50 µM biotin-14-deoxycytidine-triphosphate along with 500 nM of SolP1-A primer, 1 U of hot-start Taq, 2 U of uracil-specific excision reagent, USER enzyme mix (New England Biolabs) and 1× Taq buffer. The reaction was incubated for 10 min at 37°C to allow uracil DNA glycosylase to degrade uracil containing bisulfite treated DNA before denaturation for 2 min at 94°C and extension of the template for 10 min at 65°C. A final aliquot containing 2.5 nmol of unlabeled deoxycytidine-triphosphate was then added, with a further 5 min incubation at 65°C. The product was purified with a QiaQUICK PCR purification kit (Qiagen) and eluted in 200 µL of EB.
8. The DNA was then incubated with streptavidin beads in order to bind material labelled with biotin. A series of wash steps selectively enriched for this bound material. Heat denaturation in water allowed the enriched material to be recovered. Bovine serum albumin was used as a blocking agent and a surfactant in the wash steps used to increase the stringency of enrichment. Briefly, Invitrogen M-270 streptavidin magnetic beads were prepared according to the manufacturer's specifications then washed in 500 µL of 0.1 mg/mL bovine serum albumin before resuspension in 200 µL of 2× binding and wash (B&W) buffer (2 mol/L NaCl, 20 mmol/L Tris-HCl [pH 7.2], 2 mmol/L EDTA and 0.2% v/v Tween80) and the addition of equivolume purified labelled DNA solution. In some instances 10 pg of each control annealed oligonucleotide DNA was also 'spiked-in'. The 400 µL mixed solution was incubated with gentle rocking for 30 min at 37°C. The tube was placed on a magnet for 3 min before the supernatant was aspirated and kept. Two extra washes were performed - the first with 500 µL 1× B&W buffer and the second with 550 µL water. Then beads were resuspended in 45 µL of water and the tube heated to 90°C for 2' and placed immediately on a magnet. The heated water was aspirated as soon as the magnetic beads cleared from solution, 5 µL of 100 mM Tris-HCl pH 8.0, 1 mM EDTA solution was added and the tube. The captured and released fraction was aspirated, fractions were ethanol precipitated and resuspended in 50 µL of 10 mM Tris-HCl pH 8.0, 0.1 mM EDTA.
9. Captured DNA was amplified for a limited number of cycles with a hot start Taq polymerase in the presence of 1000 nM of the standard SOLiD P1 and P2 primers. The reverse primer was used to modify the amplicon sequence to match that of the original Applied Biosystems primer 2 sequence. The illegitimate mismatch priming in the first few amplification cycles required a lower annealing temperature. The amplification used 10 sec denaturation steps at 94°C and 30 second extension steps at 72°C with annealing steps at 50°C for the first four cycles before moving to a 62°C annealing temperature for subsequent cycles. PCR products were purified and quantitated then another few final rounds of PCR were performed with 2 U Phusion Taq (Finnzymes) in 100 µL Phusion Taq High-Fidelity buffer, 1000 nM primers and 10 sec denaturation steps at 98°C, 30 second annealing steps at 62°C and 30 second extension steps at 72°C.
10. Reaction products were run on Low-Range agarose (Biorad) gels, stained with SYBR Gold (Invitrogen) and 125-200 nucleotide DNA was cut from the agarose with a scalpel under blue light on a Safe Imager (Invitrogen). Cut agar was processed with a Wizard SV Gel extraction and PCR clean up kit (Promega) according to the manufacturer's instructions, excepting that the agarose was dissolved at room temperature.

The PCR products were purified using a Qiagen MinElute kit, quantitated with a spectrophotometer (Nanodrop) and sequenced on a Applied Biosystems SOLiD apparatus using SOLiD version 3 chemistry.

The bisulfite sequencing reads were aligned to the human genome and the number of reads of a CpG at each potential position within the 50 base reading distance on each side of a Csp61 site was determined for each DNA sample. Each read of a CpG in he bisulfite sequencing reads corresponds to a methylated CpG in the original DNA. For each DNA sample the sums of methylated CpG sites read in the region from 2 kb upstream to 1 kb downstream of the most upstream annotated transcription start site of a gene was computed. This allowed identification of genes and promoter regions where there was significant differential methylation comparing each of the colon cancer cell line DNAs with blood DNA. The bisulfite-tag methylation profiles of these genes obtained from analysis of 8 pooled cancer DNA and 8 pooled normal DNA samples (Figure 2) were then used to develop a list of genes identified through the biotin capture method that also showed significant differential methylation in clinical samples (Table 4). In Table 4, the gene or locus name is shown in the first column. The chromosomal locations of the associated genes are shown in the next two columns and the fourth and fifth columns give the positions of the first and last CpG sites sequenced in the biotin capture analysis. The number of CpG sites analysed and the total number of methylated CpG counted in each DNA sample are provide in the subsequent columns. The clear differences in methylation levels between the colon cancer cell line DNAs and blood DNA in these regions are evident.

Notably, five genes identified with an asterisk in Table 4, IRX1, DLX5, SALL1, SLC32A1 and ZNF471, had been also identified as highly differentially methylated genes using the bisulfite-tag method for primary screening.

Also notably, related members of the same gene families were found by one or both methods to be differentially methylated in colon cancer compared with normal tissue. This included genes of the IRX family, IRX1 and IRX2, of the FOXD family, FOXD2 and FOXD3, of the HOXA family, HOXA2 and HOXA5, and of the NKX2 family, NKX2-2, NKX2-3 and NKX2-6.

**Table 4**

| **Gene** | **Chrom** | **Gene Location (Hg19)** | **CG positions** | | **number** | **Number of methylated CGs** | | | |
|---|---|---|---|---|---|---|---|---|---|
| | | | **Start** | **End** | **CGs** | **HT29** | **HCT116** | **SW480** | **Blood** |
| ADCY8 | 8 | 131792547..132052835 | 132051842 | 132052875 | 11 | 312 | 170 | 171 | 2 |
| BCAT1 | 12 | 24964278..25102308 | 25102018 | 25102062 | 7 | 113 | 96 | 414 | 0 |
| CCDC48 | 3 | 128749292..128759583 | 128720532 | 128720688 | 18 | 289 | 96 | 315 | 0 |
| CNKR2 | X | 21542357..21690352 | 21391914 | 21393363 | 16 | 257 | 116 | 264 | 0 |
| CNNM1 | 10 | 101088856..101154087 | 101088918 | 101089825 | 15 | 181 | 172 | 222 | 1 |
| CYP24A1 | 20 | 52769988..52790516 | 52789985 | 52791441 | 13 | 274 | 156 | 209 | 3 |
| *DLX5 | 7 | 96649702..96654143 | 96653644 | 96654348 | 6 | 135 | 61 | 169 | 5 |
| EVX1 | 7 | 27282164..27286192 | 27281415 | 27281581 | 4 | 85 | 48 | 114 | 2 |
| FOXB1 | 15 | 60296421..60298142 | 60296891 | 60297252 | 14 | 179 | 187 | 213 | 0 |
| FOXI2 | 10 | 129535538..129539450 | 129534433 | 129535909 | 20 | 208 | 300 | 295 | 0 |
| GHSR | 3 | 172162951..172166203 | 172165903 | 172167853 | 4 | 96 | 63 | 134 | 4 |
| GRASP | 12 | 52400748..52409671 | 52401554 | 52401644 | 10 | 198 | 163 | 298 | 0 |
| GSX1 | 13 | 28366780..28368089 | 28366410 | 28367139 | 12 | 195 | 99 | 241 | 3 |
| HSD17B14 | 19 | 49316274..49339934 | 49339630 | 49340542 | 13 | 330 | 137 | 285 | 0 |
| IKZF1 | 7 | 50344378...50472799 | 50343280 | 50344174 | 36 | 623 | 258 | 999 | 0 |
| IRF4 | 6 | 391752..411443 | 391866 | 392501 | 15 | 222 | 118 | 212 | 0 |
| *IRX1 | 5 | 3596168..3601517 | 3594712 | 3596293 | 20 | 315 | 156 | 387 | 1 |
| KRBA1 | 7 | 149412148..149431664 | 149411473 | 149412383 | 19 | 240 | 170 | 367 | 0 |
| NKX2-3 | 10 | 101292690..101296281 | 101290914 | 101291177 | 13 | 144 | 208 | 185 | 0 |
| NPY | 7 | 24323809..24331477 | 24323101 | 24323905 | 15 | 273 | 152 | 442 | 0 |
| OTOP1 | 4 | 4190530..4228621 | 4228767 | 4229372 | 6 | 195 | 88 | 104 | 4 |
| PCDH7 | 4 | 30722037..31148421 | 30723110 | 30723305 | 17 | 215 | 196 | 182 | 0 |
| PDE1B | 12 | 54943404..54973023 | 54943115 | 54943207 | 11 | 148 | 112 | 247 | 0 |
| PPYR1 | 10 | 47083534..47088320 | 47083548 | 47083625 | 12 | 258 | 220 | 310 | 2 |
| *SALL1 | 16 | 51169886..51185183 | 51186808 | 51187120 | 16 | 170 | 154 | 223 | 0 |
| *SLC32A1 | 20 | 37353101..37358016 | 37352042 | 37352086 | 4 | 114 | 97 | 36 | 3 |
| SNCB | 5 | 176047210..176057557 | 176057982 | 176057996 | 3 | 70 | 59 | 88 | 4 |
| SOX21 | 13 | 95361876..95364389 | 95363446 | 95364116 | 25 | 348 | 135 | 454 | 0 |
| ST8SIA1 | 12 | 22346325..22487648 | 22486936 | 22488797 | 20 | 261 | 173 | 421 | 0 |
| SUSD5 | 3 | 33191537..33260707 | 33260578 | 33260615 | 6 | 84 | 87 | 213 | 0 |
| *ZNF471 | 19 | 57019212..57040270 | 57018864 | 57019470 | 20 | 415 | 127 | 440 | 7 |
| ZNF582 | 19 | 56894648..56904889 | 56904842 | 56904923 | 12 | 311 | 154 | 173 | 0 |

### EXAMPLE 3

### DNA METHYLATION PROVILES OF SELECTED GENES IN COLORECTAL CANCER AND NORMAL TISSUE DNA

Primers were designed for methylation status independent amplification of gene and/or promoter regions for a set of genes identified in the previous Examples. The genes, primers and chromosomal co-ordinates of amplicons are shown in Table 5,

The primers were used for PCR from bisulfite treated DNA of 10 colorectal cancer specimens, their matched normal tissue and normal blood DNA. Amplification was done using Promega GoTaq master mix (without SybrGreen), 4mM MgCl₂ and with primers at 200nM and 10ng of input DNA. Cycling conditions were 95°C, 2 min (1 cycle), followed by 50 cycles of 95°C 15 sec, N°C 30 sec; 72°C 30 sec. where the annealing temperature N for each amplicon is shown in Table 5. For some amplicons an additional 200µM of dATP and dTTP was added to enable comparable amplification of both methylated and unmethylated DNA sequences. Amplified bands of DNA were purified and equivalent amounts of the separate amplicons derived from each DNA sample were pooled and ligated with linkers for sequencing on the Roche 454 Titanium FLX system. Samples from individual patient's cancer or normal DNA and the blood DNA sample were separately ligated with bar coded "MID" linkers (Roche Cat No 05619211001) so that sequence reads could later be assigned to individual samples for sequence alignment and scoring. Libraries were prepared following protocols provided with the Roche Library preparation kit and reagents and sequenced on two halves of a flow cell; one half contained all the cancer samples and one the equivalently bar-coded normal samples. The bisulphite sequencing reads were segregated to individual samples using the bar-code sequences and aligned with the bisulphite converted sequence of each amplicon. After best alignment, the fraction of cytosines at each potential CpG methylation site was determined for each sample.

Profiles across individual amplicons are shown in Figures 3-27. The data for 57 amplicons representing 24 genes or regions is summarised in Table 6. The table shows the approximate range of methylation levels at CpG sites across each amplicon for the individual cancer samples, A-J and combined data for the 10 matched normal DNAs. Columns show headed m/10 and p/10 the number of cancer samples out of 10 showing high level (>60%) or partial (30-60%) methylation across the amplicon. Methylation of two of these genes, ADAMTS1 and TMEFF2 has been previously reported in colorectal cancer and they show partial or high level methylation in 9 or 10 cancer samples respectively. Among the 23 new genes tested, only the FGFR2 gene did not show differential methylation between cancer and normal samples. Most genes showed differential methylation in a high proportion of samples. 9 genes - DLX5, FOXD2, IRX1, MEIS1, MMP2, NPY, PDX1, SUSD5 and TCF21- showed high or partial methylation in all 10 samples, 9 genes - COL1A2, COL41, EFEMP, FGF5, FOXF1, GRASP, SDC2, SOX21 and ZNF471 - in 9 samples, FOXB1 in 8 samples, PPP1R14A in seven, FBN1 and EDIL3 in six and MEIS in three samples. In some cases, eg EDIL3, FBN1, GRASP Region 2, MEIS1 and SDC2, the level of methylation in matched normal colonic tissue was consistently very low. For other genes or regions, eg. DLX5, GRASP Region 3, IRX1, MMP2, NPY, PDX1 and TCF21, significant levels of methylation were evident in the matched normal tissue but methylation was always significantly increased in the cancer tissue. The data also demonstrate that for a given gene, not all regions show equivalent cancer-specific methylation. For example, for the COL4A gene(s) Regions 1 and 5 show high or partial methylation in 9 of 10 cancer samples, while Regions 2 and 3 are methylated in only 4 or 2 samples respectively. COL4A Region 1 lies within the COL4A1 gene, while COL4A Region 5 lies within the neighbouring, divergently transcribed COL4A2 gene.

The sequencing data thus demonstrates colorectal cancer-specific DNA methylation for 23 novel genes and specific regions that may be used for development of assays to distinguish cancer from normal DNA.

### EXAMPLE 4

### ANALYSIS OF DNA METHYLATION IN CLINICAL SAMPLES USING METHYLATION SELECTIVE PCR

DNA was extracted from colon tissue specimens comprising 10 adenomas, 15 Stage I, 18 Stage B, 28 Stage C, 7 Stage IV, 6 matched normal colon specimens and 7 other normal colon tissue. Isolated DNA was bisulphite converted using the Zymo EZ Gold bisulphite conversion kit as recommended by manufacturer. PCR assays were done in 15uL reaction mixtures containing a final concentration of 200nM of oligonucleotides and MgCl2 as shown in Table 7 and a 1:120,000 dilution of Molecular Probe SYBR Green (Invitrogen). GRASP and NPY amplifications were done 1x Platinum Buffer (Invitrogen) using 0.15 µL Platinum Taq DNA polymerase (Invitrogen). COL4A and SDC2 amplifications were done in Amplitaq, Taqman Buffer A using 0.1 µL (0.5 units) of AmpliTaq Gold (Appllied Biosystems). The PCR amplifications were performed in a Roche LightCycler 480 real-time PCR instrument using 384-well plates using the cycling conditions as shown in Table 7 for each amplicon. Levels of methylation were quantified using a standard curve of fully methylated DNA, 40 pg to 5 ng mixed with peripheral blood leukocyte DNA to give a total input of 5 ng. The proportion of methylated DNA in a sample was determined by dividing the amount of methylated DNA, calculated from the standard curve for fully methylated DNA, by the amount of input DNA

**TABLE 7**

| Gene | Primer Pair | PCR Conditions |
|---|---|---|
| | | |
| GRASP | MSPF1: 5'CGGAAGTCGCGTTCGTC (SEQ ID NO:72) | Plat Taq buffer containing 4mM MgCl2. 95°C 2 min, followed by 50 cycles of (85°C 15 sec, 64°C 15 sec, 72°C 20 sec). |
| | MSPR1: 5' GCGTACAACTCGTCCGCTAA (SEQ ID NO:73) | |
| | | |
| COL4A | MSP F3 5' GAATGTATTTGGTCGTGTTACGC (SEQ ID NO:74) | TaqMan Gold Buffer A containing 3.3 mM MgCl2. 95°C 10 min, followed by 50 cycles of (95°C 15 sec, 63°C 15 sec, 72°C 20 sec). |
| | MSP R2 5'CCCGACGTCCCGCC (SEQ ID NO:75) | |
| | | |
| SDC2 | MSPF1 5' TTTAGTATTTTCGGACGCGTTTC (SEQ ID NO:76) | TaqMan Gold Buffer A containing 4mM MgCl2. 95°C 10 min, followed by 50 cycles of (95°C 15 sec, 63°C 15 sec, 72°C 20 sec). |
| | MSPR1 5'GAAATAACCGACACTACGTAAAATCG (SEQ ID NO:77) | |
| | | |
| NPY | MSPF2 5' CGGAGACGTTCGTTCGATAGT (SEQ ID NO:78) | Plat Taq buffer containing 2mM MgCl2. 95°C 2 min, followed by 3 cycles of (95°C 15 sec, 63°C 15 sec, 72°C 20 sec) and 50 cycles of (84°C 15 sec, 64°C 15 sec, 72°C 20 sec). |
| | MSPR2 5' CAAACGAATCGTAACACTCACG (SEQ ID NO:79) | |

DNA Samples were scored as positive if the fraction of methylated DNA was >1% for GRASP or >2% for NPY, SDC2 and COL4A1. The number and % of positive samples for each biomarker assay are shown in Table 8. For cancer DNA samples a high proportion of samples, ranging from 88 to 94% were positive for methylation of each marker. By contrast the proportion of positive samples among matched normal controls was low (0-25%). Within the normal DNA samples, the level of methylation measured by the MSP assays was quantitatively low compared with that of the cancer DNA samples.

**TABLE 8**

| | | **GRASP** | | **NPY** | | **SDC2** | | **COL4A** | |
|---|---|---|---|---|---|---|---|---|---|
| | Total samples | Number positive (1% cut-off) | % positive | Number positive (2% cut-off) | % positive | Number positive (2% cut-off) | % positive | Number positive (2% cut-off) | % positive |

| **Adenoma** | **10** | **6** | **60** | **7** | **70** | **7** | **70** | **8/9** | **89** |
|---|---|---|---|---|---|---|---|---|---|
| | | | | | | | | | |
| Cancer A | 15 | 12 | 80 | 14 | 93 | 14/14 | 100 | 10/12 | 86 |
| Cancer B | 18 | 18 | 100 | 14/17 | 82 | 17 | 94 | 13/14 | 93 |
| Cancer C | 28 | 25 | 89 | 25 | 89 | 27 | 96 | 17/18 | 94 |
| Cancer D | 7 | 7 | 100 | 6 | 86 | 5 | 72 | 5/6 | 83 |
| **Cancer Total** | **68** | **62** | 91 | **59** (/67) | 88 | **63** (/67) | **94** | **45**(/50) | **90** |
| | | | | | | | | | |
| Matched normal | 6 | 0 | 0 | 1 | 16 | 1 | 16 | 1/4 | 25 |
| Other normal colon | 7 | 1 | 14 | 3 | 42 | 2 | 28 | 2/4 | 50 |
| Sm Int; stomach; rectum | 3 | 0 | 0 | 1 | rectum | 0 | | Sm int, rectum | |
| | | | | | | | | | |

### EXAMPLE 5

### ANALYSIS OF METHYLATION OF THE GRASP AND COL4A1 GENES IN DNA ISOLATED FROM PLASMA

Assays for GRASP and COL4A1 genes were also done on plasma DNA samples from patients with colorectal cancer, colorectal adenomas or patients without colorectal neoplasia as determined by colonoscopy. DNA was extracted 4mL of human blood plasma using the QIAmp Isolation of free circulating nucleic acids from serum/plasma (QIAGEN). Isolated DNA was bisulphite converted using the Zymo bisulphite conversion kit as recommended by manufacturer. A total of 36uL of bisulphite converted DNA was retrieved from 4mL of plasma. The presence of methylated COL4A1 sequences was determined using the MSP assay described in Example 4 except that a specific detection probe COL4A1 Probe BS1, HEX-5'CTAAACCCGTCCGCCTACCCCTC-BHQ (SEQ ID NO:80) was included at 100nM.

Detection of methylated GRASP sequences was done using a semi-nested PCR. First round PCRs was done in 30 µL (2.5uL of bisulphite converted DNA input) consisting of a final concentration of 1x Platinum Taq Buffer, 0.1 µL Platinum Taq DNA polymerase (Invitrogen), 3.3mM MgCl2, 200uM dNTPs (New England BioLabs) and 33 nM of oligonucleotides. The first round amplification used the forward primer GrspA-F1 5'-CGGATTTTCGATTCGGAAGT (SEQ ID NO:81) and GRASP MSPR1 from Table 7, Example 4.

Cycling conditions were 95'C for 2min, followed by eleven cycles of 92'C, 15sec; 60'C, 30 sec and 72'C 30 sec. The PCR amplifications were performed in PALM end-point PCR cycler using 96-well plates. Second round PCR used the primers and reaction conditions from Example 4, Table 7 and was performed on 1uL of material from PCR round 1 into a total PCR reaction of 15 µL. Levels of methylation were quantified using a standard curve of fully methylated DNA, 40 pg to 5 ng mixed with peripheral blood leukocyte DNA to give a total input of 5 ng. Standard curve were based on input into the single round for COL4A1 or the first round for GRASP.

The proportion of plasma DNA samples scoring positive in at least 2 or 3 triplicate PCRs is shown in Table 9. The data show that both

**TABLE 9**

| **75** | **n (%)** | **Ave.age** | **F/M** | **% pos COL4A1** | **% pos GRASP** |
|---|---|---|---|---|---|
| **Normal** | 44 | 63 | 19/25 | 11% | 11% |
| **Adenoma** | 44 | 63 | 21/22 | 9% | 18% |
| **LGD** | 18 | 68 | 7/11 | 0% | 11% |
| **HGD** | 1 | 30 | - | 0% | 100% |
| **>3 lesions** | 21 | 62 | 6/12 | 5% | 14% |
| **<3 lesions** | 23 | 65 | 12/11 | 13% | 22% |
| **TA** | 31 | 62 | 14/17 | 3% | 16% |
| **TVA** | 3 | 76 | 1/2 | 0% | 33% |
| **VA** | 4 | 59 | 3/1 | 50% | 25% |
| **other** | 6 | 59 | 3/3 | 17% | 17% |
| **>10mm** | 17 | 66 | 6/11 | 0% | 6% |
| **<10mm** | 27 | 69 | 15/12 | 15% | 26% |
| **Cancer** | 44 | 61.1 | 21/23 | 43% | 48% |
| **I** | - | - | - | - | - |
| **II** | 12 | 63 | 8/4 | 42% | 42% |
| **III** | 11 | 64.6 | 5/6 | 36% | 64% |
| **IV** | 8 | 55.4 | 4/4 | 75% | 63% |
| **Stage unk** | 13 | 68 | 4/9 | 31% | 31% |

Those skilled in the art will appreciate that the invention described herein is susceptible to variations and modifications other than those specifically described. It is to be understood that the invention includes all such variations and modifications. The invention also includes all of the steps, features, compositions and compounds referred to or indicated in this specification, individually or collectively, and any and all combinations of any two or more of said steps or features.

**TABLE 5**

| **Gene** | **Region** | **Chrom.** | **Start** | **End** | **Primer Sequence** | **CpGs** | **Assay conditions** |
|---|---|---|---|---|---|---|---|
| ADAMTS1 | Region 1 | 21 | 28217946 | 28218109 | gtgattagtattttgtattgttggggt | 10 | 58°C |
| | | | | | aaccRaaccctccctcctaaa | | |
| ADAMTS1 | Region 2 | 21 | 28218494 | 28218777 | TTGGAATGGGTGATTTGGG | 13 | 58°C |
| | | | | | CTTCTTTCCCCCTCTACACRCTTACTAA | | |
| COL1A2 | Region 2 | 7 | 94024141 | 94024345 | GGAGGTATTTTAGGGTTAGGGAAAT | 11 | 56°C |
| | | | | | ATATCTAACACTTAAACATACAAACTCCTTATAT | | |
| COL4A | Region 1 | 13 | 110959094 | 110959330 | AAAGGAGGTTYGGTTTATTAATG | 25 | 56°C |
| | | | | | CCTTCTACTCCACRAAAAACACA | | |
| COL4A | Region 3 | 13 | 110960787 | 110961141 | AGTGTGTTAGTTATTAGGTAGTGAGAYGTT | 20 | 58°C |
| | | | | | CAATACAATCRCCAACTACACCCAC | | |
| COL4A | Region 4 | 13 | 110961331 | 11096165 | GATGGAGTAGTTTTGTTTTGTGGTAGT | 10 | 56°C |
| | | | | | AACCTAAAATACTCTAATCAAAATCTCC | | |
| COL4A | Region 5 | 13 | 110959932 | 110960181 | TAGYGTAGGATGAGGGAGGT | 20 | 56°C, 2X dAdT |
| | | | | | CRCCTTATACAAACTAAAACTACAC | | |
| DLX5 | Region 1 | 7 | 96650026 | 96650127 | AGAGTAAGAGAGAGTAGTTTATTTAATAGAGTGTTT | 6 | 56°C, 2X dAdT |
| | | | | | CCAACTCAATCAATTCCCACCTA | | |
| DLX5 | Region 2 | 7 | 96651454 | 96651618 | GTGTAGTTTAGGTAGGTTTAGTGTATGGTAG | 9 | 56°C, 2X dAdT |
| | | | | | CCAAAACTATTTATTCCAACTTTCAA | | |
| DLX5 | Region 3 | 7 | 96653553 | 96653732 | TTGTTTAGTTTTTTTTTTGTTTATGTATTTG | 13 | 56°C, 2X dAdT |
| | | | | | AACTCTCAACCCCTACCAATATCAAT | | |
| EDIL | region 1 | 5 | 83679544 | 83679807 | AGAAAGTTGAAGTGATTTGTGAGATTT | 23 | 56°C, 2X dAdT |
| | | | | | CAAACCCTCCCTAAACAATACRA | | |
| EDIL | Region 2 | 5 | 83679784 | 83679988 | TTYGTATTGTTTAGGGAGGGTTTG | 13 | 56°C |
| | | | | | AATCCTAAATACCCCRAAAAAATACTT | | |
| EDIL | Region 3 | 5 | 83679960 | 83680263 | ATAAGTATTTTTTYGGGGTATTTAGGATT | 31 | 56°C, 2X dAdT |
| | | | | | CTACCACCTCRACTACACTACCCTC | | |
| EDIL | Region 4 | 5 | 83680075 | 83680383 | GGGGATTTTTAGTTTATTTTTTATTTAGTTG | 22 | 58°C |
| | | | | | AAAACTCCTCTCTTTAATCACCACTCT | | |
| EFEMP | Region 1 | 2 | 56150356 | 56150606 | GGTTTTAGGTTGTTTAGGATYGGAA | 13 | 56°C, 2X dAdT |
| | | | | | CAACCRACAAAACTTTACCCATAA | | |
| EFEMP | Region 2 | 2 | 56150304 | 56150523 | TAGGAGTTGGTTAGAAGTTGG | 14 | 56°C, 2X dAdT |
| | | | | | ACRACTAATTCTCTTTTATCTTATCA | | |
| FBN1 | Region 3 (-) | 15 | 48938136 | 48938384 | ATTGGTTTTTTTTYGGGTTAGGGTT | 13 | 56°C, 2X dAdT |
| | | | | | CAATAAAAAAAAACAAAAATAATCAAATCCTA | | |
| FGF5 | Region 1 | 4 | 81186918 | 81187228 | GYGGTTAGTTATATGTGTTGGTGTTTAGT | 18 | 56° |
| | | | | | CAACCCAACTTAAAAAACCTTTAATATAAC | | |
| FGF5 | Region 2 | 4 | 81187326 | 81187578 | ATAGTAGGGTTTATAGGGTAAAGAGAGGAG | 16 | 58°C |
| | | | | | CACRCATCCCAACAATTCTC | | |
| FGF5 | Region 3 | 4 | 81187571 | 81187792 | GATGYGTGGTTTTGGTATGGG | 17 | 56°C |
| | | | | | TTTATAACRCCAATAATCTCACTACTCC | | |
| FGFR2 | Region 2 | 10 | 123355549 | 123355736 | GGTGTTGGGAATATAGGTTTAGTATGT | 6 | 56°C |
| | | | | | CCATCCATCAAAAAAAAAATAACAA | | |
| FOXB1 | Region 1 | 15 | 60296522 | 60296719 | GAGGGAGGATATGGAGGTAGTTATT | 24 | 56°C, 2X dAdT |
| | | | | | AACCTAAACTCTTAAACTCAACCCC | | |
| FOXB1 | Region 2 | 15 | 60297024 | 60297305 | AGTTTGTTATTYGGTTTGGTTGATTT | 21 | 56°C, 2X dAdT |
| | | | | | AAAAAACRATCCATAATAAACTTATAAATCTC | | |
| FOXD2 | Region 1 | 1 | 47899091 | 47899337 | TTTTATTATTTYGGGAATTTTGTGATT | 12 | 56°C, 2X dAdT |
| | | | | | TTCTCTTTCCRCTACTCCTAACCACTA | | |
| FOXD2 | Region 2 | 1 | 47909944 | 47910172 | AAGGATTTTGTAAGAGAAAGGGAGA | 13 | 58°C |
| | | | | | AACCCCAACCTACACAACATTTTA | | |
| FOXF1 | Region 1 | 16 | 86544560 | 86544770 | GAGTTTATGTTYGAGGAGGGTTTTT | 16 | 56°C, 2X dAdT |
| | | | | | AATAACCRTTCATCATACCCAAACC | | |
| FOXF1 | Region 2 | 16 | 86544265 | 86544584 | GTTYGGTTYGTTTAAGGTTAAGAAGATTAA | 20 | 56°C, 2X dAdT |
| | | | | | AAAACCCTCCTCRAACATAAACTC | | |
| FOXF1 | Region 3 | 16 | 86544795 | 86545110 | TTTTGTTTAGTTATTYGGTGTTTTATTTGT | 19 | 56°C, 2X dAdT |
| | | | | | RACTACTCCAAAAAATACRTAAAAAAACTAC | | |
| GRASP | Region 1 | 12 | 52399672 | 52399922 | GATGGGTGTTGGGATATGGA | 9 | 56°C, 2X dAdT |
| | | | | | TTCAAAACTAAAAAAATAACCATACTCAAC | | |
| GRASP | Region 2 | 12 | 52400821 | 52401119 | TAGGAAGTTGTAGTAGAAGGAGGAGG | 35 | 56°C, 2X dAdT |
| | | | | | CRAAATCAACAAACCCTATAAAAAATC | | |
| GRASP | Region 3 | 12 | 52401407 | 52401664 | GATAGAGATAGTTTTAGGTAAGTTGAAGGTT | 22 | 56°C, 2X dAdT |
| | | | | | CCTACRACCCCTCCCACTAC | | |
| IRX1 | Region 1 | 5 | 3597227 | 3597480 | TGYGTTATATAATGGAGGATTAAAGAAAATA | 10 | 56°C, 2X dAdT |
| | | | | | AAAAAAACTACCTCCCAAAACCC | | |
| IRX1 | Region 2 (-) | 5 | 3599990 | 3600175 | GAATTTGTYGATGTGGTAGGTGTATAGTT | 20 | 56°C, 2X dAdT |
| | | | | | CCRCACRACAAACCCAAAATCTAAT | | |
| IRX1 | Region 3 (-) | 5 | 3600160 | 3600352 | GATYGAGGTTTTGTTTTTATTGAGTGT | 13 | 56°C, 2X dAdT |
| | | | | | CCACATCRACAAATTCTCCAACTAA | | |
| IRX1 | Region 4 | 5 | 3594657 | 3594847 | GGAGTTYGGTGTGTTGGAGAGT | 15 | 56°C, 2X dAdT |
| | | | | | CCTCCTCRCCTCCCTAAACCTA | | |
| MEIS1 | Region 1 | 2 | 66662009 | 66662219 | GAGGGAGAAAAGAATATTGAAAATAAAGT | 13 | 56°C, 2X dAdT |
| | | | | | CRTCTTACACAATACATTAAACTACAACAAAT | | |
| MEIS1 | Region 2 | 2 | 66662177 | 66662430 | TYGAGGAGTTTATTTGTTGTAGTTTAATGT | 10 | 56°C, 2X dAdT |
| | | | | | TCTCTCTCCCTCTTTACAAATACTACACTA | | |
| MMP2 | Region 2 (-) | 16 | 55512662 | 55512856 | TAGTTTTAGAATTTTTTGTTGGGAAATAT | 6 | 56°C |
| | | | | | AAAATTTCTATCTCTAACCATCTATCATTATAA | | |
| MMP2 | Region 3 | 16 | 55513916 | 55514215 | TAATTTTTGGATATATTTGGGTAGTTGT | 9 | 58°C |
| | | | | | AAAAAATACRCTCTCTACTTCCCTCCTA | | |
| NPY | Region 1 | 7 | 24323765 | 24323936 | GGYGAGGAAGTTTTATAAAAGTTTTG | 15 | 56°C, 2X dAdT |
| | | | | | CCTTTCTCTCCCACCCCTAA | | |
| NPY | Region 2 | 7 | 24324150 | 24324342 | TTATTTTTTAGTAGATATGGAGGGAGAATT | 14 | 56°C, 2X dAdT |
| | | | | | AACCCAAAAATCCAAAAAAATAACA | | |
| NPY | Region 3 | 7 | 24324513 | 24324717 | GGGAGAAAAGTGATTTAGTAGGAAGAAT | 15 | 56°C, 2X dAdT |
| | | | | | AACCCAAAAATAACTAACACCACCTTA | | |
| PDX1 | Region 1 | 13 | 28502100 | 28502311 | GGTTGGGTTYGGGATTAGAGTT | 10 | 56°C, 2X dAdT |
| | | | | | CCRCCTCCTTATAAACCCACTATATAA | | |
| PDX1 | Region 2 | 13 | 28502417 | 28502603 | TTTTTAGGGAGGTTTAGGGAGGTA | 12 | 56°C, 2X dAdT |
| | | | | | TCTAATCCAAAAAATATCRATCCAACTA | | |
| PDX1 | Region 3 (-) | 13 | 28503006 | 28503210 | GAAGATTTTAAGAGGAATGGAGGGT | 11 | 56°C, 2X dAdT |
| | | | | | AAAAAAAACAAAACTCCTACCAAAACTA | | |
| PPP1R14A | Region 3 (-) | 19 | 38747251 | 38747424 | ATAAATGGATGGATGAGTGAATGAAT | 13 | 56°C, 2X dAdT |
| | | | | | AAATCCTATAAAACRAAAACCTAAAACAAA | | |
| PPP1R14A | Region 4 | 19 | 38746653 | 38746912 | T**Y**GTTTTTTTGTGTAGATTAGGTTGGT | 26 | 56°C, 2X dAdT |
| | | | | | CAAAAAACAATCCCRAAAAACTACAA | | |
| QK1 | Region 3 | 6 | 163835521 | 163835719 | GGGGYGGYGGGTAGTAGGTAG | 30 | 56°C, 2X dAdT |
| | | | | | CCTCCTCCTCCTCACTCACTTAA | | |
| SDC2 | Region 1 (-) | 8 | 97506813 | 97507045 | AATTAATTGTATGTAGTTAAATAGGAAAGT | 17 | 56°C |
| | | | | | AAAAACCCCTAAAATCACTCCCAA | | |
| SDC2 | Region 2 (-) | 8 | 97507037 | 97507257 | TYGTAGGATTGGGAATTTAGTGGT | 14 | 56°C, 2X dAdT |
| | | | | | CAATTAATTCTAAAAAAATAAAAACCAAATTTAAA | | |
| SOX21 | Region 1 (-) | 13 | 95364013 | 9536417 | GGGAGTAGAGTGGAAATTGTTTATAGAGT | 14 | 56°C, 2X dAdT |
| | | | | | AAACACCTAAAAAACTAACTTATCCTTCTTA | | |
| SOX21 | Region 2 (-) | 13 | 95364515 | 95364784 | TGAGAGTTTTTTTTGGTATTTGGTAGTA | 25 | 56°C, 2X dAdT |
| | | | | | RAACAAATTATCTCTAAAACACTCTAACTTCT | | |
| SUSD5 | Region 2 (-) | 3 | 33260566 | 33260818 | GGGGAGGAGGTTAGTTGAAAAGTAG | 21 | 56°C, 2X dAdT |
| | | | | | CCTAAAAACCTCRATACCAAAAAAAAC | | |
| TCF21 | Region 1 | 6 | 134210545 | 134210749 | GGTTTTTTTAGYGATGTGGAGGATTT | 9 | 58°C |
| | | | | | CCCCRCTCAAAAAACTCTTCTTAATAA | | |
| TCF21 | Region 2 | 6 | 134210712 | 134210951 | GAGGAAGGYGTTTATTAAGAAGAGT | 14 | 56°C |
| | | | | | TTATCRTTAACCAAAATCTACCTCAAAT | | |
| TCF21 | Region 3 (-) | 6 | 134210994 | 134211274 | GGGATTTTTTTTTTGTGTTAGTTTAATAT | 26 | 56°C |
| | | | | | TCCCRAAACTACAACTACAATCCAA | | |
| TMEFF2 | Region 5 | 2 | 193059382 | 193059532 | GTTTTTTAGAGTTTTTTTTTTATGGTAGTAGT | 11 | 56°C, 2X dAdT |
| | | | | | CRAAAAAACAACAACCAAACCC | | |
| ZNF471 | Region 1 (-) | 19 | 57018955 | 57019135 | GTTGTTGAAGGAGTGAGAGGGAT | 14 | 56°C |
| | | | | | AACTAATAACTAAAAACRCTTCTAATCTCTAAAC | | |

| **Gene** | **Region** | **Chrom.** | **Start** | **End** | **Primer Sequence** | **CpGs** | **Assay conditions** |
|---|---|---|---|---|---|---|---|
| ZNF471 | Region 2 | 19 | 57019294 | 57019573 | GTTTTGGGTGGTTTGGGAGTT | 28 | 56°C, 2X dAdT |
| | | | | | CACACTCTAACAAATCTTTTACACACAA | | |

**TABLE 6**

| **Amplicon #** | **Gene** | **Region** | **m/10** | **p/10** | **A** | **B** | **C** | **D** | **E** | **F** | **G** | **H** | **I** | **J** | | **Normals** |
|---|---|---|---|---|---|---|---|---|---|---|---|---|---|---|---|---|
| 1 | ADAMTS1 | Region 1 | 7 | 2 | 60 | 80 | 80 | 80 | 30 | 20 | 0 | 80 | 80 | 80 | | <20 |
| 5 | COL1A2 | Region 2 | 7 | 2 | 50 | 30 | 60 | 70 | 60 | 40 | 20 | 60 | 80 | 40 | | <10 |
| 6 | COL4A | Region 1 | 6 | 3 | 60 | 60 | 50 | 60 | 30 | 30 | 0 | 70 | 60 | 50 | | <10 |
| 7 | COL4A | Region 3 | 3 | 1 | 40 | 0 | 0 | 60 | 0 | 0 | 0 | 30 | 70 | 0 | | <10 |
| 8 | COL4A | Region 4 | 2 | 0 | 60 | 0 | 0 | 0 | 0 | 0 | 0 | 0 | 60 | 0 | | <20 |
| 9 | COL4A | Region 5 | 6 | 3 | 60 | 80 | 60 | 80 | 50 | 40 | 0 | 40 | 80 | 60 | | <20 |
| 10 | DLX5 | Region 1 | 7 | 3 | 70 | 70 | 70 | 80 | 40 | 50 | 90 | 70 | 80 | 30 | | 10-30 |
| 11 | DLX5 | Region 2 | 8 | 2 | 70 | 80 | 80 | 80 | 30 | 40 | 80 | 80 | 80 | 80 | | 10-40 |
| 12 | DLX5 | Region 3 | 6 | 3 | 70 | 0 | 60 | 80 | 40 | 40 | 80 | 40 | 80 | 80 | | 10-30 |
| 13 | EDIL | region 1 | 5 | 1 | 60 | 0 | 50 | 50 | 30 | 20 | 0 | 60 | 60 | 10 | | ^{∼}10 |
| 14 | EDIL | Region 2 | 5 | 1 | 80 | 10 | 80 | 80 | 40 | 10 | 0 | 80 | 90 | 10 | | <10 |
| 15 | EDIL | Region 3 | 5 | 1 | 70 | 0 | 60 | 60 | 30 | 10 | 0 | 60 | 80 | 0 | | <10 |
| 16 | EDIL | Region 4 | 5 | 1 | 70 | 10 | 60 | 60 | 20 | 10 | 0 | 60 | 80 | 0 | | <10 |
| 18 | EFEMP | Region 1 | 8 | 1 | 70 | 60 | 60 | 70 | 30 | 70 | 0 | 70 | 70 | 60 | | ^{∼}20 |
| 19 | EFEMP | Region 2 | 7 | 2 | 70 | 80 | 50 | 70 | 40 | 70 | 10 | 70 | 70 | 70 | | ^{∼}25 |
| 20 | FBN1 | Region 3 | 5 | 1 | 70 | 10 | 80 | 70 | 40 | 10 | 0 | 80 | 90 | 0 | | <10 |
| 21 | FGF5 | Region 1 | 5 | 4 | 80 | 80 | 50 | 80 | 30 | 50 | 50 | 80 | 80 | 0 | | <10 |
| 22 | FGF5 | Region 2 | 5 | 3 | 60 | 70 | 50 | 60 | 30 | 20 | 40 | 70 | 80 | 0 | | <10 |
| 23 | FGF5 | Region 3 | 4 | 4 | 60 | 30 | 40 | 70 | 20 | 10 | 30 | 70 | 80 | 0 | | <10 |
| 24 | FGFR2 | Region 2 | 9 | 1 | 80 | 80 | 80 | 80 | 40 | 60 | 80 | 80 | 80 | 80 | | 40-90 |
| 25 | FOXB1 | Region 1 | 4 | 4 | 60 | 0 | 30 | 70 | 40 | 30 | 0 | 70 | 70 | 20 | | <10 |
| 26 | FOXB1 | Region 2 | 5 | 3 | 80 | 0 | 40 | 80 | 60 | 40 | 0 | 80 | 80 | 30 | | <10 |
| 27 | FOXD2 | Region 1 | 9 | 1 | 80 | 80 | 80 | 90 | 70 | 50 | 80 | 90 | 60 | 70 | | 20-40 |
| 28 | FOXD2 | Region 2 | 6 | 2 | 70 | 70 | 40 | 80 | 30 | 20 | 10 | 80 | 80 | 70 | | <20 |
| 29 | FOXF1 | Region 1 | 5 | 4 | 60 | 50 | 40 | 60 | 50 | 40 | 0 | 60 | 70 | 60 | | 20-40 |
| 30 | FOXF1 | Region 2 | 5 | 4 | 60 | 60 | 30 | 70 | 50 | 40 | 0 | 30 | 80 | 70 | | <20 |
| 32 | GRASP | Region 1 | 4 | 2 | 60 | 70 | 70 | 20 | 60 | 30 | 0 | 30 | 20 | 10 | | 10-20 |
| 33 | GRASP | Region 2 | 7 | 2 | 70 | 80 | 70 | 70 | 40 | 50 | 0* | 80 | 90 | 30 | | <5 |
| 34 | GRASP | Region 3 | 9 | 1 | 60 | 70 | 70 | 60 | 30 | 40 | 60 | 70 | 70 | 60 | | 30-60 |
| 35 | IRX1 | Region 1 | 8 | 2 | 70 | 70 | 70 | 70 | 40 | 40 | 70 | 70 | 70 | 70 | | 10-60 |
| 36 | IRX1 | Region 2 | 8 | 2 | 70 | 70 | 70 | 70 | 40 | 40 | 70 | 70 | 70 | 70 | | 10-60 |
| 37 | IRX1 | Region 3 | 8 | 2 | 70 | 70 | 70 | 70 | 50 | 50 | 70 | 70 | 70 | 70 | | 10-60 |
| 38 | IRX1 | Region 4 | 8 | 2 | 70 | 70 | 70 | 60 | 40 | 40 | 80 | 60 | 70 | 70 | | 10-50 |
| 39 | MEIS1 | Region 1 | 3 | 0 | 0 | 0 | 0 | 70 | 40 | 0 | 0 | 0 | 50 | 0 | | <5 |
| 40 | MEIS1 | Region 2 | 2 | 1 | 0 | 0 | 0 | 70 | 20 | 0 | 0 | 0 | 50 | 0 | | <5 |
| 41 | MMP2 | Region 2 | 7 | 3 | 30 | 60 | 40 | 60 | 20 | 60 | 50 | 60 | 70 | 50 | | 20-50 |
| 42 | MMP2 | Region 3 | 2 | 8 | 20 | 40 | 20 | 30 | 20 | 20 | 0 | 20 | 50 | 50 | | <30 |
| 43 | NPY | Region 1 | 7 | 3 | 30 | 60 | 80 | 40 | 30 | 20 | 60 | 40 | 40 | 50 | | 10-60 |
| 44 | NPY | Region 2 | 8 | 2 | 70 | 80 | 80 | 80 | 40 | 40 | 80 | 80 | 80 | 80 | | 20-60 |
| 45 | NPY | Region 3 | 8 | 2 | 50 | 70 | 70 | 70 | 40 | 40 | 80 | 70 | 70 | 70 | | 10-60 |
| 46 | PDX1 | Region 1 | 7 | 3 | 70 | 90 | 90 | 80 | 50 | 40 | 80 | 70 | 70 | 40 | | 10-40 |
| 47 | PDX1 | Region 2 | 8 | 2 | 80 | 90 | 80 | 80 | 50 | 50 | 80 | 80 | 70 | 60 | | 10-50 |
| 48 | PDX1 | Region 3 | 8 | 2 | 70 | 70 | 80 | 80 | 50 | 50 | 80 | 70 | 70 | 60 | | 10-40 |
| 49 | PPP1R14A | Region 3 | 4 | 3 | 80 | 10 | 30 | 80 | 30 | 30 | 0 | 80 | 80 | 0 | | 10-50 |
| 50 | PPP1R14A | Region 4 | 3 | 2 | 70 | 30 | 50 | 70 | 10 | 20 | 0 | 10 | 30 | 10 | | 10-60 |
| 54 | SDC2 | Region 1 | 7 | 2 | 50 | 70 | 70 | 60 | 40 | 30 | 0 | 60 | 70 | 50 | | <10 |
| 55 | SDC2 | Region 2 | 6 | 2 | 70 | 90 | 70 | 80 | 30 | 10 | 0 | 90 | 80 | 40 | | <10 |
| 57 | SOX21 | Region 1 | 4 | 5 | 40 | 40 | 60 | 60 | 30 | 30 | 0 | 70 | 60 | 30 | | <20 |
| 58 | SOX21 | Region 2 | 6 | 3 | 60 | 70 | 60 | 70 | 40 | 40 | 10 | 60 | 70 | 50 | | <30 |
| 59 | SUSD5 | Region 2 | 9 | 1 | 80 | 90 | 70 | 80 | 60 | 50 | 70 | 80 | 70 | 70 | | 10-50 |
| 60 | TCF21 | Region 1 | 8 | 2 | 70 | 70 | 70 | 70 | 50 | 50 | 70 | 70 | 80 | 60 | | 10-50 |
| 61 | TCF21 | Region 2 | 10 | 0 | 70 | 90 | 80 | 90 | 60 | 60 | 90 | 80 | 80 | 80 | | 40-80 |
| 62 | TCF21 | Region 3 | 8 | 2 | 70 | 70 | 70 | 70 | 40 | 50 | 70 | 70 | 70 | 70 | | 10-50 |
| 63 | TMEFF2 | Region 5 | 6 | 4 | 60 | 70 | 70 | 80 | 50 | 50 | 50 | 80 | 80 | 50 | | 10-30 |
| 64 | ZNF471 | Region 1 | 4 | 4 | 70 | 20 | 40 | 60 | 40 | 30 | 40 | 70 | 60 | 0 | | <20 |
| 65 | ZNF471 | Region 2 | 4 | 5 | 60 | 30 | 40 | 60 | 40 | 30 | 40 | 60 | 60 | 0 | | <20 |

### BIBLIOGRAPHY

Abrams and Stanton, Methods Enzymol., 212:71-74, 1992
Adorjan et al. Nucl. Acids Res., 30: e21, 2002
Alon et al., Proc. Natl. Acad. Sci. USA: 96:6745-6750, June 1999
Ammerpohl et al. Biochim Biophys Acta. 1790:847-62, 2009
Ausubel, F. et al., "Current Protocols in Molecular Biology", John Wiley & Sons, (1998)
Beaucage, et al. Tetrahedron Letters 22:1859-1862, 1981
Bianco et al., Hum. Mutat., 14:289-293, 1999
Bonner and Laskey, Eur. J. Biochem. 46:83, 1974
Bresslauer et al., Proc. Natl. Acad. Sci. USA, 83: 3746-3750, 1986
"Cancer" National Cancer Institute 2009. http://www.cancer.gov/cancertopics/commoncancers
Caruthers et al., Methods in Enzymology, Vol. 154, pp. 287-314 (1988)
Chen and Kwok, Nucleic Acids Res. 25:347-353, 1997
Clark et al. Nat Protoc. 1:2353-64, 2006
Cottrell et al., Nucl. Acids Res. 32: e10, 2003
DeGraves, et al., Biotechniques 34(1):106-10, 112-5 (2003)
Deiman B, et al., Mol. Biotechnol. 20(2):163-79 (2002)
Deng et al. Chin. J. Cancer Res., 12:171-191, 2000
Dieffenbach and Dveksler (Eds) (In: PCR Primer: A Laboratory Manual, Cold Spring Harbor Laboratories, NY, 1995
Eads et al., Cancer Res. 59:2302-2306 (1999)
Eads et al., Nucl. Acids Res. 28: E32, 2000
Egholm et al., Am. Chem. Soc., 114:1895, 1992
Egholm et al., Nature, 365:566, 1993
Fodor et al., Science 767-773, 1991
Frommer et al., Proc. Natl. Acad. Sci. USA 89:1827-1831 (1992)
Gibson et al., Genome Research 6:995-1001 (1996)
Golub et al., Science, 286:531-537, 1999
Gonzalgo & Jones, Nucleic Acids Res. 25:2529-2531 (1997)
Gonzalgo et al., Cancer Res. 57:594-599, 1997
Herman et al., Proc. Natl. Acad. Sci. USA 93:9821-9826, (1996)
Holland et al., Proc. Natl. Acad. Sci. USA, 88:7276-7280, 1991
Kawai et al., Mol. Cell. Biol. 14:7421-7427, 1994
Kristensen and Hansen Clin Chem. 55:1471-83, 2009
Kuppuswamy et al., Proc. Natl. Acad. Sci. USA 88:1143-1147, 1991
Landegren et al., Genome Res., 8(8): 769-776, 1998
Lee et al., Nucleic Acid Res. 21:3761-3766, 1993
Markowitz and Bertagnolli (2009). N. Engl. J. Med. 361(25):2449-60
Marmur and Doty, J. Mol. Biol. 5:109, 1962
Messing, Methods Enzymol, 101, 20-78, 1983
Mhlanga and Malmberg, Methods 25: 463-471, 2001
Moore et al., BBA, 1402:239-249, 1988
Narang, et al. Meth. Enzymol 68: 90, 1979
Nielsen et al., J. Chem. Soc. Perkin Trans., 1:3423, 1997
Olek, et al. Nat. Genet. 17(3): 275-6 (1997)
Orum et al., Clin. Chem. 45:1898-1905, 1999
Orum et al., Nucl. Acids Res., 21:5332, 1993
PCT Publication No. WO 00/70090
Rand et al. Epigenetics 1:94-100, 2006
Rand et al. Nucl. Acids Res. 33:e127, 2005
Rein, et al. Nucleic Acids Res. 26 (10): 2255-64 (1998)
Robinson et al. Epigenomics 2:587-98 (2010)
Sadri & Hornsby, Nucl. Acids Res. 24:5058-5059 (1996)
Sambrook et al., Molecular Cloning, A Laboratory Manual (2nd Ed., CSHP, New York 1989)
Santa Lucia, Proc. Natl. Acad. Sci. USA, 95: 1460-1465, 1995
Shames et al. Cancer Lett. 251:187-98, 2007
Simeonov and Nikiforov, Nucleic Acids Research, 30(17): 1-5, 2002
Singer-Sam et al., Nucl. Acids Res. 18, 687, 1990
Singer-Sam et al., Nucl. Acids Res. 18:687, 1990
Singer-Sam et al., PCR Methods Appl. 1: 160-163, 1992
Singh and Wengel, Chem. Commun. 1247, 1998
Southern et al., Genomics, 13:1008-1017, 1992
Szabo and Mann, Genes Dev. 9: 3097-3108, 1995
Toyota et al., Cancer Res. 59:2307-12 (1999)
U.S. Pat. No. 5,786,146
U.S. Patent Publication 2005/0069879
Uhlmann et al., Electrophoresis, 23:4072-4079, 2002
Wedemeyer et al., Clinical Chemistry 48:9 1398-1405, 2002
Weissleder et al., Nature Medicine 6:351-355, 2000
Weitzel JN (1999). Cancer 86 (11 Suppl): 2483-92
Worm et al., Clin. Chem., 47:1183-1189, 2001
Xiong & Laird, Nucleic Acids Res. 25:2532-2534 (1997)
Zyskind et al., Recombinant DNA Laboratory Manual, (Acad. Press, 1988)

The invention may be described by reference to the following numbered paragraphs:-
1. A method of screening for the onset or predisposition to the onset of a large intestine neoplasm or monitoring the progress of a neoplasm in an individual, said method comprising assessing the methylation status of a DNA region selected from:
(i) the region defined by any one or more of Hg19 coordinates and 2kb upstream of the transcription start site:

| | |
|---|---|
| (1) chr12:52400748..52409671 | (34) chr10:101292690..101296281 |
| (2) chr5:3596168..3601517 | (35) chr4:4190530..4228621 |
| (3) chr13:95361876..95364389 | (36) chr12:54943404..54973023 |
| (4) chr4:81187742..81212171 | (37) chr5:176047210..176057557 |
| (5) chr19:57019212..57040270 | (38) chr12:22346325..22487648 |
| (6) chr3:33191537..33260707 | (39) chr19:56894648..56904889 |
| (7) chr15:60296421..60298142 | (40) chr20:21491648..21494664 |
| (8) chr13:28494168..28500451 | (41) chr1:50883225..50889141 |
| (9) chr7:96649702..96654143, | (42) chr7:27180996..27183287 |
| (10) chr8:140,811,770-141,537,860 | (43) chr11:2016406..2019065 |
| (11) chr5:2746279..2751769 | (44) chr14:57267425..57277184 |
| (12) chr18:55102917..55158530 | (45) chr4:126237567..126414087 |
| (13) chr20:37353101..37358016 | (46) chr8:23559964..23563922 |
| (14) chr8:2792875..4852328 | (47) chr10:131633547..131762091 |
| (15) chr16:66613351..66622178 | (48) chr4:62362839..62938168 |
| (16) chr5:37815753..37839782 | (49) chr1:47901689..47906363 |
| (17) chr1:63788730..63790797 | (50) chr17:77768176..77770890 |
| (18) chr15:37156644..37178734 | (51) chr17:93598762..93604831 |
| (19) chr7:27139973..27142394 | (52) chr1:33789224..33841194 |
| (20) chr20:21686297..21696620 | (53) chr9:124,004,679-124,030,840 |
| (21) chr16:51169886..51185183 | (54) chr4:158141736..158287227 |
| (22) chr12:85253267..85306606 | (55) chr12:9445136..9462559 |
| (23) chr8:6357172..6420784 | (56) chr12:24964278..25102308 |
| (24) chr14:85996488..86094270 | (57) chrX:21542357..21690352 |
| (25) chr2:182541194..182545381 | (58) chr20:52769988..52790516 |
| (26) chr7:30951468..30965131 | (59) chr3:172162951..172166203 |
| (27) chr8:131792547..132052835 | (60) chr13:28366780..28368089 |
| (28) chr3:128749292..128759583 | (61) chr7:50344378...50472799 |
| (29) chr10:101088856..101154087 | (62) chr7:149412148..149431664 |
| (30) chr7:27282164..27286192 | (63) chr7:24323809..24331477 |
| (31) chr10:129535538..129539450 | (64) chr4:30722037..31148421 |
| (32) chr19:49316274..49339934 | (65) chr10:47083534..47088320 |
| (33) chr6:391752..411443; or | |

(ii) the gene region, including 2kb upstream, of any one or more of:

| | | | |
|---|---|---|---|
| (1) GRASP | (18) ANGPT2 | (35) NKX2-6 | (52) HOXA5 |
| (2) IRX1 | (19) LHX6 | (36) PAX1 | (53) GDNF |
| (3) SOX21 | (20) NEUROD1 | (37) FOXD2 | (54) FAT4 |
| (4) FGF5 | (21) AC149644.1 | (38) SLC6A15 | (55) HOXA2 |
| (5) ZNF471 | (22) CCDC48 | (39) PHC2 | (56) LPHN3 |
| (6) SUSD5 | (23) EVX1 | (40) FLRT2 | (57) ADCYAP1 |
| (7) FOXB1 | (24) GHSR | (41) GATA2 | (58) GRIA2 |
| (8) PDX1 | (25) HSD17B14 | (42) ADCY8 | (59) AQP1 |
| (9) DLX5 | (26) KRBA1 | (43) CNNM1 | (60) BCAT1 |
| (10) ONECUT2 | (27) OTOP1 | (44) IKZF1 | (61) CYP24A1 |
| (11) DMRTA2 | (28) PPYR1 | (45) NKX2-3 | (62) FOXI2 |
| (12) CMTM2 | (29) SRMS | (46) PCDH7 | (63) GSX1 |
| (13) OTX2 | (30) ZNF582 | (47) SNCB | (64) IRF4 |
| (14) LOC145845 | (31) IRX2 | (48) ST8SIA1 | (65) NPY |
| (15) EBF3 | (32) CSMD1 | (49) TRAPPC9 | (66) PDE1B |
| (16) SALL1 | (33) MIR675,H19 | (50) NKX2-2 | |
| (17) CBX8 | (34) FOXD3 | (51) SLC32A1 | |

in a biological sample from said individual wherein a higher level of methylation of the DNA regions of group (i) and/or (ii) relative to control levels is indicative of a large intestine neoplasm or a predisposition to the onset of a large intestine neoplastic state.
2. The method according to paragraph 1 wherein said one or more DNA regions are selected from:
(i) the region defined by any one or more of Hg19 coordinates and 2kb upstream of the transcription start site:

| | |
|---|---|
| (1) chr12:52400748..52409671 | (6) chr3:33191537..33260707 |
| (2) chr5:3596168..3601517 | (7) chr15:60296421..60298142 |
| (3) chr13:95361876..95364389 | (8) chr13:28494168..28500451 |
| (4) chr4:81187742..81212171 | (9) chr7:96649702..96654143 |
| (5) chr19:57019212..57040270 | |

(ii) the gene region, including 2kb upstream, of any one or more of:

| | | | |
|---|---|---|---|
| (1) GRASP | (4) FGF5 | (6) SUSD5 | (8) PDX1 |
| (2) IRX1 | (5) ZNF471 | (7) FOXB1 | (9) DLX5 |
| (3) SOX21. | | | |

3. A method of screening for the onset or predisposition to the onset of a large intestine neoplasm or monitoring the progress of a neoplasm in an individual, said method comprising assessing the methylation status of one or more DNA regions selected from the DNA regions defined by Hg19 coordinates:

| | | | |
|---|---|---|---|
| (1) | Chr12:52399672-52399922 | (30) | Chr13:28502100-28502311 |
| (2) | Chr12:52400821-52401119 | (31) | Chr13:28502417-28502603 |
| (3) | Chr12:52401407-52401664 | (32) | Chr13:28503006-28503210 |
| (4) | Chr8:97506813-97507045 | (33) | Chr7:96650026-96650127 |
| (5) | Chr8:97507037-97507257 | (34) | Chr7:96651454-96651618 |
| (6) | Chr13:110959094-110959330 | (35) | Chr7:96653553-96653732 |
| (7) | Chr5:3597227-3597480 | (36) | Chr21:28217946-28218109 |
| (8) | Chr5:3599990-3600175 | (37) | Chr21:28218494-28218777 |
| (9) | Chr5:3600160-3600352 | (38) | Chr21:28218859-28219017 |
| (10) | Chr5:3594657-3594847 | (39) | Chr13:110960787-110961141 |
| (11) | Chr13:95364013-95364178 | (40) | Chr13:110961331-110961659 |
| (12) | Chr13:95364515-95364784 | (41) | Chr5:83679544-83679807 |
| (13) | Chr4:81186918-81187228 | (42) | Chr5:83679784-83679988 |
| (14) | Chr4:81187326-81187578 | (43) | Chr5:83679960-83680263 |
| (15) | Chr4:81187571-81187792 | (44) | Chr5:83680075-83680383 |
| (16) | Chr19:57018955-57019135 | (45) | Chr5:83680356-83680630 |
| (17) | Chr19:57019294-57019573 | (46) | Chr15:48938136-48938384 |
| (18) | Chr3:33260566-33260818 | (47) | Chr1:47899091-47899337 |
| (19) | Chr2:56150356-56150606 | (48) | Chr1:47909944-47910172 |
| (20) | Chr6:134210545-134210749 | (49) | Chr2:66662009-66662219 |
| (21) | Chr6:134210712-134210951 | (50) | Chr2:66662177-66662430 |
| (22) | Chr6:134210994-134211274 | (51) | Chr16:55512662-55512856 |
| (23) | Chr7:94023751-94023975 | (52) | Chr16:55513916-55514215 |
| (24) | Chr7:94024141-94024345 | (53) | Chr7:24323765-24323936 |
| (25) | Chr15:60296522-60296719 | (54) | Chr7:24324150-24324342 |
| (26) | Chr15:60297024-60297305 | (55) | Chr7:24324513-24324717 |
| (27) | Chr16:86544560-86544770 | (56) | Chr19:38747251-38747424 |
| (28) | Chr16:86544265-86544584 | (57) | Chr19:38746653-38746912 |
| (29) | Chr16:86544795-86545110 | (58) | Chr13:110959932-110960181 |

in a biological sample from said individual when a higher level of methylation of said DNA region relative to control levels is indicative of a large intestine neoplasia or a predisposition to the onset of a large intestine neoplastic state.
4. The method according to paragraph 3 wherein said DNA region is selected from the DNA regions defined by Hg19 coordinates:

| | | | |
|---|---|---|---|
| (1) | Chr12:52399672-52399922 | (19) | Chr2:56150356-56150606 |
| (2) | Chr12:52400821-52401119 | (20) | Chr6:134210545-134210749 |
| (3) | Chr12:52401407-52401664 | (21) | Chr6:134210712-134210951 |
| (4) | Chr8:97506813-97507045 | (22) | Chr6:134210994-134211274 |
| (5) | Chr8:97507037-97507257 | (23) | Chr7:94023751-94023975 |
| (6) | Chr13:110959094-110959330 | (24) | Chr7:94024141-94024345 |
| (7) | Chr5:3597227-3597480 | (25) | Chr15:60296522-60296719 |
| (8) | Chr5:3599990-3600175 | (26) | Chr15:60297024-60297305 |
| (9) | Chr5:3600160-3600352 | (27) | Chr16:86544560-86544770 |
| (10) | Chr5:3594657-3594847 | (28) | Chr16:86544265-86544584 |
| (11) | Chr13:95364013-95364178 | (29) | Chr16:86544795-86545110 |
| (12) | Chr13:95364515-95364784 | (30) | Chr13:28502100-28502311 |
| (13) | Chr4:81186918-81187228 | (31) | Chr13:28502417-28502603 |
| (14) | Chr4:81187326-81187578 | (32) | Chr13:28503006-28503210 |
| (15) | Chr4:81187571-81187792 | (33) | Chr7:96650026-96650127 |
| (16) | Chr19:57018955-57019135 | (34) | Chr7:96651454-96651618 |
| (17) | Chr19:57019294-57019573 | (35) | Chr7:96653553-96653732 |
| (18) | Chr3:33260566-33260818. | | |

5. The method according to paragraph 4 wherein said DNA region is selected from the DNA regions defined by Hg19 coordinates:

| | | | |
|---|---|---|---|
| (1) | Chr12:52399672-52399922 | (18) | Chr3:33260566-33260818 |
| (2) | Chr12:52400821-52401119 | (19) | Chr2:56150356-56150606 |
| (4) | Chr8:97506813-97507045 | (20) | Chr6:134210545-134210749 |
| (5) | Chr8:97507037-97507257 | (21) | Chr6:134210712-134210951 |
| (6) | Chr13:110959094-110959330 | (24) | Chr7:94024141-94024345 |
| (10) | Chr5:3594657-3594847 | (25) | Chr15:60296522-60296719 |
| (11) | Chr13:95364013-95364178 | (26) | Chr15:60297024-60297305 |
| (12) | Chr13:95364515-95364784 | (28) | Chr16:86544265-86544584 |
| (13) | Chr4:81186918-81187228 | (30) | Chr13:28502100-28502311 |
| (16) | Chr19:57018955-57019135 | (31) | Chr13:28502417-28502603 |
| (17) | Chr19:57019294-57019573 | (35) | Chr7:96653553-96653732 |

6. The method according to any one of paragraphs 1-5 wherein said neoplastic cell is an adenoma or an adenocarcinoma.
7. The method according to any one of paragraphs 1-6 wherein said control level is a non-neoplastic level.
8. The method according to any one of paragraphs 1-7 wherein said neoplasia is a colorectal neoplasia.
9. The method according to any one of paragraphs 1-8 wherein said biological sample is a faecal sample, enema wash, surgical resection, tissue biopsy or blood sample.
10. The method according to any one of paragraphs 1-9 wherein said level of expression is assessed by screening for changes to genomic DNA.
11. The method according to paragraph 10 wherein said changes to genomic DNA are changes to DNA methylation.
12. The method according to paragraph 11, said method comprising assessing the methylation of one or more cytosine residues selected from:
*Chr12:52400748-52409671 (GRASP)*

| | | | |
|---|---|---|---|
| Chr12:52399713 | Chr12:52399731 | Chr12:52399749 | Chr12:52399783 |
| Chr12:52399796 | Chr12:52399808 | Chr12:52399823 | Chr12:52399835 |
| Chr12:52399891 | | | |
| | | | |
| Chr12:52400847 | Chr12:52400850 | Chr12:52400859 | Chr12:52400866 |
| Chr12:52400869 | Chr12:52400873 | Chr12:52400881 | Chr12:52400886 |
| Chr12:52400893 | Chr12:52400895 | Chr12:52400899 | Chr12:52400902 |
| Chr12:52400907 | Chr12:52400913 | Chr12:52400919 | Chr12:52400932 |
| Chr12:52400938 | Chr12:52400958 | Chr12:52400962 | Chr12:52400971 |
| Chr12:52400973 | Chr12:52400976 | Chr12:52400998 | Chr12:52401008 |
| Chr12:52401010 | Chr12:52401012 | Chr12:52401016 | Chr12:52401019 |
| Chr12:52401025 | Chr12:52401041 | Chr12:52401044 | Chr12:52401053 |
| Chr12:52401060 | Chr12:52401064 | Chr12:52401092 | Chr12:52401118 |
| | | | |
| Chr12:52401438 | Chr12:52401448 | Chr12:52401460 | Chr12:52401465 |
| Chr12:52401474 | Chr12:52401477 | Chr12:52401479 | Chr12:52401483 |
| Chr12:52401504 | Chr12:52401514 | Chr12:52401523 | Chr12:52401540 |
| Chr12:52401553 | Chr12:52401576 | Chr12:52401588 | Chr12:52401595 |
| Chr12:52401599 | Chr12:52401604 | Chr12:52401606 | Chr12:52401634 |
| Chr12:52401640 | Chr12:52401644 | Chr12:52401659 | |

*Chr8:97505882-97624037 (SDC2)*

| | | | |
|---|---|---|---|
| Chr8:97506843 | Chr8:97506845 | Chr8:97506861 | Chr8:97506876 |
| Chr8:97506902 | Chr8:97506909 | Chr8:97506911 | Chr8:97506913 |
| Chr8:97506924 | Chr8:97506932 | Chr8:97506934 | Chr8:97506940 |
| Chr8:97506977 | Chr8:97506993 | Chr8:97507005 | Chr8:97507008 |
| Chr8:97507014 | | | |
| | | | |
| Chr8:97507038 | Chr8:97507061 | Chr8:97507064 | Chr8:97507078 |
| Chr8:97507082 | Chr8:97507084 | Chr8:97507089 | Chr8:97507119 |
| Chr8:97507128 | Chr8:97507136 | Chr8:97507144 | Chr8:97507153 |
| Chr8:97507162 | Chr8:97507214 | Chr8:97507216 | |

*Chr13:110959094-110959330 (COL4A1).*

| | | | |
|---|---|---|---|
| Chr13:97507038 | Chr13:97507061 | Chr13:97507064 | Chr13:97507078 |
| Chr13:97507082 | Chr13:97507084 | Chr13:97507089 | Chr13:97507119 |
| Chr13:97507128 | Chr13:97507136 | Chr13:97507144 | Chr13:97507153 |
| Chr13:97507162 | Chr13:97507214 | Chr13:97507216 | |

*Chr5:3596168-3601517 (IRX1)*

| | | | |
|---|---|---|---|
| Chr5:3597229 | Chr5:3597299 | Chr5:3597311 | Chr5:3597321 |
| Chr5:3597330 | Chr5:3597350 | Chr5:3597352 | Chr5:3597373 |
| Chr5:3597413 | Chr5:3597427 | Chr5:3597431 | Chr5:3597447 |
| Chr5:3597450 | Chr5:3597454 | Chr5:3597456 | |
| | | | |
| Chr5:3599998 | Chr5:3600019 | Chr5:3600037 | Chr5:3600050 |
| Chr5:3600058 | Chr5:3600061 | Chr5:3600070 | Chr5:3600074 |
| Chr5:3600076 | Chr5:3600079 | Chr5:3600089 | Chr5:3600091 |
| Chr5:3600104 | Chr5:3600116 | Chr5:3600121 | Chr5:3600124 |
| Chr5:3600131 | Chr5:3600143 | Chr5:3600149 | Chr5:3600169 |
| Chr5:3600173 | | | |
| | | | |
| Chr5:3600163 | Chr5:3600190 | Chr5:3600201 | Chr5:3600205 |
| Chr5:3600208 | Chr5:3600214 | Chr5:3600250 | Chr5:3600252 |
| Chr5:3600255 | Chr5:3600264 | Chr5:3600270 | Chr5:3600284 |
| Chr5:3600309 | Chr5:3600318 | Chr5:3600345 | |
| | | | |
| Chr5:3594663 | Chr5:3594679 | Chr5:3594684 | Chr5:3594697 |
| Chr5:3594703 | Chr5:3594711 | Chr5:3594725 | Chr5:3594738 |
| Chr5:3594745 | Chr5:3594758 | Chr5:3594768 | Chr5:3594774 |
| Chr5:3594778 | Chr5:3594794 | Chr5:3594799 | Chr5:3594818 |
| Chr5:3594840 | | | |

*Chr13:95361879-95364389 (SOX21)*

| | | | |
|---|---|---|---|
| Chr13:95364016 | Chr13:95364019 | Chr13:95364042 | Chr13:95364050 |
| Chr13:95364054 | Chr13:95364061 | Chr13:95364064 | Chr13:95364074 |
| Chr13:95364080 | Chr13:95364082 | Chr13:95364106 | Chr13:95364119 |
| Chr13:95364123 | Chr13:95364125 | Chr13:95364128 | Chr13:95364141 |
| Chr13:95364163 | Chr13:95364171 | | |
| | | | |
| Chr13:95364543 | Chr13:95364545 | Chr13:95364548 | Chr13:95364551 |
| Chr13:95364560 | Chr13:95364571 | Chr13:95364582 | Chr13:95364584 |
| Chr13:95364587 | Chr13:95364602 | Chr13:95364623 | Chr13:95364627 |
| Chr13:95364640 | Chr13:95364649 | Chr13:95364653 | Chr13:95364656 |
| Chr13:95364665 | Chr13:95364684 | Chr13:95364696 | Chr13:95364712 |
| Chr13:95364729 | Chr13:95364732 | Chr13:95364734 | Chr13:95364748 |
| Chr13:95364751 | | | |

*Chr4:81187742-81212171 (FGF5)*

| | | | |
|---|---|---|---|
| Chr4:81186919 | Chr4:81186947 | Chr4:81186964 | Chr4:81186968 |
| Chr4:81186973 | Chr4:81186975 | Chr4:81186983 | Chr4:81187001 |
| Chr4:81187011 | Chr4:81187041 | Chr4:81187056 | Chr4:81187062 |
| Chr4:81187093 | Chr4:81187116 | Chr4:81187125 | Chr4:81187161 |
| Chr4:81187191 | Chr4:81187196 | Chr4:81187198 | |
| | | | |
| Chr4:81187367 | Chr4:81187389 | Chr4:81187458 | Chr4:81187467 |
| Chr4:81187495 | Chr4:81187498 | Chr4:81187504 | Chr4:81187512 |
| Chr4:81187514 | Chr4:81187530 | Chr4:81187539 | Chr4:81187547 |
| Chr4:81187549 | Chr4:81187551 | Chr4:81187554 | Chr4:81187556 |
| Chr4:81187575 | | | |
| | | | |
| Chr4:81187575 | Chr4:81187592 | Chr4:81187601 | Chr4:81187605 |
| Chr4:81187610 | Chr4:81187648 | Chr4:81187652 | Chr4:81187679 |
| Chr4:81187685 | Chr4:81187691 | Chr4:81187693 | Chr4:81187707 |
| Chr4:81187712 | Chr4:81187733 | Chr4:81187735 | Chr4:81187752 |
| Chr4:81187758 | Chr4:81187764 | Chr4:81187784 | |

*Chr19:57019212-57040269 (ZNF471)*

| | | | |
|---|---|---|---|
| Chr19:57018990 | Chr19:57018994 | Chr19:57019003 | Chr19:57019010 |
| Chr19:57019018 | Chr19:57019020 | Chr19:57019025 | Chr19:57019029 |
| Chr19:57019044 | Chr19:57019047 | Chr19:57019067 | Chr19:57019073 |
| Chr19:57019084 | Chr19:57019101 | Chr19:57019118 | |
| | | | |
| Chr19:57019315 | Chr19:57019321 | Chr19:57019346 | Chr19:57019351 |
| Chr19:57019355 | Chr19:57019361 | Chr19:57019364 | Chr19:57019366 |
| Chr19:57019371 | Chr19:57019373 | Chr19:57019385 | Chr19:57019387 |
| Chr19:57019405 | Chr19:57019428 | Chr19:57019433 | Chr19:57019435 |
| Chr19:57019437 | Chr19:57019443 | Chr19:57019451 | Chr19:57019456 |
| Chr19:57019463 | Chr19:57019465 | Chr19:57019470 | Chr19:57019483 |
| Chr19:57019487 | Chr19:57019492 | Chr19:57019502 | Chr19:57019505 |

*Chr3:33191537-33260707 (SUSD5)*

| | | | |
|---|---|---|---|
| Chr3:33260601 | Chr3:33260621 | Chr3:33260631 | Chr3:33260640 |
| Chr3:33260651 | Chr3:33260665 | Chr3:33260676 | Chr3:33260694 |
| Chr3:33260698 | Chr3:33260711 | Chr3:33260715 | Chr3:33260732 |
| Chr3:33260742 | Chr3:33260748 | Chr3:33260755 | Chr3:33260760 |
| Chr3:33260769 | Chr3:33260776 | Chr3:33260778 | Chr3:33260780 |
| Chr3:33260788 | Chr3:33260806 | | |

*Chr2:56093097-56151298 (EFEMP1)*

| | | | |
|---|---|---|---|
| Chr2:56150376 | Chr2:56150389 | Chr2:56150394 | Chr2:56150415 |
| Chr2:56150419 | Chr2:56150423 | Chr2:56150433 | Chr2:56150473 |
| Chr2:56150475 | Chr2:56150478 | Chr2:56150499 | Chr2:56150537 |
| Chr2:56150549 | Chr2:56150580 | Chr2:56150601 | |

*Chr6:134210259-134216675 (TCF21)*

| | | | |
|---|---|---|---|
| Chr6:134210556 | Chr6:134210598 | Chr6:134210615 | Chr6:134210640 |
| Chr6:134210649 | Chr6:134210667 | Chr6:134210692 | Chr6:134210694 |
| Chr6:134210697 | Chr6:134210720 | Chr6:134210745 | |
| | | | |
| Chr6:134210720 | Chr6:134210745 | Chr6:134210776 | Chr6:134210781 |
| Chr6:134210784 | Chr6:134210790 | Chr6:134210792 | Chr6:134210794 |
| Chr6:134210800 | Chr6:134210806 | Chr6:134210812 | Chr6:134210868 |
| Chr6:134210894 | Chr6:134210906 | Chr6:134210919 | Chr6:134210946 |
| | | | |
| Chr6:134211050 | Chr6:134211061 | Chr6:134211076 | Chr6:134211081 |
| Chr6:134211103 | Chr6:134211110 | Chr6:134211121 | Chr6:134211125 |
| Chr6:134211131 | Chr6:134211153 | Chr6:134211155 | Chr6:134211162 |
| Chr6:134211179 | Chr6:134211182 | Chr6:134211184 | Chr6:134211208 |
| Chr6:134211210 | Chr6:134211212 | Chr6:134211218 | Chr6:134211220 |
| Chr6:134211227 | Chr6:134211233 | Chr6:134211241 | Chr6:134211245 |
| Chr6:134211247 | Chr6:134211270 | | |

*Chr7:94023873-94060544 (COL1A2)*

| | | | |
|---|---|---|---|
| Chr7:94024172 | Chr7:94024191 | Chr7:94024214 | Chr7:94024230 |
| Chr7: 94024254 | Chr7:94024266 | Chr7:94024268 | Chr7 :94024272 |
| Chr7:94024288 | Chr7:94024291 | Chr7:94024310 | |

*Chr15:60296421-60298142 (FOXB1)*

| | | | |
|---|---|---|---|
| Chr15:60296555 | Chr15:60296561 | Chr15:60296563 | Chr15:60296578 |
| Chr15:60296585 | Chr15:60296598 | Chr15:60296601 | Chr15:60296614 |
| Chr15:60296616 | Chr15:60296619 | Chr15:60296627 | Chr15:60296633 |
| Chr15:60296639 | Chr15:60296643 | Chr15:60296647 | Chr15:60296654 |
| Chr15:60296665 | Chr15:60296668 | Chr15:60296670 | Chr15:60296675 |
| Chr15:60296679 | Chr15:60296684 | Chr15:60296689 | Chr15:60296694 |
| | | | |
| Chr15:60297035 | Chr15:60297050 | Chr15:60297053 | Chr15:60297109 |
| Chr15:60297118 | Chr15:60297121 | Chr15:60297126 | Chr15:60297128 |
| Chr15:60297130 | Chr15:60297152 | Chr15:60297169 | Chr15:60297174 |
| Chr15:60297178 | Chr15:60297185 | Chr15:60297192 | Chr15:60297203 |
| Chr15:60297212 | Chr15:60297221 | Chr15:60297228 | Chr15:60297252 |
| Chr15:60297266 | Chr15:60297273 | Chr15:60297298 | |

*Chr16:86544133-86548070 (FOXF1)*

| | | | |
|---|---|---|---|
| Chr16:86544571 | Chr16:86544587 | Chr16:86544590 | Chr16:86544593 |
| Chr16:86544597 | Chr16:86544599 | Chr16:86544601 | Chr16:86544608 |
| Chr16:86544624 | Chr16:86544652 | Chr16:86544658 | Chr16:86544675 |
| Chr16:86544685 | Chr16:86544699 | Chr16:86544703 | Chr16:86544706 |
| Chr16:86544714 | Chr16:86544720 | Chr16:86544735 | Chr16:86544745 |
| Chr16:86544763 | | | |
| | | | |
| Chr16:86544268 | Chr16:86544273 | Chr16:86544295 | Chr16:86544298 |
| Chr16:86544305 | Chr16:86544308 | Chr16:86544312 | Chr16:86544321 |
| Chr16:86544337 | Chr16:86544339 | Chr16:86544346 | Chr16:86544377 |
| Chr16:86544384 | Chr16:86544391 | Chr16:86544416 | Chr16:86544431 |
| Chr16:86544460 | Chr16:86544464 | Chr16:86544477 | Chr16:86544484 |
| Chr16:86544518 | Chr16:86544523 | Chr16:86544547 | Chr16:86544552 |
| Chr16:86544559 | Chr16:86544571 | | |
| | | | |
| Chr16:86544810 | Chr16:86544832 | Chr16:86544835 | Chr16:86544843 |
| Chr16:86544853 | Chr16:86544859 | Chr16:86544862 | Chr16:86544865 |
| Chr16:86544867 | Chr16:86544870 | Chr16:86544874 | Chr16:86544877 |
| Chr16:86544885 | Chr16:86544892 | Chr16:86544900 | Chr16:86544907 |
| Chr16:86544915 | Chr16:86544928 | Chr16:86544931 | Chr16:86544934 |
| Chr16:86544951 | Chr16:86544955 | Chr16:86544958 | Chr16:86544966 |
| Chr16:86544972 | Chr16:86544975 | Chr16:86544978 | Chr16:86544987 |
| Chr16:86544993 | Chr16:86544996 | Chr16:86545000 | Chr16:86545002 |
| Chr16:86545005 | Chr16:86545015 | Chr16:86545018 | Chr16:86545060 |
| Chr16:86545062 | Chr16:86545078 | Chr16:86545092 | |

*Chr13:28494168-28500451 (PDX1)*

| | | | |
|---|---|---|---|
| Chr13:28502109 | Chr13:28502153 | Chr13:28502161 | Chr13:28502177 |
| Chr13:28502179 | Chr13:28502191 | Chr13:28502198 | Chr13:28502205 |
| Chr13:28502207 | Chr13:28502210 | Chr13:28502245 | Chr13:28502309 |
| | | | |
| Chr13:28502442 | Chr13:28502449 | Chr13:28502461 | Chr13:28502464 |
| Chr13:28502475 | Chr13:28502507 | Chr13:28502512 | Chr13:28502538 |
| Chr13:28502544 | Chr13:28502549 | Chr13:28502559 | Chr13:28502564 |
| Chr13:28502585 | | | |
| | | | |
| Chr13:28503045 | Chr13:28503049 | Chr13:28503081 | Chr13:28503099 |
| Chr13:28503114 | Chr13:28503127 | Chr13:28503138 | Chr13:28503147 |
| Chr13:28503155 | Chr13:28503157 | Chr13:28503179 | |

*Chr7:96649702-96654143 (DLX5)*

| | | | |
|---|---|---|---|
| Chr7:96650062 | Chr7:96650072 | Chr7:96650078 | Chr7:96650096 |
| Chr7:96650099 | Chr7:96650102 | | |
| | | | |
| Chr7:96651485 | Chr7:96651488 | Chr7:96651518 | Chr7:96651523 |
| Chr7:96651532 | Chr7:96651535 | Chr7:96651537 | Chr7:96651542 |
| Chr7:96651550 | Chr7:96651586 | | |
| | | | |
| Chr7:96653596 | Chr7:96653605 | Chr7:96653607 | Chr7:96653617 |
| Chr7:96653620 | Chr7:96653623 | Chr7:96653644 | Chr7:96653656 |
| Chr7:96653683 | Chr7:96653686 | Chr7:96653692 | Chr7:96653698 |
| Chr7:96653701 | | | |

*Chr21:28208606-28217728 (ADAMTS1)*

| | | | |
|---|---|---|---|
| Chr21:28217973 | Chr21:28218002 | Chr21:28218015 | Chr21:28218018 |
| Chr21:28218047 | Chr21:28218051 | Chr21:28218057 | Chr21:28218072 |
| Chr21:28218074 | Chr21:28218084 | Chr21:28218105 | |
| | | | |
| Chr21:28218514 | Chr21:28218516 | Chr21:28218550 | Chr21:28218568 |
| Chr21:28218579 | Chr21:28218586 | Chr21:28218596 | Chr21:28218635 |
| Chr21:28218638 | Chr21:28218646 | Chr21:28218671 | Chr21:28218684 |
| Chr21:28218688 | Chr21:28218704 | Chr21:28218729 | Chr21:28218741 |
| | | | |
| Chr21:28218893 | Chr21:28218906 | Chr21:28218914 | Chr21:28218916 |
| Chr21:28218928 | Chr21:28218934 | Chr21:28218938 | Chr21:28218949 |
| Chr21:28218953 | Chr21:28218959 | Chr21:28218974 | Chr21:28218976 |
| Chr21:28218978 | Chr21:28218984 | Chr21:28218986 | Chr21:28218996 |
| Chr21:28219008 | Chr21:28219016 | | |

*Chr13:110959631-111165373 (COL4A2)*

| | | | |
|---|---|---|---|
| Chr13:110960813 | Chr13:110960827 | Chr13:110960849 | Chr13:110960875 |
| Chr13:110960925 | Chr13:110960930 | Chr13:110960938 | Chr13:110960978 |
| Chr13:110961001 | Chr13:110961003 | Chr13:110961025 | Chr13:110961045 |
| Chr13:110961049 | Chr13:110961069 | Chr13:110961072 | Chr13:110961088 |
| Chr13:110961091 | Chr13:110961095 | Chr13:110961110 | Chr13:110961116 |
| Chr13:110961131 | Chr13:110960786 | | |
| | | | |
| Chr13:110961358 | Chr13:110961386 | Chr13:110961404 | Chr13:110961406 |
| Chr13:110961445 | Chr13:110961579 | Chr13:110961594 | Chr13:110961606 |
| Chr13:110961631 | | | |
| | | | |
| Chr13:110959935 | Chr13:110959965 | Chr13:110959968 | Chr13:110959974 |
| Chr13:110959979 | Chr13:110959981 | Chr13:110959986 | Chr13:110959988 |
| Chr13:110959997 | Chr13:110959999 | Chr13:110960004 | Chr13:110960006 |
| Chr13:110960011 | Chr13:110960027 | Chr13:110960035 | Chr13:110960046 |
| Chr13:110960051 | Chr13:110960071 | Chr13:110960099 | Chr13:110960108 |
| Chr13:110960115 | Chr13:110960156 | Chr13:110960180 | |

*Chr5:83238126-83680611 (EDIL3)*

| | | | |
|---|---|---|---|
| Chr5:83679825 | Chr5:83679827 | Chr5:83679836 | Chr5:83679843 |
| Chr5:83679846 | Chr5:83679860 | Chr5:83679864 | Chr5:83679883 |
| Chr5:83679894 | Chr5:83679900 | Chr5:83679906 | Chr5:83679910 |
| Chr5:83679937 | Chr5:83679953 | Chr5:83679957 | Chr5:83679961 |
| Chr5:83679976 | Chr5:83679982 | Chr5:83679987 | Chr5:83680003 |
| Chr5:83680007 | Chr5:83680011 | Chr5:83680026 | Chr5:83680032 |
| Chr5:83680086 | Chr5:83680090 | Chr5:83680103 | Chr5:83680106 |
| Chr5:83680122 | Chr5:83680126 | | |
| | | | |
| Chr5:83679546 | Chr5:83679568 | Chr5:83679571 | Chr5:83679585 |
| Chr5:83679602 | Chr5:83679617 | Chr5:83679630 | Chr5:83679640 |
| Chr5:83679667 | Chr5:83679675 | Chr5:83679689 | Chr5:83679693 |
| Chr5:83679699 | Chr5:83679701 | Chr5:83679733 | |
| | | | |
| Chr5:83679973 | Chr5:83679989 | Chr5:83679992 | Chr5:83680001 |
| Chr5:83680003 | Chr5:83680006 | Chr5:83680012 | Chr5:83680015 |
| Chr5:83680019 | Chr5:83680033 | Chr5:83680036 | Chr5:83680045 |
| Chr5:83680051 | Chr5:83680061 | Chr5:83680063 | Chr5:83680066 |
| Chr5:83680108 | Chr5:83680115 | Chr5:83680118 | Chr5:83680132 |
| Chr5:83680147 | Chr5:83680159 | Chr5:83680174 | Chr5:83680181 |
| Chr5:83680185 | Chr5:83680198 | Chr5:83680205 | Chr5:83680209 |
| Chr5:83680217 | Chr5:83680232 | Chr5:83680235 | Chr5:83680237 |
| Chr5:83680253 | | | |
| | | | |
| Chr5:83680108 | Chr5:83680115 | Chr5:83680118 | Chr5:83680132 |
| Chr5:83680147 | Chr5:83680159 | Chr5:83680174 | Chr5:83680181 |
| Chr5:83680185 | Chr5:83680198 | Chr5:83680205 | Chr5:83680209 |
| Chr5:83680217 | Chr5:83680232 | Chr5:83680235 | Chr5:83680237 |
| Chr5:83680253 | Chr5:83680264 | Chr5:83680284 | Chr5:83680310 |
| Chr5:83680326 | Chr5:83680355 | | |
| | | | |
| Chr5:83680401 | Chr5:83680406 | Chr5:83680409 | Chr5:83680411 |
| Chr5:83680413 | Chr5:83680430 | Chr5:83680438 | Chr5:83680441 |
| Chr5:83680444 | Chr5:83680458 | Chr5:83680463 | Chr5:83680474 |
| Chr5:83680483 | Chr5:83680485 | Chr5:83680488 | Chr5:83680492 |
| Chr5:83680497 | Chr5:83680499 | Chr5:83680507 | Chr5:83680509 |
| Chr5:83680513 | Chr5:83680518 | Chr5:83680529 | Chr5:83680531 |
| Chr5:83680548 | Chr5:83680559 | Chr5:83680564 | Chr5:83680568 |
| Chr5:83680572 | Chr5:83680579 | Chr5:83680589 | Chr5:83680591 |
| Chr5:83680593 | Chr5:83680596 | Chr5:83680602 | |

*Chr15:48700503-48937985 (FBN1)*

| | | | |
|---|---|---|---|
| Chr15:48938149 | Chr15:48938163 | Chr15:48938172 | Chr15:48938181 |
| Chr15:48938183 | Chr15:48938218 | Chr15:48938236 | Chr15:48938238 |
| Chr15:48938254 | Chr15:48938267 | Chr15:48938273 | Chr15:48938280 |
| Chr15:48938291 | Chr15:48938349 | | |

*Chr1:47901689-47906363 (FOXD2)*

| | | | |
|---|---|---|---|
| Chr1:47899102 | Chr1:47899126 | Chr1:47899129 | Chr1:47899143 |
| Chr1:47899152 | Chr1:47899168 | Chr1:47899184 | Chr1:47899205 |
| Chr1:47899212 | Chr1:47899230 | Chr1:47899232 | Chr1:47899253 |
| Chr1:47899286 | Chr1:47899327 | | |
| | | | |
| Chr1:47909988 | Chr1:47909995 | Chr1:47910005 | Chr1:47910036 |
| Chr1:47910051 | Chr1:47910056 | Chr1:47910062 | Chr1:47910074 |
| Chr1:47910103 | Chr1:47910122 | Chr1:47910137 | Chr1:47910142 |
| Chr1:47910144 | Chr1:47910146 | | |

*Chr2:66662532-66799891 (MEIS1)*

| | | | |
|---|---|---|---|
| Chr2:66662043 | Chr2:66662047 | Chr2:66662049 | Chr2:66662063 |
| Chr2:66662073 | Chr2:66662107 | Chr2:66662111 | Chr2:66662124 |
| Chr2:66662145 | Chr2:66662156 | Chr2:66662161 | Chr2:66662163 |
| Chr2:66662178 | Chr2:66662218 | | |
| | | | |
| Chr2:66662178 | Chr2:66662218 | Chr2:66662220 | Chr2:66662246 |
| Chr2:66662265 | Chr2:66662280 | Chr2:66662306 | Chr2:66662310 |
| Chr2:66662323 | Chr2:66662333 | Chr2:66662337 | |

*Chr16:55513081-55540586 (MMP2)*

| | | | |
|---|---|---|---|
| Chr16:55512695 | Chr16:55512711 | Chr16:55512732 | Chr16:55512750 |
| Chr16:55512753 | Chr16:55512793 | | |
| Chr16:55513961 | Chr16:55513997 | Chr16:55514025 | Chr16:55514059 |
| Chr16:55514061 | Chr16:55514092 | Chr16:55514120 | Chr16:55514131 |
| Chr16:55514158 | Chr16:55514205 | | |

*Chr7:24323807-24331484 (NPY)*

| | | | |
|---|---|---|---|
| Chr7:24323767 | Chr7:24323792 | Chr7:24323794 | Chr7:24323799 |
| Chr7:24323817 | Chr7:24323834 | Chr7:24323840 | Chr7:24323844 |
| Chr7:24323848 | Chr7:24323866 | Chr7:24323876 | Chr7:24323880 |
| Chr7:24323882 | Chr7:24323884 | Chr7:24323894 | Chr7:24323905 |
| Chr7:24323910 | Chr7:24323930 | Chr7:24323934 | |
| | | | |
| Chr7:24324180 | Chr7:24324186 | Chr7:24324206 | Chr7:24324217 |
| Chr7:24324222 | Chr7:24324225 | Chr7:24324244 | Chr7:24324249 |
| Chr7:24324251 | Chr7:24324268 | Chr7:24324274 | Chr7:24324280 |
| Chr7:24324311 | Chr7:24324313 | | |
| | | | |
| Chr7:24324549 | Chr7:24324570 | Chr7:24324592 | Chr7:24324595 |
| Chr7:24324597 | Chr7:24324599 | Chr7:24324621 | Chr7:24324625 |
| Chr7:24324633 | Chr7:24324635 | Chr7:24324668 | Chr7:24324672 |
| Chr7:24324676 | Chr7:24324679 | Chr7:24324683 | Chr7:24324690 |

*Chr19:38741877-38747172 (PPPIR14A)*

| | | | |
|---|---|---|---|
| Chr19:38747280 | Chr19:38747296 | Chr19:38747300 | Chr19:38747313 |
| Chr19:38747319 | Chr19:38747322 | Chr19:38747328 | Chr19:38747334 |
| Chr19:38747355 | Chr19:38747361 | Chr19:38747371 | Chr19:38747382 |
| Chr19:38747389 | Chr19:38747410 | | |
| | | | |
| Chr19:38746654 | Chr19:38746680 | Chr19:38746690 | Chr19:38746701 |
| Chr19:38746715 | Chr19:38746726 | Chr19:38746728 | Chr19:38746732 |
| Chr19:38746749 | Chr19:38746759 | Chr19:38746770 | Chr19:38746784 |
| Chr19:38746786 | Chr19:38746804 | Chr19:38746808 | Chr19:38746811 |
| Chr19:38746826 | Chr19:38746834 | Chr19:38746837 | Chr19:38746849 |
| Chr19:38746852 | Chr19:38746865 | Chr19:38746871 | Chr19:38746873 |
| Chr19:38746875 | Chr19:38746877 | Chr19:38746882 | Chr19:38746898 |

or a corresponding cytosine at position n+1 on the opposite DNA strand, in a biological sample from said individual wherein a higher level of methylation of one or more of said residues relative to the methylation level of a corresponding residue in a control sample is indicative of a large intestine neoplasm or a predisposition to the onset of a neoplastic state.
13. The method according to paragraph 12 wherein said one or more cytosine residues are selected from:
*Chr12:52400748-52409671 (GRASP)*

| | | | |
|---|---|---|---|
| Chr12:52399713 | Chr12:52399731 | Chr12:52399749 | Chr12:52399783 |
| Chr12:52399796 | Chr12:52399808 | Chr12:52399823 | Chr12:52399835 |
| Chr12:52399891 | | | |
| | | | |
| Chr12:52400847 | Chr12:52400850 | Chr12:52400859 | Chr12:52400866 |
| Chr12:52400869 | Chr12:52400873 | Chr12:52400881 | Chr12:52400886 |
| Chr12:52400893 | Chr12:52400895 | Chr12:52400899 | Chr12:52400902 |
| Chr12:52400907 | Chr12:52400913 | Chr12:52400919 | Chr12:52400932 |
| Chr12:52400938 | Chr12:52400958 | Chr12:52400962 | Chr12:52400971 |
| Chr12:52400973 | Chr12:52400976 | Chr12:52400998 | Chr12:52401008 |
| Chr12:52401010 | Chr12:52401012 | Chr12:52401016 | Chr12:52401019 |
| Chr12:52401025 | Chr12:52401041 | Chr12:52401044 | Chr12:52401053 |
| Chr12:52401060 | Chr12:52401064 | Chr12:52401092 | Chr12:52401118 |

*Chr8:97505882-97624037 (SDC2)*

| | | | |
|---|---|---|---|
| Chr8:97506843 | Chr8:97506845 | Chr8:97506861 | Chr8:97506876 |
| Chr8:97506902 | Chr8:97506909 | Chr8:97506911 | Chr8:97506913 |
| Chr8:97506924 | Chr8:97506932 | Chr8:97506934 | Chr8:97506940 |
| Chr8:97506977 | Chr8:97506993 | Chr8:97507005 | Chr8:97507008 |
| Chr8:97507014 | | | |
| | | | |
| Chr8:97507038 | Chr8:97507061 | Chr8:97507064 | Chr8:97507078 |
| Chr8:97507082 | Chr8:97507084 | Chr8:97507089 | Chr8:97507119 |
| Chr8:97507128 | Chr8:97507136 | Chr8:97507144 | Chr8:97507153 |
| Chr8:97507162 | Chr8:97507214 | Chr8:97507216 | |

*Chr13:110959094-110959330 (COL4A1).*

| | | | |
|---|---|---|---|
| Chr13:97507038 | Chr13:97507061 | Chr13:97507064 | Chr13:97507078 |
| Chr13:97507082 | Chr13:97507084 | Chr13:97507089 | Chr13:97507119 |
| Chr13:97507128 | Chr13:97507136 | Chr13:97507144 | Chr13:97507153 |
| Chr13:97507162 | Chr13:97507214 | Chr13:97507216 | |
| Chr5:3594663 | Chr5:3594679 | Chr5:3594684 | Chr5:3594697 |
| Chr5:3594703 | Chr5:3594711 | Chr5:3594725 | Chr5:3594738 |
| Chr5:3594745 | Chr5:3594758 | Chr5:3594768 | Chr5:3594774 |
| Chr5:3594778 | Chr5:3594794 | Chr5:3594799 | Chr5:3594818 |
| Chr5:3594840 | | | |

*Chr13:95361879-95364389 (SOX21)*

| | | | |
|---|---|---|---|
| Chr13:95364016 | Chr13:95364019 | Chr13:95364042 | Chr13:95364050 |
| Chr13:95364054 | Chr13:95364061 | Chr13:95364064 | Chr13:95364074 |
| Chr13:95364080 | Chr13:95364082 | Chr13:95364106 | Chr13:95364119 |
| Chr13:95364123 | Chr13:95364125 | Chr13:95364128 | Chr13:95364141 |
| Chr13:95364163 | Chr13:95364171 | | |
| | | | |
| Chr13:95364543 | Chr13:95364545 | Chr13:95364548 | Chr13:95364551 |
| Chr13:95364560 | Chr13:95364571 | Chr13:95364582 | Chr13:95364584 |
| Chr13:95364587 | Chr13:95364602 | Chr13:95364623 | Chr13:95364627 |
| Chr13:95364640 | Chr13:95364649 | Chr13:95364653 | Chr13:95364656 |
| Chr13:95364665 | Chr13:95364684 | Chr13:95364696 | Chr13:95364712 |
| Chr13:95364729 | Chr13:95364732 | Chr13:95364734 | Chr13:95364748 |
| Chr13:95364751 | | | |

*Chr4:81187742-81212171 (FGF5)*

| | | | |
|---|---|---|---|
| Chr4:81186919 | Chr4:81186947 | Chr4:81186964 | Chr4:81186968 |
| Chr4:81186973 | Chr4:81186975 | Chr4:81186983 | Chr4:81187001 |
| Chr4:81187011 | Chr4:81187041 | Chr4:81187056 | Chr4:81187062 |
| Chr4:81187093 | Chr4:81187116 | Chr4:81187125 | Chr4:81187161 |
| Chr4:81187191 | Chr4:81187196 | Chr4:81187198 | |

*Chr19:57019212-57040269 (ZNF471)*

| | | | |
|---|---|---|---|
| Chr19:57018990 | Chr19:57018994 | Chr19:57019003 | Chr19:57019010 |
| Chr19:57019018 | Chr19:57019020 | Chr19:57019025 | Chr19:57019029 |
| Chr19:57019044 | Chr19:57019047 | Chr19:57019067 | Chr19:57019073 |
| Chr19:57019084 | Chr19:57019101 | Chr19:57019118 | |
| Chr19:57019315 | Chr19:57019321 | Chr19:57019346 | Chr19:57019351 |
| Chr19:57019355 | Chr19:57019361 | Chr19:57019364 | Chr19:57019366 |
| Chr19:57019371 | Chr19:57019373 | Chr19:57019385 | Chr19:57019387 |
| Chr19:57019405 | Chr19:57019428 | Chr19:57019433 | Chr19:57019435 |
| Chr19:57019437 | Chr19:57019443 | Chr19:57019451 | Chr19:57019456 |
| Chr19:57019463 | Chr19:57019465 | Chr19:57019470 | Chr19:57019483 |
| Chr19:57019487 | Chr19:57019492 | Chr19:57019502 | Chr19:57019505 |

*Chr3:33191537-33260707 (SUSD5)*

| | | | |
|---|---|---|---|
| Chr3:33260601 | Chr3:33260621 | Chr3:33260631 | Chr3:33260640 |
| Chr3:33260651 | Chr3:33260665 | Chr3:33260676 | Chr3:33260694 |
| Chr3:33260698 | Chr3:33260711 | Chr3:33260715 | Chr3:33260732 |
| Chr3:33260742 | Chr3:33260748 | Chr3:33260755 | Chr3:33260760 |
| Chr3:33260769 | Chr3:33260776 | Chr3:33260778 | Chr3:33260780 |
| Chr3:33260788 | Chr3:33260806 | | |

*Chr2:56093097-56151298 (EFEMP1)*

| | | | |
|---|---|---|---|
| Chr2:56150376 | Chr2:56150389 | Chr2:56150394 | Chr2:56150415 |
| Chr2:56150419 | Chr2:56150423 | Chr2:56150433 | Chr2:56150473 |
| Chr2:56150475 | Chr2:56150478 | Chr2:56150499 | Chr2:56150537 |
| Chr2:56150549 | Chr2:56150580 | Chr2:56150601 | |

*Chr6:134210259-134216675 (TCF21)*

| | | | |
|---|---|---|---|
| Chr6:134210556 | Chr6:134210598 | Chr6:134210615 | Chr6:134210640 |
| Chr6:134210649 | Chr6:134210667 | Chr6:134210692 | Chr6:134210694 |
| Chr6:134210697 | Chr6:134210720 | Chr6:134210745 | |
| | | | |
| Chr6:134210720 | Chr6:134210745 | Chr6:134210776 | Chr6:134210781 |
| Chr6:134210784 | Chr6:134210790 | Chr6:134210792 | Chr6:134210794 |
| Chr6:134210800 | Chr6:134210806 | Chr6:134210812 | Chr6:134210868 |
| Chr6:134210894 | Chr6:134210906 | Chr6:134210919 | Chr6:134210946 |

*Chr7:94023873-94060544 (COL1A2)*

| | | | |
|---|---|---|---|
| Chr7:94024172 | Chr7:94024191 | Chr7:94024214 | Chr7:94024230 |
| Chr7: 94024254 | Chr7:94024266 | Chr7:94024268 | Chr7 :94024272 |
| Chr7:94024288 | Chr7:94024291 | Chr7:94024310 | |

*Chr15:60296421-60298142 (FOXB1)*

| | | | |
|---|---|---|---|
| Chr15:60296555 | Chr15:60296561 | Chr15:60296563 | Chr15:60296578 |
| Chr15:60296585 | Chr15:60296598 | Chr15:60296601 | Chr15:60296614 |
| Chr15:60296616 | Chr15:60296619 | Chr15:60296627 | Chr15:60296633 |
| Chr15:60296639 | Chr15:60296643 | Chr15:60296647 | Chr15:60296654 |
| Chr15:60296665 | Chr15:60296668 | Chr15:60296670 | Chr15:60296675 |
| Chr15:60296679 | Chr15:60296684 | Chr15:60296689 | Chr15:60296694 |
| | | | |
| Chr15:60297035 | Chr15:60297050 | Chr15:60297053 | Chr15:60297109 |
| Chr15:60297118 | Chr15:60297121 | Chr15:60297126 | Chr15:60297128 |
| Chr15:60297130 | Chr15:60297152 | Chr15:60297169 | Chr15:60297174 |
| Chr15:60297178 | Chr15:60297185 | Chr15:60297192 | Chr15:60297203 |
| Chr15:60297212 | Chr15:60297221 | Chr15:60297228 | Chr15:60297252 |
| Chr15:60297266 | Chr15:60297273 | Chr15:60297298 | |

*Chr16:86544133-86548070 (FOXF1)*

| | | | |
|---|---|---|---|
| Chr16:86544268 | Chr16:86544273 | Chr16:86544295 | Chr16:86544298 |
| Chr16:86544305 | Chr16:86544308 | Chr16:86544312 | Chr16:86544321 |
| Chr16:86544337 | Chr16:86544339 | Chr16:86544346 | Chr16:86544377 |
| Chr16:86544384 | Chr16:86544391 | Chr16:86544416 | Chr16:86544431 |
| Chr16:86544460 | Chr16:86544464 | Chr16:86544477 | Chr16:86544484 |
| Chr16:86544518 | Chr16:86544523 | Chr16:86544547 | Chr16:86544552 |
| Chr16:86544559 | Chr16:86544571 | | |

*Chr13:28494168-28500451 (PDX1)*

| | | | |
|---|---|---|---|
| Chr13:28502109 | Chr13:28502153 | Chr13:28502161 | Chr13:28502177 |
| Chr13:28502179 | Chr13:28502191 | Chr13:28502198 | Chr13:28502205 |
| Chr13:28502207 | Chr13:28502210 | Chr13:28502245 | Chr13:28502309 |
| | | | |
| Chr13:28502442 | Chr13:28502449 | Chr13:28502461 | Chr13:28502464 |
| Chr13:28502475 | Chr13:28502507 | Chr13:28502512 | Chr13:28502538 |
| Chr13:28502544 | Chr13:28502549 | Chr13:28502559 | Chr13:28502564 |
| Chr13:28502585 | | | |

*Chr7:96649702-96654143 (DLX5)*

| | | | |
|---|---|---|---|
| Chr7:96653596 | Chr7:96653605 | Chr7:96653607 | Chr7:96653617 |
| Chr7:96653620 | Chr7:96653623 | Chr7:96653644 | Chr7:96653656 |
| Chr7:96653683 | Chr7:96653686 | Chr7:96653692 | Chr7:96653698 |
| Chr7:96653701 | | | |

14. The method according to any one of paragraphs 1 to 13 wherein said level of expression is assessed by screening for changes to the chromatin protein with which said gene is associated.
15. A method of screening for the onset or predisposition to the onset of or monitoring a large intestine neoplasm in an individual, said method comprising assessing the level of expression of a DNA region selected from:
(i) the region defined by any one or more of Hg19 coordinates and 2kb upstream of the transcription start site:

| | |
|---|---|
| (1) chr12:52400748..52409671 | (34) chr10:101292690..101296281 |
| (2) chr5:3596168..3601517 | (35) chr4:4190530..4228621 |
| (3) chr13:95361876..95364389 | (36) chr12:54943404..54973023 |
| (4) chr4:81187742..81212171 | (37) chr5:176047210..176057557 |
| (5) chr19:57019212..57040270 | (38) chr12:22346325..22487648 |
| (6) chr3:33191537..33260707 | (39) chr19:56894648..56904889 |
| (7) chr15:60296421..60298142 | (40) chr20:21491648..21494664 |
| (8) chr13:28494168..28500451 | (41) chr1:50883225..50889141 |
| (9) chr7:96649702..96654143, | (42) chr7:27180996..27183287 |
| (10) chr8:140,811,770-141,537,860 | (43) chr11:2016406..2019065 |
| (11) chr5:2746279..2751769 | (44) chr14:57267425..57277184 |
| (12) chr18:55102917..55158530 | (45) chr4:126237567..126414087 |
| (13) chr20:37353101..37358016 | (46) chr8:23559964..23563922 |
| (14) chr8:2792875..4852328 | (47) chr10:131633547..131762091 |
| (15) chr16:66613351..66622178 | (48) chr4:62362839..62938168 |
| (16) chr5:37815753..37839782 | (49) chr1:47901689..47906363 |
| (17) chr1:63788730..63790797 | (50) chr17:77768176..77770890 |
| (18) chr15:37156644..37178734 | (51) chr17:93598762..93604831 |
| (19) chr7:27139973..27142394 | (52) chr1:33789224..33841194 |
| (20) chr20:21686297..21696620 | (53) chr9:124,004,679-124,030,840 |
| (21) chr16:51169886..51185183 | (54) chr4:158141736..158287227 |
| (22) chr12:85253267..85306606 | (55) chr12:9445136..9462559 |
| (23) chr8:6357172..6420784 | (56) chr12:24964278..25102308 |
| (24) chr14:85996488..86094270 | (57) chrX:21542357..21690352 |
| (25) chr2:182541194..182545381 | (58) chr20:52769988..52790516 |
| (26) chr7:30951468..30965131 | (59) chr3:172162951..172166203 |
| (27) chr8:131792547..132052835 | (60) chr13:28366780..28368089 |
| (28) chr3:128749292..128759583 | (61) chr7:50344378...50472799 |
| (29) chr10:101088856..101154087 | (62) chr7:149412148..149431664 |
| (30) chr7:27282164..27286192 | (63) chr7:24323809..24331477 |
| (31) chr10:129535538..129539450 | (64) chr4:30722037..31148421 |
| (32) chr19:49316274..49339934 | (65) chr10:47083534..47088320 |
| (33) chr6:391752..411443 | |

(ii) the gene region, including 2kb upstream of any one or more of:

| | | | |
|---|---|---|---|
| (1) GRASP | (18) ANGPT2 | (35) NKX2-6 | (52) HOXA5 |
| (2) IRX1 | (19) LHX6 | (36) PAX1 | (53) GDNF |
| (3) SOX21 | (20) NEUROD1 | (37) FOXD2 | (54) FAT4 |
| (4) FGF5 | (21) AC149644.1 | (38) SLC6A15 | (55) HOXA2 |
| (5) ZNF471 | (22) CCDC48 | (39) PHC2 | (56) LPHN3 |
| (6) SUSD5 | (23) EVX1 | (40) FLRT2 | (57) ADCYAP1 |
| (7) FOXB1 | (24) GHSR | (41) GATA2 | (58) GRIA2 |
| (8) PDX1 | (25) HSD17B14 | (42) ADCY8 | (59) AQP1 |
| (9) DLX5 | (26) KRBA1 | (43) CNNM1 | (60) BCAT1 |
| (10) ONECUT2 | (27) OTOP1 | (44) IKZF1 | (61) CYP24A1 |
| (11) DMRTA2 | (28) PPYR1 | (45) NKX2-3 | (62) FOXI2 |
| (12) CMTM2 | (29) SRMS | (46) PCDH7 | (63) GSX1 |
| (13) OTX2 | (30) ZNF582 | (47) SNCB | (64) IRF4 |
| (14) LOC145845 | (31) IRX2 | (48) ST8SIA1 | (65) NPY |
| (15) EBF3 | (32) CSMD1 | (49) TRAPPC9 | (66) PDE1B |
| (16) SALL1 | (33) MIR675,H19 | (50) NKX2-2 | |
| (17) CBX8 | (34) FOXD3 | (51) SLC32A1 | |

in a biological sample from said individual wherein a lower level of expression of the DNA regions of group (i) and/or (ii) relative to control levels is indicative of a large intestine neoplasm or a predisposition to the onset of a neoplastic state.
16. The method according to paragraph 15 wherein said one or more genes or transcripts selected from:
(i) the region defined by any one or more of Hg19 coordinates and 2kb upstream of the transcription start site:

| | |
|---|---|
| (1) chr12:52400748..52409671 | (6) chr3:33191537..33260707 |
| (2) chr5:3596168..3601517 | (7) chr15:60296421..60298142 |
| (3) chr13:95361876..95364389 | (8) chr13:28494168..28500451 |
| (4) chr4:81187742..81212171 | (9) chr7:96649702..96654143, |
| (5) chr19:57019212..57040270 | |

(ii) the gene region, including 2kb upstream of any one or more of:

| | | | |
|---|---|---|---|
| (1) GRASP | (4) FGF5 | (6) SUSD5 | (8) PDX1 |
| (2) IRX1 | (5) ZNF471 | (7) FOXB1 | (9) DLX5 |
| (3) SOX21 | | | |

17. The method according to any one of paragraphs 15 or 16 wherein said level of expression is mRNA expression or protein expression.
18. The method according to any one of paragraphs 1-17 wherein said individual is a human.
19. A molecular array, which array comprises a plurality of:
(i) nucleic acid molecules comprising a nucleotide sequence corresponding to any one or more of the DNA regions listed in any one of paragraphs 1-5 or a sequence exhibiting at least 80% identity thereto or a functional derivative, fragment, variant or homologue of said nucleic acid molecule; or
(ii) nucleic acid molecules comprising a nucleotide sequence capable of hybridising to any one or more of the sequences of (i) under medium stringency conditions or a functional derivative, fragment, variant or homologue of said nucleic acid molecule; or
(iii) nucleic acid probes or oligonucleotides comprising a nucleotide sequence capable of hybridising to any one or more of the sequences of (i) under medium stringency conditions or a functional derivative, fragment, variant or homologue of said nucleic acid molecule; or
(iv) probes capable of binding to any one or more of the proteins encoded by the nucleic acid molecules of (i) or a derivative, fragment or, homologue thereof
wherein the level of expression of said marker genes of (i) or proteins of (iv) is indicative of the neoplastic state of a cell or cellular subpopulation derived from the large intestine.

## Claims

1. A method of screening for the onset or predisposition to the onset of a colorectal neoplasm or monitoring the progress of a colorectal neoplasm in an individual, said method comprising assessing the methylation status of the DNA region defined by Hg19 coordinates chr12:24964278..25102308 and 2kb upstream of the transcription start site of the BCAT1 gene therein comprised in a biological sample from said individual wherein a higher level of methylation of the DNA regions specified above relative to control levels is indicative of a colorectal neoplasm or a predisposition to the onset of a colorectal neoplasm.

2. The method according to claim 1 wherein said neoplasm is an adenoma or an adenocarcinoma.

3. The method according to any one of claims 1-2 wherein said control level is a non-neoplastic level.

4. The method according to claim 3 wherein a decrease in the level of methylation relative to said control level is indicative of the clearance of the neoplasm.

5. The method according to any one of claim 1-4 wherein said biological sample is a faecal sample, enema wash, surgical resection, tissue biopsy or blood sample.

6. The method according to claim 5 wherein said blood sample is whole blood, serum or plasma.

7. A method of screening for the onset or predisposition to the onset of or monitoring a colorectal neoplasm in an individual, said method comprising assessing the level of expression of the DNA region defined by Hg19 coordinates chr12:24964278..25102308 whereby said expression is specific for the gene BCAT1 comprised in said DNA region in a biological sample from said individual wherein a lower level of expression of the DNA region specified above relative to control levels is indicative of a colorectal neoplasm or a predisposition to the onset of a colorectal neoplasm.

8. The method according to claim 7 wherein said level of expression is mRNA expression or protein expression.

9. The method according to any one of claims 7 and 8 wherein said control level is a non-neoplastic level.

10. The method according to any one of claims 7-9 wherein said biological sample is a faecal sample, enema wash, surgical resection, tissue biopsy or blood sample.

11. The method according to claim 10 wherein said blood sample is whole blood, serum or plasma.

12. The method according to any one of claims 1-6 wherein said DNA region is the promoter region.

13. The method according to any one of claims 1-12 wherein said individual is a human.

## Patentansprüche

1. Verfahren zum Screenen des Beginns oder der Prädisposition für den Beginn eines kolorektalen Neoplasmas oder zum Überwachen des Fortschritts eines kolorektalen Neoplasmas in einem Individuum, wobei das Verfahren das Beurteilen des Methylierungsstatus der DNA-Region, die durch Hg19-Koordinaten chr12:24964278..25102308 und 2kB stromaufwärts der Transkriptionsstartstelle des darin enthaltenen BCAT1-Gens definiert ist, in einer biologischen Probe von dem Individuum umfasst, wobei eine höhere Methylierungsstufe der vorstehend angegebenen DNA-Region relativ zu den Kontrollstufen ein kolorektales Neoplasma oder eine Prädisposition für den Beginn eines kolorektalen Neoplasmas anzeigt.

2. Verfahren nach Anspruch 1, wobei das Neoplasma ein Adenom oder ein Adenokarzinom ist.

3. Verfahren nach einem der Ansprüche 1-2, wobei die Kontrollstufe eine nichtneoplastische Stufe ist.

4. Verfahren nach Anspruch 3, wobei eine Abnahme der Methylierungsstufe relativ zu der Kontrollstufe das Verschwinden des Neoplasmas anzeigt.

5. Verfahren nach einem der Ansprüche 1-4, wobei die biologische Probe eine Stuhlprobe, Klistierwäsche, chirurgische Resektion, Gewebebiopsie oder Blutprobe ist.

6. Verfahren nach Anspruch 5, wobei die Blutprobe Vollblut, Serum oder Plasma ist.

7. Verfahren zum Screenen des Beginns oder der Prädisposition für den Beginn oder die Überwachung eines kolorektalen Neoplasmas in einem Individuum, wobei das Verfahren das Beurteilen der Expressionsstufe der durch die Hg19-Koordinaten chr12:24964278..25102308 definierten DNA-Region, wobei die Expression spezifisch für das in dieser DNA-Region enthaltene BCAT1-Gen ist, in einer biologischen Probe des Individuums umfasst, wobei eine niedrigere Expressionsstufe der vorstehend spezifizierten DNA-Region relativ zu Kontrollstufen ein kolorektales Neoplasma oder eine Prädisposition für den Beginn eines kolorektalen Neoplasmas anzeigt.

8. Verfahren nach Anspruch 7, wobei die Expressionsstufe mRNA-Expression oder Proteinexpression ist.

9. Verfahren nach einem der Ansprüche 7 und 8, wobei die Kontrollstufe eine nichtneoplastische Stufe ist.

10. Verfahren nach einem der Ansprüche 7-9, wobei die biologische Probe eine Stuhlprobe, Klistierwäsche, chirurgische Resektion, Gewebebiopsie oder Blutprobe ist.

11. Verfahren nach Anspruch 10, wobei die Blutprobe Vollblut, Serum oder Plasma ist.

12. Verfahren nach einem der Ansprüche 1-6, wobei die DNA-Region die Promotor-Region ist.

13. Verfahren nach einem der Ansprüche 1-12, wobei das Individuum ein Mensch ist.

## Revendications

1. Procédé de dépistage de l'apparition ou de la prédisposition à l'apparition d'un néoplasme colorectal ou de surveillance de la progression d'un néoplasme colorectal chez un individu, ledit procédé comprenant l'évaluation du statut de méthylation de la région d'ADN définie par les coordonnées d'Hg19 chr12:24964278..25102308 et 2kb en amont du site de début de transcription du gène BCAT1 contenu dans un échantillon biologique dudit individu dans lequel un niveau supérieur de méthylation de la région d'ADN spécifiée ci-dessus par rapport à des niveaux de contrôle indique un néoplasme colorectal ou une prédisposition à l'apparition d'un néoplasme colorectal.

2. Procédé selon la revendication 1, dans lequel ledit néoplasme est un adénome ou un adénocarcinome.

3. Procédé selon l'une quelconque des revendications 1 et 2, dans lequel ledit niveau de contrôle est un niveau non néoplasique.

4. Procédé selon la revendication 3, dans lequel une diminution du taux de méthylation par rapport audit niveau de contrôle indique l'élimination du néoplasme.

5. Procédé selon l'une quelconque des revendications 1 à 4, dans lequel ledit échantillon biologique est un échantillon fécal, un lavement, une résection chirurgicale, une biopsie tissulaire ou un échantillon de sang.

6. Procédé selon la revendication 5, dans lequel ledit échantillon de sang est du sang total, du sérum ou du plasma.

7. Procédé de dépistage de l'apparition ou de la prédisposition à l'apparition ou de surveillance d'un néoplasme colorectal chez un individu, ledit procédé comprenant l'évaluation du niveau d'expression de la région d'ADN définie par les coordonnées d'Hg19 chr12:24964278..25102308 où ladite expression est spécifique du gène BCAT1 compris dans ladite région d'ADN dans un échantillon biologique dudit individu dans lequel un niveau d'expression inférieur de la région d'ADN spécifiée ci-dessus par rapport à des niveaux de contrôle indique un néoplasme colorectal ou une prédisposition à l'apparition d'un néoplasme colorectal.

8. Procédé selon la revendication 7, dans lequel ledit niveau d'expression est l'expression de l'ARNm ou l'expression de la protéine.

9. Procédé selon l'une quelconque des revendications 7 et 8, dans lequel ledit niveau de contrôle est un niveau non néoplasique.

10. Procédé selon l'une quelconque des revendications 7 à 9, dans lequel ledit échantillon biologique est un échantillon fécal, un lavement, une résection chirurgicale, une biopsie tissulaire ou un prélèvement sanguin.

11. Procédé selon la revendication 10, dans lequel ledit échantillon sanguin est du sang total, du sérum ou du plasma.

12. Procédé selon l'une quelconque des revendications 1 à 6, dans lequel ladite région d'ADN est la région promotrice.

13. Procédé selon l'une quelconque des revendications 1 à 12, dans lequel ledit individu est un humain.
